# EUROPEAN PATENT SPECIFICATION

(11) **EP 4 114 825 B1**
(45) Date of publication and mention of the grant of the patent: **20.11.2024**
(21) Application number: 21709000.0
(22) Date of filing: 02.03.2021
(51) Int. Cl.: C07D 209/86, C08F 2/48, G03F 7/004, C07D 405/06, C07D 409/12, C07F 1/00

(54) **OXIME ESTER PHOTOINITIATORS**
OXIMESTER-PHOTOINITIATOREN
PHOTO-INITIATEURS À BASE D'ESTER D'OXIME

(30) Priority: 04.03.2020 EP 20160969; 14.12.2020 EP 20213955
(43) Date of publication of application: 11.01.2023
(73) Proprietor: BASF SE, 67056 Ludwigshafen am Rhein (DE)
(72) Inventor: KUNIMOTO, Kazuhiko, Kawanishi, Hyogo 663-8141 (JP); MATSUOKA, Yuki, Fukuoka 807-0071 (JP); FUKUZUMI, Takeo, 67056 Ludwigshafen am Rhein (DE); KURA, Hisatoshi, Hyogo 665-0013 (JP); SAMESHIMA, Kaori, Hyogo 660-0083 (JP)
(74) Representative: BASF IP Association
(86) International application number: PCT/EP2021/055184
(87) International publication number: WO 2021/175855

(56) References cited:
- CN-A- 107 793 502
- JP-A- 2009 179 619
- US-A1- 2015 307 481

## Description

The present invention relates to novel α-oxo oxime ester compounds based on carbazole derivatives which have specific substituent groups and their use as photoinitiators in photopolymerizable compositions, particularly in photoresist formulations for display applications, e.g. liquid crystal display (LCD), organic light emitting diode (OLED) and touch panel.

From WO2002100903 it is known that certain α-oxo oxime ester compounds based on carbazole derivatives having an acyl substituent are photoinitiators. Different carbazole α-oxo oxime ester compounds having an acyl substituent are disclosed in JP2009221334**,** WO2009131189**,** JP2011074042**,** KR1225695**,** KR2013038151**,** CN103130919**,** CN103204960**,** JP2013142087**,** WO2014121701**,** CN104276995**,** WO2015080503**.**

Carbazole α-oxo oxime ester photoinitiators having O-substituted benzoyl group are disclosed in the following patent applications.

JP2009179619 discloses α,α-dioxo oxime ester compounds based on carbazole derivatives having m-cyclohexyloxy- or m-phenoxybenzoyl substituent.

CN 103130919 discloses carbazole ketoxime ester photoinitiators having a ring-shaped substituent and p-methoxy- or p-phenoxybenzoyl substituent.

CN 107793502 discloses a fused carbazole ketoxime ester photoinitiator having p-methoxybenzoyl substituent.

In photopolymerization technology there still exists a strong demand for photoinitiators with higher photosensitivity in order to reduce the energy consumption, to achieve a shorter tact time for higher productivity and/or more freedom in choice of materials for display application. For example, color filters for LCD and white OLED, which comprise a black matrix and red, green and blue color pixels on a glass substrate, are manufactured by photolithography using radically photopolymerizable resists. In the color filter resist applications, high loading of the colorants is required to attain high color quality properties. With the increase of the colorant content, however, curing of the color resists becomes more difficult because the colorants reduce transmission of the exposed light for photolithography. Hence, photoinitiators having a high sensitivity are required. In addition, brightness of the color filters is also reduced by the increase of the colorant content, and therefore, discoloration by the photoinitiator should be minimized in the visible region during photolithography and subsequent post-bake processes to avoid reduction of the brightness. However, there is still an unmet demand in the market for a photoinitiator, which meets at the same time both high sensitivity and high brightness.

Accordingly, the present invention provides compounds of the formula I, II, III and IV 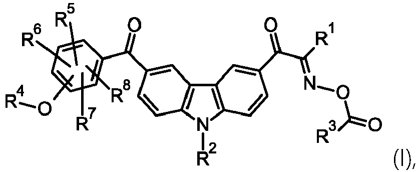 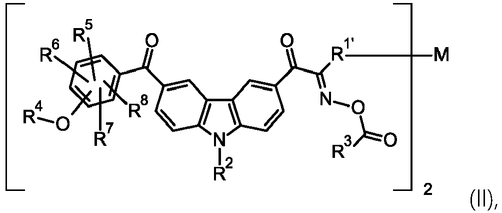 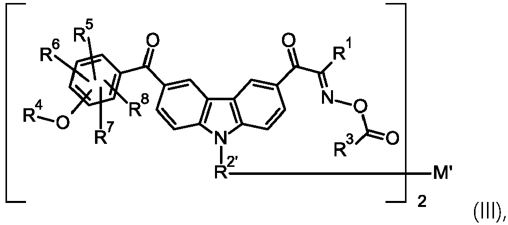 and 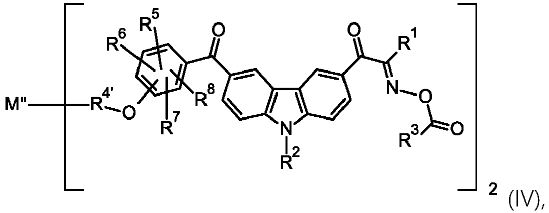 wherein
**R¹** is hydrogen, C₁-C₂₀alkyl, C₁₋C₆alkyl-C₃₋C₆cycloalkyl, C₃-C₂₀cycloalkyl, C₂-C₁₂alkenyl, wherein C₃-C₂₀cycloalkyl or C₂-C₁₂alkenyl is uninterrupted or interrupted by one or more O, S, CO, NR¹⁰ or COOR⁴; or
**R¹** is C₁-C₂₀alkyl which is unsubstituted or substituted by one or more halogen, OR⁴, SR⁹, NR¹⁰R¹¹, COOR⁴ CONR¹⁰R¹¹, CN, PO(OR^{3a})₂, S(O)ₘ-R^{3a}, 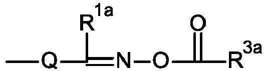 , C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹, NR¹⁰R¹¹, PO(OR^{3a})₂ or S(O)ₘ-R^{3a}; or
**R¹** is C₂-C₂₀alkyl which is interrupted by one or more O, CO, S, C(O)O, OC(O), SO, SO₂, phenylene, naphthylene or NR¹⁰, wherein the interrupted C₂-C₂₀alkyl is unsubstituted or substituted by one or more halogen, C₃-C₈cycloalkyl, OH, SH, OR⁴, SR⁹, COOR⁴, O(CO)-R^{3a}, CONR¹⁰R¹¹, NR¹⁰R¹¹, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR⁴, SR⁹ or NR¹⁰R¹¹; or
**R¹** is C₆-C₂₀aryl or C₃-C₂₀heteroaryl each of which is unsubstituted or substituted by one or more C₁-C₂₀alkyl, phenyl, halogen, C₁-C₄haloalkyl, CN, NO₂, OR⁴, SR⁹, NR¹⁰R¹¹, COOR⁴, (CO)-R^{3a}, (CO)NR¹⁰R¹¹, PO(OR^{3a})₂, S(O)ₘ-R^{3a} or group 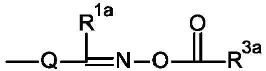 ; or by one or more C₂-C₂₀alkyl which is interrupted by one or more O, S, or NR¹⁰; or by one or more C₁-C₂₀alkyl which is unsubstituted or substituted by one or more halogen, COOR⁴, CONR¹⁰R¹¹, phenyl, C₃-C₈cycloalkyl, C₃-C₂₀heteroaryl, C₆-C₂₀aryloxycarbonyl, C₃-C₂₀heteroaryloxycarbonyl, OR⁴, SR⁹ or NR¹⁰R¹¹; or by one or more phenyl, naphthyl, benzoyl or naphthoyl, each of which is unsubstituted or substituted by OR⁴, SR⁹ or NR¹⁰R¹¹;
**R¹** is C₂-C₂₀alkanoyl or benzoyl which is unsubstituted or substituted by one or more C₁-C₆alkyl, phenyl, OR⁴, SR⁹ or NR¹⁰R¹¹; or
**R¹** is C₂-C₁₂alkoxycarbonyl optionally interrupted by one or more -O- and/or optionally substituted by one or more hydroxyl groups; or
**R¹** is phenoxycarbonyl which is unsubstituted or substituted by C₁-C₆alkyl, halogen, phenyl, OR⁴, SR⁹ or NR¹⁰R¹¹; or
**R¹** is CN, (CO)-R^{3a}, COOR⁴, CONR¹⁰R¹¹, NO₂, PO(OR^{3a})₂ or S(O)ₘ-R^{3a}.
**R^{1'}** is C₁-C₂₀alkylene, C₁-C₂₀alkylene which is interrupted by one or more O, S, (CO)O, O(CO), phenylene, naphthylene or NR⁸, wherein the C₁-C₂₀alkylene and interrupted C₁-C₂₀alkylene is unsubstituted or substituted by halogen or OR⁴, or
**R^{1'}** is C₂-C₂₀alkenylene, C₂-C₂₀alkenylene which is interrupted by one or more O, S, (CO)O, O(CO), phenylene, naphthylene or by NR¹⁰, wherein the C₂-C₂₀alkenylene and interrupted C₂-C₂₀alkenylene is unsubstituted or substituted by halogen or OR⁴, or 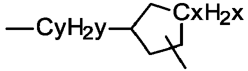
**R^{1'}** is C₅-C₈cycloalkylene, C₅-C₈cycloalkenylene, 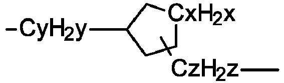 , wherein the groups C_{y}H_{2y} and C_{z}H_{2z} are uninterrupted or interrupted by one or more O, S or by NR¹⁰;
**R^{1a}** is hydrogen, C₁-C₂₀alkyl, CN, (CO)-R^{3a}, COOR^{4a}, CONR^{10a}R^{11a}, NO₂, PO(OR^{3a})₂ or S(O)ₘ-R^{3a}; or
**R^{1a}** is C₁-C₂₀alkyl substituted by one or more halogen, OR^{4a}, SR^{9a}, NR^{10a}R^{11a}, CN, COOR^{4a}, CONR^{10a}R^{11a}, PO(OR^{3a})₂, S(O)ₘ-R^{3a}, C₃-C₈cycloalkyl which is uninterrupted or interrupted by one or more O, S, CO or NR^{10a}; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR^{4a}, SR^{9a}, NR^{10a}R^{11a}, PO(OR^{3a})₂ or S(O)ₘ-R^{3a}; or
**R^{1a}** is C₁-C₂₀alkyl interrupted by one or more O, S, NR^{10a}, CO, SO or SO₂, which is unsubstituted or substituted by C₃-C₈cycloalkyl, OH, SH, O(CO)R^{3a}, COOR^{4a}, CONR^{10a}R^{11a}, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR^{4a}, SR^{9a} or NR^{10a}R^{11a}; or
**R^{1a}** is C₂-C₁₂alkenyl or C₃-C₂₀cycloalkyl, each of which is uninterrupted or interrupted by one or more O, S, CO, NR^{10a} or COOR^{4a}; or
**R^{1a}** is C₆-C₂₀aryl or C₃-C₂₀heteroaryl, each of which is unsubstituted or substituted by one or more halogen, CN, NO₂, OR^{4a}, SR^{9a}, NR^{10a}R^{11a}, COOR^{4a}, (CO)-R^{3a} CONR^{10a}R^{11a}, PO(OR^{3a})₂ or S(O)ₘ-R^{3a}; or by one or more C₁-C₂₀alkyl which is unsubstituted or substituted by one or more halogen, COOR^{4a}, CONR^{10a}R^{11a}, phenyl, C₃-C₈cycloalkyl, C₃-C₂₀heteroaryl, OR^{4a}, SR^{9a} or NR^{10a}R^{11a}; or by one or more C₂-C₂₀alkyl which is interrupted by one or more O, S or NR^{10a}; or by one or more phenyl, naphthyl, benzoyl or naphthoyl, each of which is unsubstituted or substituted by OR^{4a}, SR^{9a} or N R^{10a}R^{11a};
**R²** is hydrogen, C₁-C₂₀alkyl or C₁-C₆alkyl-C₃-C₆-cycloalkyl which is unsubstituted or substituted by one or more halogen, OR⁴, SR⁹, COOR⁴ CONR¹⁰R¹¹, NR¹⁰R¹¹, PO(OR^{3a})₂; COR^{3a}, or
**R²** is C₂-C₂₀alkyl or C₁-C₆alkyl-C₃-C₆-cycloalkyl which is interrupted by one or more O, CO, S, C(O)O, OC(O), SO, SO₂, phenylene, naphthylene or NR¹⁰, wherein the interrupted C₂-C₂₀alkyl is unsubstituted or substituted by one or more halogen, OR⁴, SR⁹, COOR⁴, CONR¹⁰R¹¹, NR¹⁰R¹¹; or
**R²** is C₂-C₄hydroxyalkyl, C₂-C₁₀alkoxyalkyl, C₃-C₅alkenyl, C₃-C₈cycloalkyl, phenyl-C₁₋C₃alkyl, C₂-C₈alkanoyl, C₃-C₁₂alkenoyl, benzoyl; or
**R²** is C₆-C₂₀aryl or C₃-C₂₀heteroaryl each of which is unsubstituted or substituted by one or more C₁-C₁₂alkyl, C₁-C₄haloalkyl, phenyl, halogen, CN, NO₂, OR⁴, SR⁹, NR¹⁰R¹¹, (CO)-R^{3a}, or by C₂-C₂₀alkyl which is interrupted by one or more O, S, or NR¹⁰, or each of which is substituted by one or more C₁-C₂₀alkyl which is unsubstituted or substituted by one or more halogen, COOR⁴, CONR¹⁰R¹¹, phenyl, C₃-C₈cycloalkyl, C₃-C₂₀heteroaryl, C₆-C₂₀aryloxycarbonyl, C₃-C₂₀heteroaryloxycarbonyl, OR⁴- SR⁹ or NR¹⁰R¹¹; or
**R²** is a group 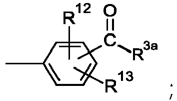
**R^{2'}** is C₁-C₂₀alkylene, C₁-C₂₀alkylene which is interrupted by one or more O, S, (CO)O, O(CO), phenylene, naphthylene or NR⁸, wherein the C₁-C₂₀alkylene and interrupted C₁-C₂₀alkylene is unsubstituted or substituted by halogen or OR⁴, or
**R^{2'}** is C₂-C₂₀alkenylene, C₂-C₂₀alkenylene which is interrupted by one or more O, S, (CO)O, O(CO), phenylene, naphthylene or by NR¹⁰, wherein the C₂-C₂₀alkenylene and interrupted C₂-C₂₀alkenylene is unsubstituted or substituted by halogen or OR⁴, or 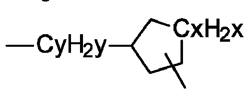
**R^{2'}** is C₅-C₈cycloalkylene, C₅-C₈cycloalkenylene, 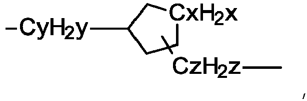 wherein the groups C_{y}H_{2y} and C_{z}H_{2z} are uninterrupted or interrupted by one or more O, S or by NR¹⁰;
**R³** is hydrogen or C₁-C₂₀alkyl; or
**R³** is C₁-C₂₀alkyl substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹ or NR¹⁰R¹¹; or
**R³** is C₁-C₂₀alkyl interrupted by one or more O, S, NR¹⁰, CO, SO or SO₂, which is unsubstituted or substituted by C₃-C₈cycloalkyl, OH, SH, O(CO)-R^{3a}, COOR⁴, CONR¹⁰R¹¹ C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR⁴, SR⁹ or NR¹⁰R¹¹; or
**R³** is C₂-C₁₂alkenyl or C₃-C₂₀cycloalkyl, each of which is uninterrupted or interrupted by one or more O, S, CO, NR¹⁰ or COOR⁴; or
**R³** is C₆-C₂₀aryl or C₃-C₂₀heteroaryl, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR⁴, SR⁹, NR¹⁰R¹¹, COOR⁴, (CO)-R^{3a} or SO₂-R^{3a}; or
**R³** is C₁-C₂₀alkoxy, which is unsubstituted or substituted by one or more C₁-C₁₀alkyl, C₁-C₄haloalkyl, halogen, phenyl, C₁₋C₂₀alkylphenyl or C₁-C₈alkoxyphenyl; or
**R³** is C₁-C₂₀alkoxy, which is interrupted by one or more O, S, NR¹⁰, CO, SO or SO₂; or
**R³** is C₆-C₂₀aryloxy or C₃-C₂₀heteroaryloxy, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR⁴, SR⁹, NR¹⁰R¹¹, COOR⁴, (CO)-R^{3a} or SO₂-R^{3a};
**R^{3a}** is hydrogen or C₁-C₂₀alkyl; or
**R^{3a}** is C₁-C₂₀alkyl substituted by one or more halogen, OR^{4a}, SR^{9a}, NR^{10a}R^{11a}, CN, COOR^{4a}, CONR^{10a}R^{11a}, C₃-C₈cycloalkyl which is uninterrupted or interrupted by one or more O, S, CO or NR^{10a}; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR^{4a}, SR^{9a} or NR^{10a}R^{11a}; or
**R^{3a}** is C₁-C₂₀alkyl interrupted by one or more O, S, NR^{10a}, CO, SO or SO₂, which is unsubstituted or substituted by C₃-C₈cycloalkyl, OH, SH, O(CO)-(C₁-C₈alkyl), COOR^{4a}, CONR^{10a}R^{11a}, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR^{4a}, SR^{9a} or NR^{10a}R^{11a}; or
**R^{3a}** is C₂-C₁₂alkenyl or C₃-C₂₀cycloalkyl, each of which is uninterrupted or interrupted by one or more O, S, CO, NR^{10a} or COOR^{4a}; or
**R^{3a}** is C₆-C₂₀aryl or C₃-C₂₀heteroaryl, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR^{4a}, SR^{9a}, NR^{10a}R^{11a}, COOR^{4a}, (CO)-(C₁-C₈alkyl) or SOr(C₁-C₄haloalkyl); or
**R^{3a}** is C₁-C₂₀alkoxy, which is unsubstituted or substituted by one or more C₁-C₁₀alkyl, C₁-C₄haloalkyl, halogen, phenyl, C₁₋C₂₀alkylphenyl or C₁-C₈alkoxyphenyl; or
**R^{3a}** is C₁-C₂₀alkoxy, which is interrupted by one or more O, S, NR^{10a}, CO, SO or SO₂; or
**R^{3a}** is C₆-C₂₀aryloxy or C₃-C₂₀heteroaryloxy, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR^{4a}, SR^{9a}, NR^{10a}R^{11a}, COOR^{4a}, (CO)-(C₁-C₈alkyl) or SO₂-(C₁-C₄haloalkyl); or
**R^{3a}** is a group 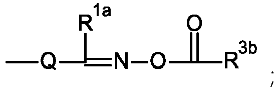
**R^{3b}** is hydrogen or C₁-C₂₀alkyl; or
**R^{3b}** is C₁-C₂₀alkyl substituted by one or more halogen, OR^{4a}, SR^{9a}, NR^{10a}R^{11a}, CN, COOR^{4a}, CONR^{10a}R^{11a}, C₃-C₈cycloalkyl which is uninterrupted or interrupted by one or more O, S, CO or NR^{10a}; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR^{4a}, SR^{9a} or NR^{10a}R^{11a}; or
**R^{3b}** is C₁-C₂₀alkyl interrupted by one or more O, S, NR^{10a}, CO, SO or SO₂, which is unsubstituted or substituted by C₃-C₈cycloalkyl, OH, SH, O(CO)-(C₁-C₈alkyl), COOR^{4a}, CONR^{10a}R^{11a}, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR^{4a}, SR^{9a} or NR^{10a}R^{11a}; or
**R^{3b}** is C₂-C₁₂alkenyl or C₃-C₂₀cycloalkyl, each of which is uninterrupted or interrupted by one or more O, S, CO, NR^{10a} or COOR^{4a}; or
**R^{3b}** is C₆-C₂₀aryl or C₃-C₂₀heteroaryl, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR^{4a}, SR^{9a}, NR^{10a}R^{11a}, COOR^{4a}, (CO)-(C₁-C₈alkyl) or SO₂-(C₁-C₄haloalkyl); or
**R^{3b}** is C₁-C₂₀alkoxy, which is unsubstituted or substituted by one or more C₁-C₁₀alkyl, C₁-C₄haloalkyl, halogen, phenyl, C₁₋C₂₀alkylphenyl or C₁-C₈alkoxyphenyl; or
**R^{3b}** is C₁-C₂₀alkoxy, which is interrupted by one or more O, S, NR^{10a}, CO, SO or SO₂; or
**R^{3b}** is C₆-C₂₀aryloxy or C₃-C₂₀heteroaryloxy, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR^{4a}, SR^{9a}, NR^{10a}R^{11a}, COOR^{4a}, (CO)-(C₁-C₈alkyl) or SO₂-(C₁-C₄haloalkyl);
**R⁴** is hydrogen, (CO)-R^{3a}, COOR^{4a}, CONR¹⁰R¹¹, S(O)ₘ-R^{3a} or PO(OR^{3a})₂; or
**R⁴** is C₁-C₂₀alkyl, which is substituted by one or more halogen, OR^{4a}, SR^{9a}, NR^{10a}R^{11a}, CN, COOR^{4a}, CONR^{10a}R^{11a}, PO(OR^{3a})₂, S(O)ₘ-R^{3a}, C₃-C₈cycloalkyl which is uninterrupted or interrupted by one or more O, S, CO or NR^{10a}; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR^{4a}, SR^{9a}, NR^{10a}R^{11a}, PO(OR^{3a})₂ or S(O)ₘ-R^{3a}; or
**R⁴** is C₁-C₂₀alkyl interrupted by one or more O, S, NR^{10a}, CO, SO or SO₂, which is unsubstituted or substituted by C₃-C₈cycloalkyl, OH, SH, O(CO)-R^{3a}, COOR^{4a}, CONR^{10a}R^{11a}, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR^{4a}, SR^{9a} or NR^{10a}R^{11a}; or
**R⁴** is C₂-C₁₂alkenyl or C₃-C₂₀cycloalkyl, each of which is uninterrupted or interrupted by one or more O, S, CO, NR^{10a} or COOR^{4a}; or
**R⁴** is C₆-C₂₀aryl, which is substituted by one or more halogen, CN, NO₂, OR^{4a}, SR^{9a}, NR^{10a}R^{11a}, COOR^{4a}, (CO)-R^{3a}, CONR^{10a}R^{11a}, PO(OR^{3a})₂, S(O)ₘ-R^{3a} or group 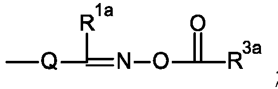 or by one or more C₁-C₂₀alkyl which is unsubstituted or substituted by one or more halogen, COOR^{4a}, CONR^{10a}R^{11a}, phenyl, C₃-C₈cycloalkyl, C₃-C₂₀heteroaryl, OR^{4a}, SR^{9a} or NR^{10a}R^{11a}; or by one or more C₂-C₂₀alkyl which is interrupted by one or more O, S or NR^{10a}; or by one or more phenyl, naphthyl, benzoyl or naphthoyl, each of which is unsubstituted or substituted by OR^{4a}, SR^{9a} or NR^{10a}R^{11a}; or
**R⁴** is C₃-C₂₀heteroaryl, each of which is unsubstituted or substituted by one or more halogen, CN, NO₂, OR^{4a}, SR^{9a}, NR^{10a}R^{11a}, COOR^{4a}, (CO)-R^{3a}, CONR^{10a}R^{11a}, PO(OR^{3a})₂, S(O)ₘ-R^{3a} or group 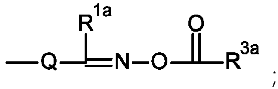 or by one or more C₁-C₂₀alkyl which is unsubstituted or substituted by one or more halogen, COOR^{4a}, CONR^{10a}R^{11a}, phenyl, C₃-C₈cycloalkyl, C₃-C₂₀heteroaryl, OR^{4a}, SR^{9a} or NR^{10a}R^{11a}; or by one or more C₂-C₂₀alkyl which is interrupted by one or more O, S or NR^{10a}; or by one or more phenyl, naphthyl, benzoyl or naphthoyl, each of which is unsubstituted or substituted by OR^{4a}, SR^{9a} or NR^{10a}R^{11a}; or
**R⁴** together with one of the carbon atom of R¹ forms a 5- or 6-membered saturated or unsaturated ring which is uninterrupted or interrupted by O, S or NR^{10a}, and which 5- or 6-membered saturated or unsaturated ring is unsubstituted or substituted by one or more C₁-C₂₀alkyl, OR^{4a}, SR^{9a}, NR^{10a}R^{11a}, (CO)-R^{3a}, NO₂, halogen, C₁-C₄haloalkyl, CN, phenyl, C₁-C₂₀alkylphenyl, C₁-C₈alkoxyphenyl, 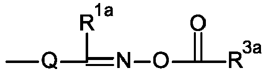 or C₃-C₂₀cyclalkyl which is uninterrupted or interrupted by one or more O, S, CO or NR^{10a};
**R^{4'}** is -(CO)-, -(CO)O-, -(CO)N-, -S(O)ₘ-, -PO(O)₂-, or -OP(O)₂;
**R^{4a}** is hydrogen, C₁-C₂₀alkyl, (CO)O(C₁-C₈alkyl) or CON(C₁-C₈alkyl)₂; or
**R^{4a}** is C₁-C₂₀alkyl substituted by one or more halogen, OH, SH, CN, C₃-C₈alkenoxy, OCH₂CH₂CN, OCH₂CH₂(CO)O(C₁-C₈alkyl), O(CO)-(C₁-C₈alkyl), O(CO)-(C₂-C₄)alkenyl, O(CO)-phenyl, (CO)OH, (CO)O(C₁-C₈alkyl), C₃-C₈cycloalkyl, SO₂-(C₁-C₄haloalkyl), O(C₁-C₄haloalkyl), phenyl, C₁-C₈alkylphenyl, C₁-C₈alkoxyphenyl or C₃-C₈cycloalkyl which is interrupted by one or more O; or
**R^{4a}** is C₂-C₂₀alkyl interrupted by one or more O, S, N(C₁-C₈alkyl), CO, SO or SO₂, which is unsubstituted or substituted by C₃-C₈cycloalkyl, OH, SH, O(CO)(C₁-C₈alkyl), (CO)O(C₁-C₈alkyl), (CO)N(C₁-C₈alkyl)₂, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, C₁-C₈alkoxy, C₁-C₈alkylsulfanyl or N(C₁-C₈alkyl)₂; or
**R^{4a}** is C₂-C₁₂alkenyl, (CO)O(C₁-C₈alkenyl) or C₃-C₈cycloalkyl, each of which is uninterrupted or interrupted by one or more O, S, CO, N(C₁-C₈alkyl) or COO(C₁-C₈alkyl); or
**R^{4a}** is C₆-C₂₀aryl, C₃-C₂₀heteroaryl, C₆-C₂₀aroyl or C₃-C₂₀heteroarylcarbonyl, each of which is unsubstituted or substituted by one or more halogen, CN, NO₂, OH, C₁-C₈alkyl, C₁-C₄haloalkyl, C₁-C₈alkoxy, phenyl-C₁-C₃alkyloxy, phenoxy, C₁-C₈alkylsulfanyl, phenylsulfanyl, N(C₁-C₈alkyl)₂, diphenylamino, (CO)O(C₁-C₈alkyl), (CO)-C₁-C₈alkyl or (CO)N(C₁-C₈)₂, phenyl or benzoyl; or
**R^{4a}** is C₁-C₂₀alkanoyl, C₃-C₁₂alkenoyl, each of which is unsubstituted or substituted by one or more halogen, phenyl, C₁-C₈alkylphenyl, C₁-C₈alkoxyphenyl, OH, C₁-C₈alkoxy, phenoxy, C₁-C₈alkylsulfanyl, phenylsulfanyl, N(C₁-C₈alkyl)₂ or diphenylamino;
**R⁵**, **R⁶**, **R⁷** and **R⁸** independently of each other are hydrogen, C₁-C₂₀alkyl, C₆-C₂₀aryl, C₁-C₂₀alkoxy, C₆-C₂₀arylC₁-C₂₀alkyl, hydroxyC₁-C₂₀alkyl, hydroxyC₁-C₂₀alkoxyC₁-C₂₀alkyl, C₃-C₁₀cycloalkyl, amino, CN, NO₂, hydroxy, 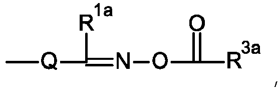 (CO)-R^{3a}, OR^{4a} or COOR⁴;
**R⁹** is hydrogen or C₁-C₂₀alkyl; or
**R⁹** is C₁-C₂₀alkyl substituted by one or more halogen, OR^{4a}, SR^{9a}, NR^{10a}R^{11a}, CN, COOR^{4a}, CONR^{10a}R^{11a}, PO(OR^{3a})₂, S(O)ₘ-R^{3a}, C₃-C₈cycloalkyl which is uninterrupted or interrupted by one or more O, S, CO or NR^{10a}; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR^{4a}, SR^{9a}, NR^{10a}R^{11a}, PO(OR^{3a})₂ or S(O)ₘ-R^{3a}; or
**R⁹** is C₁-C₂₀alkyl interrupted by one or more O, S, NR^{10a}, CO, SO or SO₂, which is unsubstituted or substituted by C₃-C₈cycloalkyl, OH, SH, O(CO)-R^{3a}, COOR^{4a}, CONR^{10a}R^{11a}, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR^{4a}, SR^{9a} or NR^{10a}R^{11a}; or
**R⁹** is C₂-C₁₂alkenyl or C₃-C₂₀cycloalkyl, each of which is uninterrupted or interrupted by one or more O, S, CO, NR^{10a} or COOR^{4a}; or
**R⁹** is C₆-C₂₀aryl or C₃-C₂₀heteroaryl, each of which is unsubstituted or substituted by one or more halogen, CN, NO₂, OR^{4a}, SR^{9a}, NR^{10a}R^{11a}, COOR^{4a}, (CO)-R^{3a} CONR^{10a}R^{11a}, PO(OR^{3a})₂, S(O)ₘ-R^{3a} or group 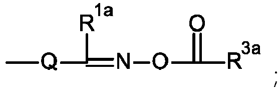 or by one or more C₁-C₂₀alkyl which is unsubstituted or substituted by one or more halogen, COOR^{4a}, CONR^{10a}R^{11a}, phenyl, C₃-C₈cycloalkyl, C₃-C₂₀heteroaryl, OR^{4a}, SR^{9a} or NR^{10a}R^{11a}; or by one or more C₂-C₂₀alkyl which is interrupted by one or more O, S or NR^{10a}; or by one or more phenyl, naphthyl, benzoyl or naphthoyl, each of which is unsubstituted or substituted by OR^{4a}, SR^{9a} or NR^{10a}R^{11a}; or
**R⁹** together with one of the carbon atom of R¹ forms a 5- or 6-membered saturated or unsaturated ring which is uninterrupted or interrupted by O, S or NR^{10a}, and which 5- or 6-membered saturated or unsaturated ring is unsubstituted or substituted by one or more C₁-C₂₀alkyl, OR^{4a}, SR^{9a}, NR^{10a}R^{11a}, (CO)-R^{3a}, NO₂, halogen, C₁-C₄haloalkyl, CN, phenyl, C₁-C₂₀alkylphenyl, C₁-C₈alkoxyphenyl, 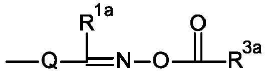 or C₃-C₂₀cyclalkyl which is uninterrupted or interrupted by one or more O, S, CO or NR^{10a};
**R^{9a}** is hydrogen or C₁-C₂₀alkyl; or
**R^{9a}** is C₁-C₂₀alkyl substituted by one or more halogen, OH, SH, CN, C₃-C₈alkenoxy, OCH₂CH₂CN, OCH₂CH₂(CO)O(C₁-C₈alkyl), O(CO)-(C₁-C₈alkyl), O(CO)-(C₂-C₄)alkenyl, O(CO)-phenyl, (CO)OH, (CO)O(C₁-C₈alkyl), C₃-C₈cycloalkyl, SO₂-(C₁-C₄haloalkyl), O(C₁-C₄haloalkyl), phenyl, C₁-C₈alkylphenyl, C₁-C₈alkoxyphenyl or C₃-C₈cycloalkyl which is interrupted by one or more O; or
**R^{9a}** is C₂-C₂₀alkyl interrupted by one or more O, S, N(C₁-C₈alkyl), CO, SO or SO₂, which is unsubstituted or substituted by C₃-C₈cycloalkyl, OH, SH, O(CO)(C₁-C₈alkyl), (CO)O(C₁-C₈alkyl), (CO)N(C₁-C₈alkyl)₂, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, C₁-C₈alkoxy, C₁-C₈alkylsulfanyl or N(C₁-C₈alkyl)₂; or
**R^{9a}** is C₂-C₁₂alkenyl or C₃-C₈cycloalkyl, each of which is uninterrupted or interrupted by one or more O, S, CO, N(C₁-C₈alkyl) or COO(C₁-C₈alkyl); or
**R^{9a}** is C₆-C₂₀aryl, C₃-C₂₀heteroaryl, C₆-C₂₀aroyl or C₃-C₂₀heteroarylcarbonyl, each of which is unsubstituted or substituted by one or more halogen, CN, NO₂, OH, C₁-C₈alkyl, C₁-C₄haloalkyl, C₁-C₈alkoxy, phenyl-C₁-C₃alkyloxy, phenoxy, C₁-C₈alkylsulfanyl, phenylsulfanyl, N(C₁-C₈alkyl)₂, diphenylamino, (CO)O(C₁-C₈alkyl), (CO)-C₁-C₈alkyl or (CO)N(C₁-C₈)₂, phenyl or benzoyl; or
**R^{9a}** is C₁-C₂₀alkanoyl, C₃-C₁₂alkenoyl, each of which is unsubstituted or substituted by one or more halogen, phenyl, C₁-C₈alkylphenyl, C₁-C₈alkoxyphenyl, OH, C₁-C₈alkoxy, phenoxy, C₁-C₈alkylsulfanyl, phenylsulfanyl, N(C₁-C₈alkyl)₂ or diphenylamino;
**R¹⁰** and **R¹¹** independently of each other are hydrogen, C₁-C₂₀alkyl, S(O)ₘ-R^{3a}, O(CO)-R^{3a} (CO)-R^{3a} or CONR^{10a}R^{11a}; or
**R¹⁰** and **R¹¹** independently of each other are C₁-C₂₀alkyl substituted by one or more halogen, OR^{4a}, SR^{9a}, NR^{10a}R^{11a}, CN, COOR^{4a}, CONR^{10a}R^{11a}, PO(OR^{3a})₂, S(O)ₘ-R^{3a}, C₃-C₈cycloalkyl which is uninterrupted or interrupted by one or more O, S, CO or NR^{10a}; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR^{4a}, SR^{9a}, NR^{10a}R^{11a}, PO(OR^{3a})₂ or S(O)ₘ-R^{3a}; or
**R¹⁰** and **R¹¹** independently of each other are C₁-C₂₀alkyl interrupted by one or more O, S, NR^{10a}, CO, SO or SO₂, which is unsubstituted or substituted by C₃-C₈cycloalkyl, OH, SH, O(CO)-R^{3a}, COOR^{4a}, CONR^{10a}R^{11a}, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR^{4a}, SR^{9a} or NR^{10a}R^{11a}; or
**R¹⁰** and **R¹¹** independently of each other are C₂-C₁₂alkenyl or C₃-C₂₀cycloalkyl, each of which is uninterrupted or interrupted by one or more O, S, CO, NR^{10a} or COOR^{4a}; or
**R¹⁰** and **R¹¹** independently of each other are C₆-C₂₀aryl or C₃-C₂₀heteroaryl, each of which is unsubstituted or substituted by one or more halogen, CN, NO₂, OR^{4a}, SR^{9a}, NR^{10a}R^{11a}, COOR^{4a}, (CO)-R^{3a} CONR^{10a}R^{11a}, PO(OR^{3a})₂, S(O)ₘ-R^{3a} or group 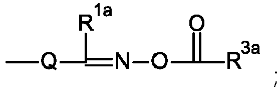 or by one or more C₁-C₂₀alkyl which is unsubstituted or substituted by one or more halogen, COOR^{4a}, CONR^{10a}R^{11a}, phenyl, C₃-C₈cycloalkyl, C₃-C₂₀heteroaryl, OR^{4a}, SR^{9a} or NR^{10a}R^{11a}; or by one or more C₂-C₂₀alkyl which is interrupted by one or more O, S or NR^{10a}; or by one or more phenyl, naphthyl, benzoyl or naphthoyl, each of which is unsubstituted or substituted by OR^{4a}, SR^{9a} or NR^{10a}R^{11a}; or
**R¹⁰** and **R¹¹** independently of each other are C₁-C₂₀alkoxy, which is unsubstituted or substituted by one or more halogen, phenyl, C₁-C₈alkylphenyl or C₁-C₈alkoxyphenyl; or
**R¹⁰** and **R¹¹** independently of each other are C₁-C₂₀alkoxy, which is interrupted by one or more O, S, NR^{10a}, CO, SO or SO₂; or
**R¹⁰** and **R¹¹** independently of each other are C₆-C₂₀aryloxy or C₃-C₂₀heteroaryloxy, each of which is unsubstituted or substituted by one or more halogen, C₁-C₈alkyl, C₁-C₄haloalkyl, phenyl, C₁-C₈alkylphenyl, C₁-C₈alkoxyphenyl, CN, NO₂, OR^{4a}, SR^{9a}, NR^{10a}R^{11a}, COOR^{4a}, (CO)-R^{3a} or SO₂-R^{3a}; or
**R¹⁰** together with one of the carbon atom of R¹ forms a 5- or 6-membered saturated or unsaturated ring which is uninterrupted or interrupted by O, S or NR^{10a}, and which 5- or 6-membered saturated or unsaturated ring is unsubstituted or substituted by one or more C₁-C₂₀alkyl, OR^{4a}, SR^{9a}, NR^{10a}R^{11a}, (CO)-R^{3a}, NO₂, halogen, C₁-C₄-haloalkyl, CN, phenyl, C₁-C₂₀alkylphenyl, C₁-C₈alkoxyphenyl, 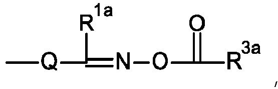 or C₃-C₂₀cyclaIkyl which is uninterrupted or interrupted by one or more O, S, CO or NR^{10a}; or
**R¹⁰** and **R¹¹** together with the N-atom to which they are attached form a 5- or 6-membered saturated or unsaturated ring which is uninterrupted or interrupted by O, S or NR^{10a}, and which 5- or 6-membered saturated or unsaturated ring is unsubstituted or substituted by one or more C₁-C₂₀alkyl, OR^{4a}, SR^{9a}, NR^{10a}R^{11a}, (CO)-R^{3a}, NO₂, halogen, C₁-C₄-haloalkyl, CN, phenyl, 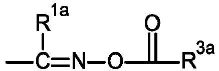 or C₃-C₂₀cyclalkyl which is uninterrupted or interrupted by one or more O, S, CO or NR^{10a};
**R^{10a}** and **R^{11a}** independently of each other are hydrogen, C₁-C₂₀alkyl, S(O)ₘ-(C₁-C₈alkyl), O(CO)(C₁ C₈alkyl), (CO)(C₁-C₈alkyl), (CO)O(C₁-C₈alkyl) or CON(C₁-C₈alkyl)₂; or
**R^{10a}** and **R^{11a}** independently of each other are C₁-C₂₀alkyl substituted by one or more halogen, OH, SH, CN, C₃-C₈alkenoxy, OCH₂CH₂CN, OCH₂CH₂(CO)O(C₁-C₈alkyl), O(CO)-(C₁-C₈alkyl), O(CO)-(C₂-C₄)alkenyl, O(CO)-phenyl, (CO)OH, (CO)O(C₁-C₈alkyl), C₃-C₈cycloalkyl, SO₂-(C₁-C₄haloalkyl), O(C₁-C₄haloalkyl), phenyl, C₁C₈alkylphenyl, C₁-C₈alkoxyphenyl or C₃-C₈cycloalkyl which is interrupted by one or more O; or
**R^{10a}** and **R^{11a}** independently of each other are C₂-C₂₀alkyl interrupted by one or more O, S, N(C₁-C₈alkyl), CO, SO or SO₂, which is unsubstituted or substituted by C₃-C₈cycloalkyl, OH, SH, O(CO)(C₁-C₈alkyl), (CO)O(C₁-C₈alkyl), (CO)N(C₁-C₈alkyl)₂, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, C₁-C₈alkoxy, C₁C₈alkylsulfanyl or N(C₁-C₈alkyl)₂; or
**R^{10a}** and **R^{11a}** independently of each other are C₂-C₁₂alkenyl or C₃-C₈cycloalkyl, each of which is uninterrupted or interrupted by one or more O, S, CO, N(C₁-C₈alkyl) or COO(C₁-C₈alkyl); or
**R^{10a}** and **R^{11a}** independently of each other are C₆-C₂₀aryl, C₃-C₂₀heteroaryl, C₆-C₂₀aroyl or C₃-C₂₀heteroarylcarbonyl, each of which is unsubstituted or substituted by one or more halogen, CN, NO₂, OH, C₁-C₈alkyl, C₁-C₄haloalkyl, C₁-C₈alkoxy, phenyl-C₁₋C₃alkyloxy, phenoxy, C₁-C₈alkylsulfanyl, phenylsulfanyl, N(C₁-C₈alkyl)₂, diphenylamino, (CO)O(C₁-C₈alkyl), (CO)-C₁-C₈alkyl or (CO)N(C₁-C₈alkyl)₂, phenyl or benzoyl; or
**R^{10a}** and **R^{11a}** independently of each other are C₁-C₂₀alkanoyl, C₃-C₁₂alkenoyl, each of which is unsubstituted or substituted by one or more halogen, phenyl, C₁-C₈alkylphenyl, C₁-C₈alkoxyphenyl, OH, C₁-C₈alkoxy, phenoxy, C₁C₈alkylsulfanyl, phenylsulfanyl, N(C₁-C₈alkyl)₂ or diphenylamino; or
**R^{10a}** and **R^{11a}** independently of each other are C₁-C₂₀alkoxy, which is unsubstituted or substituted by one or more halogen, phenyl, C₁C₈alkylphenyl or C₁-C₈alkoxyphenyl; or
**R^{10a}** and **R^{11a}** independently of each other are C₁-C₂₀alkoxy, which is interrupted by one or more O, S, N(C₁-C₈alkyl), CO, SO or SO₂; or
**R^{10a}** and **R^{11a}** independently of each other are C₆-C₂₀aryloxy or C₃-C₂₀heteroaryloxy, each of which is unsubstituted or substituted by one or more halogen, C₁-C₈alkyl, C₁-C₄haloalkyl, phenyl, C₁-C₈alkylphenyl, C₁-C₈alkoxyphenyl, CN, NO₂, C₁-C₈alkoxy, C₁C₈alkylsulfanyl, N(C₁-C₈alkyl)₂, CO(OC₁-C₈alkyl), (CO)-(C₁-C₈alkyl) or SO₂-(C₁-C₈alkyl); or
**R^{10a}** and **R^{11a}** together with the N-atom to which they are attached form a 5- or 6-membered saturated or unsaturated ring which is uninterrupted or interrupted by O, S or N(C₁-C₈alkyl), and which 5- or 6-membered saturated or unsaturated ring is unsubstituted or substituted by one or more C₁-C₈alkyl, C₁-C₈alkoxy, C₁C₈alkylsulfanyl, N(C₁-C₈alkyl)₂, NO₂, halogen, C₁-C₄haloalkyl, CN, phenyl or C₃-C₂₀cyclalkyl which is uninterrupted or interrupted by one or more O, S, CO or N(C₁-C₈alkyl);
**R¹²** and **R¹³** independently of each other are hydrogen, C₁-C₁₂alkyl optionally substituted by one or more halogen, phenyl, CN, -OH, -SH, C₁-C₄alkoxy, (CO)OH or (CO)O(C₁-C₄alkyl); or
**R¹²** and **R¹³** are phenyl optionally substituted by one or more C₁-C₆alkyl, halogen, CN, OR⁴, SR⁹ or NR¹⁰R¹¹; or
**R¹²** and **R¹³** are halogen, CN, OR⁴, SR⁹, SOR⁹, SO₂R⁹ or NR¹⁰R¹¹, wherein the substituents OR⁴, SR⁹ or NR¹⁰R¹¹ optionally form 5- or 6-membered rings via the radicals R⁴, R⁹, R¹⁰ and/or R¹¹ with one of the carbon atoms of the phenyl, naphthyl, benzoyl or naphthoyl group or that of the substituent R^{3a}; or
**R¹²** and **R¹³** together are a group 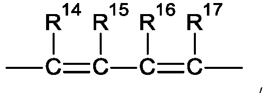 wherein R¹⁴, R¹⁵, R¹⁶ and R¹⁷ independently of one another are hydrogen, C₁-C₁₂alkyl optionally substituted by one or more halogen, phenyl, CN, -OH, -SH, C₁₋C₄alkoxy, (CO)OH or (CO)O(C₁-C₄alkyl); or R¹⁴, R¹⁵, R¹⁶ and R¹⁷ are phenyl optionally substituted by one or more C₁₋C₆alkyl, halogen, CN, OR⁴, SR⁹ or NR¹⁰R¹¹; or R¹⁴, R¹⁵, R¹⁶ and R¹⁷ are halogen, CN, OR⁴, SR⁹ or NR¹⁰R¹¹; or
**R¹²** and **R¹³** together are a group 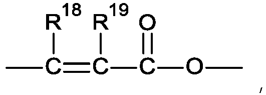 wherein R¹⁸ and R¹⁹ independently of each other are hydrogen, C₁-C₁₂alkyl optionally substituted by one or more halogen, phenyl, CN, -OH, -SH, C₁₋C₄-alkoxy, (CO)OH or (CO)O(C₁-C₄alkyl); or R¹⁸ and R¹⁹ are phenyl optionally substituted by one or more C₁-C₆alkyl, halogen, CN, OR⁴, SR⁹ or NR¹⁰R¹¹;
**M** is a direct bond or a divalent linking group C₁-C₂₀alkylene or C₁-C₂₀alkylene substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹ or NR¹⁰R¹¹; or
**M** is C₁-C₂₀alkylene interrupted by one or more O, S, NR¹⁰, CO, SO or SO₂, which is unsubstituted or substituted by C₃-C₈cycloalkyl, OH, SH, O(CO)-R^{3a}, COOR⁴, CONR¹⁰R¹¹, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR⁴, SR⁹ or NR¹⁰R¹¹; or
**M** is C₂-C₁₂alkenylene or C₃-C₂₀cycloalkylene, each of which is uninterrupted or interrupted by one or more O, S, CO, NR¹⁰ or COOR⁴; or
**M** is C₆-C₂₀arylene or C₃-C₂₀heteroarylene, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR⁴, SR⁹, NR¹⁰R¹¹, COOR⁴, (CO)-R^{3a} or SO₂-R^{3a};
**M'** is a direct bond or a divalent linking group selected from C₁-C₂₀alkylene or C₁-C₂₀lkylene substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈koxyphenyl, C₁-C₄haloalkyl, CN, NO₂, OR⁴, SR⁹ or NR¹⁰R¹¹; or
**M'** is C₁-C₂₀alkylene interrupted by one or more O, S, NR¹⁰, CO, SO or SO₂, which is unsubstituted or substituted by C₃-C₈cycloalkyl, OH, SH, O(CO)-R^{3a}, COOR⁴, CONR¹⁰R¹¹, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR⁴, SR⁹ or NR¹⁰R¹¹; or
**M'** is C₂-C₁₂alkenylene or C₃-C₂₀cycloalkylene, each of which is uninterrupted or interrupted by one or more O, S, CO, NR¹⁰ or COOR⁴; or
**M'** is C₆-C₂₀arylene or C₃-C₂₀heteroarylene, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR⁴, SR⁹, NR¹⁰R¹¹, COOR⁴, (CO)-R^{3a} or SO₂-R^{3a};
**M"** is a direct bond or a divalent linking group selected from C₁-C₂₀alkylene or C₁-C₂₀alkylene substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹ or NR¹⁰R¹¹; or
**M"** is C₁-C₂₀alkylene interrupted by one or more O, S, NR¹⁰, CO, SO or SO₂, which is unsubstituted or substituted by C₃-C₈cycloalkyl, OH, SH, O(CO)-R^{3a}, COOR⁴, CONR¹⁰R¹¹, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR⁴, SR⁹ or NR¹⁰R¹¹; or
**M"** is C₂-C₁₂alkenylene or C₃-C₂₀cycloalkylene, each of which is uninterrupted or interrupted by one or more O, S, CO, NR¹⁰ or COOR⁴; or
**M"** is C₆-C₂₀arylene or C₃-C₂₀heteroarylene, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR⁴, SR⁹, NR¹⁰R¹¹, COOR⁴, (CO)-R^{3a} or SO₂-R^{3a};
**m** is 1 or 2;
**Q** is CO or a direct bond; and
**x, y** and **z** independently of each other are an integer 1, 2, 3 or 4.

*C₁-C₂₀alkyl* is linear or branched and is, for example, C₁-C₁₈-, C₁-C₁₄-, C₁-C₁₂-, C₁-C₈-, C₁-C₆- or C₁-C₄alkyl. Examples are methyl, ethyl, propyl, isopropyl, n-butyl, sec-butyl, isobutyl, tert-butyl, pentyl, hexyl, heptyl, 2,4,4-trimethylpentyl, 2-ethylhexyl, octyl, nonyl, decyl, dodecyl, tetradecyl, pentadecyl, hexadecyl, octadecyl and icosyl.

*C₁-_{C20}haloalkyl* is C₁-C₂₀alkyl and *C₁-C₄haloalkylis* C₁-C₄alkyl, which are mono- or poly-substituted by halogen up to the exchange of all H-atoms by halogen. Examples are chloromethyl, trichloromethyl, trifluoromethyl or 2-bromopropyl, especially trifluoromethyl or trichloromethyl. *C₂-C₂₀alkyl interrupted by* one or more O, S, NR¹⁶, CO, SO or SO₂ is for example interrupted 1-9, 1-7 or once or twice by the defined radicals. In case the groups are interrupted by more than one O, said O-atoms are separated from one another by at least one methylene group, i.e. the O-atoms are non-consecutive. The alkyl groups in the interrupted alkyl are linear or branched. Examples are the following structural units -CH₂-O-CH₃, -CH₂CH₂-O-CH₂CH₃, -[CH₂CH₂O]_{y}-CH₃, with y = 1-9, -(CH₂CH₂O)₇-CH₂CH₃, -CH₂-CH(CH₃)-O-CH₂-CH₂CH₃, or -CH₂-CH(CH₃)-O-CH₂CH₃, etc.

*C₃-C₂₀cycloalkyl* or C₃-C₈cycloalkyl is a mono- or polycyclic aliphatic ring, for example a mono-, bi- or tricyclic aliphatic ring, e.g. C₃-C₁₈-, C₃-C₁₂-, C₃-C₁₀cycloalkyl. Examples of monocyclic rings are cyclopropyl, cyclobutyl, cyclopentyl, cyclohexyl, or cycloheptyl, especially cyclopentyl and cyclohexyl. Examples of polycyclic rings are perhydroanthracyl, perhydrophenyathryl, perhydronaphthyl, perhydrofluorenyl, perhydrochrysenyl, perhydropicenyl, adamantyl, bicyclo[1.1.1]pentyl, bicyclo[4.2.2]decyl, bicyclo[2.2.2]octyl, bicyclo[3.3.2]decyl, bicyclo[4.3.2]undecyl, bicyclo[4.3.3]dodecyl, bicyclo[3.3.3]undecyl, bicyclo[4.3.1]decyl, bicyclo[4.2.1]nonyl, bicyclo[3.3.1]nonyl, bicyclo[3.2.1]octyl, 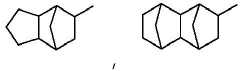 and the like.

Also "*spiro*"-cycloalkyl compounds are covered by the definition C₃-C₂₀cycloalkyl in the present context, e.g. spiro[5.2]octyl, spiro[5.4]decyl, spiro[5.5]undecyl. More examples of polycyclic cycloalkyl groups, which are subject of the respective definition in the compounds of the present invention are listed in EP 878738, page 11 and 12, wherein to the formulae (1)-(46) a bond to achieve the *"y*/*'* has to be added. The person skilled in the art is aware of this fact. *C₃-C₈cycloalkyl which is interrupted* by one or more O, S, CO or NR¹⁶ refers to C₃-C₈cycloalkyl as defined above, wherein at least one C-atom is replaced by O, S, CO or NR¹⁶.

*C₂-C₂alkenyl* is mono or polyunsaturated, linear or branched and is for example C₂-C₈-, C₂-C₆- or C₂-C₄alkenyl. Examples are allyl, methallyl, vinyl, 1,1-dimethylallyl, 1-butenyl, 3-butenyl, 2-butenyl, 1,3-pentadienyl, 5-hexenyl or 7-octenyl, especially allyl or vinyl.

*C₁-C₂₀alkoxy* is linear or branched and is for example C₁-C₁₈-, C₁-C₁₆-, C₁-C₁₂-, C₁-C₈-, C₁-C₆- or C₁-C₄-alkoxy. Examples are methoxy, ethoxy, propoxy, isopropoxy, n-butyloxy, sec-butyloxy, iso-butyloxy, tert-butyloxy, pentyloxy, hexyloxy, heptyloxy, 2,4,4-trimethylpetyloxy, 2-ethylhexyloxy, octyloxy, nonyloxy, decyloxy, dodecyloxy, hexadecyloxy, octadecyloxy or icosyloxy, in particular methoxy, ethoxy, propoxy, isopropoxy, n-butyloxy, sec-butyloxy, iso-butyloxy, tert-butyloxy, especially methoxy.

*C₂-C₂₀alkoxy, which is interrupted* by one or more O, S, NR¹⁶, CO, SO or SO₂ is for example interrupted 1-9, 1-7, 1-4 or once or twice by the defined radicals. In case the groups are interrupted by more than one O, said O-atoms are separated from one another by at least one methylene group, i.e. the O-atoms are non-consecutive. Examples are the following structural units -O-CH₂-O-CH₃, -O-CH₂CH₂-O-CH₂CH₃, -O-[CH₂CH₂O]ᵥCH₃, with v = 1-4, -O-(CH₂CH₂O)₄CH₂CH₃, -O-CH₂-CH(CH₃)-O-CH₂-CH₂CH₃, or -O-CH₂-CH(CH₃)-O-CH₂CH₃, etc. *C₁C₈alkylsulfanyl*(=C₁-C₈alkylthio) is C₁-C₈alkyl (as defined above), which at the *"yl"* moiety bears one-S-atom. C₁-C₈alkylsulfanyl is linear or branched, for example, methylsulfanyl, ethylsulfanyl, propylsulfanyl, isopropylsulfanyl, n-butylsulfanyl, sec-butylsulfanyl, isobutylsulfanyl, tert-butylsulfanyl, etc.

*C₁-C₂₀alkanoyl*(=C₁-C₂₀alkylcarbonyl) is linear or branched and is, for example,C₁₋C₁₈-, C₁-C₁₄-, C₁-C₁₂-, C₁-C₈-, C₁-C₆- or C₁-C₄alkanoyl or C₄-C₁₂- or C₄-C₈alkanoyl. Examples are formyl, acetyl, propionyl, butanoyl, isobutanoyl, pentanoyl, hexanoyl, heptanoyl, octanoyl, nonanoyl, decanoyl, dodecanoyl, tetradecanoyl, pentadecanoyl, hexadecanoyl, octadecanoyl, icosanoyl, preferably acetyl.

*C₃-C₁₂alkenoyl* is mono or polyunsaturated. Examples are propenoyl, 2-methyl-propenoyl, butenoyl, pentenoyl, 1,3-pentadienoyl, 5-hexenoyl, etc.

*Halogen* is fluorine, chlorine, bromine and iodine, especially fluorine, chlorine and bromine, preferably fluorine and chlorine.

*C₁-C₂₀alkylphenyl* corresponds to phenyl that is substituted once or more times by alkyl at the phenyl ring and is for example C₁-C₁₂alkyl-, C₁-C₈alkyl- or C₁-C₄alkylphenyl, wherein the number of the alkyl corresponds to the total number of all C-atoms in all alkyl-subtstituents at the phenyl ring. Examples are tolyl, xylyl, mesityl, ethylphenyl, diethylphenyl, in particular tolyl and mesityl. *C₁-C₈alkoxyphenyl* corresponds to phenyl that is substituted once or more times by alkoxyl at the phenyl ring and is for example C₁-C₄alkoxyphenyl, wherein the number of the alkoxy corresponds to the total number of all C-atoms in all alkoxy-subtstituents at the phenyl ring. Examples are methoxyphenyl, dimethoxyphenyl, trimethoxyphenyl, ethoxyphenyl, diethoxyphenyl, ect.. *C₆-C₂₀aryl* is for example phenyl, naphthyl, anthryl or phenanthryl, in particular phenyl or naphthyl, preferably phenyl.

*Naphthyl corresponds* to 1-naphthyl and 2-naphthyl.

*C₆-C₂₀aroyl* corresponds to C₆-C₂₀aryl-CO-, wherein C₆-C₂₀aryl is defined as above. Examples are benzoyl, naphthoyl etc..

*C₆-C₂₀aryloxy* corresponds to C₆-C₂₀aryl-O-, wherein C₆-C₂₀aryl is defined as above. Examples are phenyoxy, naphthyloxy etc..

*Phenyl-C₁-C₃alkyloxy* corresponds to C₁-C₃alkyloxy (=C₁-C₃alkoxy) which is substituted by phenyl. *C₃-C₂₀heteroaryl* is meant to comprise either a one ring or a multiple ring system, e.g. a fused ring-system. Examples are thienyl, benzo[b]thienyl, naphtho[2,3-b]thienyl, thianthrenyl, furyl, dibenzofuryl, benzofuryl, chromenyl, xanthenyl, thioxanthyl, phenoxathiinyl, pyrrolyl, imidazolyl, pyrazolyl, pyrazinyl, pyrimidinyl, pyridazinyl, indolizinyl, isoindolyl, indolyl, indazolyl, purinyl, quinolizinyl, isoquinolyl, quinolyl, phthalazinyl, naphthyridinyl, quinoxalinyl, quinazolinyl, cinnolinyl, pteridinyl, carbazolyl, β-carbolinyl, phenanthridinyl, acridinyl, perimidinyl, phenanthrolinyl, phenazinyl, isothiazolyl, phenothiazinyl, isoxazolyl, furazanyl, phenoxazinyl, 7-phenanthryl, anthraquinone-2-yl (= 9,10-dioxo-9,10-dihydroanthracen-2-yl), 3-benzo[b]thienyl, 5-benzo[b]thienyl, 2-benzo[b]thienyl, 4-dibenzofuryl, 4,7-dibenzofuryl, 4-methyl-7-dibenzofuryl, 2-xanthenyl, 8-methyl-2-xanthenyl, 3-xanthenyl, 2-phenoxyathiinyl, 2,7-phenoxathiinyl, 2-pyrrolyl, 3-pyrrolyl, 5-methyl-3-pyrrolyl, 2-imidazolyl, 4-imidazolyl, 5-imidazolyl, 2-methyl-4-imidazolyl, 2-ethyl-4-imidazolyl, 2-ethyl-5-imidazolyl, 1*H-*tetrazol-5-yl, 3-pyrazolyl, 1-methyl-3-pyrazolyl, 1-propyl-4-pyrazolyl, 2-pyrazinyl, 5,6-dimethyl-2-pyrazinyl, 2-indolizinyl, 2-methyl-3-isoindolyl, 2-methyl-1-isoindolyl, 1-methyl-2-indolyl, 1-methyl-3-indolyl, 1,5-dimethyl-2-indolyl, 1-methyl-3-indazolyl, 2,7-dimethyl-8-purinyl, 2-methoxy-7-methyl-8-purinyl, 2-quinolizinyl, 3-isoquinolyl, 6-isoquinolyl, 7-isoquinolyl, 3-methoxy-6-isoquinolyl, 2-quinolyl, 6-quinolyl, 7-quinolyl, 2-methoxy-3-quinolyl, 2-methoxy-6-quinolyl, 6-phthalazinyl, 7-phthalazinyl, 1-methoxy-6-phthalazinyl, 1,4-dimethoxy-6-phthalazinyl, 1,8-naphthyridin-2-yl, 2-quinoxalinyl, 6-quinoxalinyl, 2,3-dimethyl-6-quinoxalinyl, 2,3-dimethoxy-6-quinoxalinyl, 2-quinazolinyl, 7-quinazolinyl, 2-dimethylamino-6-quinazolinyl, 3-cinnolinyl, 6-cinnolinyl, 7-cinnolinyl, 3-methoxy-7-cinnolinyl, 2-pteridinyl, 6-pteridinyl, 7-pteridinyl, 6,7-dimethoxy-2-pteridinyl, 2-carbazolyl, 3-carbazolyl, 9-methyl-2-carbazolyl, 9-methyl-3-carbazolyl, β-carbolin-3-yl, 1-methyl-β-carbolin-3-yl, 1-methyl-β-carbolin-6-yl, 3-phenanthridinyl, 2-acridinyl, 3-acridinyl, 2-perimidinyl, 1-methyl-5-perimidinyl, 5-phenanthrolinyl, 6-phenanthrolinyl, 1-phenazinyl, 2-phenazinyl, 3-isothiazolyl, 4-isothiazolyl, 5-isothiazolyl, 2-phenothiazinyl, 3-phenothiazinyl, 10-methyl-3-phenothiazinyl, 3-isoxazolyl, 4-isoxazolyl, 5-isoxazolyl, 4-methyl-3-furazanyl, 2-phenoxazinyl, 10-methyl-2-phenoxazinyl, etc..

*C₃-C₂₀heteroaryl* in particular is thienyl, benzo[b]thienyl, furyl, benzofuryl, thianthrenyl, thioxanthyl, 1-methyl-2-indolyl or 1-methyl-3-indolyl.

*C₃-C₂₀heteroarylcarbonyl* corresponds to C₃-C₂₀heteroaryl-CO-, wherein C₃-C₂₀heteroaryl is defined as above.

*C₃-C₂₀heteroaryloxy* corresponds to C₃-C₂₀heteroaryl-O-, wherein C₃-C₂₀heteroaryl is defined as above.

*C₁-C₂₀alkylene* is linear or branched and is for example , C₁-C₁₈-, C₁-C₁₄-, C₁-C₁₂-, C₁-C₈-, C₁-C₆- or C₁-C₄alkylene. Examples are methylene, ethylene, propylene, 1-methylethylene 1,1-dimethylethylene, butylene, 1-methylpropylene, 2-methyl-propylene, pentylene, hexylene, heptylene, octylene, nonylene, decylene, dodecylene, tetradecylene, hexadecylene or octadecylene.

*C₂-C₂₀alkylene interrupted by* one or more O, S, NR₅, CO, SO or SO₂ is, for example, interrupted 1-9 times, for example 1-7 times or once or twice by the defined substituents and the C₂-C₂₀alkylene is linear or branched. This produces structural units such as, for example, -CH₂-O-CH₂-, -CH₂-S-CH₂-, -CH₂-N(CH₃)-CH₂-, -CH₂CH₂-O-CH₂CH₂-, -[CH₂CH₂O]_{y}-, -[CH₂CH₂O]_{y}-CH₂-, where y = 1-9, -(CH₂CH₂O)₇CH₂CH₂-, -CH₂-CH(CH₃)-O-CH₂-CH(CH₃)- or -CH₂-CH(CH₃)-O-CH₂-CH₂CH₂-. Interrupting O-atoms are non-successive.

*C₂-C₁₂alkenylene* is mono- or polyunsaturated, linear or branched and is, for example, C₁-C₈-, C₁-C₆- or C₁-C₄alkenylene. Examples are ethenylene, 1-propenylene, 1-butenylene, 3-butenylene, 2-butenylene, 1,3-pentadienylene, 5-hexenylene, 7-octenylene, etc..

*C₃-C₂₀cycloalkylene* consists of a two valent mono- or polycyclic aliphatic ring, for example a mono-, bi- or tricyclic aliphatic ring, e.g. C₃-C₁₈-, C₃-C₁₂-, C₃-C₁₀cycloalkylene. Examples are cyclopropylene, cyclopentylene, cyclohexylene, cyclooctylene, cyclododecylene, 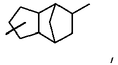 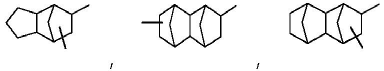 especially cyclopentylene and cyclohexylene, preferably cyclohexylene.

*C₆-C₂₀arylene* is for example phenylene, biphenylene, o-, m- and p-terphenylene, triphenylphenylene, naphthylene, binaphthylene, anthracenylene, phenanthrylene or pyrenylene, in particular phenylene or naphthylene, especially phenylene.

*C₃-C₂₀heteroarylene* refers to C₃-C₂₀heteroaryl as defined above however instead of being one-valent C₃-C₂₀heteroarylene groups are two valent.

If *R¹⁶ and R¹⁷ together with the N-atom* to which they are attached form a 5- or 6-membered saturated or unsaturated ring which is uninterrupted or interrupted by O, S or NR₅ₐ, saturated or unsaturated rings are formed, for example aziridine, pyrrole, thiazole, pyrrolidine, oxazole, pyridine, 1,3-diazine, 1,2-diazine, piperidine or morpholine. Preferably, if R¹⁶ and R¹⁷ together with the N-atom to which they are attached form a 5- or 6-membered saturated or unsaturated ring which optionally is interrupted by O, S or NR^{6a}, 5- or 6-membered saturated rings which are not interrupted or which are interrupted by O or NR^{6a}, in particular by O, are formed.

If *R^{16a} and R^{17a} together with the N-atom* to which they are attached form a 5- or 6-membered saturated or unsaturated ring which is uninterrupted or interrupted by O, S or N(C₁-C₈alkyl), saturated or unsaturated rings are formed as described above for R₅ and R₆ forming a ring with the N-atom.

*Substituted aryl* radicals phenyl, naphthyl, C₆-C₂₀aryl, C₅-C₂₀heteroaryl, C₆-C₂₀arylene or C₃-C₂₀heteroarylene etc. are substituted 1 to 7, 1 to 6 or 1 to 4 times respectively, in particular one, two or three times. It is evident that a defined aryl or heteroaryl radical cannot have more substituents than free *"CH'* or *" NH'* positions are at the defined ring.

Substituents on the phenyl ring are preferably in positions 4 or in 3,4-, 3,4,5-, 2,6-, 2,4- or 2,4,6-configuration on the phenyl ring.

*Interrupted radicals which* are interrupted once or more times are for example interrupted 1-19, 1-15, 1-12, 1-9, 1-7, 1-5, 1-4, 1-3 or once or twice (it is evident, that the number of interrupting atoms depends on the number of C-atoms to be interrupted).

*Substituted radicals,* which are substituted once or more times have for example 1-7, 1-5, 1-4, 1-3 or one or two identical or different substituents.

A radical substituted by *one or more defined* substituents is meant to have either one substituent or more substituents of identical or different definitions as given

The terms *"and*/*or"* or *"or*/*and'* in the present context are meant to express that not only one of the defined alternatives (substituents) may be present, but also several of the defined alternatives (substituents) together, namely mixtures of different alternatives (substituents).

The term *"at least"* is meant to define one or more than one, for example one or two or three, preferably one or two.

The term *"optionally substituted"* means, that the radical to which it refers is either unsubstituted or substituted.

Throughout this specification and the claims which follow, unless the context requires otherwise, the word *"comprise",* or variations such as *"comprises"* or *"comprising",* will be understood to imply the inclusion of a stated integer or step or group of integers or steps but not the exclusion of any other integer or step or group of integers or steps.

The term *"(meth)acrylate"* in the context of the present application is meant to refer to the acrylate as well as to the corresponding methacrylate.

The *preferences* indicated above for the compounds according to the present invention in the context of this invention are intended to refer to all categories of the claims, that is to the compositions, use, process claims as well.

It is to be understood that this invention is not limited to particular compounds, configurations, method steps, substrates, and materials disclosed herein as such compounds, configurations, method steps, substrates, and materials may vary somewhat. It is also to be understood that the terminology employed herein is used for the purpose of describing particular embodiments only and is not intended to be limiting since the scope of the present invention is limited only by the appended claims and equivalents thereof.

It must be noted that, as used in this specification and the appended claims, the singular forms *"a", "an"* and " *the"* include plural referents unless the context clearly dictates otherwise.

If nothing else is defined, any terms and scientific terminology used herein are intended to have the meanings commonly understood by those of skill in the art to which this invention pertains.

### General procedure of novel oxime esters

The novel oxime ester compounds of the present invention can be synthesized, for example, by the following method, but are not limited to this.

Oxime esters of formula I are prepared by methods described in the literature. Oxime esters of formula II, III or IV can be prepared analogously from the corresponding precursor compounds. For example, the compounds according to the invention can be prepared by reaction of the corresponding oximes with an acyl halide, in particular a chloride, or an anhydride in an inert solvent such as for example t-butyl methyl ether (TBME), tetrahydrofurane (THF), dimethoxyethane (DME), dimethylacetamide (DMA), dichloromethane (DCM), ethyl acetate or dimethylformamide (DMF) in the presence of a base or a mixture of bases, for example triethylamine or pyridine, or in a basic solvent such as pyridine. As example in the following the preparation of compounds of the formula I is described:

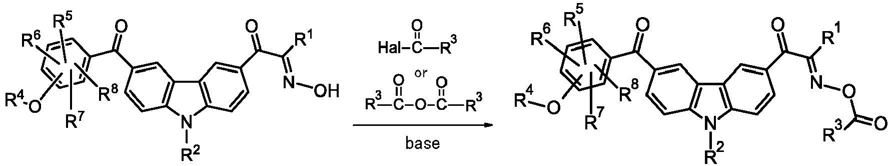

R¹ to R⁸ have the meanings as given above, and R³ is preferably methyl. Hal means a halogen atom, in particular CI. Such reactions are well known to those skilled in the art such as WO2012045736, and are generally carried out at temperatures of -15 to +50°C, preferably 0 to 25°C.

Every oxime ester group can exist in two configurations, (*Z*) or (*E*), wherein Z means that higher-priority groups attached to the C=N double bond are *cis* to each other and E means that higher-priority groups are *trans* to each other. According to the present invention, isomers of formulae I, II, III and IV are considered to be in Z configuration if the oxygen atom of the oxime ester group bonded to the N atom in the double bond on the one hand, and the keto group attached to both the C atom of the double bond and the carbazole group on the other hand are *cis* to each other, while isomers of formulae I, II, III and IV are considered to be in E configuration if the oxygen atom of the oxime ester group bonded to the N atom in the double bond on the one hand, and the keto group attached to both the C atom of the double bond and the carbazole group on the other hand are *trans* to each other:

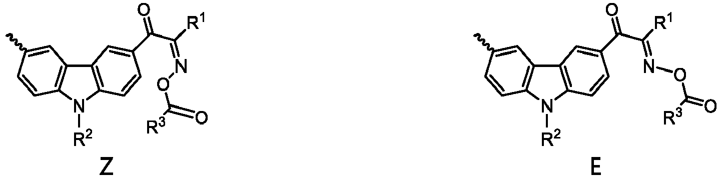

Only illustrative parts of the relevant structures are exemplified above on the basis of parts of e.g. formula I. In case more than one oxime ester group is present in the molecule, such as in compounds of formula II, III or IV, one of the oxime ester groups may be in e.g. E configuration and the other may be in E or Z configuration.

It is possible according to the present invention to use isomeric E/Z mixtures as such as photoinitiator species. On the other hand, it is also possible to use mixtures enriched in one of the Z or E isomers.

Z and E isomers can be separated by conventional methods, e.g. by column chromatography on silica gel, as described, for example, in T. Tasso et al., Chem. Eur. J. 2015, 21, 4817-4824, or Y. Zhang et al., Dyes and Pigments 2016, 133, 354-362.

The α-ketoximes required as starting materials can be obtained by a variety of methods described in standard chemistry textbooks (for instance in J. March, Advanced Organic Chemistry, 4th Edition, Wiley Interscience, 1992), or in specialized monographs, for example, S.R. Sandler & W. Karo, Organic functional group preparations, Vol. 3, Academic Press. One of the most convenient methods is, for example, the nitrosation of *"active"* methylene groups with nitrous acid or an alkyl nitrite. Both alkaline conditions, as described for example in Organic Synthesis coll. Vol. VI (J. Wiley & Sons, New York, 1988), pp 199 and 840, and acidic conditions, as described, for example, in Organic Synthesis coll. Vol. V, pp 32 and 373, coll. Vol. III, pp 191 and 513, coll. Vol. II, pp 202, 204 and 363, are suitable for the preparation of the oximes used as starting materials in the invention. Nitrous acid is usually generated from sodium nitrite. The alkyl nitrite can be for example methyl nitrite, ethyl nitrite, isopropyl nitrite, butyl nitrite, amyl nitrite, or isoamyl nitrite.

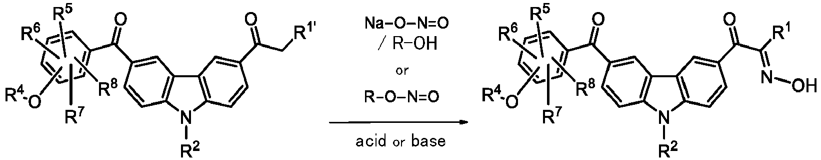

The corresponding ketone intermediates are for example prepared by the methods described in the literature, for example, in standard chemistry textbooks (for instance in J. March. Advanced Organic Chemistry, 4th Edition, Wiley Interscience, 1992). In addition, successive Friedel-Clafts reaction is effective for synthesis of the intermediates. Such reactions are well known to those skilled in the art.

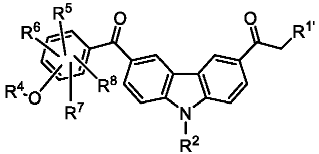

The corresponding ketone intermediates can be synthesized, for example, by the following method, but are not limited to this. A plausible synthetic scheme is described as follows. Arylation and acylation of [A], and demethylation reaction of [B] give the corresponding ketone intermediate [C]. Coupling reaction of [C] with [D] gives the corresponding ketone intermediate [E]. Alternatively, introduction of [D] can also be done after the subsequent oximation or oxime esterification (Scheme 1).

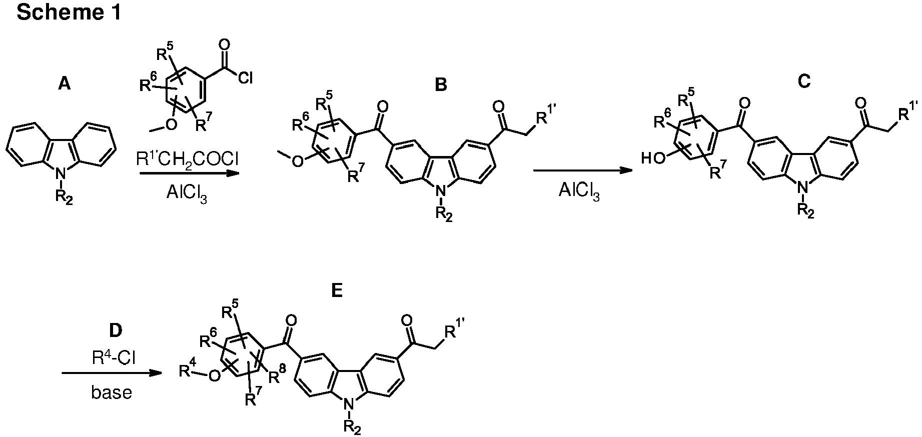

Subject of the invention also is a process for the preparation of a compound of the formula I, II, III or IV as defined above by reacting the corresponding oxime compounds of the formula IA, IIA, IIIA or IVA 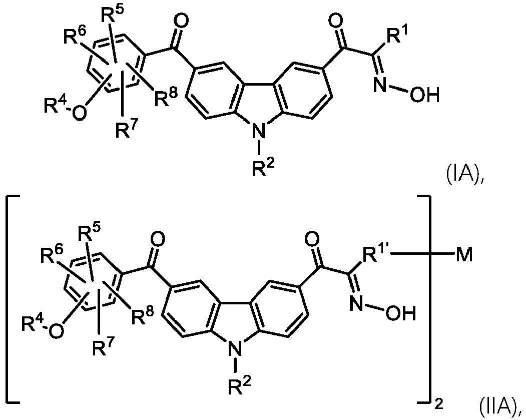 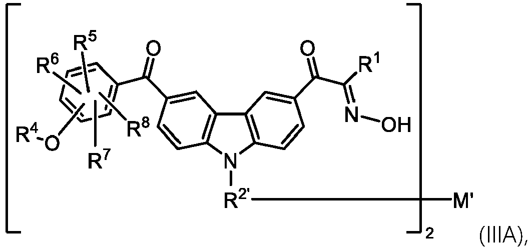 and 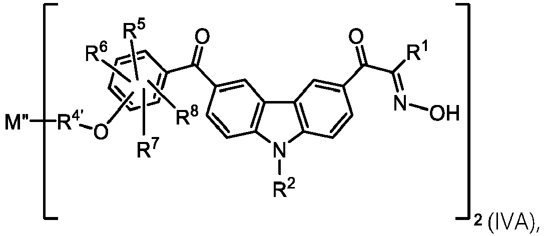 wherein
**R¹**, **R²**, **R⁴** to **R⁸**, **R^{1'}**, **R^{2'}**, **R^{4'}**, **M**, **M'** and **M"** are as defined above,
with an acyl halide of the formula I' or an anhydride of the formula I" 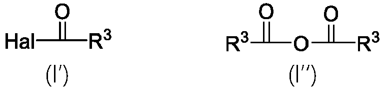
wherein Hal is a halogen, in particular CI, and R³ is as defined above, in the presence of a base or a mixture of bases.

Intereseting compounds of the formula I, II, III and IV as shown above include compounds of the formula la, Ila, IIIa or IVa: 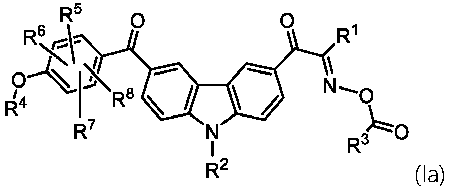 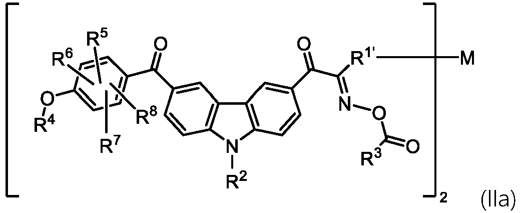 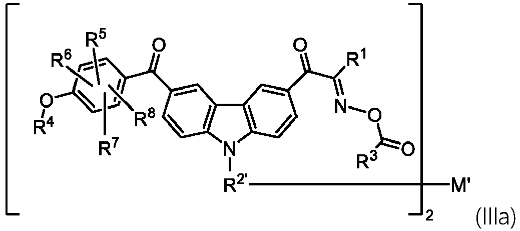 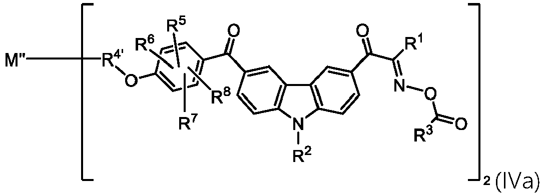 wherein
**R¹** to **R⁸**, **R^{1'}**, **R^{2'}**, **R^{4'}**, **M**, **M'** and **M"** are defined as above.

Preferred compounds of the formula I, II, III and IV according to the present invention include compounds of the formula formula Ib, IIb, IIIb or IVb: 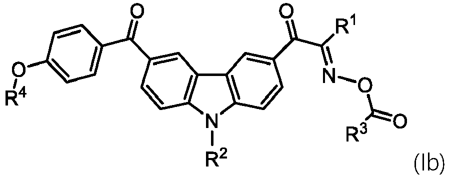 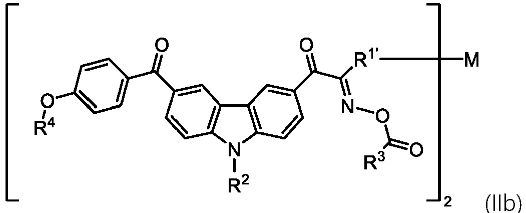 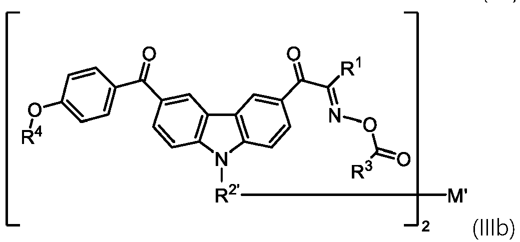 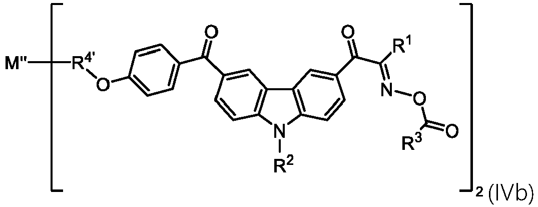 wherein
**R¹** is hydrogen, C₁-C₂₀alkyl, C₁-C₆alkyl-C₃-C₆cycloalkyl, C₃-C₂₀cycloalkyl, C₂-C₁₂alkenyl, wherein C₃-C₂₀cycloalkyl or C₂-C₁₂alkenyl is uninterrupted or interrupted by one or more O, S, CO, NR¹⁰ or COOR⁴; or
**R¹** is C₂-C₂₀alkyl which is interrupted by one or more O, CO, S, C(O)O, OC(O), SO, SO₂, phenylene, naphthylene or NR¹⁰, wherein the interrupted C₂-C₂₀alkyl is unsubstituted or substituted by one or more halogen, C₃-C₈cycloalkyl, OH, SH, OR⁴, SR⁹, COOR⁴, O(CO)-R^{3a}, CONR¹⁰R¹¹, NR¹⁰R¹¹, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR⁴, SR⁹ or NR¹⁰R¹¹; or
**R¹** is C₆-C₂₀aryl or C₃-C₂₀heteroaryl each of which is unsubstituted or substituted by one or more C₁-C₂₀alkyl;
**R^{1'}** is C₁-C₂₀alkylene, C₁-C₂₀alkylene which is interrupted by one or more O, S, (CO)O, O(CO), phenylene, naphthylene or NR⁸, wherein the C₁-C₂₀alkylene and interrupted C₁-C₂₀alkylene is unsubstituted or substituted by halogen or OR⁴,
**R²** is hydrogen, C₁-C₂₀alkyl which is unsubstituted or substituted by one or more halogen, OR⁴, SR⁹, COOR⁴ CONR¹⁰R¹¹, NR¹⁰R¹¹, PO(OR^{3a})₂; (CO)-R^{3a}, or
**R²** is C₂-C₂₀alkyl or C₁-C₆alkyl-C₃-C₆-cycloalkyl which is interrupted by one or more O, CO, S, C(O)O, OC(O), SO, SO₂, phenylene, naphthylene or NR¹⁰; or
**R²** is C₆-C₂₀aryl or C₃-C₂₀heteroaryl each of which is unsubstituted or substituted by one or more C₁-C₁₂alkyl, (CO)-R^{3a};
**R^{2'}** is C₁-C₂₀alkylene, C₁-C₂₀alkylene which is interrupted by one or more O, S, (CO)O, O(CO), phenylene, naphthylene or NR⁸, wherein the C₁-C₂₀alkylene and interrupted C₁-C₂₀alkylene is unsubstituted or substituted by halogen or OR⁴,
**R³** is hydrogen or C₁-C₂₀alkyl; or
**R³** is C₁-C₂₀alkyl substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹ or NR¹⁰R¹¹;
**R^{3a}** is hydrogen or C₁-C₂₀alkyl; or
**R^{3a}** is C₁-C₂₀alkyl interrupted by one or more O, S, NR^{10a}, CO, SO or SO₂, which is unsubstituted or substituted by C₃-C₈cycloalkyl, OH, SH, O(CO)-(C₁-C₈alkyl), COOR^{4a}, CONR^{10a}R^{11a}, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR^{4a}, SR^{9a} or NR^{10a}R^{11a}; or
**R^{3a}** is C₆-C₂₀aryl or C₃-C₂₀heteroaryl, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR^{4a}, SR^{9a}, NR^{10a}R^{11a}, COOR^{4a}, (CO)-(C₁-C₈alkyl) or SO₂-(C₁-C₄haloalkyl);
**R⁴** is hydrogen, (CO)-R^{3a}, COOR^{4a}, CONR¹⁰R¹¹, S(O)m-R^{3a} or PO(OR^{3a})₂;
**R^{4'}** is -(CO)-, -(CO)O-, -(CO)N-, -S(O)ₘ-, -PO(O)₂-, or -OP(O)₂;
**R^{4a}** is hydrogen, C₁C₂₀alkyl, (CO)O(C₁-C₈alkyl) or CON(C₁-C₈alkyl)₂; or
**R^{4a}** is C₂-C₂₀alkyl interrupted by one or more O, S, N(C₁-C₈alkyl), CO, SO or SO₂, which is unsubstituted or substituted by C₃-C₈cycloalkyl, OH, SH, O(CO)(C₁-C₈alkyl), (CO)O(C₁-C₈alkyl), (CO)N(C₁-C₈alkyl)₂, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, C₁-C₈alkoxy, C₁C₈alkylsulfanyl or N(C₁-C₈alkyl)₂; or
**R^{4a}** is C₆-C₂₀aryl, C₃-C₂₀heteroaryl, C₆-C₂₀aroyl or C₃-C₂₀heteroarylcarbonyl, each of which is unsubstituted or substituted by one or more halogen, CN, NO₂, OH, C₁-C₈alkyl, C₁-C₄haloalkyl, C₁-C₈alkoxy, phenyl-C₁-C₃alkyloxy, phenoxy, C₁C₈alkylsulfanyl, phenylsulfanyl, N(C₁-C₈alkyl)₂, diphenylamino, (CO)O(C₁-C₈alkyl), (CO)-C₁-C₈alkyl or (CO)N(C₁-C₈)₂, phenyl or benzoyl;
**R⁸** is hydrogen, C₁-C₂₀alkyl or C₆-C₂₀aryl;
**R⁹** is hydrogen or C₁-C₂₀alkyl;
**R^{9a}** is hydrogen or C₁-C₂₀alkyl;
**R¹⁰** and **R¹¹** independently of each other are hydrogen, C₁-C₂₀alkyl, S(O)ₘ-R^{3a}, O(CO)-R^{3a} (CO)-R^{3a} or CONR^{10a}R^{11a}_{;}
**R^{10a}** and **R^{11a}** independently of each other are hydrogen, C₁-C₂₀alkyl, S(O)ₘ-(C₁-C₈alkyl), O(CO)(C₁ C₈alkyl), (CO)(C₁-C₈alkyl), (CO)O(C₁-C₈alkyl) or CON(C₁-C₈alkyl)₂;
**M** is a direct bond or a divalent linking group C₁-C₂₀alkylene or C₁-C₂₀alkylene substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹ or NR¹⁰R¹¹;
**M'** is a direct bond or a divalent linking group selected from C₁-C₂₀alkylene or C₁-C₂₀alkylene substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹ or NR¹⁰R¹¹;
**M"** is a direct bond or a divalent linking group selected from C₁-C₂₀alkylene or C₁-C₂₀alkylene substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹ or NR¹⁰R¹¹; and
**m** is 1 or 2.

It is preferred according to the invention that the compounds of the formula I, II, III and IV are compounds of the above formula Ib, wherein
**R¹** is C₁-C₂₀alkyl, C₁-C₆alkyl-C₃-C₆cycloalkyl, C₃-C₂₀cycloalkyl; or
**R¹** is C₂-C₂₀alkyl which is interrupted by one or more O, CO, S, C(O)O, OC(O), SO, or SO₂; or
**R¹** is C₆-C₂₀aryl which is unsubstituted or substituted by one or more C₁-C₂₀alkyl;
**R²** is C₁-C₂₀alkyl; or
**R²** is C₂-C₂₀alkyl or C₁-C₆alkyl-C₃-C₆-cycloalkyl which is interrupted by one or more O, CO, S, C(O)O, OC(O), SO or SO₂; or
**R²** is C₆-C₂₀aryl which is unsubstituted or substituted by one or more C₁-C₁₂alkyl or (CO)-R^{3a};
**R³** is C₁-C₂₀alkyl;
**R^{3a}** is C₁-C₂₀alkyl, C₆-C₂₀aryl or C₃-C₂₀heteroaryl;
**R⁴** is (CO)-R^{3a}, COOR^{4a}, CONR¹⁰R¹¹, S(O)ₘ-R^{3a} or PO(OR^{3a})₂;
**R^{4a}** is C₁-C₂₀alkyl, C₆-C₂₀aryl or C₃-C₂₀heteroaryl;
**R¹⁰** and **R¹¹** are C₁-C₂₀alkyl; and
**m is** 1 or 2.

The C=N double bonds present in oxime ester groups in the above compounds of formulae I, II, III or IV may be present in Z configuration or in E configuration as outlined above. Thus, the present invention also relates to compounds of the above formulae I, II, III and IV, wherein the C=N double bonds present in oxime ester groups are in either Z configuration or in E configuration.

In another embodiment, the present invention relates to compounds of formulae I, II, III and IV, wherein more than 50% of the C=N double bonds present in oxime ester groups are in Z configuration. Preferably, 55% or more, or 75% or more of the C=N double bonds present in oxime ester groups in the molecule are in Z configuration. For example, 90% or more, 95% or more, such as 99% or more of the C=N double bonds present in oxime ester groups in the molecule are in Z configuration.

In an alternative embodiment, the present invention relates to compounds of the above formulae I, II, III and IV, wherein more than 50% of the C=N double bonds present in oxime ester groups are in E configuration. Preferably, 55% or more, or 75% or more of the C=N double bonds present in oxime ester groups in the molecule are in E configuration. For example, 90% or more, 95% or more, such as 99% or more of the C=N double bonds present in oxime ester groups in the molecule are in E configuration.

For example, the present invention relates to compounds of formula I, wherein more than 50%, preferably 55% or more, or 75% or more, or 90% or more, or 95% or more, or 99% or more of the C=N double bonds present in the oxime ester group are in Z configuration:

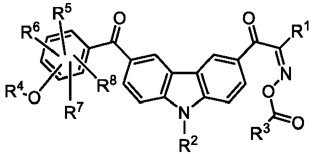

In another illustrative embodiment, the present invention relates to compounds of formula I, wherein more than 50%, preferably 55% or more, or 75% or more, or 90% or more, or 95% or more, or 99% or more of the C=N double bonds present in the oxime ester group are in E configuration:

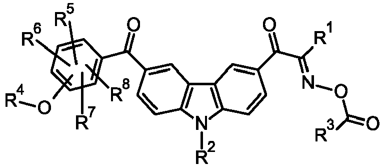

In a specific example, the present invention relates to a compound of formula OE17, wherein more than 50%, preferably 55% or more, or 75% or more, or 90% or more, or 95% or more, or 99% or more of the C=N double bonds present in the oxime ester group are in Z configuration: 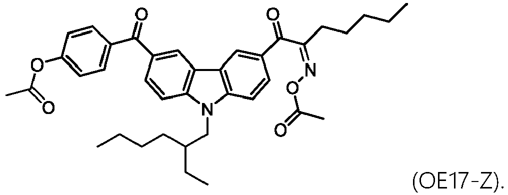 In another illustrative example, the present invention relates to a compound of formula OE17, wherein more than 50%, preferably 55% or more, or 75% or more, or 90% or more, or 95% or more, or 99% or more of the C=N double bonds present in the oxime ester group are in Z configuration:

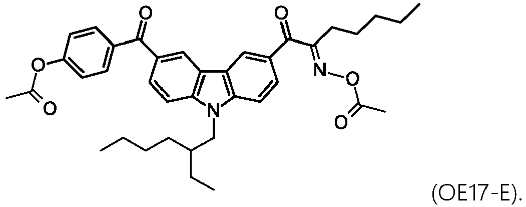

The compounds of the formulae I, II, III and IV are suitable as radical photoinitiators. Accordingly, subject of the invention is the use of a compound of the formula I, II, III and IV as defined above for the photopolymerization of a composition comprising at least one ethylenically unsaturated photopolymerizable compound.

Another subject of the present invention therefore is a photopolymerizable composition comprising
(a) at least one ethylenically unsaturated photopolymerizable compound and
(b) as photoinitiator, at least one compound of the formula I, II, III or IV as defined above.

The composition may comprise additionally to the photoinitiator (b) at least one further photoinitiator (c) or other additives (d), or both, at least one further photoinitiator (c) and other additives (d).

Other additives (d) for example are one or more components selected from thermal radical initiators, pigments, fillers, dispersants and sensitizers.

Said additives are described in more detail below.

Interesting is a photopolymerizable composition as described above, comprising 0.05 to 25 % by weight of the photoinitiator (b), or the photoinitiators (b) and (c), based on the composition.

The unsaturated compounds (a) may include one or more olefinic double bonds. They may be of low (monomeric) or high (oligomeric) molecular mass.

Component (a) for example comprises a resin obtained by the reaction of a saturated or unsaturated polybasic acid anhydride with a product of the reaction of an epoxy resin and an unsaturated monocarboxylic acid.

Subject of the invention accordingly also is a photopolymerizable composition as described above, wherein the component (a) is a resin obtained by the reaction of a saturated or unsaturated polybasic acid anhydride with a product of the reaction of an epoxy resin and an unsaturated monocarboxylic acid.

Component (a) for example comprises an alkaline developable resin.

The polymerizable composition according to the invention preferably comprises the component (a) in an amount of from 2 to 98% by weight, more preferably from 5 to 90% by weight, in particular from 10 to 80% by weight, based on the whole solid contents of the polymerizable composition (i.e. the amount of all components without the solvent(s)).

Preferably, the alkaline developable resin has free carboxylic groups. The acid number is preferably from 50 to 600 mg KOH/g, more preferably 100 to 300 mg KOH/g. The acid numbers stated here are the acid number according to DIN EN 12634.

Examples of alkali developable resins are acrylic polymers having carboxylic acid function as a pendant group, such as copolymers obtained by copolymerizing an ethylenic unsaturated carboxylic acid such as (meth)acrylic acid, 2-carboxyethyl (meth)acrylic acid, 2-carboxypropyl (meth)acrylic acid, itaconic acid, citraconic acid, mesaconic acid, fumaric acid, crotonic acid, maleic acid, maleic anhydride, fumaric anhydride, half-ester of maleic acid, cinnamic acid, mono[2-(meth)acryloyloxyethyl] succinate, mono[2-(meth)acryloyloxyethyl] adipate, mono[2-(meth)acryloyloxyethyl] phthalate, mono[2-(meth)acryloyloxyethyl] hexahydrophthalate, mono[2-(meth)acryloyloxyethyl] maleate, mono[2-(meth)acryloyloxypropyl] succinate, mono[2-(meth)acryloyloxypropyl] adipate, mono[2-(meth)acryloyloxypropyl] phthalate, mono[2-(meth)acryloyloxypropyl] hexahydrophthalate, mono[2-(meth)acryloyloxypropyl] maleate, mono[2-(meth)acryloyloxybutyl] succinate, mono[2-(meth)acryloyloxybutyl] adipate, mono[2-(meth)acryloyloxybutyl] phthalate, mono[2-(meth)acryloyloxybutyl] hexahydrophthalate, mono[2-(meth)acryloyloxybutyl] maleate, 3-(alkylcarbamoyl)acrylic acid, α-chloroacrylic acid, monoesterified maleic acid, and ω-carboxypolycaprolactone mono(meth)acrylate, with one or more monomers selected from esters of (meth)acrylic acid, such as methyl (meth)acrylate, ethyl (meth)acrylate, propyl (meth)acrylate, butyl (meth)acrylate, benzyl (meth)acrylate, 2-ethylhexyl (meth)acrylate, hydroxyethyl (meth)acrylate, hydroxypropyl (meth)acrylate, tetrahydrofurfuryl (meth)acrylate, hydroxybutyl (meth)acrylate, glycerol mono(meth)acrylate, dihydroxypropyl (meth)acrylate, allyl (meth)acrylate, cyclohexyl (meth)acrylate, phenyl (meth)acrylate, methoxyphenyl (meth)acrylate, methoxyethyl (meth)acrylate, phenoxyethyl (meth)acrylate, methoxydiethyleneglycol (meth)acrylate, methoxytriethyleneglycol (meth)acrylate, methoxypropyl (meth)acrylate, methoxydipropyleneglycol (meth)acrylate, (3-trimethoxysilyl)propyl (meth)acrylate, (meth)acrylic acid trimethylsilyl ester, isobornyl meth(acrylate), dicyclopentadienyl (meth)acrylate, 2-hydroxy-3-phenoxypropyl (meth)acrylate, tricyclo[5.2.1.0^{2,6}]decan-8-yl (meth)acrylate, aminoethyl (meth)acrylate, N, N-dimethylaminoethyl (meth)acrylate, aminopropyl (meth)acrylate, N, N-dimethylaminopropyl (meth)acrylate, glycidyl (meth)acrylate, 2-methylglycidyl (meth)acrylate, 3,4-epoxybutyl (meth)acrylate, 6,7-epoxyheptyl (meth)acrylate; vinyl aromatic compounds, such as styrene, α-methylstyrene, vinyltoluene, p-chlorostyrene, polychlorostyrene, fluorostyrene, bromostyrene, ethoxymethyl styrene, methoxystyrene, 4-methoxy-3-methystyrene, dimethoxystyrene, vinylbenzyl methyl ether, vinylbenzyl glycidyl ether, indene, 1-methylindene; 1-ethenyl-4-silyl-benzen, 1-ethenyl-4-trimethylsilyl-benzene, t-buthyl dimethylsilyl *p*-vinyl phenyl ester; amide type unsaturated compounds, such as (meth)acrylamide, diacetone acrylamide, N-methylolacrylamide, N-butoxymethacrylamide, N,N-dimethyl (meth)acrylamide, N,N-diethyl (meth)acrylamide, N,N-dibutyl (meth)acrylamide, N,N-diethylhexyl (meth)acrylamide, N,N-dicyclohexyl (meth)acrylamide, N,N-diphenyl (meth)acrylamide, N-methyl-N-phenyl (meth)acrylamide, N-hydroxyethyl-N-methyl (meth)acrylamide, N-methyl (meth)acrylamide, N-ethyl (meth)acrylamide, N-propyl (meth)acrylamide, N-butyl (meth)acrylamide, N-hydroxyethyl (meth)acrylamide, N-heptyl (meth)acrylamide, N-octyl (meth)acrylamide, N-ethyhexyl (meth)acrylamide, N-hydroxyethyl (meth)acrylamidecyclohexyl, N-benzyl (meth)acrylamide, N-phenyl (meth)acrylamide, N-tolyl (meth)acrylamide, N-hydroxyphenyl (meth)acrylamide, N-naphthyl (meth)acrylamide, N-phenylsulfonyl (meth)acrylamide, N-methylphenylsulfonyl (meth)acrylamide and N-(meth)acryloylmorpholine; acetal ester or ketal ester compounds, such as norbornene, 2,3-bis(trimethylsilyloxycarbonyl)-5-norbornene, 2,3-bis(triethylsilyloxycarbonyl)-5-norbornene, 2,3-bis(*t-*butyldimethylsilyloxycarbonyl)-5-norbornene, 2,3-bis(trimethylgermyloxycarbonyl)-5-norbornene, 2,3-bis(triethylgermyloxycarbonyl)-5-norbornene, 2,3-bis(*t*-butyldimethylgermyloxycarbonyl)-5-norbornene, 2,3-bis(*t*-butyloxycarbonyl)-5-norbornene, 2,3-bis(benzyloxycarbonryl)-5-norbornene, 2,3-bis(tetrahydrofurane-2-yloxycarbonyl)-5-norbornene, 2,3-bis(cyclopentyloxycarbonyl)-5-norbornene, 2,3-bis(cyclohexyloxycarbonyl)-5-norbornene, 2,3-bis(cycloheptyloxycarbonyl)-5-norbornene, 2,3-bis(1-methoxyethoxycarbonyl)-5-norbornene, 2,3-bis(1-*t*-butoxyethoxycarbonyl)-5-norbornene, 2,3-bis(1-benzyloxyethoxycarbonyl)-5-norbornene, 2,3-bis[(cyclohexyl)(ethoxy)methoxycarbonyl]-5-norbornene, 2,3-bis(1-methyl-1-methoxyethoxycarbonyl)-5-norbornene, 2,3-bis(1-methyl-1-*i*-butoxyethoxycarbonyl)-5-norbornene, 2,3-bis[(benzyl)(ethoxy)methoxycarbonyl]-5-norbornene; 1-alkylcycloalkylester compounds, such as 1-metylcyclopropyl (meth)acrylate, 1-methylcyclobutyl (meth)acrylate, 1-methylcyclopentyl (meth)acrylate, 1-methylcyclohexyl (meth)acrylate, 1-methylcycloheptyl (meth)acrylate, 1-methylcyclooctyl (meth)acrylate, 1-methylcyclononyl (meth)acrylate, 1-ethylcyclodecyl (meth)acrylate, 1-ethylcyclopropyl (meth)acrylate, 1-ethylcyclobutyl (meth)acrylate, 1-ethylcyclopentyl (meth)acrylate, 1-ethylcyclohexyl (meth)acrylate, 1-ethylcycloheptyl (meth)acrylate, 1-ethylcyclooctyl (meth)acrylate, 1-ethylcyclononyl (meth)acrylate, 1-ethylcyclodecyl (meth)acrylate, 1-*i*-propylcyclopropyl (meth)acrylate, 1-*i*-propylcyclopentyl (meth)acrylate, 1-*i*-propylcyclohexyl (meth)acrylate, 1-*i*-propylcycloheptyl (meth)acrylate, 1-*i*propylcyclooctyl (meth)acrylate, 1-*i*-propylcyclononyl (meth)acrylate, 1-*i*-propylcyclodecyl (meth)acrylate, 1-*i*-butylcyclopropyl (meth)acrylate, 1-*i*-butylcyclobutyl (meth)acrylate, 1-*i-*butylcyclopentyl (meth)acrylate, 1-*i*-butylcyclohexyl (meth)acrylate, 1-*i*-butylcyclooctyl (meth)acrylate, 1-*i*-butylcyclononyl (meth)acrylate, 1-*i*-butylcyclodecyl (meth)acrylate, 1-*i-*pentylcyclopropyl (meth)acrylate, 1-*i*-pentylcyclopentyl (meth)acrylate, 1-*i*-pentylcyclohexyl (meth)acrylate, 1-*i*-pentylcycloheptanyl (meth)acrylate, 1-*i*-pentylcyclooctyl (meth)acrylate, 1-*i-*pentylcyclononyl (meth)acrylate, 1-*i*-pentylcyclodecyl (meth)acrylate, 1-*i*-octylcyclopropyl (meth)acrylate, 1-*i*-octylcyclobutyl (meth)acrylate,1-*i*-octylcycloheptyl (meth)acrylate, 1-*i-*octylcyclooctyl (meth)acrylate, 1-*i*-octylcyclononyl (meth)acrylate, 1-*i*-octylcyclodecyl (meth)acrylate; methacryl acids, such as 3-(methacryloyloxymethyl)oxetane, 3-(methacryloyloxymethyl)-3-ethyloxetane, 3-(methacryloyloxymethyl)-2-methyloxetane, 3-(methacryloyloxymethyl)-2-trifluoromethyloxetane, 3-(methacryloyloxynethyl)-2-pentafluoroethyloxetane, 3-(methacryloyloxymethyl)-2-phenyloxetane, 3-(methacryloyloxymethyl)-2,2-difluorooxetane, 3-(methacryloyloxymethyl)-2,2,4,-trifluorooxetane, 3-(methacryloyloxymethyl)-2,2,4,4-tetrafluorooxetane, 3-(methacryloyloxyethyl)oxetane, 3-(methacryloyloxyethyl)-3-ethyloxetane, 2-ethyl-3-(methacryloyloxyethyl)oxetane, 3-(methacryloyloxyethyl)-2-trifluoromethyloxetane, 3-(methacryloyloxyethyl)-2-pentafluoroethyloxetane, 3-(methacryloyloxyethyl)-2-phenyloxetane, 2,2-difluoro-3-(methacryloyloxyethyl)oxetane, 3-(methacryloyloxyethyl)-2,2,4-trifluorooxetane, 3-(methacryloyloxyethyl)-2,2,4,4,-tetrafluorooxetane; polycyclic compounds or anhydride, such as 5-carboxybicyclo[2.2.1]hept-2-ene, 5,6-dicarboxybicyclo[2.2.1]hept-2-ene, 5-carboxy-5-methylbicyclo[2.2.1]hept-2-ene, 5-carboxy-6-ethylbicyclo[2.2.1]hept-2-ene, 5-carboxy-6-methylbicyclo[2.2.1]hept-2-ene, 5-carboxy-6-ethylbicyclo[2.2.1]hept-2-ene, 5,6-dicarboxybicyclo[2.2.1]hept-2-ene anhydride; vinyl or allyl esters, such as vinyl acetate, vinyl propionate, vinyl butylate, vinyl pivalate, vinyl benzoate, vinyl trimethylacetate, vinyl diethylacetate, vinyl borate, vinyl caproate, vinyl chloroacetate, vinyl dichloroacetate, vinyl methoxyacetate, vinyl butoxyacetate, vinyl phenylacetate, vinyl acetate, vinyl acetoacetate, vinyl lactate, vinyl phenylbutylate, vinyl cyclohexylcarboxylate, vinyl salicylate, vinyl chlorobenzoate, vinyl tetrachlorobenzoate, vinyl naphthoate, vinyl triethoxysilane, allyl acetate, allyl propionate, allyl butylate, allyl pivalate, allyl benzoate, allyl caproate, allyl stearate, allyl acetoacetate, allyl lactate; vinyl or allyl ethers, such as vinyl methyl ether, vinyl ethyl ether, vinyl hexyl ether, vinyl octyl ether, vinyl ethylhexyl ether, vinyl methoxyethyl ether, vinyl ethoxyethyl ether, vinyl chloroethyl ether, vinyl hydroxyethyl ether, vinyl ethybutyl ether, vinyl hydroxyethoxyethyl ether, vinyl dimethylaminoethyl ether, vinyl diethylaminoethyl ether, vinyl butylaminoethyl ether, [(ethenyloxy)methyl]triethylsilane, vinyl benzyl ether, vinyl tetrahydrofurfuryl ether, vinyl phenyl ether, vinyl tolyl ether, vinyl chlorophenyl ether, vinyl chloroethyl ether, vinyl dichlorophenyl ether, vinyl naphthyl ether, vinyl anthryl ether, allyl glycidyl ether; crotonates, such as butyl crotonate, hexyl crotonate, glycerine monocrotonate; itaconates, such as dimethyl itaconate, diethyl itaconate, dibutyl itaconate; and maleates or fumarates, such as dimethyl maleate, dibutyl fumarate; polyolefin type compounds, such as butadiene, isoprene, chloroprene and the like; methacrylonitrile, methyl isopropenyl ketone, vinyl acetate, vinyl propionate, vinyl pivalate, maleimide, N-phenylmaleimide, N-methylphenylmaleimide, N-methoxyphenylmaleimide, N-cyclohexylmaleimide, N-alkylmaleimide, maleic anhydride, polystyrene macromonomer, polymethyl (meth)acrylate macromonomer, polybutyl (meth)acrylate macromonomer. Examples of copolymers are copolymers of acrylates and methacrylates with acrylic acid or methacrylic acid and with styrene or substituted styrene, phenolic resins, for example novolak, (poly)hydroxystyrene, and copolymers of hydroxystyrene with alkyl acrylates, acrylic acid and/or methacrylic acid. Preferable examples of copolymers are copolymers of methyl (meth)acrylate/(meth)acrylic acid, copolymers of benzyl (meth)acrylate/(meth)acrylic acid, copolymers of methyl (meth)acrylate/ethyl (meth)acrylate/(meth)acrylic acid, copolymers of benzyl (meth)acrylate/(meth)acrylic acid/styrene, copolymers of benzyl (meth)acrylate/(meth)acrylic acid/hydroxyethyl (meth)acrylate, copolymers of benzyl (meth)acrylate/(meth)acrylic acid/glycidyl (meth)acrylate, copolymers of benzyl (meth)acrylate/(meth)acrylic acid/3-(methacryloyloxymethyl)oxetane, copolymers of methyl (meth)acrylate/butyl (meth)acrylate/(meth)acrylic acid/styrene, copolymers of methyl (meth)acrylate/benzyl (meth)acrylate/(meth)acrylic acid/hydroxyphenyl (meth)acrylate, copolymers of methyl (meth)acrylate/(meth)acrylic acid/polymethyl (meth)acrylate macromonomer, copolymers of benzyl (meth)acrylate/(meth)acrylic acid/polymethyl (meth)acrylate macromonomer, copolymers of tetrahydrofurfuryl (meth)acrylate/styrene/(meth)acrylic acid, copolymers of methyl (meth)acrylate/(meth)acrylic acid/polystyrene macromonomer, copolymers of benzyl (meth)acrylate/(meth)acrylic acid/polystyrene macromonomer, copolymers of benzyl (meth)acrylate/(meth)acrylic acid/2-hydroxyethyl (meth)acrylate/polystyrene macromonomer, copolymers of benzyl (meth)acrylate/(meth)acrylic acid/2-hydroxypropyl (meth)acrylate/polystyrene macromonomer, copolymers of benzyl (meth)acrylate/(meth)acrylic acid/2-hydroxy-3-phenoxypropyl (meth)acrylate/polymethyl (meth)acrylate macromonomer, copolymers of methyl (meth)acrylate/(meth)acrylic acid/2-hydroxyethyl (meth)acrylate/polystyrene macromonomer, copolymers of benzyl (metha)crylate/(meth)acrylic acid/2-hydroxyethyl (meth)acrylate/polymethyl (meth)acrylate macromonomer, copolymers of N-phenylmaleimide/benzyl (meth)acrylate/(meth)acrylic acid and styrene, copolymers of benzyl (meth)acrylate/(meth)acrylic acid/N-phenylmaleimide/mono-[2-(meth)acryloyloxyethyl] succinate/styrene, copolymers of allyl (meth)acrylate/(meth)acrylic acid/N-phenylmaleimide/mono-[2-(meth)acryloyloxyethyl] succinate/styrene, copolymers of benzyl (meth)acrylate/(meth)acrylic acid/N-phenylmaleimide/glycerol mono(meth)acrylate/styrene, copolymers of benzyl (meth)acrylate/ω-carboxypolycaprolactone mono(meth)acrylate/(meth)acrylic acid/N-phenylmaleimide/glycerol mono(meth)acrylate/styrene, and copolymers of benzyl (meth)acrylate/(meth)acrylic acid/N-cyclohexylmaleimide/styrene. Example of commercial product is Ripoxy SPC-2000 provided by Showa Denko K.K. The term "*(meth)acrylate*" in the context of the present application is meant to refer to the acrylate as well as to the corresponding methacrylate.

One or more monomers selected from the group consisting of silicon containing monomers such as silanes, such as tetraethylorthosilicate or tetraethoxysilane or chloro- or alkoxy functional silanes, olefins such as ethylene, propylene, styrene, vinylpyrrolidone, oxygen- or nitrogen-containing monomers such as acrylic derivatives, e.g. acrylic ester and acrylic acid, methacrylic acid and -ester, urethanes, mono- and di-functional alcohols, carboxylic acids, amines, isocyanates, epoxides, aromatic compounds such as aromatics carrying substituents such as alkyl groups and sulfonated aromatics, aromatic resins, imidazole and imidazole derivatives, pyrazoles, quartenary ammonium compounds, polyurethane prepolymers and epoxy resins.

The functional groups in the compound, which result in good alkaline solubility, are preferably carboxylic groups. However, also other groups, which result in alkaline solubility, are possible. Examples for such groups are phenolic groups, sulfonic acid groups and anhydride groups.

When the number of ethylenically unsaturated bonds, which are present in the molecular unit of the resin curable by the activated energy ray, is small, it is possible to use bis-phenol A type epoxy compounds to lower the viscosity of the ink.

The novolak type epoxy compounds are represented by phenol novolak type epoxy resins and cresol novolak type epoxy resins. Such compounds are typically produced by reacting epichlorohydrin with a novolak resin.

Typical examples of the aforementioned acid anhydride are dibasic acid anhydrides such as for example maleic anhydride, succinic anhydride, itaconic anhydride, phthalic anhydride, tetrahydrophthalic anhydride, hexahydrophthalic anhydride, methylhexahydrophthalic anhydride, endo-methylenetetrahydrophthalic anhydride, methyl-endo-methylenetetrahydrophthalic anhydride, chlorendic anhydride, and methyltetrahydrophthalic anhydride; aromatic polycarboxylic anhydrides such as for example trimellitic anhydride, pyromellitic anhydride and benzophenone-tetracarboxylic dianhydride: and polycarboxylic anhydride derivatives such as 5-(2,5-dioxotetrahydrofuryl)-3-methyl-3-cyclohexene-1, 2-dicarboxylic anhydride.

Further examples of alkaline developable resins (d) are polymers or oligomers having at least two ethylenically unsaturated groups and at least one carboxyl function within the molecule structure, such as a resin obtained by the reaction of a saturated or unsaturated polybasic acid anhydride with a product of the reaction of an epoxy compound and an unsaturated monocarboxylic acid (for example, EB9696 from UCB Chemicals; KAYARAD TCR1025 from Nippon Kayaku Co.Ltd.; NK OLIGO EA-6340, EA-7440 from Shin-Nakamura Chemical Co.,Ltd.). Other examples of such binders are described in JP2002-206014A, JP2004-69754A, JP2004-302245A, JP2005-77451A, JP2005-316449A, JP2005-338328A and JP3754065B2.

Further examples of alkaline developable resins (d) are the above-mentioned polymers or oligomers having at least one ethylenically unsaturated groups.

Further examples are reaction products obtained by adding an epoxy group containing unsaturated compound to a part of the carboxyl groups of a carboxylic acid group containing polymer (for ex., ACA200, ACA200M, ACA210P, ACA230AA, ACA250, ACA300, ACA320 from Daicel Co. and Ripoxy SPC-1000 provided by Showa Denko K. K.). As the carboxylic acid containing polymer, the abovementioned binder polymers which are resulting from the reaction of an unsaturated carboxylic acid compound with one or more polymerizable compounds, for example, copolymers of (meth)acrylic acid, benzyl (meth)acrylate, styrene and 2-hydroxyethyl (meth)acrylate, copolymers of (meth)acrylic acid, styrene and α-methylstyrene, copolymers of (meth)acrylic acid, N-phenylmaleimide, styrene and benzyl (meth)acrylate, copolymers of (meth)acrylic acid and styrene, copolymers of (meth)acrylic acid and benzyl (meth)acrylate, copolymers of tetrahydrofurfuryl (meth)acrylate, styrene and (meth)acrylic acid and the like.

Examples of the unsaturated compounds having an epoxy group are given below in the formula (V-1) - (V-15); 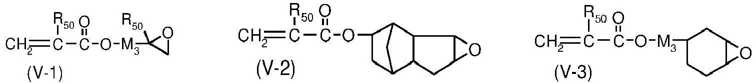 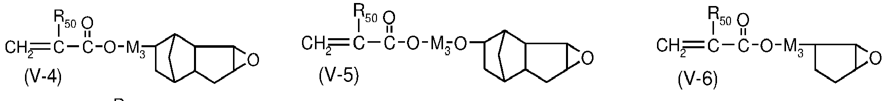 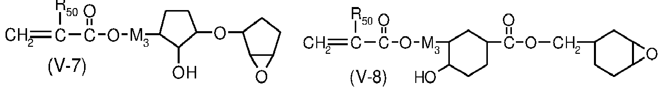 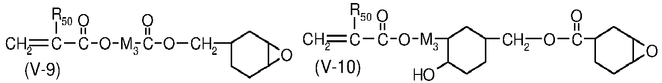 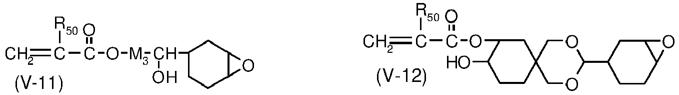 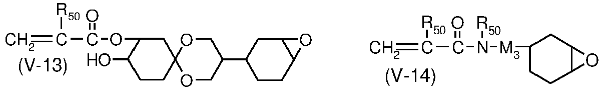 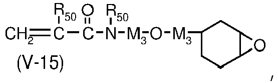 wherein R₅₀ is hydrogen or methyl group, M₃ is substituted or unsubstituted alkylene having 1 to 10 carbon atoms.

Among these compounds, compounds having alicyclic epoxy groups are particularly preferred, because these compounds have a high reactivity with carboxyl group-containing resins, accordingly the reaction time can be shortened. These compounds further do not cause gelation in the process of reaction and make it possible to carry out the reaction stably. On the other hand, glycidyl acrylate and glycidyl methacrylate are advantageous from the viewpoint of sensitivity and heat resistance because they have a low molecular weight and can give a high conversion of esterification.

Concrete examples of the abovementioned compounds are, for example a reaction product of a copolymer of styrene, α-methylstyrene and acrylic acid or a copolymer of methyl methacrylate and acrylic acid with 3,4-epoxycyclohexylmethyl (meth)acrylate.

Other examples are products obtained by addition reaction of an epoxy group containing unsaturated compounds to a part of or all of the carboxyl groups of a carboxylic acid group containing polymers followed by further reaction with polybasic acid anhydride (for ex., Ripoxy SPC-3000 provided by Showa Denko K.K.).

Unsaturated compounds having a hydroxy group such as 2-hydroxyethyl (meth)acrylate and glycerol mono(meth)acrylate can be used instead of the abovementioned epoxy group containing unsaturated compounds as the reactant for carboxylic acid group containing polymers.

Other examples are half esters of anhydride containing polymers, for example reaction products of a copolymer of maleic anhydride and one or more other polymerizable compounds with (meth)acrylates having an alcoholic hydroxyl group such as 2-hydroxyethyl (meth)acrylate or having an epoxy group for example such as the compounds described in the formula (V-1) - (V-15).

Reaction products of polymers having alcoholic hydroxyl groups such as copolymers of 2-hydroxyethyl (meth)acrylate, (meth)acrylic acid, benzyl (meth)acylate and styrene, with (meth)acrylic acid or (meth)acryl chloride can also be used.

Other examples are reaction products of polyester with terminal unsaturated groups, which is obtained from the reaction of a dibasic acid anhydride and a compound having at least two epoxy groups followed by further reaction with an unsaturated compound, with a polybasic acid anhydride.

Further examples are resins obtained by the reaction of a saturated or unsaturated polybasic acid anhydride with a reaction product obtained by adding epoxy groups containing (meth)acrylic compounds to all of the carboxyl groups of a carboxylic acid containing polymer as mentioned above.

Other example is polyimide resin having ethylenically unsaturated groups and at least one carboxyl function. The polyimide binder resin in the present invention can be a polyimide precursor, for example, a poly(amic acid).

Specific examples of alkaline developable resins are:
Acrylpolymer type resins such as 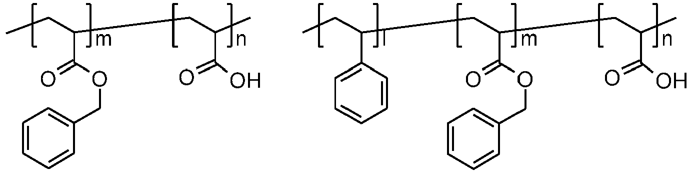 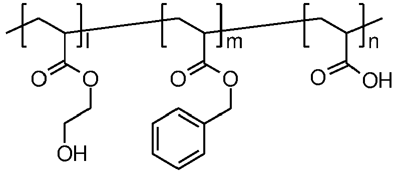 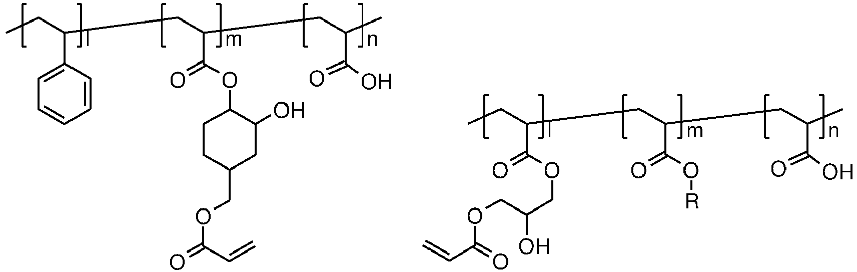 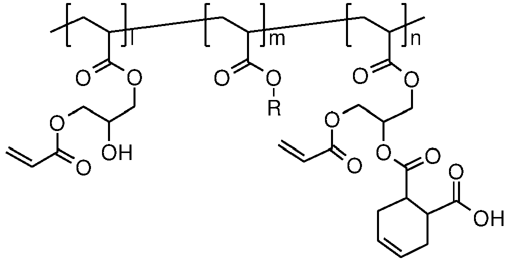
Cardo type resin (fluorene epoxy acrylate based resin)

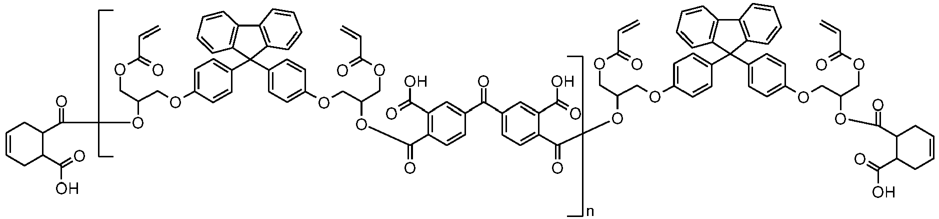

For example, a content of the binder resin may be 2-98%, preferably 5-90% and especially 10-80% by weight based on a total weight of the solid contents in the radically polymerizable composition.

Component (a) for example comprises an acrylate monomer.

The acrylate monomer refers to an acrylate monomer or oligomer that contains one or more acryloyl or methacryloyl moieties or combinations thereof.

Examples of compounds containing a double bond are (meth)acrylic acid, alkyl, hydroxyalkyl or aminoalkyl (meth)acrylates, for example methyl, ethyl, n-butyl, i-butyl, t-butyl, n-propyl, i-propyl, n-hexyl, cyclohexyl, 2-ethylhexyl, isobornyl, benzyl, 2-hydroxyethyl, 2-hydroxypropyl, methoxyethyl, ethoxyethyl, glycerol, phenoxyethyl, methoxydiethylene glycol, ethoxydiethylene glycol, polyethylene glycol, polypropylene glycol, glycidyl, N, N-dimethylaminoethyl, and N, N-diethylaminoethyl (meth)acrylates. Other examples are (meth)acrylonitrile, (meth)acrylamide, N-substituted (meth)acrylamides such as N, N-dimethyl (meth)acrylamide, N, N-diethyl (meth)acrylamide, N, N-dibutyl (meth)acrylamide, N-methyl (meth)acrylamide, N-ethyl (meth)acrylamide, N-butyl (meth)acrylamide, and N-(meth)acryloylmorpholine, vinyl esters such as vinyl acetate, vinyl ethers such as i-butyl vinyl ether, styrene, alkyl-, hydroxy- and halostyrenes, N-vinylpyrrolidone, N-vinylcaprolactam, N-vinyl acetoamide, N-vinyl formamide, vinyl chloride and vinylidene chloride.

Examples of polyunsaturated compounds of relatively high molecular mass (oligomers) are polyesters, polyurethanes, polyethers and polyamides, which contain ethylenically unsaturated carboxylates.

Particularly suitable examples are esters of an ethylenically unsaturated carboxylic acid with a polyol or polyepoxide.

Examples of unsaturated carboxylic acids are acrylic acid, methacrylic acid, crotonic acid, itaconic acid, cinnamic acid, and unsaturated fatty acids such as linolenic acid or oleic acid. Acrylic and methacrylic acids are preferred.

Suitable polyols are aromatic, in particular, aliphatic and cycloaliphatic polyols. Examples of aromatic polyols are hydroquinone, 4,4'-dihydroxybiphenyl, 2,2-bis(4-hydroxyphenyl)ethane, 2,2-bis(4-hydroxyphenyl)propane, 2,2-bis(4-hydroxyphenyl)hexafluoropropane, 9,9-bis(4-hydroxyphenyl)fluorene, novolacs and resols. Examples of aliphatic and cycloaliphatic polyols are alkylenediols having preferably 2 to 12 carbon atoms, such as ethylene glycol, 1,2- or 1,3-propanediol, 1,2-, 1,3- or 1,4-butanediol, pentanediol, hexanediol, octanediol, dodecanediol, diethylene glycol, triethylene glcyol, polyethylene glycols having molecular weights of preferably from 200 to 1500, 1,3-cyclopentanediol, 1,2-, 1,3- or 1,4-cyclohexanediol, 1,4-dihydroxymethylcyclohexane, glycerol, triethanolamine, trimethylolethane, trimethylolpropane, pentaerythritol, pentaerythritol monooxalate, dipentaerythritol, ethers of pentaerythritol with ethylene glycol or propylene glycol, ethers of dipentaerythritol with ethylene glycol or propylene glycol, sorbitol, 2,2-bis[4-(2-hydroxyethoxy)phenyl]methane, 2,2-bis[4-(2-hydroxyethoxy)phenyl]propane and 9,9-bis[4-(2-hydroxyethoxy)phenyl]fluorene. Other suitable polyols are polymers and copolymers containing hydroxyl groups in the polymer chain or in side groups, examples being homopolymers or copolymers comprising vinyl alcohol or comprising hydroxyalkyl (meth)acrylates. Further polyols which are suitable are esters and urethanes having hydroxyl end groups.

The polyols may be partially or completely esterified with one unsaturated carboxylic acid or with different unsaturated carboxylic acids, and in partial esters the free hydroxyl groups may be modified, for example etherified or esterified with other carboxylic acids.

Examples of esters based on polyols are trimethylolpropane tri(meth)acrylate, trimethylolpropane tri(acryloyloxypropyl) ether, trimethylolethane tri(meth)acrylate, ethylene glycol di(meth)acrylate, diethylene glycol di(meth)acrylate, triethylene glycol di(meth)acrylate, tetraethylene glycol di(meth)acrylate, tetramethylene glycol di(meth)acrylate, neopentyl glycol di(meth)acrylate, pentaerythritol di(meth)acrylate, pentaerythritol tri(meth)acrylate, pentaerythritol tetra(meth)acrylate, pentaerythritol tri(meth)acrylate monooxalate, dipentaerythritol di(meth)acrylate, dipentaerythritol tri(meth)acrylate, dipentaerythritol tetra(meth)acrylate, dipentaerythritol penta(meth)acrylate, dipentaerythritol hexa(meth)acrylate, dipentaerythritol penta(meth)acrylate mono(2-hydroxyethyl) ether, tripentaerythritol octa(meth)acrylate, 1,3-butanediol di(meth)acrylate, 1,4-butanediol diitaconate, hexanediol di(meth)acrylate, 1,4-cyclohexanediol di(meth)acrylate, sorbitol tri(meth)acrylate, sorbitol tetra(meth)acrylate, sorbitol penta(meth)acrylate, sorbitol hexa(meth)acrylate, oligoester (meth)acrylates, glycerol di(meth)acrylate and tri(meth)acrylate, di(meth)acrylates of polyethylene glycol with a molecular weight of from 200 to 1500, pentaerythritol diitaconate, dipentaerythritol trisitaconate, dipentaerythritol pentaitaconate, dipentaerythritol hexaitaconate, ethylene glycol diitaconate, propylene glycol diitaconate, 1,3-butanediol diitaconate, 1,4-butanediol diitaconate, tetramethylene glycol diitaconate, sorbitol tetraitaconate, ethylene glycol dicrotonate, tetramethylene glycol dicrotonate, pentaerythritol dicrotonate, ethylene glycol dimaleate, tiethylene glycol dimaleate, pentaerythritol dimaleate, sorbitol tetramaleate, or mixtures thereof.

Other examples are pentaerythritol and dipentaerythritol derivatives shown in the following formula (XII) and (XIII): 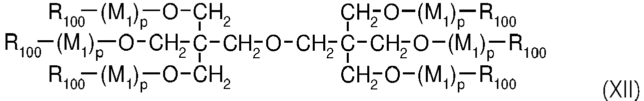 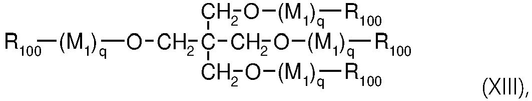 wherein
M₁ is -(CH₂CH₂O)- or-[CH₂CH(CH₃)O]-,
R₁₀₀ is -COCH=CH₂ or -COC(CH₃)=CH₂,
p is 0 to 6 (total of p: 3 - 24), and q is 0 to 6 (total of q: 2 - 16).

Examples of polyepoxides are those based on the abovementioned polyols and epichlorohydrin. Typical examples are bis(4-glycidyloxyphenyl)methane, 2,2-bis(4-glycidyloxyphenyl)propane, 2,2-bis(4-glycidyloxyphenyl)hexafluoropropane, 9,9-bis(4-glycidyloxyphenyl)fluorene, bis[4-(2-glycidyloxyethoxy)phenyl]methane, 2,2-bis[4-(2-glycidyloxyethoxy)phenyl]propane, 2,2-bis[4-(2-glycidyloxyethoxy)phenyl]hexafluoropropane, 9,9-bis[4-(2-glycidyloxyethoxy)phenyl]fluorene, bis[4-(2-glycidyloxypropoxy)phenyl]methane, 2,2-bis[4-(2-glycidyloxypropoxy)phenyl]propane, 2,2-bis[4-(2-glycidyloxypropoxy)phenyl]hexafluoropropane, 9,9-bis[4-(2-glycidyloxypropoxy)phenyl]fluorene, glycerol diglycidyl ether and glycidyl ethers of phenol and cresol novolacs.

Typical examples based on polyepoxides are 2,2-bis[4-{(2-hydroxy-3-acryloxy)propoxy}phenyl] propane, 2,2-bis[4-{(2-hydroxy-3-acryloxy)propoxyethoxy}phenyl] propane, 9,9-bis[4-{(2-hydroxy-3-acryloxy)propoxy}phenyl]fluorene, 9,9-bis[4-{(2-hydroxy-3-acryl-oxy)propoxyethoxy}phenyl]fluorene, glycerol 1,3-diglycerolate diacrylate and reaction products of epoxy resins based on novolacs with (meth)acrylic acid.

Preferred multifunctional (meth)acrylate monomers or oligomers include pentaerythritol tetraacrylate, dipentaerythritol pentaacrylate, dipentaerythritol hexaacrylate, ditrimethylolpropane tetraacrylate, pentaerythritol triacrylate, tris(2-hydroxy ethyl) isocyanurate triacrylate.

Specific examples are:
Dipentaerythritol-hexaacrylate (DPHA) 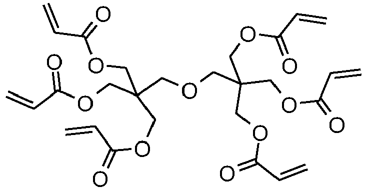
Dipentaerythritol-pentaacrylate (DPPA)

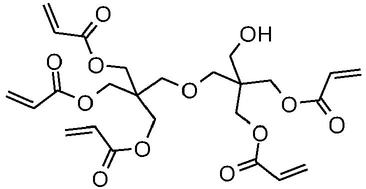

Examples of commercially available acrylate monomer or oligomers having two acryloyl or methacryloyl moieties are Aronix^{®}M-210, Aronix^{®}M-240, Aronix^{®}M-6200 (TOAGOSEI Co., LDT.) KAYARAD HDDA, KAYARAD HX-220, KAYARAD HX-620, KAYARAD R-526, KAYARAD UX-2201, KAYARAD MU-2100 (NIPPON KAYAKU Co., LTD.), VISCOAT-260, VISCOAT-355HP (OSAKA ORGANIC CHEMICAL INDUSTRY LTD.).

Examples of commercially available acrylate monomer or oligomers having three or more acryloyl or methacryloyl moieties are Aronix^{®}M-309, Aronix^{®}M-400, Aronix^{®}M-1310, Aronix^{®}M-1960, Aronix^{®}M-7100, Aronix^{®}M-8530, Aronix^{®}TO-1450 (TOAGOSEI Co., LDT.), KAYARAD TMPTA, KAYARAD DPHA, KAYARAD DPCA-20, KAYARAD MAX-3510 (NIPPON KAYAKU Co., LTD.), VISCOAT-295, VISCOAT-300, VISCOAT-GPT, VISCOAT-3PA, VISCOAT-400 (OSAKA ORGANIC CHEMICAL INDUSTRY LTD.).

Examples of commercially available urethane acrylate monomer or oligomers having two or more acryloyl or methacryloyl moieties are NEW FRONTIER R-1150 (DAI-ICHI KOGYO SEIYAKU CO., LTD.) KAYARAD DPHA-40H, KAYARAD UX-5000 (NIPPON KAYAKU Co., LTD.), UN-9000H (Negami Chemical Industrial Co., Ltd.)

The amount of acrylate present in the radiation curable composition ranges from about 2% to 80% and preferably from about 5% to 70% based on the whole solid contents of the composition, i.e. the amount of all components without the solvent(s).

It is of course possible to add other known photoinitiators (c) to the photocurable composition.

The use of the photoinitiator is not critical. The photoinitiator is for example selected from benzophenones, bis-imidazole, aromatic α-hydroxyketones, benzylketals, aromatic α-aminoketones, phenylglyoxalic acid esters, mono-acylphosphinoxides, bis-acylphosphinoxides, tris-acylphosphinoxides, oximesters derived from aromatic ketones and/or oxime esters of the carbazol type.

Examples of photoinitiators are camphor quinone; benzophenone, benzophenone derivatives, such as 2,4,6-trimethylbenzophenone, 2-methylbenzophenone, 3-methylbenzophenone, 4-methylbenzophenone, 2-methoxycarbonylbenzophenone, 4,4'-bis(chloromethyl)benzophenone, 4-chlorobenzophenone, 4-phenylbenzophenone, 3,3'-dimethyl-4-methoxy-benzophenone, [4-(4-methylphenylthio)phenyl]-phenylmethanone, methyl-2-benzoylbenzoate, 3-methyl-4'-phenylbenzophenone, 2,4,6-trimethyl-4'-phenylbenzophenone, 4,4'-bis(dimethylamino)benzophenone, 4,4'-bis(diethylamino)benzophenone, thioxanthones, thioxanthone derivatives, polymeric thioxanthones as for example OMNIPOL TX; ketal compounds, as for example benzildimethylketal (IRGACURE^{®} 651); acetophenone, acetophenone derivatives, for example α-hydroxycycloalkyl phenyl ketones or α-hydroxyalkyl phenyl ketones, such as for example 2-hydroxy-2-methyl-1-phenyl-propanone (DAROCURE^{®}1173), 1-hydroxycyclohexyl-phenyl-ketone (IRGACURE^{®}184), 1-(4-dodecylbenzoyl)-1-hydroxy-1-methyl-ethane, 1-(4-i-propylbenzoyl)-1-hydroxy-1-methyl-ethane, 1-[4-(2-hydroxyethoxy)-phenyl]-2-hydroxy-2-methyl-1-propan-1-one (IRGACURE^{®}2959); 2-hydroxy-1-{4-[4-(2-hydroxy-2-methyl-propionyl)-benzyl]-phenyl}-2-methyl-propan-1-one (IRGACURE^{®}127); 2-hydroxy-1-{4-[4-(2-hydroxy-2-methyl-propionyl)-phenoxy]-phenyl}-2-methyl-propan-1-one; dialkoxyacetophenones, α-hydroxy- or α-aminoacetophenones, e.g.(4-methylthiobenzoyl)-1-methyl-1-morpholinoethane (IRGACURE^{®} 907), (2-Benzyl-2-(dimethylamino)-1-[4-(4-morpholinyl)phenyl]-1-butanone) (IRGACURE^{®} 369), 2-(4-Methylbenzyl)-2-(dimethylamino)-1-[4-(4-morpholinyl)phenyl]-1-butanone (IRGACURE^{®} 379), (4-(2-hydroxyethyl)aminobenzoyl)-1-benzyl-1-dimethylaminopropane), (3,4-dimethoxybenzoyl)-1-benzyl-1-dimethylaminopropane; 1-[4-[4-(2-hydroxyethoxy)phenyl]sulfanylphenyl]-2-methyl-2-morpholino-propan-1-one, 4-aroyl-1,3-dioxolanes, benzoin alkyl ethers and benzyl ketals, e.g. dimethyl benzyl ketal, phenylglyoxalic esters and derivatives thereof, e.g. methyl α-oxo benzeneacetate, oxo-phenyl-acetic acid 2-(2-hydroxy-ethoxy)-ethyl ester, dimeric phenylglyoxalic esters, e.g. oxo-phenyl-acetic acid 1-methyl-2-[2-(2-oxo-2-phenyl-acetoxy)-propoxy]-ethyl ester (IRGACURE^{®} 754); ketosulfones, e.g. ESACURE KIP 1001 M; oximeesters, e.g. 1,2-octanedione 1-[4-(phenylthio)phenyl]-2-(O-benzoyloxime) (IRGACURE^{®} OXE01), ethanone 1-[9-ethyl-6-(2-methylbenzoyl)-9H-carbazol-3-yl]-1-(O-acetyloxime) (IRGACURE^{®} OXE02), Methanone,[8-[[(acetyloxy)imino][2-(2,2,3,3-tetrafluoropropoxy)phenyl]methyl]-11-(2-ethylhexyl)-11H-benzo[a]carbazol-5-yl](2,4,6-trimethylphenyl), [1-[4-[4-(benzofuran-2-carbonyl)phenyl]sulfanylphenyl]-4-methyl-pentylidene]amino] acetate, ethanone 1-[9-ethyl-6-(2-methyl-4-(2,2-dimethyl-1,3-dioxolanyl)methoxybenzoyl)-9H-carbazol-3-yl]-1-(O-acetyloxime), [3-cyclopentyl-1-[9-ethyl-6-(thiophene-2-carbonyl)carbazol-3-yl]propylidene]amino] acetate, *N*-Acetoxy-*N*-{3-[9-ethyl-6-(naphthalene-1-carbonyl)-9H-carbazol-3-yl]-1-methyl-3-acetoxyimino-propyl}-acetamide, 9H-thioxanthene-2-carboxaldehyde 9-oxo-2-(O-acetyloxime), [[1-(cyclohexylmethyl)-2-oxo-2-(4-phenylsulfanylphenyl)ethylidene]amino] cyclopropanecarboxylate), [[1-(cyclohexylmethyl)-2-oxo-2-(4-phenylsulfanylphenyl)ethylidene]amino] acetate,[1-(cyclohexylmethyl)-2-[9-ethyl-6-(thiophene-2-carbonyl)carbazol-3-yl]-2-oxo-ethylidene]amino] acetate, [1-(cyclohexylmethyl)-2-[9-ethyl-6-(furan-2-carbonyl)carbazol-3-yl]-2-oxo-ethylidene]amino] acetate, [1-(o-tolyl)-2-oxo-2-(4-phenylsulfanylphenyl)ethylidene]amino] acetate, 1-[1-(4-benzoylphenyl)indole-3-carbonyl]heptylideneamino] acetate, 1-[9-(4-benzoylphenyl)carbazol-3-yl]propylideneamino] acetate, [5-(4-isopropylphenyl)sulfanyl-1-oxo-indan-2-ylidene]amino] acetate, 1-(9,9-dibutyl-7-nitro-fluoren-2-yl)ethylideneamino] acetate, [2-(9,9-diethylfluoren-2-yl)-1-methyl-2-oxo-ethylidene]amino] acetate, [(7-nitro-9,9-dipropyl-fluoren-2-yl)-(o-tolyl)methylene]amino] acetate, [2-(9,9-dibutylfluoren-2-yl)-1-(o-tolyl)-2-oxo-ethylidene]amino] acetate, the oxime esters described in WO 07/062963, WO 07/071797, WO 07/071497WO 05/080337, JP2010-049238, WO2008078678, JP2010-15025 and JP2010-49238, peresters, e,g. benzophenone tetracarboxylic peresters as described for example in EP 126541, monoacyl phosphine oxides, e.g. (2,4,6-trimethylbenzoyl)diphenylphosphine oxide (DAROCURE^{®} TPO), ethyl (2,4,6 trimethylbenzoyl phenyl) phosphinic acid ester; bisacylphosphine oxides, e.g. bis(2,6-dimethoxy-benzoyl)-(2,4,4-trimethyl-pentyl)phosphine oxide, bis(2,4,6-trimethylbenzoyl)-phenylphosphine oxide (IRGACURE^{®} 819), bis(2,4,6-trimethylbenzoyl)-2,4-dipentoxyphenylphosphine oxide, trisacylphosphine oxides, halomethyltriazines, e.g. 2-[2-(4-methoxy-phenyl)-vinyl]-4,6-bistrichloromethyl-[1,3,5]triazine, 2-(4-methoxy-phenyl)-4,6-bis-trichloromethyl-[1,3,5]triazine, 2-(3,4-dimethoxy-phenyl)-4,6-bis-trichloromethyl-[1,3,5]triazine, 2-methyl-4,6-bis-trichloromethyl-[1,3,5]triazine, hexaarylbisimidazole / coinitiators systems, e.g. ortho-chlorohexaphenyl-bisimidazole combined with 2-mercaptobenzothiazole, ferrocenium compounds, or titanocenes, e.g. bis(cyclopentadienyl)-bis(2,6-difluoro-3-pyrryl-phenyl)titanium (IRGACURE^{®}784). Further, borate compounds can be used as coinitiators. As additional photoinitiators oligomeric compounds such as for example oligomeric alpha hydroxyl ketones, e.g. 2-hydroxy-1-{1-[4-(2-hydroxy-2-methyl-propionyl)-phenyl]-1,3,3-trimethyl-indan-5-yl}-2-methyl-propan-1-one, ESACURE KIP provided by Fratelli Lamberti, or oligomeric alpha amino ketones may be employed as well.

### Specific examples are:

### IRGACURE^{®}369

### (2-Benzyl-2-(dimethylamino)-1-[4-(4-morpholinyl)phenyl]-1-butanone)

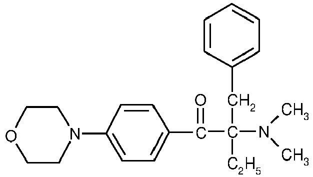

### IRGACURE^{®}379

### 2-(4-Methylbenzyl)-2-(dimethylamino)-1-[4-(4-morpholinyl)phenyl]-1-butanone

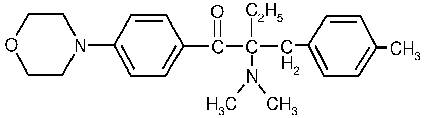

### IRGACURE^{®}OXE01

### 1,2-Octanedione 1-[4-(phenylthio)phenyl]-2-(O-benzoyloxime)

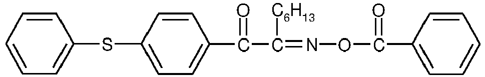

### IRGACURE^{®}OXE02

### Ethanone 1-[9-ethyl-6-(2-methylbenzoyl)-9H-carbazol-3-yl]-1-(O-acetyloxime)

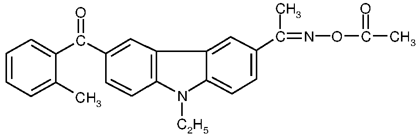

The composition according to the invention may comprise as component (d) a pigment, or a mixture of pigments, or a dye, or a mixture of dyes, or a mixture of one or more pigments with one or more dyes. The pigments which can be comprised in the composition according to the present invention, including a pigmented color filter resist composition, are preferably processed pigments.

The red pigment comprises, for example, an anthraquinone type pigment alone, a diketopyrolopyrole type pigment alone, a mixture of them or a mixture consisting of at least one of them and a disazo type yellow pigment or an isoindoline type yellow pigment, in particular C. I. Pigment Red 177 alone, C. I. Pigment Red 254 alone, a mixture of C. I. Pigment Red 177 and C. I. Pigment Red 254 or a mixture consisting of at least one member of C. I. Pigment Red 177, C. I. Pigment Red 242 and C. I. Pigment Red 254, and C. I. Pigment Yellow 83 or C. I. Pigment Yellow 139 ("*C.I*" refers to the Color Index, known to the person skilled in the art and publicly available). Further suitable examples for the pigment are C.I. Pigment Red 9, 97, 105, 122, 123, 144, 149, 168, 176, 179, 180, 185, 202, 206, 207, 209, 214, 222, 224, 244, 255, 264, 272 and C.I. Pigment Yellow 12, 13, 14, 17, 20, 24, 31, 53, 55, 83, 93, 95, 109, 110, 117, 128, 129, 138, 139, 150, 153, 154, 155, 166, 168, 180, 185, 199, 213 and C.I. Pigment Orange 38, 43, 64, 71, 72 and 73.

Examples of the dyes for red color are C.I. Solvent Red 25, 27, 30, 35, 36, 42, 43, 44, 45, 46, 47, 48, 49, 72, 73, 83, 89, 100, 109, 122, 138, 140, 141, 149, 150, 160, 179, 218, 230, 237, 246, C. I. Direct Red 20, 28, 37, 39, 44, 83 and C. I. Acid Red 6, 8, 9, 13, 14, 18, 26, 27, 37, 51, 52, 87, 88, 89, 92, 94, 97, 111, 114, 115, 134, 145, 151, 154, 180, 183, 184, 186, 198, 289, 388, C. I. Reactive Red 17, 120, C. I. Basic Red 1, 8, 12, 13, 18, C. I. Mordant Red 7, C. I. Disperse Red 5, 7, 13, 17, 58 and 60. The Red dyes can be used in combination with yellow and/or orange dyes.

The green pigment comprises for instance a halogenated phthalocyanine type pigment alone or its mixture with a bisazo type yellow pigment, an quinophthalone type yellow pigment or a metal complex, in particular C. I. Pigment Green 7 alone, C. I. Pigment Green 36 alone, C.I.Pigment 58 alone, C.I.Pigment Green 62 alone, C.I.Pigment Green 63 alone, or a mixture consisting of at least one member of C. I. Pigment Green 7, C. I. Pigment Green 36, C.I.Pigment Green 58, C.I.Pigment Green 59, C.I.Pigment Green 62, C.I.Pigment Green 63 and C. I. Pigment Yellow 83, C. I. Pigment Yellow 138, Pigment Yellow 139 or C. I. Pigment Yellow 150. Other suitable green pigments are C.I. Pigment Green 15, 25 and 37.

Examples for suitable green dyes are C. I. Acid Green 3, 9, 16, 25, C. I. Direct Green 28, 59, C. I. Basic Green 1 and 4.

Examples for suitable blue pigments are phthalocyanine type pigments, used either alone or in combination with an dioxazine type violet pigment, for instance, C. I. Pigment Blue 15:6 alone, a combination of C. I. Pigment Blue 15:6 and C. I. Pigment Violet 23. Further examples for blue pigments are such of C. I. Pigment Blue 15, 15:1, 15:2, 15:3, 15:4, 16, 22, 28, 60, 64 and 66. Other suitable pigments are C. I. Pigment Violet 1, 1:1, 2, 2:2, 3, 3:1, 5, 5:1, 14,15, 16, 19, 23, 25, 27, 29, 31, 32, 37, 39, 42, 44, 47, 49, 50, 177 and C. I. Orange 73.

The blue dye comprises, for example, a methane type dye, an anthraquinone type dye, an azo type dye, a metal complex azo type dye, a triaryl methane type dye or a phthalocyanine type dye.

Examples for suitable blue dyes are C. I. Solvent Blue 11, 25, 37, 45,49, 68, 78, 94, C. I. Direct Blue 25, 86, 90, 108, C.I. Acid Blue 1, 3, 7, 9, 15, 29, 83, 90, 103, 104, 158, 161, 249, C. I. Basic Blue 1, 3, 5, 7, 9, 24, 25, 26, 41, 105, C. I. Reactive Blue 19, 49, and C. I. Disperse Blue 56, 60, 165, 198, C. I. Vat Blue 4, 5, and C.I.Mordant Blue 1.

The pigment of the photopolymeric composition for black matrix preferably comprises at least one member selected from the group consisting of carbon black, titanium black, iron oxide, lactone, lactam and perylene. Preferred example is carbon black. However, a mixture of other pigments which, in total, give the black appearance, can also be used. For example, also C. I. Pigment Black 1, 6, 7, 12, 20, 31 and 32 can be used alone or in combination.

Examples for suitable black dyes are C. I. Mordant Black 7 and C. I. Reactive Black 5.

Other examples of the dyes used for color filter are C. I. Solvent Yellow 2, 5, 14, 15, 16, 19, 21, 33, 56, 62, 77, 83, 93, 162, 104, 105, 114, 129, 130, 162, C. I. Disperse Yellow 3, 4, 7, 31, 42, 54, 61, 64, 201, C. I. Reactive Yellow 2, C. I. Mordant Yellow 5, C. I. Direct Yellow 1, 11, 12, 28, C. I. Acid Yellow 1, 3, 11, 17, 23, 38, 40, 42, 76, 98, C. I. Basic Yellow 1, C. I. Solvent Violet 2, 10, 13, 33, 45, 46, C. I. Disperse Violet 22, 24, 26, 28, 31, C. I. Acid Violet 9, 49, C. I. Basic Violet 1, 2, 3, 7, 10, 14, C. I. Solvent Orange 1, 2, 5, 6, 37, 45, 62, 99, C. I. Acid Orange 1, 3, 7, 8, 10, 20, 24, 28, 33, 56, 74, C.I. Direct Orange 1, 26, C. I. Disperse Orange 5, C. I. Direct Brown 6, 58, 95, 101, 173, C. I. Acid Brown 14, C.I. Solvent Black 3, 5, 7, 27, 28, 29, 35, 45 and 46.

In some special cases of manufacturing color filters, complementary colors, yellow, magenta, cyan and optionally green, are used instead of red, green and blue. As yellow for this type of color filters, the abovementioned yellow pigments and dyes can be employed. Examples of the colorants suitable for magenta color are C. I. Pigment Red 122, 144, 146, 169, 177, C. I. Pigment Violet 19 and 23. Examples of cyan color are aluminum phthalocyanine pigments, titanium phthalocyanine pigments, cobalt phthalocyanine pigments, and tin phthalocyanine pigments.

The pigments in the color filter resist composition have preferably a mean particle diameter smaller than the wavelength of visible light (400 nm to 700 nm). Particularly preferred is a mean pigment diameter of < 100 nm.

The concentration of the pigment in the total solid component (pigments of various colors and resin) is for example in the range of 5% to 80% by weight, in particular in the range of 20% to 65 % by weight.

The concentration of the dye in the total solid component (dyes of various colors and resin) is for example in the range of 0.5% to 95% by weight, in particular in the range of 0.5% to 70% by weight.

If necessary, the pigments may be stabilized in the photosensitive composition by pretreatment of the pigments with a dispersant to improve the dispersion stability of the pigment in the liquid formulation. Suitable additives are described below.

Additives (d) are optionally present in the composition of the invention, such as dispersing agents, surfactant, adhesion promoters, photosensitizer and the like.

It is preferred to apply a surface treatment to the pigments in order to make the pigment easy to disperse and to stabilize the resultant pigment dispersion. The surface treatment reagents are, for example, surfactants, polymeric dispersants, general texture improving agents, pigment derivatives and mixtures thereof. It is especially preferred when the colorant composition according to the invention comprises at least one polymeric dispersant and/or at least pigment derivative.

Suitable surfactants include anionic surfactants such as alkylbenzene- or alkylnaphthalene-sulfonates, alkylsulfosuccinates or naphthalene formaldehyde sulfonates; cationic surfactants including, for example, quaternary salts such as benzyl tributyl ammonium chloride; or nonionic or amphoteric surfactants such as polyoxyethylene surfactants and alkyl- or amidopropyl betaines, respectively.

Illustrative examples of the surfactant include polyoxyethylene alkyl ethers such as polyoxyethylene lauryl ether, polyoxyethylene stearyl ether and polyoxyethylene oleyl ether; polyoxyethylene alkylphenyl ethers such as polyoxyethylene octylphenyl ether and polyoxyethylene nonylphenyl ether; polyethylene glycol diesters such as polyethylene glycol dilaurate and polyethylene glycol distearate; sorbitan fatty acid esters; fatty acid modified polyesters; tertiary amine modified polyurethanes; polyethyleneimines; those available under the trade names of KP (a product of Shin-Etsu Chemical Co., Ltd), Polyflow (a product of KYOEISHA CHEMICAL Co., Ltd), F-Top (a product of Tochem Products Co., Ltd), MEGAFAC (a product of Dainippon Ink & Chemicals, Inc.), Fluorad (a product of Sumitomo 3M Ltd), Asahi Guard and Surflon (products of Asahi Glass Co., Ltd); and the like.

These surfactants may be used alone or in admixture of two or more.

The surfactant is generally used in an amount of 50 parts or less by weight, preferably 0 to 30 parts by weight, based on 100 parts by weight of the colorant composition.

Polymeric dispersants include high molecular weight polymers with pigment affinic groups. Examples are: statistical co-polymers comprised from, for instance, styrene derivatives, (meth)acrylates and (meth)acrylamides, and such statistical co-polymers modified by post modification; block co-polymers and/or comb polymers comprised from, for instance, styrene derivatives, (meth)acrylates and (meth)acrylamides, and such block co-polymers and/or comb polymers modified by post modification; polyethylenimines, which for instance is crafted with polyesters; polyamines, which for instance is crafted with polyesters; and many kinds of (modified) polyurethanes.

Polymeric dispersants may also be employed. Suitable polymeric dispersants are, for example, BYK's DISPERBYK^{®} 101, 115, 130, 140, 160, 161, 162, 163, 164, 166, 168, 169, 170, 171, 180, 182, 2000, 2001, 2009, 2020, 2025, 2050, 2090, 2091, 2095, 2096, 2150, Ciba's Ciba^{®} EFKA@ 4008, 4009, 4010, 4015, 4046, 4047, 4050, 4055, 4060, 4080, 4300, 4310, 4330, 4340, 4400, 4401, 4402, 4403, 4406, 4500, 4510, 4520, 4530, 4540, 4550, 4560, Ajinomoto Fine Techno's PB^{®}711, 821, 822, 823, 824, 827, Lubrizol's SOLSPERSE@ 1320, 13940, 17000, 20000, 21000, 24000, 26000, 27000, 28000, 31845, 32500, 32550, 32600, 33500, 34750, 36000, 36600, 37500, 39000, 41090, 44000 ,53095 and combinations thereof.

It is preferred to use Ciba^{®} EFKA@ 4046, 4047, 4060, 4300, 4310, 4330, 4340, DISPERBYK^{®} 161, 162, 163, 164, 165, 166, 168, 169, 170, 2000, 2001, 2020, 2050, 2090, 2091, 2095, 2096, 2105, 2150, PB^{®}711, 821, 822, 823, 824, 827, SOLSPERSE@ 24000, 31845, 32500, 32550, 32600, 33500, 34750, 36000, 36600, 37500, 39000, 41090, 44000, 53095 and combinations thereof as dispersant.

Suitable texture improving agents are, for example, fatty acids such as stearic acid or behenic acid, and fatty amines such as laurylamine and stearylamine. In addition, fatty alcohols or ethoxylated fatty alcohols, polyols such as aliphatic 1,2-diols or epoxidized soy bean oil, waxes, resin acids and resin acid salts may be used for this purpose.

Suitable pigment derivatives are, for example, copper phthalocyanine derivatives such as Ciba's Ciba^{®} EFKA@ 6745, Lubrizol's SOLSPERSE@ 5000, 12000, BYK's SYNERGIST 2100 and azo derivatives such as Ciba^{®} EFKA@ 6750, SOLSPERSE@ 22000 and SYNERGIST 2105.

The above mentioned dispersants and surfactants for pigments are for example employed in compositions of the present invention which are used as resist formulations, in particular in color filter formulations.

Subject of the invention also is a photopolymerizable composition as described above as further additive comprising a dispersant or a mixture of dispersants as well as a photopolymerizable composition as described above as further additive comprising a pigment or a mixture of pigments.

In the invention, the content of the dispersing agent is preferably from 1 to 80% by mass, more preferably from 5 to 70% by mass, even more preferably from 10 to 60% by mass, based on the mass of the pigment.

Further suitable additives (d) are for example adhesion improving agents. The curable composition of the invention may contain an adhesion improving agent for increasing adhesion to a hard surface, such as of a support. The adhesion improving agent may be a silane coupling agent, a titanium coupling agent or the like.

Photopolymerization can also be accelerated by adding as component (d) further photosensitizers or coinitiators which shift or broaden the spectral sensitivity. These are, in particular, aromatic compounds, for example benzophenone and derivatives thereof, thioxanthone and derivatives thereof, anthraquinone and derivatives thereof, coumarin and phenothiazine and derivatives thereof, and also 3-(aroylmethylene)thiazolines, rhodanine, camphorquinone, but also eosine, rhodamine, erythrosine, xanthene, thioxanthene, acridine, e.g. 9-phenylacridine, 1,7-bis(9-acridinyl)heptane, 1,5-bis(9-acridinyl)pentane, cyanine and merocyanine dyes.

### Specific examples of such compounds are

1.Thioxanthones
   Thioxanthone, 2-isopropylthioxanthone, 2-chlorothioxanthone, 1-chloro-4-propoxythioxanthone, 2-dodecylthioxanthone, 2,4-diethylthioxanthone, 2,4-dimethylthioxanthone, 1-methoxycarbonylthioxanthone, 2-ethoxycarbonylthioxanthone, 3-(2-methoxyethoxycarbonyl)-thioxanthone, 4-butoxycarbonylthioxanthone, 3-butoxycarbonyl-7-methylthioxanthone, 1-cyano-3chlorothioxanthone, 1-ethoxycarbonyl-3-chlorothioxanthone, 1-ethoxycarbonyl-3-ethoxythioxanthone, 1-ethoxycarbonyl-3-aminothioxanthone, 1-ethoxycarbonyl-3-phenylsulfurylthioxanthone, 3,4-di-[2-(2-methoxyethoxy)ethoxycarbonyl]-thioxanthone, 1,3-dimethyl-2-hydroxy-9Hthioxanthen-9-one 2-ethylhexylether, 1-ethoxycarbonyl-3-(1-methyl-1-morpholinoethyl)-thioxanthone, 2-methyl-6-dimethoxymethyl-thioxanthone, 2-methyl-6-(1,1-dimethoxybenzyl)-thioxanthone, 2-morpholinomethylthioxanthone, 2-methyl-6-morpholinomethylthioxanthone, N-allylthioxanthone-3,4-dicarboximide, N-octylthioxanthone-3,4-dicarboximide, N-(1,1,3,3-tetramethylbutyl)-thioxanthone-3,4-dicarboximide, 1-phenoxythioxanthone, 6-ethoxycarbonyl-2-methoxythioxanthone, 6-ethoxycarbonyl-2-methylthioxanthone, thioxanthone-2-carboxylic acid polyethyleneglycol ester, 2-hydroxy-3-(3,4-dimethyl-9-oxo-9H-thioxanthon-2-yloxy)N,N,N-trimethyl-1-propanaminium chloride;
2. Benzophenones
   benzophenone, 4-phenyl benzophenone, 4-methoxy benzophenone, 4,4'-dimethoxy benzophenone, 4,4'-dimethyl benzophenone, 4,4'-dichlorobenzophenone 4,4'-bis(dimethylamino)benzophenone, 4,4'-bis(diethylamino)benzophenone, 4,4'-bis(methylethylamino)benzophenone, 4,4'-bis(*p*-isopropylphenoxy)benzophenone, 4-methyl benzophenone, 2,4,6-trimethylbenzophenone, 4-(4-methylthiophenyl)-benzophenone, 3,3'-dimethyl-4-methoxy benzophenone, methyl-2-benzoylbenzoate, 4-(2-hydroxyethylthio)-benzophenone, 4-(4-tolylthio)benzophenone, 1-[4-(4-benzoyl-phenylsulfanyl)-phenyl]-2-methyl-2-(toluene-4-sulfonyl)-propan-1-one, 4-benzoyl-N,N,N-trimethylbenzenemethanaminium chloride, 2-hydroxy-3-(4-benzoylphenoxy)-N,N,N-trimethyl-1-propanaminium chloride monohydrate, 4-(13-acryloyl1,4,7,10,13-pentaoxatridecyl)-benzophenone, 4-benzoyl-N,N-dimethyl-N-[2-(1-oxo-2-propenyl)oxy]ethyl-benzenemethanaminium chloride;
3. Coumarins
   Coumarin 1, Coumarin 2, Coumarin 6, Coumarin 7, Coumarin 30, Coumarin 102, Coumarin 106, Coumarin 138, Coumarin 152, Coumarin 153, Coumarin 307, Coumarin 314, Coumarin 314T, Coumarin 334, Coumarin 337, Coumarin 500, 3-benzoyl coumarin, 3-benzoyl-7-methoxycoumarin, 3-benzoyl-5,7-dimethoxycoumarin, 3-benzoyl-5,7-dipropoxycoumarin, 3-benzoyl-6,8-dichlorocoumarin, 3-benzoyl-6-chloro-coumarin, 3,3'-carbonyl-bis[5,7-di(propoxy)coumarin], 3,3'-carbonyl-bis(7-methoxycoumarin), 3,3'-carbonyl-bis(7-diethylamino-coumarin), 3-isobutyloylcoumarin, 3-benzoyl-5,7-dimethoxy-coumarin, 3-benzoyl-5,7-diethoxy-coumarin, 3-benzoyl-5,7-dibutoxycoumarin, 3-benzoyl-5,7-di(methoxyethoxy)-coumarin, 3-benzoyl-5,7-di(allyloxy)coumarin, 3-benzoyl-7-dimethylaminocoumarin, 3-benzoyl-7-diethylaminocoumarin, 3-isobutyloyl-7-dimethylaminocoumarin, 5,7-dimethoxy-3-(1-naphthoyl)-coumarin, 5,7-diethoxy-3-(1-naphthoyl)-coumarin, 3-benzoylbenzo[f]coumarin, 7-diethylamino-3-thienoylcoumarin, 3-(4-cyanobenzoyl)-5,7-dimethoxycoumarin, 3-(4-cyanobenzoyl)-5,7-dipropoxycoumarin, 7-dimethylamino-3-phenylcoumarin, 7-diethylamino-3-phenylcoumarin, the coumarin derivatives disclosed in JP09-179299-A and JP09-325209-A, for example 7-[{4-chloro-6-(diethylamino)-S-triazine-2-yl}amino]-3-phenylcoumarin;
4. 3-(aroylmethylene)-thiazolines
   3-methyl-2-benzoylmethylene-β-naphthothiazoline, 3-methyl-2-benzoylmethylene-benzothiazoline, 3-ethyl-2-propionylmethylene-β-naphthothiazoline;
5. Rhodanines
   4-dimethylaminobenzalrhodanine, 4-diethylaminobenzalrhodanine, 3-ethyl-5-(3-octyl-2-benzothiazolinylidene)-rhodanine, the rhodanine derivatives, formulae [1], [2], [7], disclosed in JP08-305019A;
6. Other compounds
   Acetophenone, 3-methoxyacetophenone, 4-phenylacetophenone, benzil, 4,4'-bis(dimethylamino)benzil, 2-acetylnaphthalene, 2-naphthaldehyde, dansyl acid derivatives, 9,10-anthraquinone, anthracene, pyrene, aminopyrene, perylene, phenanthrene, phenanthrenequinone, 9-fluorenone, dibenzosuberone, curcumin, xanthone, thiomichler's ketone, α-(4-dimethylaminobenzylidene) ketones, e.g. 2,5-bis(4-diethylaminobenzylidene)cyclopentanone, 2-(4-dimethylamino-benzylidene)-indan-1-one, 3-(4-dimethylamino-phenyl)-1-indan-5-yl-propenone, 3-phenylthiophthalimide, N-methyl-3,5-di(ethylthio)-phthalimide, N-methyl-3,5-di(ethylthio)phthalimide, phenothiazine, methylphenothiazine, amines, e.g. N-phenylglycine, ethyl 4-dimethylaminobenzoate, butoxyethyl 4-dimethylaminobenzoate, 4-dimethylaminoacetophenone, triethanolamine, methyldiethanolamine, dimethylaminoethanol, 2-(dimethylamino)ethyl benzoate, poly(propylenegylcol)-4-(dimethylamino) benzoate.

A photosensitizer may be selected from the group consisting of benzophenone and its derivatives, thioxanthone and its derivatives, anthraquinone and its derivatives, or coumarin and its derivatives.

To accelerate the photopolymerization, it is possible to add amines, for example triethanolamine, N-methyldiethanolamine, ethyl-p-dimethylaminobenzoate, 2-(dimethylamino)ethyl benzoate, 2-ethylhexyl-p-dimethylaminobenzoate, octyl-para-N,N-dimethylaminobenzoate, N-(2-hydroxyethyl)-N-methyl-para-toluidine or Michler's ketone. The action of the amines can be intensified by the addition of aromatic ketones of the benzophenone type. Examples of amines which can be used as oxygen scavengers are substituted N,N-dialkylanilines, as are described in EP339841. Other accelerators, coinitiators and autoxidizers are thiols, thioethers, disulfides, phosphonium salts, phosphine oxides or phosphines, as described, for example, in EP438123, in GB2180358 and in JP KokaiHei 6-68309.

To accelerate the polymerization thermally, it is possible to add thermal curing promoter, e.g., oxime sulfonates, as are described, for example, in WO2012/101245, hydroxylamine esters, as are described, for example, in WO2012/108835, in WO2001090113, in WO03029332 and in WO04081100, peroxides, such as organic peroxides or hydroperoxides, as are described, for example, in JP2003015288 and in JP10010718, and azo compounds, are described, for example, in JP2003015288.

The choice of additive(s) is made depending on the field of application and on properties required for this field. The additives described above are customary in the art and accordingly are added in amounts which are usual in the respective application.

Thermal inhibitors as optional further additives (d) are intended to prevent premature polymerization, examples being hydroquinone, hydroquinone derivatives, p-methoxyphenol, β-naphthol or sterically hindered phenols, such as 2,6-di-t-butyl-p-cresol. In order to increase the stability on storage in the dark it is possible, for example, to use copper compounds, such as copper naphthenate, stearate or octoate, phosphorus compounds, for example triphenylphosphine, tributylphosphine, triethylphosphine, triphenyl phosphate or tribenzyl phosphate, quaternary ammonium compounds, for example tetramethylammonium chloride or trimethylbenzylammonium chloride, or hydroxylamine derivatives, for example N-diethylhydroxylamine. To exclude atmospheric oxygen during the polymerization it is possible to add paraffin or similar wax-like substances which, being of inadequate solubility in the polymer, migrate to the surface in the beginning of polymerization and form a transparent surface layer which prevents the ingress of air. It is also possible to apply an oxygen-impermeable layer. Light stabilizers which can be added in a small quantity are UV absorbers, for example those of the hydroxyphenylbenzotriazole, hydroxyphenyl-benzophenone, oxalamide or hydroxyphenyl-s-triazine type. These compounds can be used individually or in mixtures, with or without sterically hindered amines (HALS).

Thermal inhibitors can be used to prevent deterioration of color properties like transparency of the compositions, preferably phenol derivatives or sterically hindered phenols as are described, for example, in US4994628, in JP6128195, JP7206771, and in WO0198249.

Further, the latent thermal inhibitors are also used as antioxidant to prevent premature polymerization or discoloration. The latent thermal inhibitors are a compound having a protective group capable of being desorbed by heating, which is a compound that develops an antioxidant function by desorption of the protecting group. As preferable latent thermal inhibitors, the compounds which synthesized with a combination of phenol derivatives and acid anhydride, Boc reagent such as di-t-butyl dicarbonate, acid chloride, alkyl halide derivatives, allyl ether derivatives or cyclochloride derivatives are known, for example, in WO14021023, in WO17043353, in WO2016056290, in JP2017008219, in JP2017066370, in JP201513937 in WO2018062105.

The compositions according to the invention may also comprise one or more solvents. Examples of suitable solvents are ketones, ethers and esters, such as methyl ethyl ketone, isobutyl methyl ketone, cyclopentanone, cyclohexanone, N-methylpyrrolidone, dioxane, tetrahydrofuran, ethylene glycol monomethyl ether, ethylene glycol monoethyl ether, ethylene glycol monopropyl ether, ethylene glycol monobutyl ether, ethylene glycol dimethyl ether, ethylene glycol diethyl ether, ethylene glycol dipropyl ether, propylene glycol monomethyl ether, propylene glycol monoethyl ether, propylene glycol monopropyl ether, propylene glycol monobutyl ether, propylene glycol dimethyl ether, propylene glycol diethyl ether, propylene glycol dipropyl ether, diethylene glycol monomethyl ether, diethylene glycol monoethyl ether, diethylene glycol dimethyl ether, diethylene glycol diethyl ether, ethylene glycol monomethyl ether acetate, ethylene glycol monoethyl ether acetate, ethylene glycol monopropyl ether acetate, ethylene glycol monobutyl ether acetate, propylene glycol monomethyl ether acetate, propylene glycol monoethyl ether acetate, propylene glycol monopropyl ether acetate, 2-methoxybutyl acetate, 3-methoxybutyl acetate, 4-methoxybutyl acetate, 2-methyl-3-methoxybutyl acetate, 3-methyl-3-methoxybutyl acetate, 3-ethyl-3-methoxybutyl acetate, 2-ethoxybutyl acetate, 4-ethoxybutyl acetate, 4-propoxybutyl acetate, 2-methoxypentyl acetate, 3-methoxypentyl acetate, 4-methoxypentyl acetate, 2-methyl-3-methoxypentyl acetate, 3-methyl-3-methoxypentyl acetate, 3-methyl-4-methoxypentyl acetate, 4-methyl-4-methoxypentyl acetate, ethyl acetate, n-butyl acetate, ethyl propionate, propyl propionate, butyl propionate, ethyl 3-ethoxypropionate, methyl 3-methoxypropionate, 2-heptanone, 2-pentanone, and ethyl lactate.

The compositions according to this invention can comprise additionally a compound which generates radical thermally, examples being organic peroxide, azo derivatives, benzoin derivatives, benzoin ether derivatives, acetophenone derivatives, hydroxylamine esters, oxime derivatives and hydrogen peroxides.

Examples of commercially available peroxide are dilauroyl, 1,1,3,3-tetramethylbutyl peroxy-2-ethylhexanoate, t-butyl peroxy-2-ethylhexanoate, 1,1-di(t-butylperoxy)-2-methylcyclohexane, 1,1-di(t-hexylperoxy)-3,3,5-trimethylcyclohexane, t-butylperoxymaleic acid, t-butyl peroxylaurate, t-butyl peroxy 2-ethylhexyl monocarbonate, t-hexyl peroxybenzoate, t-butyl peroxyacetate, t-butyl peroxybenzoate, Dicymyl peroxide, di-t-butyl peroxide, 2,5-dimethyl-2,5-di(t-butylperoxy)hexyne-3, 1,1,3,3-tetramethylbutyl hydroperoxide, cumene hydroperoxide, 2,3-dimethyl-2,3-diphenylbutane, (NOF Co., LTD.), Kayamek A, Kayamek M, Kayamek R, Kayamek L, Kayamec LH,(Kayaku Akzo Co., LTD.) for example described in JP2013-014675.

Examples of commercially available azo derivatives are 2,2'-azobis(isobutyronitrile), 2,2'-azobis(2-methylbutyronitrile), 1,1'-azobis(cyclohexane-1-1-carbonitrile), (2,4-dimethylvaleronitrile), 1-[(1-cyano-1-methylethyl)azo]formamide, 2,2'-azobis(2-amidinopropane) dihydrochloride, 2,2'-azobis[N-(2-propenyl)-2-methylpropionamide], 2,2'-azobis(N-butyl-2-methylpropionamide), 2,2'-azobis[2-(2-imidazolin-2-yl)propane], 2,2'-azobis[2-methyl-N-(2-hydroxyethyl)propionamide], Dimethyl 2,2'-azobis(isobutyrate), (Wako pure Chemical Industries., LTD.)

Other thermal radical initiators are hydroxylamine derivatives, as described, for example, oxime derivatives, in WO10/108835 and oxime derivatives such as oxime sulfonate, as described, for example, in WO12/101245 and WO16/030790.

The compositions according to this invention can comprise additionally a crosslinking agent which is activated by an acid or a base, for example as described in JP 10 221843-A, and a compound which generates acid or base thermally or by actinic radiation and which activates a crosslinking reaction. Use is made, in addition to the free-radical hardeners, of cationic photo or thermal initiators such as sulfonium-, phosphonium- or iodonium salts, for example IRGACURE^{®}250, San-Aid SI series, SI-60L, SI-80L, SI-100L, SI-110L, SI-145, SI-150, SI-160, SI-180L produced by Sanshin Chemical, cyclopentadienyl-arene-iron(II) complex salts, for example (η⁶-iso-propylbenzene)(η⁵-cyclopentadienyl)iron(II) hexafluorophosphate, as well as oxime sulfonic acid esters, for example described in EP 780729. Also pyridinium and i-quinolinium salts as described e.g. in EP 497531 and EP 441232 may be used in combination with the new photoinitiators. Examples of bases are imidazole and its derivatives for example Curezole OR series and CN series provided by Shikoku Chemicals.

The crosslinking agents which can be activated by acid or base include compounds having epoxy or oxetane groups. There may be used a solid or liquid known epoxy or oxetane compound and said compound is used depending on required characteristics. A preferred epoxy resin is a bisphenol S type epoxy resin such as BPS-200 produced by Nippon Kayaku Co., Ltd., EPX-30 produced by ACR Co., Epiculon EXA-1514 produced by Dainippon Ink & Chemicals Inc., etc.; a bisphenol A type epoxy resin such as Epiculon N-3050, N-7050, N-9050 produced by Dainippon Ink & Chemicals Inc., XAC-5005, GT-7004, 6484T, 6099 ; a bisphenol F type epoxy resin such as YDF-2004, YDF2007 produced by NSCC Epoxy Manufacturing Co., Ltd., etc. ; a bisphenol fluorene type epoxy resin such as OGSOL PG, PG-100, EG, EG-210 produced by Osaka Gas Chemicals; a diglycidyl phthalate resin such as Blemmer DGT produced by Nippon Oil and Fats Co., Ltd., etc.; a heterocyclic epoxy resin such as TEPIC produced by Nissan Chemical Industries, Ltd., Araldite PT810 produced by Ciba Specialty Chemicals Inc., etc.; a bixylenol type epoxy resin such as YX-4000 produced by Yuka Shell Co., etc.; a biphenol type epoxy resin such as YL-6056 produced by Yuka Shell Co., etc.; a tetraglycidyl xylenoylethane resin such as ZX-1063 produced by NSCC Epoxy Manufacturing Co., Ltd., etc.; a novolak type epoxy resin such as EPPN-201, EOCN-103, EOCN-1020, EOCN-1025 and BRRN produced by Nippon Kayaku Co., Ltd., ECN-278, ECN-292 and ECN-299 produced by Asahi Chemical Industry Co., Ltd., GY-1180, ECN-1273 and ECN-1299 produced by BASF Japan Ltd., YDCN-220L, YDCN-220HH, YDCN-702, YDCN-704, YDPN-601 and YDPN-602 produced by NSCC Epoxy Manufacturing Co., Ltd., Epiculon-673, N-680, N-695, N-770 and N-775 produced by Dainippon Ink & Chemicals Inc., etc.; a novolak type epoxy resin of bisphenol A such as EPX-8001, EPX-8002, EPPX-8060 and EPPX-8061 produced by Asahi Chemical Industry Co., Ltd., Epiculon N-880 produced by Dainippon Ink & Chemicals Inc., etc.; a chelate type epoxy resin such as EPX-49-69 and EPX-49-30 produced by Asahi Denka Kogyo K.K., etc.; a glyoxal type epoxy resin such as YDG-414 produced by NSCC Epoxy Manufacturing Co., Ltd., etc.; an amino group-containing epoxy resin such as YH-1402 and ST-110 produced by NSCC Epoxy Manufacturing Co., Ltd.., YL-931 and YL-933 produced by Yuka Shell Co., etc.; a rubber-modified epoxy resin such as Epiculon TSR-601 produced by Dainippon Ink & Chemicals Inc., EPX-84-2 and EPX-4061 produced by Asahi Denka Kogyo K.K., etc.; a dicyclopentadiene phenolic type epoxy resin such as DCE-400 produced by Sanyo-Kokusaku Pulp Co., Ltd., etc.; a silicone-modified epoxy resin such as X-1359 produced by Asahi Denka Kogyo K.K., etc.; an e-caprolactone-modified epoxy resin such as Plaque G-402 and G-710 produced by Daicel Co., etc. and others. Further, partially esterified compounds of these epoxy compounds (e.g. esterified by (meth)acrylates) can be used in combination. Examples of oxetane compounds are 3-ethyl-3-hydroxymethyloxetane (oxetane alcohol), 2-ethylhexyloxetane, xylene bisoxetane, 3-ethyl-3[[(3-ethyloxetane-3-yl)methoxy]methyl]oxetane (Aron Oxetane series) provided by Toagosei Co., Ltd.

The photopolymerizable composition according to the present invention can be used for various purposes. Specific and intended applications and uses include but are not limted to
- Resists to manufacture color filters for a variety of display applications (e.g. LCD, OLED),
- Spacers for LCD,
- Overcoat layer for color filter or LCD,
- Bank/pixel definition layer of OLED,
- Sealant for LCD and OLED,
- Insulation/passivation layer for LCD and OLED,
- Insulation for metal wiring/transparent conductive film for touch panel,
- Coating for touch panel such as anti-fingerprint, hard coat and optical coat,
- Decorative ink for touch panel,
- Protective film for touch panel,
- Etching resists for touch panel.

Other potential applications or uses may e.g. include but are not limited to
- Optical films for a variety of display applications such as hard coat, anti-reflective film, anti-glare film, retardation film, NIR absorbing film, prism sheet, brightness enhancement film and the like,
- Other resists, photosensitive compositions or thermosetting compositions to generate structures or layers in the manufacturing processes of plasma-display panels, electroluminescence displays and LCD,
- Solder resists,
- Photoresist material used for forming dielectric layers in a sequential build-up layer of a printed circuit board,
- Photoresists for electronics, electroplating resists, etch resists, both liquid and dry films,
- Anisotropy conducting adhesive to connect with liquid crystal display and tape carrier package (TCP) or with TCP and printed board on electronic connection of fine circuit,
- Polymerization for oligomers, co-oligomers, polymers and copolymers, for example, random block, multi-block, star or gradient copolymers,
- For the controlled degradation of polymer and for the controlled build-up the molecular weight or crosslinking,
- Coating agent for building, building materials, automobile parts, electrical instrument, and precision instrument,
- Pressure-sensitive adhesive optical film including an optical film and a pressure-sensitive adhesive layer for LCD and an organic electroluminescence (EL) display,
- Adhesive and printed circuit board having adhesive layer as automobile parts and electrical instrument and the like,
- Dental materials,
- Sealer for building and building materials.

For example, the photopolymerizable compositions can be used as printing ink, e.g. screen printing inks, inks for offset- or flexo printing, as a clear finish, as a white or colored finish, for example for wood or metal, as powder coating, as a coating material, inter alia for paper, wood, metal or plastic, as a daylight-curable coating for the marking of buildings and roadmarking, for photographic reproduction techniques, for holographic recording materials, for image recording techniques or to produce printing plates which can be developed with organic solvents or with aqueous alkalis, for producing masks for screen printing, as dental filling compositions, as adhesives, as pressure-sensitive adhesives, as laminating resins, as etch resists, solder resists, electroplating resists, or permanent resists, both liquid and dry films, as photostructurable dielectric, for printed circuit boards and electronic circuits, as resists to manufacture color filters for a variety of display applications or to generate structures in the manufacturing process of plasma-display panels and electroluminescence displays, (as for example described in US5853446, EP863534, JP 09-244230-A, JP10-62980-A, JP08-171863-A, US5840465, EP855731, JP05-271576-A, JP 05-67405-A) for the production of holographic data storage (HDS) material, for the production of optical switches, optical lattices (interference lattice), light circuits, for producing three-dimensional articles by mass curing (UV curing in transparent moulds) or by the stereo-lithography technique, as is described, for example, in US4575330, to produce composite materials (for example styrenic polyesters, which may, if desired, contain glass fibres and/or other fibres and other auxiliaries) and other thick-layered compositions, for coating or sealing electronic components and integrated circuits, or as coatings for optical fibres, or for producing optical lenses, e.g. contact lenses or Fresnel lenses. The compositions according to the invention are further suitable for the production of medical equipment, auxiliaries or implants. Further, the compositions according to the invention are suitable for the preparation of gels with thermotropic properties, as for example described in DE19700064 and EP678534.

The novel photoinitiators may additionally be employed as initiators for emulsion polymerizations, pearl polymerizations or suspension polymerizations, as polymerization initiators for fixing ordered states of liquid-crystalline monomers and oligomers, or as initiators for fixing dyes on organic materials.

In coating materials, use is frequently made of mixtures of a prepolymer with polyunsaturated monomers, which may additionally include a monounsaturated monomer as well. It is the prepolymer here which primarily dictates the properties of the coating film, and by varying it the skilled worker is able to influence the properties of the cured film. The polyunsaturated monomer functions as a crosslinking agent which renders the film insoluble. The monounsaturated monomer functions as a reactive diluent, which is used to reduce the viscosity without the need to employ a solvent.

Unsaturated polyester resins are usually used in two-component systems together with a monounsaturated monomer, preferably with styrene. For photoresists, specific one-component systems are often used, for example polymaleimides, polychalcones or polyimides, as described in DE 2308830.

The novel photoinitiators can also be used for the polymerization of radiation-curable powder coatings. The powder coatings can be based on solid resins and monomers containing reactive double bonds, for example maleates, vinyl ethers, acrylates, acrylamides and mixtures thereof.

The novel photocurable compositions are suitable, for example, as coating materials for substrates of all kinds, for example wood, textiles, paper, ceramics, glass, plastics such as polyesters, polyethylene terephthalate, polyolefins or cellulose acetate, especially in the form of films, and also metals such as Al, Cu, Ni, Fe, Zn, Mg or Co and GaAs, Si or Si02 to which it is intended to apply a protective layer or, by means of imagewise exposure, to generate an image.

The novel radiation-sensitive compositions further find application as negative resists, having a very high sensitivity to light and being able to be developed in an aqueous alkaline medium without swelling. They are suitable for the production of printing forms for relief printing, planographic printing, photogravure or of screen printing forms, for the production of relief copies, for example for the production of texts in braille, for the production of stamps, for use in chemical milling or as a microresist in the production of integrated circuits. The compositions further may be used as photopatternable dielectric layer or coating, encapsulating material and isolating coating in the production of computer chips, printed boards and other electric or electronic components. The possible layer supports, and the processing conditions of the coating substrates, are just as varied.

The novel composition also relates to a photosensitive thermosetting resin composition and a method of forming a solder resist pattern by the use thereof, and more particularly relates to a novel photosensitive thermosetting resin composition useful as materials for the production of printed circuit boards, the precision fabrication of metallic articles, the etching of glass and stone articles, the relief of plastic articles, and the preparation of printing plates and particularly useful as a solder resist for printed circuit boards and to a method of forming a solder resist pattern by the steps of exposing a layer of the resin composition selectively to an actinic ray through a photomask having a pattern and developing the unexposed part of the layer.

The solder resist is a substance which is used during the soldering of a given part to a printed circuit board for the purpose of preventing molten solder from adhering to irrelevant portions and protecting circuits. It is, therefore, required to possess such properties as high adhesion, insulation resistance, resistance to soldering temperature, resistance to solvents, resistance to alkalis, resistance to acids, and resistance to plating.

Because the photocurable compositions according to the invention have a good thermal stability and are sufficiently resistant to inhibition by oxygen, they are particularly suitable for the production of color filters or color mosaic systems, such as described, for example, in EP320264. Color filters usually are employed in the manufacturing of flat panel displays such as LCD's, PDP(plasma panel display), EL (electroluminessence) display, and projection systems, image sensors, CCD (charge coupled device), and CMOS (complementary metal oxide semiconductor) sensors for scanner, digital camera and video camera. The color filters usually are prepared by forming red, green and blue pixels and a black matrix on a glass substrate. In these processes photocurable compositions according to the invention can be employed. A particularly preferred method of use comprises adding of the coloring matters, dyes and pigments of red, green and blue colors to the light-sensitive resin composition of the present invention, coating of the substrate with the composition, drying of the coating with a short heat treatment, patternwise exposure of the coating to actinic radiation and subsequent development of the pattern in an aqueous alkaline developer solution and optionally a heat treatment. Thus, by subsequently applying a red, green and blue pigmented coating, in any desired order, on top of each other with this process a color filter layer with red, green and blue color pixels can be produced.

The development is carried out by washing out the areas which were not polymerized with a suitable alkali developing solution. This process is repeated to form the image having plural colors.

In the light-sensitive resin composition of the present invention, with a process in which at least one or more picture elements are formed on a transparent substrate and then an exposure is given from a side of the transparent substrate, on which the above picture elements are not formed, the above picture elements can be utilized as a light-shielding mask. In this case, for example, in the case where an overall exposure is given, a position adjustment of a mask gets unnecessary and a concern on a position slippage thereof is removed. And, it is possible to cure all of the part on which the above picture elements are not formed. Further, in this case, it is possible as well to develop and remove a part of the portion on which the above picture elements are not formed by using partially a light-shielding mask.

Since in either case, no gap is formed between the picture elements which are formed formerly and those which are formed later, the composition of the present invention is suitable for, for example, a forming material for a color filter. To be concrete, the coloring matters, dyes and pigments of red, green and blue colors are added to the light-sensitive resin composition of the present invention, and the processes for forming an image are repeated to form the picture elements of red, green and blue colors. Then, the light-sensitive resin composition to which, for example, the black coloring materials, dyes and pigments are added is provided on an overall face. An overall exposure (or a partial exposure via a light-shielding mask) can be provided thereon to form the picture elements of a black color all over the spaces (or all but a partial region of the light-shielding mask) between the picture elements of red, green and blue colors. In addition to a process in which the light-sensitive resin composition is coated on a substrate and dried, the light-sensitive resin composition of the present invention can be used as well for a layer transfer material. That is, the light-sensitive resin composition is layer-wise provided directly on a temporary support, preferably on a polyethylene terephthalate film, or on a polyethylene terephthalate film on which an oxygen-shielding layer and a peeling layer or the peeling layer and the oxygen-shielding layer are provided. Usually, a removable cover sheet made of a synthetic resin is laminated thereon for a protection in handling. Further, there can be applied as well a layer structure in which an alkali soluble thermoplastic resin layer and an intermediate layer are provided on a temporary support and further a light-sensitive resin composition layer is provided thereon (JP 5-173320-A).

The above cover sheet is removed in use and the light-sensitive resin composition layer is laminated on a permanent support. Subsequently, peeling is carried out between those layer and a temporary support when an oxygen-shielding layer and a peeling layer are provided, between the peeling layer and the oxygen-shielding layer when the peeling layer and the oxygen-shielding layer are provided, and between the temporary support and the light-sensitive resin composition layer when either the peeling layer or the oxygen-shielding layer is not provided, and the temporary support is removed.

A metal support, glass, ceramics, and a synthetic resin film can be used as a support for a color filter. Glass and a synthetic resin film which is transparent and have an excellent dimension stability is particularly preferred. The thickness of the light-sensitive resin composition layer is usually 0.1 to 50 micrometers, in particular 0.5 to 5 micrometers.

The developer solution can be used in all forms known to the person skilled in the art, for example in form of a bath solution, puddle, or a spraying solution. In order to remove the non-cured portion of the light-sensitive resin composition layer, there can be combined the methods such as rubbing with a rotary brush and rubbing with a wet sponge. Usually, the temperature of the developing solution is preferably at and around room temperature to 40°C. The developing time is changeable according to the specific kind of the light-sensitive resin composition, the alkalinity and temperature of the developing solution, and the kind and concentration of the organic solvent in the case where it is added. Usually, it is 10 seconds to 2 minutes. It is possible to put a rinsing step after the development processing.

A final heat treatment is preferably carried out after the development processing. Accordingly, a support having a layer which is photopolymerized by exposing (hereinafter referred to as a photocured layer) is heated in an electric furnace and a drier, or the photocured layer is irradiated with an infrared lamp or heated on a hot plate. The heating temperature and time depend on the composition used and the thickness of the formed layer. In general, heating is preferably applied at about 120°C to about 250°C, for about 5 to about 60 minutes.

The compositions according to this invention can also comprise latent pigments which are transformed into finely dispersed pigments during the heat treatment of the latent pigment containing photosensitive pattern or coating. The heat treatment can be performed after exposure or after development of the latent pigment-containing photoimageable layer. Such latent pigments are soluble pigment precursors which can be transformed into insoluble pigments by means of chemical, thermal, photolytic or radiation induced methods as described, for example, in US5879855. This transformation of such latent pigments can be enhanced by adding a compound which generates acid at actinic exposure or by adding an acidic compound to the composition. Therefore, a color filter resist can also be prepared, which comprises a latent pigment in a composition according to this invention.

Examples of color filters, especially with respect to the above described combinations of pigments and ionic impurity scavenger are given in EP320264. It is understood, that the photoinitiators according to the present invention, i.e. the compounds of the formula I in the color filter formulations described in EP 320264 can replace the triazine initiator compounds. Suitable components for a color filter compositions are described above in more detail.

Examples for color filter resists, the composition of such resists and the processing conditions are given by T. Kudo et al., Jpn. J. Appl. Phys. Vol. 37 (1998) 3594; T. Kudo et al., J. Photopolym. Sci. Technol. Vol 9 (1996) 109; K. Kobayashi, Solid State Technol. Nov. 1992, p. S15-S18; US5368976; US5800952; US5882843; US5879855; US5866298; US5863678; JP 06-230212A; EP320264; JP 09-269410A; JP 10-221843A; JP 01 -090516A; JP 10-171119A, US5821016, US5847015, US5882843, US5719008, EP881541 , or EP902327.

The photoinitiators of the present invention can be used in color filter resists, for exampie, such as those given as examples above, or can partially or fully replace the known photoinitiators in such resists. It is understood by a person skilled in the art that the use of the new photoinitiators of the present invention is not limited to the specific binder resins, crosslinkers and formulations of the color filter resist examples given hereinbefore but can be used in conjunction with any radically polymerizable component in combination with a dye or color pigment or latent pigment to form a photosensitive color filter ink or color filter resist.

Accordingly, subject of the invention also is a color filter prepared by providing red, green and blue (RGB) colour elements and, optionally a black matrix, all comprising a photosensitive resin and a pigment on a transparent substrate and providing a transparent electrode either on the surface of the substrate or on the surface of the color filter layer, wherein said photosensitive resin comprises a polyfunctional acrylate monomer, an organic polymer binder and a photopolymerization initiator of the present invention as described above. The monomer and binder components, as well as suitable pigments are as described above. In the manufacture of color filters the transparent electrode layer can either be applied on the surface of the transparent substrate or can be provided on the surface of the red, green and blue picture elements and the black matrix. The transparent substrate is for example a glass substrate which can additionally have an electrode layer on its surface.

It is preferred to apply a black matrix between the color areas of different color in order to improve the contrast of a color filter. The photosensitive compositions of the present invention, as already stated above, are also suitable for the preparation of the black matrix of color filters.

Said black matrix composition for example comprises
a photoinitiator compound of the of the present invention,
an organic binder, in particular an organic binder, which is an epoxy acrylate resin having a carboxyl group,
a black coloring material,
a polymer dispersant, in particular a polymer dispersant containing a basic functional group. The person skilled in the art is familiar with such formulations. Examples of suitable black matrix compositions and the components (other than the photoinitiator) as described above are given in JP Patent No. 3754065, the disclosure of which hereby is incorporated by reference.

Instead of forming a black matrix using a photosensitive composition and patterning the black photosensitive composition photolithographically by patternwise exposure (i.e. through a suitable mask) to form the black pattern separating the red green and blue coloured areas on the transparent substrate it is alternatively possible to use an inorganic black matrix. Such inorganic black matrix can be formed from deposited (i.e. sputtered) metal (i.e. chromium) film on the transparent substrate by a suitable imaging process, for example utilizing photolithographic patterning by means of an etch resist, etching the inorganic layer in the areas not protected by the etch resist and then removing the remaining etch resist.

There are different methods known how and at which step in the color filter manufacturing process the black matrix can be applied. It can either be applied directly on the transparent substrate prior to formation of the red, green and blue (RGB) colour filter as already mentioned above, or it can be applied after the RGB colour filter is formed on the substrate.

In a different embodiment of a color filter for a liqid crystal display, according to US626796, the black matrix can also be applied on the substrate opposite to the RGB color filter element-carrying substrate, which is separated from the former by a liquid crystal layer. If the transparent electrode layer is deposited after applying the RGB color filter elements and - optionally - the black matrix, an additional overcoat film as aprotective layer can be applied on the color filter layer prior to deposition of the electrode layer, for example, as described in US 5650263.

To form an overcoat layer of a color filter, photosensitive resin or thermosetting resin compositions are employed. The photosensitive composition of the present invention can also be used to form such overcoat layers, because a cured film of the composition is excellent in flatness, hardness, chemical and thermal resistance, transparency especially in a visible region, adhesion to a substrate, and suitability for forming a transparent conductive film, e.g., an ITO film, thereon. In the production of a protective layer, there has been a demand that unnecessary parts of the protective layer, for example on scribing lines for cutting the substrate and on bonding pads of solid image sensors should be removed from the substrate as described in JP57-42009A, JP1-130103A and JP1-134306A. In this regard, it is difficult to selectively form a protective layer with good precision using the above-mentioned thermosetting resins. The photosensitive composition, however, allows to easily remove the unnecessary parts of the protective layer by photolithography. It is obvious to those skilled in the art, that the photosensitive compositions of the present invention can be used for generating red, green and blue color pixels and a black matrix, for the manufacture of a color filter, regardless of the above described differences in processing, regardless, of additional layers which can be applied and regardless of differences in the design of the color filter. The use of a composition according to the present invention to form colored elements shall not be regarded as limited by different designs and manufacturing processes of such color filters.

The photosensitive composition of the present invention can suitably be used for forming a color filter but will not be limited to this application. It is useful as well for a recording material, a resist material, a protective layer, a dielectric layer, in display applications and display elements, a paint, and a printing ink.

The photosensitive compositions according to the invention are also suitable for manufacturing interlayer insulating layers or dielectric layers in a liquid crystal display, and more particularly in a reflection type liquid crystal display including an active matrix type display having a thin film transistor (TFT) as a switching device, and a passive matrix type without a switching device.

In recent years, liquid crystal displays have, for example, been widely used for pocket-type TV sets and terminal devices for communication by virtue of its small thickness and light weight. A reflection type liquid crystal display without necessity of using a back light is in particular in demand because it is ultra-thin and light-weight, and it can significantly reduce power consumption. However, even if a back light is removed out of a presently available transmission type color liquid crystal display and a light reflection plate is added to a lower surface of the display, it would cause a problem in that the efficiency of utilizing lights is low, and it is not possible to have practical brightness. As a solution to this problem, there have been suggested various reflection type liquid crystal displays for enhancing an efficiency of utilizing lights. For instance, a certain reflection type liquid crystal display is designed to include a pixel electrode having reflection function.

The reflection type liquid crystal display includes an insulating substrate and an opposing substrate spaced away from the insulating substrate. A space between the substrates is filled with liquid crystals. A gate electrode is formed on the insulating substrate, and both the gate electrode and the insulating substrate are covered with a gate insulating film. A semiconductor layer is then formed on the gate insulating film above the gate electrode. A source electrode and a drain electrode are also formed on the gate insulating film in contact with the semiconductor layer. The source electrode, the drain electrode, the semiconductor layer, and the gate electrode cooperate with one another to thereby constitute a bottom gate type TFT as a switching device.

An interlayer insulating film is formed covering the source electrode, the drain electrode, the semiconductor layer, and the gate insulating film therewith. A contact hole is formed throughout the interlayer insulating film on the drain electrode. A pixel electrode made of aluminum is formed on both the interlayer insulating film and an inner sidewall of the contact hole. The drain electrode of the TFT is eventually in contact with the pixel electrode through the interlayer insulating film. The interlayer insulating layer is generally designed to have a roughened surface by which the pixel electrode acts as a reflection plate which diffuses lights to get a wider angle for viewing (angle of visibility).

The reflection type liquid crystal display remarkably enhances an efficiency of using lights by virtue that the pixel electrode acts as a light reflection plate.

In the above-mentioned reflection type liquid crystal display, the interlayer insulating film is designed to have projections and recesses by photolithography. To form and control a fine shape of the projections and recesses in micrometer order for surface roughness and to form contact holes, photolithography methods using positive and negative photoresists are used. For these resists the compositions according to the invention are especially suitable.

The photosensitive compositions according to the invention can further be used for manufacturing spacers, which control a cell gap of the liquid crystal part in liquid crystal display panels. A transparent column spacer has been widely used in the LCD technology, but the transparent spacer disturbs polarized light reducing the contrast ratio. One of a possible solution is to mix with a black colorant not to scatter but to absorb the polarized light, i.e. a black column spacer. Black column spacer is also used in the LCD technology. In case of black column spacer, one or more further black colorants or mixture of other color colorants described above colorant is used.

Since the properties of light transmitted or reflected through the liquid crystal layer in a liquid crystal display are dependent on the cell gap, the thickness accuracy and uniformity over the pixel array are critical parameters for the performance of the liquid crystal display unit. In a liquid crystal cell, the spacing between the substrates in the cell is maintained constant by sparsely distributing glass or polymer spheres about several micrometers in diameter as spacers between the substrates. The spacers are thus held between the substrates to maintain the distance between the substrates at a constant value. The distance is determined by the diameter of the spacers. The spacers assure the minimum spacing between the substrates; i.e., they prevent a decrease in distance between the substrates. However, they cannot prevent the substrates from being separated apart from each other, i.e. the increase in distance between the substrates. Additionally, this method of using spacer beads has problems of the uniformity in the diameter of spacer beads and difficulty in the even dispersion of spacer beads on the panel, as well as non-uniform orientation and decrease in brightness and/or optical aperture depending on the location of spacers on pixel array region. Liquid crystal displays having a large image display area have recently been attracting much attention. However, the increase in the area of a liquid crystal cell generally produces the distortion of the substrates constituting the cell. The layer structure of the liquid crystal tends to be destroyed due to the deformation of the substrate. Thus, even when spacers are used for maintaining the spacing between the substrates constant, a liquid crystal display having a large image display area is unfeasible because the display experiences disturbances. Instead of the above spacer sphere dispersion method, a method of forming columns in the cell gap as spacers has been proposed. In this method, columns of a resin are formed as spacers in the region between the pixel array region and the counter electrode to form a prescribed cell gap. Photosensitive materials having adhesive properties with photolithography are commonly used, for instance, in the manufacturing process of color filters. This method is advantageous compared with the conventional method using spacer beads in the points that location, number and height of the spacers may be controlled freely. In recent years, as the spread of the touch panel type liquid crystal displays such as mobile audio players and handheld game platforms, the mechanical stress to liquid crystal panel tends to grow. The demand for spacer that controls the cell gap to raise mechanical strength becomes strong thus the multi-spacer method is used. According to the multi-spacer method, when cell gap narrows by pressure from the outside, adding to main-spacer that controls the cell gap normally lower sub-spacer supports the cell gap against external stress. The multi-spacer can follow the contraction of liquid crystal at low temperature conditions by main-spacer and prevent to generate bubbles inside the liquid crystal.

The multi-spacer which contains main-spacer and sub-spacer is formed in the same step using, for example, a halftone mask as described in JPA-201 1065133. The photosensitive compositions according to the invention are eligible for manufacturing process using halftone mask.

In a color liquid crystal display panel, such spacers are formed in the nonimaging area under black matrix of color filter elements. Therefore, the spacers formed using photosensitive compositions do not decrease brightness and optical aperture. Photosensitive compositions for producing protective layer with spacers for color filters are disclosed in JP 2000-81701A and dry film type photoresists for spacer materials are also disclosed in JP 11-174459A and JP 11-174464A. As described in the documents, the photosensitive compositions, liquid and dry film photoresists, are comprising at least an alkaline or acid soluble binder polymer, a radically polymerizable monomer, and a radical initiator. In some cases, thermally crosslinkable components such as epoxide and carboxylic acid may additionally be included.

The steps to form spacers using a photosensitive composition are as follows:
a photosensitive composition is applied to the substrate, for instance a color filter panel and after the substrate is prebaked, it is exposed to light through a mask. Then, the substrate is developed with a developer and patterned to form the desired spacers. When the composition contains some thermosetting components, usually a postbaking is carried out to thermally cure the composition.

The photocurable compositions according to the invention are suitable for producing spacers for liquid crystal displays (as described above) because of their high sensitivity.

The photosensitive compositions according to the invention are also suitable for manufacturing microlens arrays used in liquid crystal display panels, image sensors and the like.

Microlenses are microscopic passive optical components that fit on active optoelectronic devices such as detectors, displays, and light emitting devices (light-emitting diodes, transversal and vertical cavity lasers) to improve their optical input or output quality. The areas of applications are wide and cover areas such as telecommunications, information technology, audio-visual services, solar cells, detectors, solid-state light sources, and optical interconnects.

Present optical systems use a variety of techniques to obtain efficient coupling between microlenses and microoptical devices.

The microlens arrays are used for condensing illuminating light on the picture element regions of a nonluminescent display device, such as a liquid crystal display devices, to increase the brightness of the display, for condensing incident light or as a means for forming an image on the photoelectric conversion regions of a line image sensor used for example in facsimiles and the like to improve the sensitivity of these devices, and for forming an image to be printed on a photosensitive means used in liquid crystal printers or light emitting diode (LED) printers.

The most common application is their use to improve the efficiency of photodetector arrays of a solid-state image sensing device such as a charge coupled device (CCD). In a detector array, the collection of as much light as possible in each detector element or pixel is wanted. If a microlens is put on top of each pixel, the lens collects incoming light and focuses it onto an active area that is smaller than the size of the lens.

According to the prior art, microlens arrays can be produced by a variety of methods; for each of them compositions according to the present invention may be employed.
(1) A method for obtaining convex lenses wherein a pattern of the lenses in a planar configuration is drawn on a thermoplastic resin by a conventional photolithographic technique or the like, and then the thermoplastic resin is heated to a temperature above the softening point of the resin to have flowability, thereby causing a sag in the pattern edge (so called " *reflowing"*) (see, e.g., JP 60-38989A, JP 60-165623A, JP 61-67003A, and JP 2000-39503A). In this method, when the thermoplastic resin used is photosensitive, a pattern of the lenses can be obtained by exposure of this resin to light.
(2) A method for forming a plastic or glass material by the use of a mold or a stamper. As lens material, a photocurable resin and a thermosetting resin can be used in this method (see, e.g., WO99/38035).
(3) A method for forming convex lenses on the basis of a phenomenon in which when a photosensitive resin is exposed to light in a desired pattern by the use of an aligner, unreacted monomers move from the unexposed regions to the exposed regions, resulting in a swell of the exposed regions (see, e.g., Journal of the Research Group in Microoptics Japanese Society of Applied Physics, Colloquium in Optics, Vol. 5, No. 2, pp. 118-123 (1987) and Vol. 6, No. 2, pp. 87-92(1988)).
   On the upper surface of a supporting substrate, a photosensitive resin layer is formed. Thereafter, with the use of a separate shading mask, the upper surface of the photosensitive resin layer is illuminated with light from a mercury lamp or the like, so that the photosensitive resin layer is exposed to the light. As a result, the exposed portions of the photosensitive resin layer swell into the shape of convex lenses to form the light condensing layer having a plurality of microlens.
(4) A method for obtaining convex lenses wherein a photosensitive resin is exposed to light by a proximity exposure technique in which a photomask is not brought into contact with the resin, to cause a blur at the pattern edge, so that the amount of photochemical reaction products is distributed depending upon the degree of blurring at the pattern edge (see, e.g., JP 61-153602A). (5) A method for generating a lens effect wherein a photosensitive resin is exposed to light with a particular intensity distribution to form a distribution pattern of refractive index depending upon the light intensity (see, e.g., JP 60-72927A and JP 60-166946A). The photosensitive compositions according to the invention can be used in any one of the above-mentioned methods to form microlens arrays using photocurable resin compositions.

A particular class of techniques concentrates on forming microlenses in thermoplastic resins like photoresist. An example is published by Popovic et al. in the reference SPIE 898, pp.23-25 (1988). The technique, named reflow technique, comprises the steps of defining the lenses' footprint in a thermoplastic resin, e.g. by photolithography in a photosensitive resin like a photoresist, and subsequently heating this material above its reflow temperature. The surface tension draws the island of photoresist into a spherical cap with a volume equal to the original island before the reflow. This cap is a plano-convex microlens. Advantages of the technique are, amongst others, the simplicity, the reproducibility, and the possibility of integration directly on top of a light-emitting or light-detecting optoelectronic device.

In some cases, an overcoat layer is formed on the patterned lens units with a rectangular shape prior to reflowing to avoid a sagging of the island of the resin in the middle without reflow into a spherical cap in the reflow step. The overcoat acts as a permanent protective layer. The coating layer is also made of a photosensitive composition. Microlens arrays can also be fabricated by the use of a mold or a stamper as, for example, disclosed in EP0932256. A process of manufacturing the planar microlens array is as follows: a release agent is coated on a shaping surface of a stamper on which convex portions are densely arranged, and a photocurable synthetic resin material having a high refractive index is set on the shaping surface of the stamper. Next, the base glass plate is pushed onto the synthetic resin material, thereby spreading the synthetic resin material, and the synthetic resin material is cured by irradiating with ultraviolet radiation or by heating and is shaped to form the convex microlenses. Thereafter the stamper is peeled off. Then, a photocurable synthetic resin material having a low refractive index is additionally coated onto the convex microlenses as an adhesive layer and a glass substrate which is made into a cover glass plate is pushed onto the synthetic resin material, thereby spreading the same. The synthetic resin material is then cured and finally the planar microlens array is formed.

As disclosed in US5969867, a similar method using a mold is applied for the production of a prism sheet, which is used as a part of backlight units for color liquid crystal display panels to enhance the brightness. A prism sheet forming a prism row on one side is mounted on the light-emitting surface of the backlight. For fabricating a prism sheet, an active energy ray-curable composition is cast and spread in a lens mold which is made of metal, glass or resin and forms the lens shape of the prism row, etc., after which a transparent substrate sheet is placed onto it and active energy rays from an active energy ray-emitting source are irradiated through the sheet for curing. The prepared lens sheet is then released from the lens mold to obtain the lens sheet.

The active energy ray-curable composition used to form the lens section must have a variety of properties, including adhesion to the transparent substrate, and suitable optical characteristics. Lenses at least with some photoresists in the prior art are not desirable for some applications since the optical transmittance in the blue end of the optical spectrum is poor. Because the photocurable compositions according to the invention have low yellowing properties, both thermally and photochemically, they are suitable for the production of microlens arrays as described above.

The novel radiation-sensitive compositions are also suitable for photo-lithographic steps used in the production process of plasma display panels (PDP), particularly for the imaging forming process of barrier rib, phosphor layer and electrodes.

The PDP is a planar display for displaying images and information by virtue of the emission of light by gas discharge. By the construction of panel and the method of operation, it is known in two types, i.e. DC (direct current) type and AC (alternating current) type.

By way of example, the principle of the DC type color PDP will be briefly explained. In the DC type color PDP, the space intervening between two transparent substrates (generally glass plates) is divided into numerous minute cells by latticed barrier ribs interposed between the transparent substrates. In the individual cells a discharge gas, such as He or Xe, is sealed. On the rear wall of each cell there is a phosphor layer which, on being excited by the ultraviolet light generated by the discharge of the discharge gas, emits visible light of three primary colors. On the inner faces of the two substrates, electrodes are disposed as opposed to each other across the relevant cells. Generally, the cathodes are formed of a film of transparent electroconductive material such as NESA glass. When a high voltage is applied between these electrodes formed on the fore wall and the rear wall, the discharge gas which is sealed in the cells induces plasma discharge and, by virtue of the ultraviolet light radiated consequently, incites the fluorescent elements of red, blue, and green colors to emit lights and effect the display of an image. In the full-color display system, three fluorescent elements severally of the three primary colors of red, blue, and green mentioned above jointly form one picture element.

The cells in the DC type PDP are divided by the component barrier ribs of a lattice, whereas those in the AC type PDP are divided by the barrier ribs which are arranged parallel to each other on the faces of the substrates. In either case, the cells are divided by barrier ribs. These barrier ribs are intended to confine the luminous discharge within a fixed area to preclude false discharge or cross talk between adjacent discharge cells and ensure ideal display.

The compositions according to the invention also find application for the production of one- or more-layered materials for the image recording or image reproduction (copies, reprography), which may be mono- or polychromatic. Furthermore the materials are suitable for color proofing systems. In this technology formulations containing micro-capsules can be applied and for the image production the radiation curing can be followed by a thermal treatment. Such systems and technologies and their applications are for example disclosed in US5376459.

The compounds of the present invention are also suitable as photoinitiators in the holographic data storage application. Said photoinitiators generate radicals and initiate polymerization of monomer upon irradiation with blue laser radiation, suitable for holographic data storage. The wavelength range of the blue laser is 390-420 nm, preferably 400-410 nm and particularly 405 nm. Holographic storage systems (holographic recording media) are for example used to record and to retrieve a large amount of data with fast access time. The photoinitiators of the invention are for example in particular suitable for systems as described for example in WO03/021358. The holographic data storage system is preferably comprised of a matrix network of low-refractive index matrix precursors and high-refractive index photopolymerizable monomers.

The matrix precursor and photoactive monomer can be selected such that (a) the reaction by which the matrix precursor is polymerized during the cure is independent from the reaction by which the photoactive monomer will be polymerized during writing of a pattern, e.g. data, and (b) the matrix polymer and the polymer resulting from polymerization of the photoactive monomer (the photopolymer) are compatible with each other. The matrix is considered to be formed when the photorecording material, i.e. the matrix material plus the photoactive monomer, photoinitiator and/or additives, exhibits an elastic modulus of at least about 10⁵ Pa, generally about 10⁵ Pa to about 10⁹ Pa.

The media matrix is formed by in-situ polymerization which yields as cross-linked network in the presence of the photopolymerizable monomers which remain *"dissolved'* and unreacted. The matrix containing un-reacted, photopolymerizable monomers can also be formed by other means, for example by using a solid-resin matrix material in which the photoreactive, liquid monomer is homogeneously distributed. Then, monochromatic exposure generates the holographic pattern, which according to the light intensity distribution, polymerizes the photoreactive monomers in the solid pre-formed matrix. The unreacted monomers (where light intensity was at a minimum) diffuse through the matrix, producing a modulation of the refractive index that is determined by the difference between the refractive indices of the monomer and the matrix and by the relative volume fraction of the monomer. The thickness of the recording layer is in the range of several micrometers up to a thickness of one millimeter. Because of such thick holographic data storage layers it is required that the photoinitiator combines high photoreactivity with low absorbance, in order to render the layer transparent at the laser wavelength to assure that the extent of photopolymerization is as little as possible dependent on the exposure depth into the recording layer.

It was found that the photoinitiators of the present invention combine high reactivity with low absorbance at 405 nm and are suitable for this application. Dyes and sensitizers can also be added to the formulations. Suitable dyes and sensitizers for blue laser radiation are for example coumarines, xanthones, thioxanthones, see list above.

In particular relevant are thioxanthones, coumarins and benzophenones as mentioned under items 1., 2. and 3. in the list given above.

It was found that the photoinitiators allow photopolymerization of monomers in thick layers, such as required for holographic data storage, with high sensitivity and yield recording layers which are sensitive to blue laser radiation. The photoinitiators, when applied at a concentration of 2-8 wt% in the photosensitive layer of 20 micron thickness yield an absorbance of the layer which comprises the photoinitiator, of less than 0.4, preferably less than 0.2 at the laser wavelength. The photoinitiators are in particular suitable for the preparation of optical articles (for example optical waveguides) or holographic recording media e.g. comprising a polymer and an organic photoinitiator as described above, having a maximum absorption at a UV wavelength in the range of 340-450 nm, wherein the refractive index contrast adjusted sensitivity is greater than 3×10⁻⁶Δn/(mJ/cm²). For example, the polymer is formed by polymerizing a material comprising component 1 and component 2, wherein component 1 comprises a NCO-terminated prepolymer and component 2 comprises a polyol. Component 1 is, for example, diphenylmethane diisocyanate, toluene diisocyanate, hexamethylene diisocyanate, a derivative of hexamethylene diisocyanate, a methylenebiscyclohexylisocyanate, a derivative of methylenebiscyclohexylisocyanate. Component 2 is for example a polyol of propylene oxide. Preferably, the photoactive monomer is an acrylate monomer. In such media the shrinkage induced by writing is usually less than 0.25%.

Photocuring further is of great importance for printings, since the drying time of the ink is a critical factor for the production rate of graphic products, and should be in the order of fractions of seconds. UV-curable inks are particularly important for screen printing and offset inks.

As already mentioned above, the novel mixtures are highly suitable also for producing printing plates. This application uses, for example, mixtures of soluble linear polyamides or styrene/butadiene and/or styrene/isoprene rubber, polyacrylates or polymethyl methacrylates containing carboxyl groups, polyvinyl alcohols or urethane acrylates with photopolymerizable monomers, for example acrylamides and/or methacrylamides, or acrylates and/or methacrylates, and a photoinitiator. Films and plates of these systems (wet or dry) are exposed over the negative (or positive) of the printed original, and the uncured parts are subsequently washed out using an appropriate solvent or aqueous solutions.

Another field where photocuring is employed is the coating of metals, in the case, for example, of the coating of metal plates and tubes, cans or bottle caps, and the photocuring of polymer coatings, for example of floor or wall coverings based on PVC.

Examples of the photocuring of paper coatings are the colourless varnishing of labels, record sleeves and book covers.

Also of interest is the use of the novel photoinitiators for curing shaped articles made from composite compositions. The composite compound consists of a self-supporting matrix material, for example a glass fibre fabric, or alternatively, for example, plant fibres [cf. K.-P. Mieck, T. Reussmann in Kunststoffe 85 (1995), 366-370], which is impregnated with the photocuring formulation.

The compositions and compounds according to the invention can be used for the production of holographies, waveguides, optical switches wherein advantage is taken of the development of a difference in the index of refraction between irradiated and unirradiated areas.

The use of photocurable compositions for imaging techniques and for the optical production of information carriers is also important. In such applications, as already described above, the layer (wet or dry) applied to the support is irradiated imagewise, e.g. through a photomask, with UV or visible light, and the unexposed areas of the layer are removed by treatment with a developer. Application of the photocurable layer to metal can also be carried out by electrodeposition. The exposed areas are polymeric through crosslinking and are therefore insoluble and remain on the support. Appropriate colouration produces visible images. Where the support is a metallized layer, the metal can, following exposure and development, be etched away at the unexposed areas or reinforced by electroplating. In this way it is possible to produce electronic circuits and photoresists. When used in image-forming materials the novel photoinitiators provide excellent performance in generating so called printout images, whereby a color change is induced due to irradiation. To form such printout images different dyes and/or their leuco form are used and examples for such print out image systems can be fount e.g. in WO96/41240, EP706091 , EP511403, US3579339 and US4622286.

The novel photoinitiator is also suitable for a photopatternable composition for forming a dielectric layer of a multilayer layer circuit board produced by a sequential build-up process.

The invention, as described above, provides compositions, as well as a process, for producing pigmented and nonpigmented paints and varnishes, powder coatings, printing inks, printing plates, adhesives, pressure sensitive adhesives, dental compositions, gel coats, photoresists for electronics, etch resists, both liquid and dry films, solder resists, resists to manufacture color filters for a variety of display applications (a color filter resist contains pigments, pigments and dyes (i.e. hybrid systems) or dyes alone), resists to generate structures in the manufacturing processes of plasma-display panels (e.g. barrier rib, phosphor layer, electrode), electroluminescence displays and LCD (e.g. interlayer insulating layers, spacers, multi-spacers, microlens arrays), for encapsulating electrical and electronic components, for producing magnetic recording materials, micromechanical parts, waveguides, optical switches, plating masks, colour proofing systems, glass fibre cable coatings, screen printing stencils, three-dimensional objects by means of stereolithography, image recording materials for holographic recordings (e.g. for holographic data storage (HDS)), microelectronic circuits, decolorizing materials, formulations containing microcapsules, photoresist materials for a UV and visible laser direct imaging system and for forming dielectric layers in a sequential build-up layer of a printed circuit board, bank/pixel definition layers for OLED, sealants for LCD and OLED, insulation/passivation layers for LCD and OLED, insulation for metal wiring/transparent conductive films for touch panel, coatings for touch panel, decorative inks for touch panel, protective films for touch panel or etching resists for touch panel; wherein the process comprises irradiating a composition as described abbove with electromagnetic radiation in the range from 150 to 600 nm, or with electron beam or with X-rays.

Substrates used for photographic information recordings include, for example, films of polyester, cellulose acetate or polymer-coated papers; substrates for offset printing formes are specially treated aluminium, substrates for producing printed circuits are copper-clad laminates, and substrates for producing integrated circuits are, for example, silicon wafers. The layer thickness of the photosensitive layer for photographic materials and offset printing forms is generally from about 0.5 µm to 10 µm, while for printed circuits it is from 0.1 µm to about 100 µm. Following the coating of the substrates, the solvent is removed, generally by drying, to leave a coat of the photoresist on the substrate.

Coating of the substrates can be carried out by applying to the substrate a liquid composition, a solution or a suspension. The choice of solvents and the concentration depend principally on the type of composition and on the coating technique. The solvent should be inert, i.e. it should not undergo a chemical reaction with the components and should be able to be removed again, after coating, in the course of drying. Examples of suitable solvents are ketones, ethers and esters, such as methyl ethyl ketone, isobutyl methyl ketone, cyclopentanone, cyclohexanone, N-methylpyrrolidone, dioxane, tetrahydrofuran, 2-methoxyethanol, 2-ethoxyethanol, 1-methoxy-2-propanol, 1,2-dimethoxyethane, ethyl acetate, n-butyl acetate, ethyl 3-ethoxypropionate, 2-methoxypropylacetate, methyl-3-methoxypropionate, 2-heptanone, 2-pentanone, and ethyl lactate. The solution is applied uniformly to a substrate by means of known coating techniques, for example by spin coating, dip coating, knife coating, curtain coating, brushing, spraying, especially by electrostatic spraying, and reverse-roll coating, and also by means of electrophoretic deposition. It is also possible to apply the photosensitive layer to a temporary, flexible support and then to coat the final substrate, for example a copper-clad circuit board, or a glass substrate by transferring the layer via lamination.

The quantity applied (coat thickness) and the nature of the substrate (layer support) are dependent on the desired field of application. The range of coat thicknesses generally comprises values from about 0.1 µm to more than 100 µm, for example 0.1 µm to 1 cm, preferably 0.5 µm to 1000 µm. Following the coating of the substrates, the solvent is removed, generally by drying, to leave an essentially dry resist film of the photoresist on the substrate.

The photosensitivity of the novel compositions can extend in general from about 150 nm to 600 nm, for example 190-600 nm, (UV-vis region). Suitable radiation is present, for example, in sunlight or light from artificial light sources. Consequently, a large number of very different types of light sources are employed. Both point sources and arrays *("lamp carpets")* are suitable. Examples are carbon arc lamps, xenon arc lamps, low-, medium-, high- and super high-pressure mercury lamps, possibly with metal halide dopes (metal-halogen lamps), microwave-stimulated metal vapour lamps, excimer lamps, superactinic fluorescent tubes, fluorescent lamps, argon incandescent lamps, electronic flashlights, photographic flood lamps, light emitting diodes (LED, OLED), electron beams and X-rays. The distance between the lamp and the substrate to be exposed in accordance with the invention may vary depending on the intended application and the type and output of lamp, and may be, for example, from 2 cm to 150 cm. Laser light sources, for example excimer lasers, such as F2 excimer lasers at 157 nm exposure, KrF excimer lasers for exposure at 248 nm and ArF excimer lasers for exposure at 193 nm are also suitable. Lasers in the visible region can also be employed.

The term *"imagewise"* exposure includes both, exposure through a photomask comprising a predetermined pattern, for example a slide, a chromium mask, a stencil mask or a reticle, as well as exposure by means of a laser or light beam, which for example is moved under computer control over the surface of the coated substrate and in this way produces an image. Suitable UV laser exposure systems for the purpose are, for example, provided by Etec and Orbotech (DP-100^{™} DIRECT IMAGING SYSTEM). Other examples of laser light sources are, for example excimer lasers, such as F2 excimer lasers at 157 nm exposure, KrF excimer lasers for exposure at 248 nm and ArF excimer lasers for exposure at 193 nm. Further suitable are solid state UV lasers (e.g. Gemini from ManiaBarco, DI-2050 from PENTAX) and violet laser diodes with 405 nm output (DI-2080, DI-PDP from PENTAX). Lasers in the visible region can also be employed. And the computer-controlled irradiation can also be achieved by electron beams. It is also possible to use masks made of liquid crystals that can be addressed pixel by pixel to generate digital images, as is, for example, described by A. Bertsch, J.Y. Jezequel, J.C. Andre in Journal of Photochemistry and Photobiology A: Chemistry 1997, 107, p. 275-281 and by K.-P. Nicolay in Offset Printing 1997, 6, p. 34-37.

Following the imagewise exposure of the material and prior to development, it may be advantageous to carry out thermal treatment for a short time. After the development a thermal post bake can be performed to harden the composition and to remove all traces of solvents. The temperatures employed are generally 50-250°C, preferably 80-220°C; the duration of the thermal treatment is in general between 0.25 and 60 minutes.

The invention therefore also provides a process for the photopolymerization of compounds containing ethylenically unsaturated double bonds, i.e. monomeric, oligomeric or polymeric compounds containing at least one ethylenically unsaturated double bond, which comprises adding to these compounds at least one photoinitiator of the present invention as described above and irradiating the resulting composition with electromagnetic radiation, in particular light of the wavelength 150 to 600 nm, in particular 190-600 nm, with electron beam, or with X-rays. In other words, adding to these compounds compounds containing ethylenically unsaturated double bonds at least one photoinitiator of the present invention as described above and irradiating the resulting composition with electromagnetic radiation, in particular light of the wavelength 150 to 600 nm, in particular 190-600 nm, with electron beam, or with X-rays.

The invention further provides a coated substrate which is coated on at least one surface with a composition as described above. Interesting also is a process for the photographic production of relief images, in which a coated substrate as described above is subjected to imagewise exposure and then the unexposed portions are removed with a developer. Imagewise exposure may be effected by irradiating through a mask or by means of a laser or electron beam as already described above. Of particular advantage in this context is the laser beam exposure already mentioned above.

The compounds of the invention have a good thermal stability, low volatility, good storage stability and high solubility, and are also suitable for photopolymerisations in the presence of air (oxygen). Further, the compositions show increased brightness after photopolymerization.

The examples which follow illustrate the invention in more detail, without restricting the scope said examples only. Parts and percentages are, as in the remainder of the description and in the claims, by weight, unless stated otherwise. Where alkyl radicals having more than three carbon atoms are referred to in the examples without any mention of specific isomers, the n-isomers are meant in each case.

### Substance examples

### Example 1: Preparation of OE1

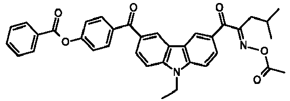

### OE1 is prepared according to the following scheme:

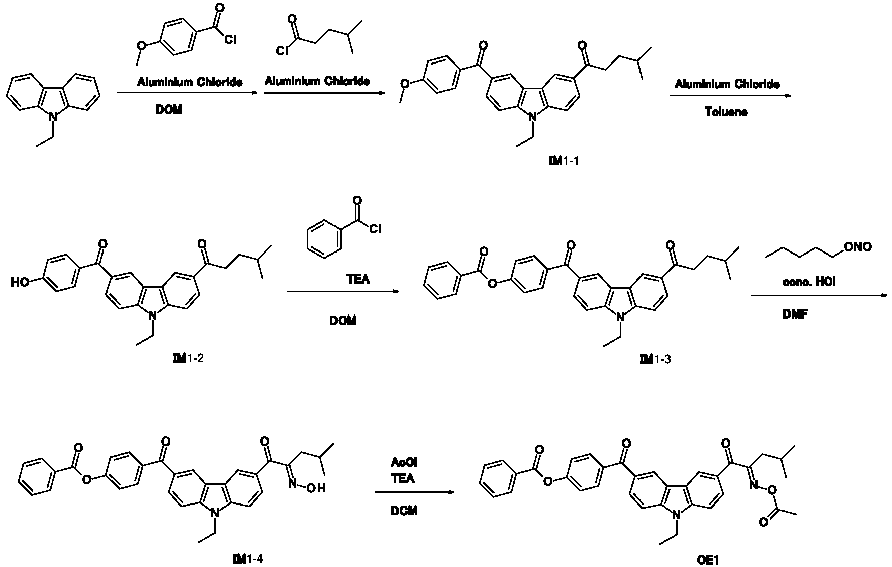

### Preparation of IM1-1

To a solution of 9-ethylcarbazole (3.0 g, 15.4 mmol) and Aluminum Chloride (2.25 g, 16.9 mmol) in DCM (100 ml) was added 4-Methoxybenzoyl chloride (2.62 g, 15.4 mmol) at 0 °C, and the mixture was stirred for 30 min. To the reaction mixture was added Aluminum Chloride (2.25 g, 16.9 mmol) and 4-Methylvaleryl Chloride (2.1 ml, 15.4 mmol), and the mixture was stirred for 30 min. The reaction mixture was quenched with cold water. The reaction mixture was extracted with DCM, and dried over anhydrous MgSO₄. The solvent was removed under reduced pressure and the crude product was purified by silica gel chromatography to give the product (3.6 g, 55%).

### Preparation of IM1-2

To a solution of IM1-1 (3.6 g, 8.4 mmol) in Toluene (100 ml) was added Aluminum Chloride (3.38 g, 25.3 mmol), and the mixture was stirred for 2 h at 80 °C. The reaction mixture was quenched with cold water. The reaction mixture was extracted with EtOAc, and dried over anhydrous MgSO₄. The solvent was removed under reduced pressure to give the product (3.0 g, 87%).

### Preparation of IM1-3

To a solution of IM1-2 (0.50 g, 1.2 mmol) and TEA (0.17 ml, 1.21 mmol) in DCM (100 ml) was added Benzoyl Chloride (0.14 ml, 1.2 mmol) at 0 °C, and the mixture was stirred for 30 min at room temperature (rt). The reaction mixture was quenched with water. The reaction mixture was extracted with DCM, and dried over anhydrous MgSO₄. The solvent was removed under reduced pressure to give the product (0.59 g, 94%).

### Preparation of IM1-4

To a solution of IM1-3 (0.59 g, 1.14 mmol) in DMF (10 ml) was added conc. HCl (0.10 ml, 1.2 mmol) and Amyl Nitrite (0.23 ml, 1.7 mmol) at 0 °C, and the mixture was stirred overnight at rt. The reaction mixture was quenched with cold water. The reaction mixture was extracted with EtOAc, washed with brine, and dried over anhydrous MgSO₄. The solvent was removed under reduced pressure and the crude product was purified by silica gel chromatography to give the product (0.32 g, 51%).

### Preparation of OE1

To a solution of the IM1-4 (0.32 g, 0.59 mmol) in DCM (30 ml) was added TEA (0.09 ml, 0.64 mmol) and AcCl (0.05 ml, 0.70 mmol) at 0 °C, and the mixture was stirred for 15 min. The reaction mixture was quenched with water. The mixture was extracted with DCM, and dried over anhydrous MgSO₄. Silica gel was added to the solution. The yellow silica gel was removed by cotton filtration, and washed with DCM. The solvent was removed under reduced pressure to give the product (0.17 g, 49%).

### Example 2: Preparation of OE2

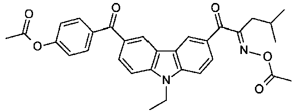

### OE2 is prepared according to the following scheme:

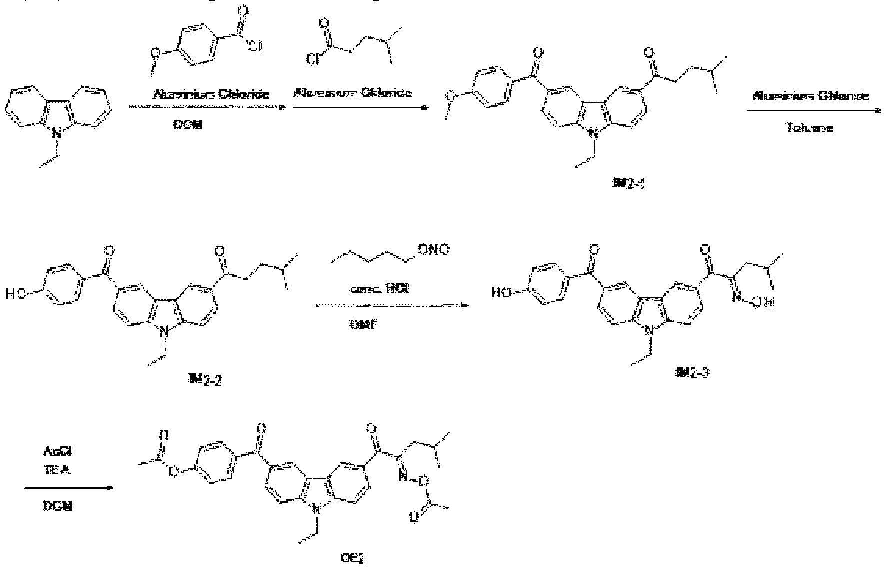

### Preparation of IM2-1

To a solution of 9-ethylcarbazole (3.0 g, 15.4 mmol) and Aluminum Chloride (2.25 g, 16.9 mmol) in DCM (100 ml) was added 4-Methoxybenzoyl chloride (2.62 g, 15.4 mmol) at 0 °C, and the mixture was stirred for 30 min. To the reaction mixture was added Aluminum Chloride (2.25 g, 16.9 mmol) and 4-Methylvaleryl Chloride (2.1 ml, 15.4 mmol), and the mixture was stirred for 30 min. The reaction mixture was quenched with cold water. The reaction mixture was extracted with DCM, and dried over anhydrous MgSO₄. The solvent was removed under reduced pressure and the crude product was purified by silica gel chromatography to give the product (3.6 g, 55%).

### Preparation of IM2-2

To a solution of IM2-1 (3.6 g, 8.4 mmol) in Toluene (100 ml) was added Aluminum Chloride (3.38 g, 25.3 mmol), and the mixture was stirred for 2 h at 80 °C. The reaction mixture was quenched with cold water. The reaction mixture was extracted with EtOAc, and dried over anhydrous MgSO₄. The solvent was removed under reduced pressure to give the product (3.0 g, 87%).

### Preparation of IM2-3

To a solution of IM2-2 (0.50 g, 1.21 mmol) in DMF (10 ml) was added conc. HCl (0.15 ml, 1.8 mmol) and Amyl Nitrite (0.24 ml, 1.8 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure to give the product (0.35 g, 65%).

### Preparation of OE2

To a solution of the IM2-3 (0.35 g, 0.79 mmol) in DCM (20 ml) was added TEA (0.23 ml, 1.6 mmol) and AcCl (0.12 ml, 1.6 mmol) at 0 °C, and the mixture was stirred for 15 min. The reaction mixture was quenched with water. The mixture was extracted with DCM, and dried over anhydrous MgSO₄. Silica gel was added to the solution. The yellow silica gel was removed by cotton filtration, and washed with DCM. The solvent was removed under reduced pressure to give the product (0.39 g, 74%).

### Example 3: Preparation of OE3

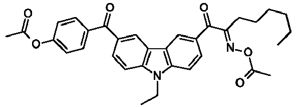

### OE3 is prepared as the following scheme:

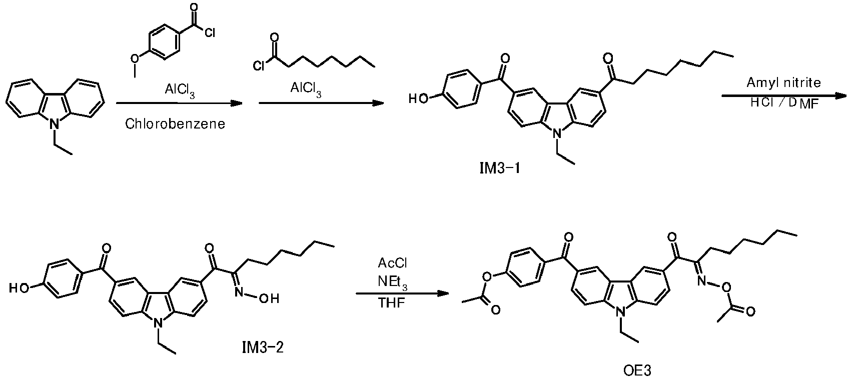

### Preparation of IM3-1

To 10.05g of 9-ethylcarbazole and 6.87g of aluminum chloride in chlorobenzene (100ml) was added 8.80g of 4-methoxybenzoyl chloride and stirred at room temperature (rt) for 2h under N₂ flow condition. To the reaction mixture was added 8.38g of n-octanoyl chloride and 13.76g of aluminum chloride and stirred at rt for 16h under N₂ flow condition, then heated at 100°C for 1h. The reaction mixture was poured into ice-water, and the precipitate was collected by filtration and washed with mixture of hexane and dichloromethane (DCM). The resulting solid was dried in vacuo to give 25.81g of IM3-1 as beige solid.

### Preparation of IM3-2

To 3.25g of IM3-1 was added mixture of DMF (40ml) and hydrochloric acid (10ml) at rt. Then, 1.25g of amyl nitrite was added and stirred for 20h. The organic layer was washed with brine and dried over anhydrous sodium sulfate. The solvent was removed under vacuum, and the crude was purified via column chromatography with hexane and DCM (90:10 to 0:100 v/v) as eluent to give 2.55g of IM3-2 as orange resin.

### Preparation of OE3

To 2.55 of IM3-2 and 3.00g of acetyl chloride in tetrahydrofuran (20ml) was added 3.96g of triethylamine at 0 °C, then stirred at room temperature for 3h. Water was added to the reaction mixture and organic layer was extracted with ethyl acetate. The organic layer was washed with brine and dried over anhydrous sodium sulfate. The solvent was removed under vacuum, and the crude was purified via column chromatography with hexane and DCM (90:10 to 0:100 v/v) as eluent to give 1.33g of OE3 as yellow resin.

### Example 4: Preparation of OE4

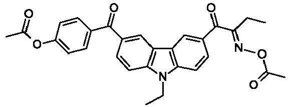

### OE4 is prepared as the following scheme:

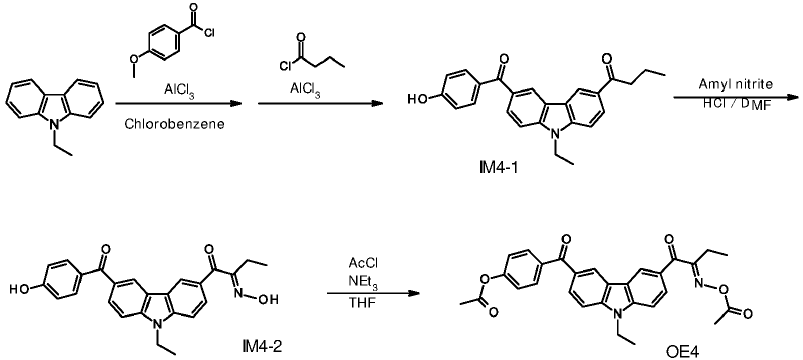

### Preparation of IM4-1

To 9.80g of 9-ethylcarbazole and 6.70g of aluminum chloride in chlorobenzene (100ml) was added 8.64g of 4-methoxybenzoyl chloride and stirred at rt for 4h under N₂ flow condition. To the reaction mixture was added 5.36g of n-butyryl chloride and 13.87g of aluminum chloride and stirred at rt for 1h under N₂ flow condition, then heated at 100°C for 3h. The reaction mixture was poured into ice-water and the precipitate was collected by filtration and dried in vacuo. The resulting solid was washed with mixture of hexane and DCM, giving 21.74g of IM4-1 as beige solid.

### Preparation of IM4-2

To 20.69g of IM4-1 was added mixture of DMF (100ml), tetrahydrofyran (200ml) and hydrochloric acid (30ml) at rt. Then, 7.45g of amyl nitrite was added and stirred for 4h. The organic layer was washed with brine and dried over anhydrous sodium sulfate. The solvent was removed under vacuum, and the crude was purified via column chromatography with hexane and DCM (90:10 to 0:100 v/v) as eluent to give 19.81g of IM4-2 as yellow powder.

### Preparation of OE4

To 19.81 of IM4-2 and 8.11g of acetyl chloride in tetrahydrofuran (200ml) was added 1046g of triethylamine at 0 °C, then stirred at room temperature for 15h. Water was added to the reaction mixture and organic layer was extracted with ethyl acetate. The organic layer was washed with brine and dried over anhydrous sodium sulfate. The solvent was removed under vacuum, and the crude was purified via column chromatography with hexane and DCM (90:10 to 0:100 v/v) as eluent to give 4.96g of OE4 as yellow resin.

### Example 5: Preparation of OE5

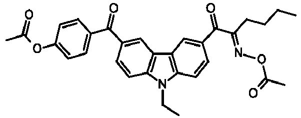

### OES is prepared according to the following scheme:

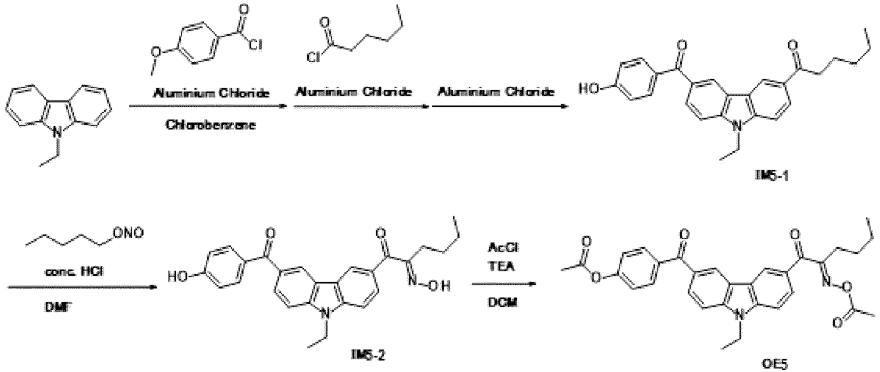

### Preparation of IMS -1

To a solution of 9-ethylcarbazole (10.0 g, 51.2 mmol) and Aluminum Chloride (7.57 g, 56.8 mmol) in Chlorobenzene (200 ml) was added 4-Methoxybenzoyl chloride (8.74 g, 51.2 mmol) at 0 °C, and the mixture was stirred for 1 h. To the reaction mixture was added Aluminum Chloride (7.57 g, 51.2 mmol) and Hexanoyl Chloride (6.89 g, 51.2 mmol), and the mixture was stirred for 1 h. To the reaction mixture was added Aluminum Chloride (7.57 g, 51.2 mmol), and the mixture was stirred for 3 h at 80 °C. The reaction mixture was quenched with cold water. The reaction mixture was extracted with DCM, and dried over anhydrous MgSO₄. The solvent was removed under reduced pressure and Hexane was added to the crude product. The precipitated solid was collected by filtration and dried to give the product (19.5 g, 92%).

### Preparation of IM5-2

To a solution of IM5-1 (6.00 g, 14.5 mmol) in DMF (50 ml) was added conc. HCl (1.8 ml, 22 mmol) and Amyl Nitrite (2.9 ml, 22 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and Hexane was added to the crude product. The precipitated solid was collected by filtration and dried to give the product (5.3 g, 83%).

### Preparation of OE5

To a solution of the IM5-2 (5.3 g, 12 mmol) in DCM (120 ml) was added TEA (3.6 ml, 25 mmol) and AcCl (1.8 ml, 25 mmol) at 0 °C, and the mixture was stirred for 15 min. The reaction mixture was quenched with water. The mixture was extracted with DCM, and dried over anhydrous MgSO₄. The solvent was removed under reduced pressure and the crude product was purified by silica gel chromatography to give the product (3.56 g, 56%).

### Example 6: Preparation of OE6

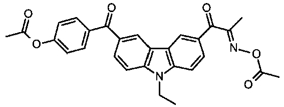

### OE6 is prepared according to the following scheme:

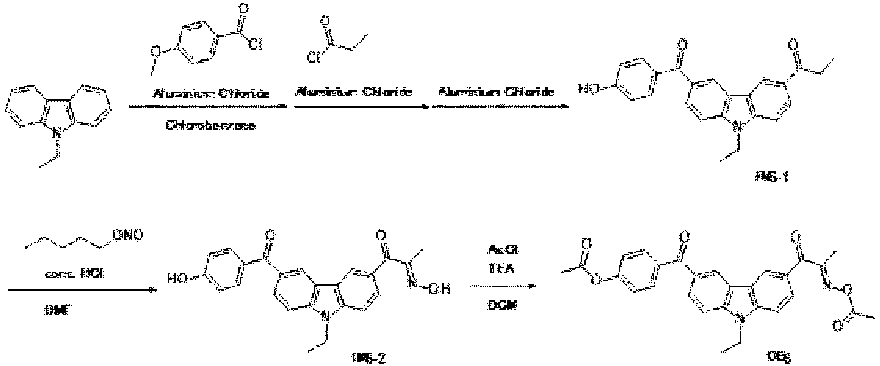

### Preparation of IM6-1

To a solution of 9-ethylcarbazole (6.00 g, 30.7 mmol) and Aluminum Chloride (4.51 g, 33.8 mmol) in Chlorobenzene (300 ml) was added 4-Methoxybenzoyl chloride (5.24 g, 30.7 mmol) at 0 °C, and the mixture was stirred for 1 h. To the reaction mixture was added Aluminum Chloride (4.51 g, 33.8 mmol) and Propionyl Chloride (2.84 g, 30.7 mmol), and the mixture was stirred for 1 h. To the reaction mixture was added Aluminum Chloride (4.51 g, 33.8 mmol), and the mixture was stirred for 3 h at 80 °C. The reaction mixture was quenched with cold water. The precipitated solid was collected by filtration and washed with water to give the product (10.1 g, 89%).

### Preparation of IM6-2

To a solution of IM6-1 (6.00 g, 16.2 mmol) in DMF (50 ml) was added conc. HCl (2.0 ml, 24 mmol) and Amyl Nitrite (3.2 ml, 24 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and DCM/Hexane was added to the crude product. The precipitated solid was collected by filtration and washed with DCM/Hexane to give the product (5.16 g, 80%).

### Preparation of OE6

To a solution of the IM6-2 (5.16 g, 12.9 mmol) in DCM (130 ml) was added TEA (3.8 ml, 27 mmol) and AcCl (1.9 ml, 27 mmol) at 0 °C, and the mixture was stirred for 15 min. The reaction mixture was quenched with water. The mixture was extracted with DCM, and dried over anhydrous MgSO₄. The solvent was removed under reduced pressure and the crude product was purified by silica gel chromatography to give the product (3.5 g, 56%).

### Example 7: Preparation of OE7

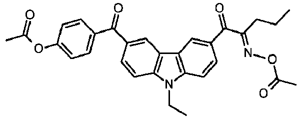

### OE7 is prepared according to the following scheme:

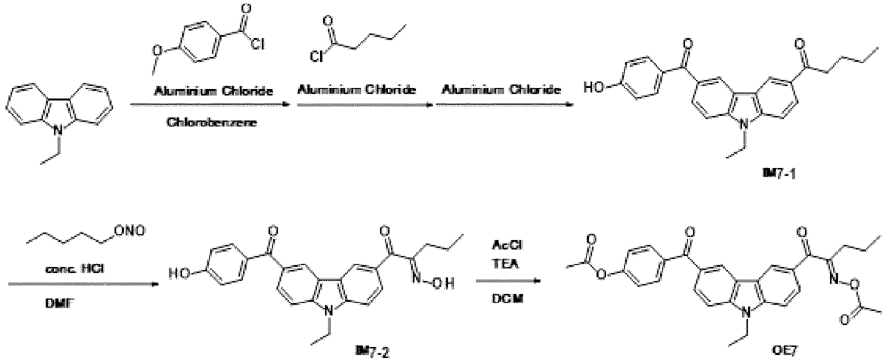

### Preparation of IM7-1

To a solution of 9-ethylcarbazole (10.0 g, 51.2 mmol) and Aluminum Chloride (7.57 g, 56.8 mmol) in Chlorobenzene (300 ml) was added 4-Methoxybenzoyl chloride (8.74 g, 51.2 mmol) at 0 °C, and the mixture was stirred for 1 h. To the reaction mixture was added Aluminum Chloride (7.57 g, 56.8 mmol) and Valeryl Chloride (6.17 g, 51.2 mmol), and the mixture was stirred for 1 h. To the reaction mixture was added Aluminum Chloride (7.57 g, 56.8 mmol), and the mixture was stirred for 3 h at 80 °C. The reaction mixture was quenched with cold water. The reaction mixture was extracted with DCM, and dried over anhydrous MgSO₄. The solvent was removed under reduced pressure to give the product (14.0 g, 68%).

### Preparation of IM7-2

To a solution of IM7-1 (6.00 g, 15.0 mmol) in DMF (80 ml) was added conc. HCl (1.9 ml, 22 mmol) and Amyl Nitrite (3.0 ml, 22 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and DCM/Hexane was added to the crude product. The precipitated solid was collected by filtration and washed with DCM/Hexane to give the product (5.9 g, 90%).

### Preparation of OE7

To a solution of the IM7-2 (5.8 g, 13 mmol) in DCM (130 ml) was added TEA (4.0 ml, 28 mmol) and AcCI (2.0 ml, 28 mmol) at 0 °C, and the mixture was stirred for 15 min. The reaction mixture was quenched with water. The mixture was extracted with DCM, and dried over anhydrous MgSO₄. The solvent was removed under reduced pressure and the crude product was purified by silica gel chromatography to give the product (3.8 g, 55%).

### Example 8: Preparation of OE8

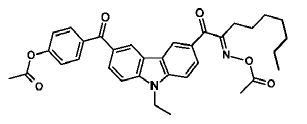

**OE8** is prepared as the following scheme:

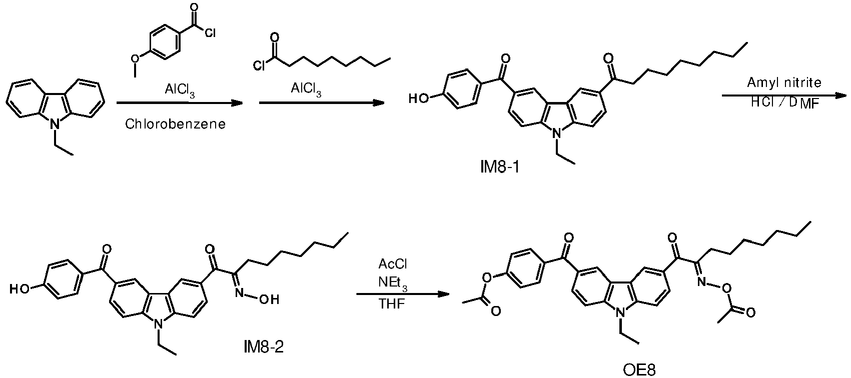

### Preparation of IM8-1

To 10.04g of 9-ethylcarbazole and 6.88g of aluminum chloride in chlorobenzene (100ml) was added 8.77g of 4-methoxybenzoyl chloride and stirred at rt for 1h under N₂ flow condition. To the reaction mixture was added 9.09g of n-nonanoyl chloride and 14.00g of aluminum chloride and stirred at rt for 2h under N₂ flow condition, then heated at 100°C for 4h. The reaction mixture was poured into ice-water and the precipitate was collected by filtration and dried in vacuo. The resulting solid was washed with mixture of hexane and DCM, giving 45.49g of IM8-1 as beige solid.

### Preparation of IMB-2

To 6.02g of IM8-1 was added mixture of DMF (50ml), tetrahydrofuran (50ml) and hydrochloric acid (10ml) at rt. Then, 1.67g of amyl nitrite was added and stirred for 4h. The organic layer was washed with brine and dried over anhydrous sodium sulfate. The solvent was removed under vacuum, and the crude was purified via column chromatography with hexane and DCM (90:10 to 0:100 v/v) as eluent to give 1.81g of IM8-2 as brown resin.

### Preparation of OE8

To 5.96 of IM8-2 and 2.10g of acetyl chloride in tetrahydrofuran (100ml) was added 2.65g of triethylamine at 0°C, then stirred at room temperature for 1h. Water was added to the reaction mixture and organic layer was extracted with ethyl acetate. The organic layer was washed with brine and dried over anhydrous sodium sulfate. The solvent was removed under vacuum, and the crude was purified via column chromatography with hexane and DCM (90:10 to 0:100 v/v) as eluent to give 4.37 of OE8 as off-white powder. The E:Z ratio was found to be 93:7.

### Example 9: Preparation of OE9

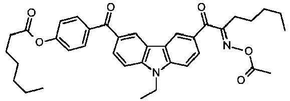

### OE9 is prepared according to the following scheme:

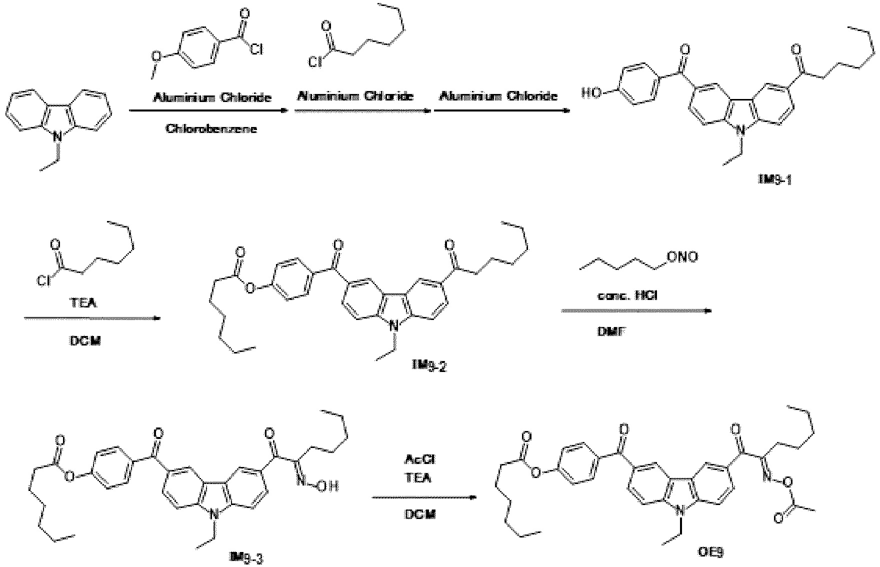

### Preparation of IM9-1

To a solution of 9-ethylcarbazole (10.0 g, 51.2 mmol) and Aluminum Chloride (7.57 g, 56.8 mmol) in Chlorobenzene (300 ml) was added 4-Methoxybenzoyl chloride (8.74 g, 51.2 mmol) at 0 °C, and the mixture was stirred for 1 h. To the reaction mixture was added Aluminum Chloride (7.57 g, 56.8 mmol) and Heptanoyl Chloride (7.61 g, 51.2 mmol), and the mixture was stirred for 1 h. To the reaction mixture was added Aluminum Chloride (7.57 g, 56.8 mmol), and the mixture was stirred for 3 h at 80 °C. The reaction mixture was quenched with cold water. The reaction mixture was extracted with DCM, and dried over anhydrous MgSO₄. The solvent was removed under reduced pressure and DCM/Hexane was added to the crude product. The precipitated solid was collected by filtration and washed with DCM/Hexane to give the product (17.8 g, 82%).

### Preparation of IM9-2

To a solution of IM9-1 (4.00 g, 9.36 mmol) and TEA (1.4 ml, 9.9 mmol) in DCM (100 ml) was added Heptanoyl Chloride (1.5 ml, 9.9 mmol) at 0 °C, and the mixture was stirred for 15 min at rt. The reaction mixture was quenched with water. The reaction mixture was extracted with DCM, and dried over anhydrous MgSO₄. The solvent was removed under reduced pressure and DCM/Hexane was added to the crude product. The precipitated solid was collected by filtration and washed with DCM/Hexane to give the product (3.85 g, 76%).

### Preparation of IM9-3

To a solution of IM9-2 (3.85 g, 7.13 mmol) in DMF (24 ml) was added conc. HCl (0.65 ml, 7.8 mmol) and Amyl Nitrite (1.05 ml, 7.8 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and the crude product was purified by silica gel chromatography to give the product (2.4 g, 59%).

### Preparation of OE9

To a solution of the IM9-3 (2.4 g, 4.2 mmol) in DCM (40 ml) was added TEA (0.62 ml, 4.4 mmol) and AcCl (0.32 ml, 4.4 mmol) at 0 °C, and the mixture was stirred for 15 min. The reaction mixture was quenched with water. The mixture was extracted with DCM, and dried over anhydrous MgSO₄. The solvent was removed under reduced pressure and Diethyl ether/Hexane was added to the crude product. The precipitated solid was collected by filtration and washed with Diethyl ether/Hexane to give the product (1.9 g, 75%).

### Example 10: Preparation of OE10

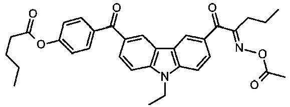

### OE10 is prepared according to the following scheme:

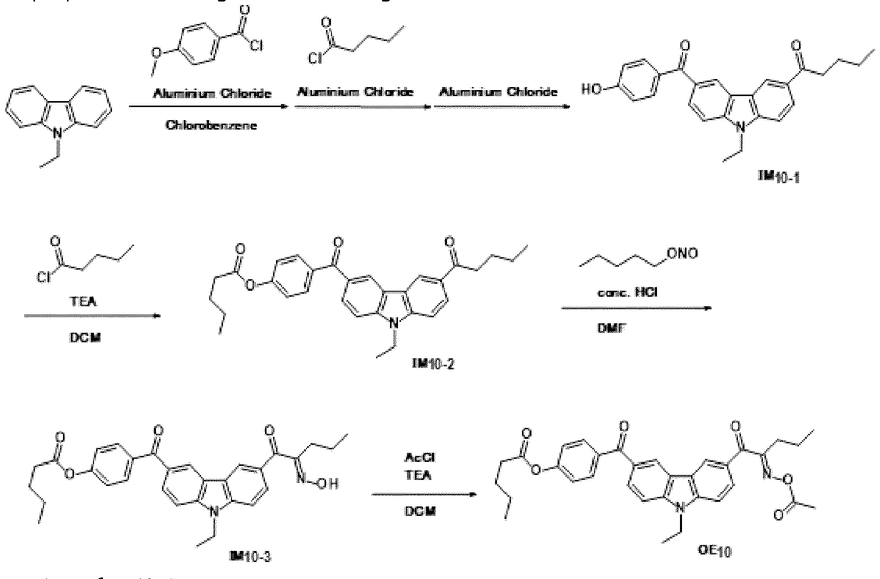

### Preparation of IM10-1

To a solution of 9-ethylcarbazole (10.0 g, 51.2 mmol) and Aluminum Chloride (7.57 g, 56.8 mmol) in Chlorobenzene (300 ml) was added 4-Methoxybenzoyl chloride (8.74 g, 51.2 mmol) at 0 °C, and the mixture was stirred for 1 h. To the reaction mixture was added Aluminum Chloride (7.57 g, 56.8 mmol) and Valeryl Chloride (6.17 g, 51.2 mmol), and the mixture was stirred for 1 h. To the reaction mixture was added Aluminum Chloride (7.57 g, 56.8 mmol), and the mixture was stirred for 3 h at 80 °C. The reaction mixture was quenched with cold water. The reaction mixture was extracted with DCM, and dried over anhydrous MgSO₄. The solvent was removed under reduced pressure to give the product (14.0 g, 68%).

### Preparation of IM10-2

To a solution of IM10-1 (4.00 g, 9.36 mmol) and TEA (1.4 ml, 9.9 mmol) in DCM (100 ml) was added Valeryl Chloride (1.2 ml, 10 mmol) at 0 °C, and the mixture was stirred for 15 min at rt. The reaction mixture was quenched with water. The reaction mixture was extracted with DCM, and dried over anhydrous MgSO₄. The solvent was removed under reduced pressure and Diethyl ether/Hexane was added to the crude product. The precipitated solid was collected by filtration and washed with Diethyl ether/Hexane to give the product (4.40 g, 91%).

### Preparation of IM10-3

To a solution of IM10-2 (4.40 g, 9.10 mmol) in DMF (30 ml) was added conc. HCl (0.83 ml, 10 mmol) and Amyl Nitrite (1.3 ml, 10 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and the crude product was purified by silica gel chromatography to give the product (2.6 g, 56%).

### Preparation of OE10

To a solution of the IM10-3 (2.6 g, 5.1 mmol) in DCM (50 ml) was added TEA (0.75 ml, 5.3 mmol) and AcCl (0.38 ml, 5.3 mmol) at 0 °C, and the mixture was stirred for 15 min. The reaction mixture was quenched with water. The mixture was extracted with DCM, and dried over anhydrous MgSO₄. The solvent was removed under reduced pressure and TBME/Hexane was added to the crude product. The precipitated solid was collected by filtration and washed with TBME/Hexane to give the product (2.4 g, 86%).

### Example 11: Preparation of OE11

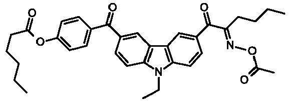

### OE11 is prepared according to the following scheme:

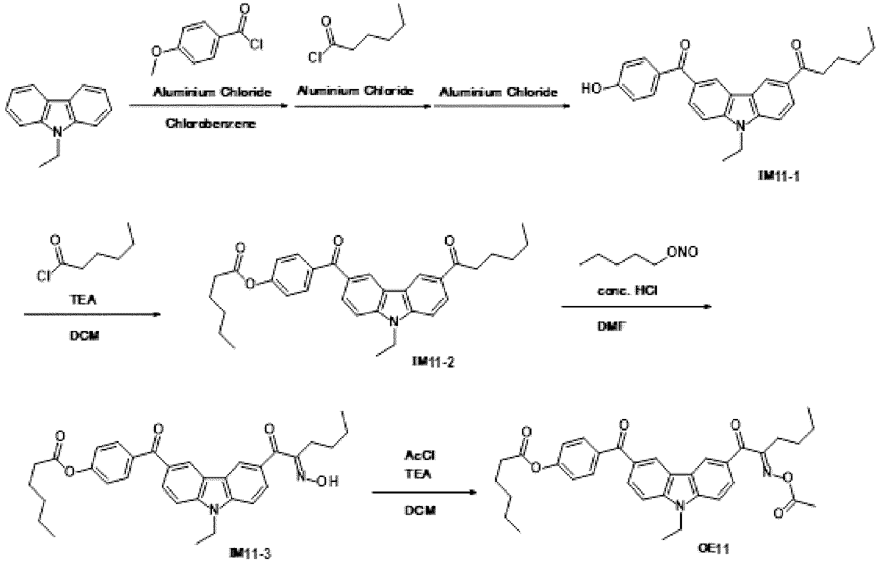

### Preparation of IM11-1

To a solution of 9-ethylcarbazole (10.0 g, 51.2 mmol) and Aluminum Chloride (7.57 g, 56.8 mmol) in Chlorobenzene (200 ml) was added 4-Methoxybenzoyl chloride (8.74 g, 51.2 mmol) at 0 °C, and the mixture was stirred for 1 h. To the reaction mixture was added Aluminum Chloride (7.57 g, 51.2 mmol) and Hexanoyl Chloride (6.89 g, 51.2 mmol), and the mixture was stirred for 1 h. To the reaction mixture was added Aluminum Chloride (7.57 g, 51.2 mmol), and the mixture was stirred for 3 h at 80 °C. The reaction mixture was quenched with cold water. The reaction mixture was extracted with DCM, and dried over anhydrous MgSO₄. The solvent was removed under reduced pressure and Hexane was added to the crude product. The precipitated solid was collected by filtration and dried to give the product (19.5 g, 92%).

### Preparation of IM11-2

To a solution of IM11-1 (7.00 g, 16.9 mmol) and TEA (2.5 ml, 18 mmol) in DCM (160 ml) was added Hexanoyl Chloride (2.4 ml, 18 mmol) at 0 °C, and the mixture was stirred for 15 min at rt. The reaction mixture was quenched with water. The reaction mixture was extracted with DCM, and dried over anhydrous MgSO₄. The solvent was removed under reduced pressure and Diethyl ether/Hexane was added to the crude product. The precipitated solid was collected by filtration and washed with Diethyl ether/Hexane to give the product (6.32 g, 73%).

### Preparation of IM11-3

To a solution of IM11-2 (4.00 g, 7.81 mmol) in DMF (40 ml) was added conc. HCl (0.98 ml, 12 mmol) and Amyl Nitrite (1.6 ml, 12 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and the crude product was purified by silica gel chromatography to give the product (2.91 g, 69%).

### Preparation of OE11

To a solution of the IM11-3 (2.91 g, 5.38 mmol) in DCM (50 ml) was added TEA (0.79 ml, 5.6 mmol) and AcCl (0.40 ml, 5.6 mmol) at 0 °C, and the mixture was stirred for 15 min. The reaction mixture was quenched with water. The mixture was extracted with DCM, and dried over anhydrous MgSO₄. The solvent was removed under reduced pressure and TBME/Hexane was added to the crude product. The precipitated solid was collected by filtration and washed with TBME/Hexane to give the product (2.44 g, 78%).

### Example 12: Preparation of OE12

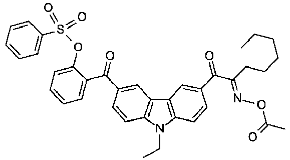

### OE12 is prepared according to the following scheme:

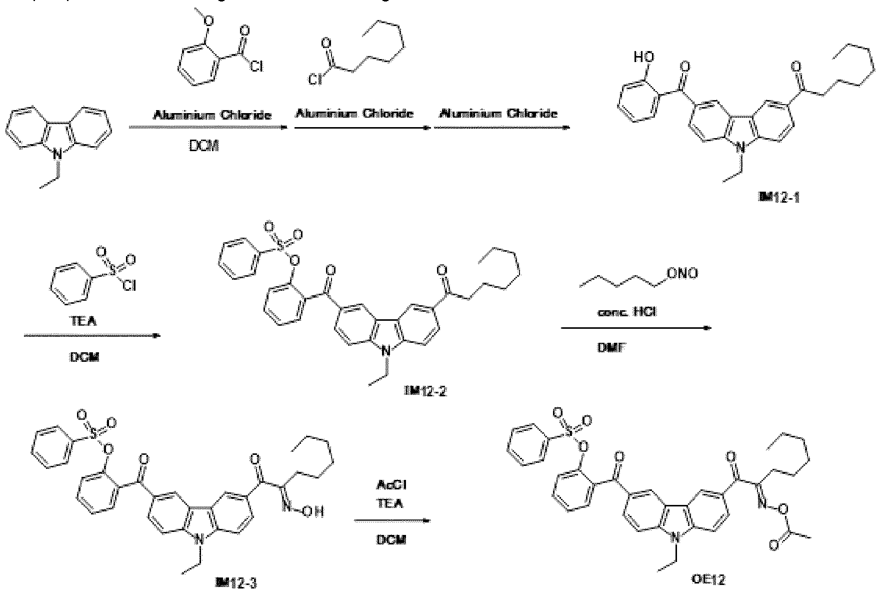

### Preparation of IM12-1

To a solution of 9-ethylcarbazole (5.00 g, 25.6 mmol) and Aluminum Chloride (3.75 g, 28.1 mmol) in DCM (250 ml) was added 2-Methoxybenzoyl chloride (4.37 g, 25.6 mmol) at 0 °C, and the mixture was stirred for 1 h. To the reaction mixture was added Aluminum Chloride (3.75 g, 28.1 mmol) and Octanoyl Chloride (4.16 g, 25.6 mmol), and the mixture was stirred for 1 h. To the reaction mixture was added Aluminum Chloride (7.5 g, 56 mmol), and the mixture was stirred for 2 h at the reflux temperature. The reaction mixture was quenched with cold water. The reaction mixture was extracted with DCM, and dried over anhydrous MgSO₄. The solvent was removed under reduced pressure and Hexane was added to the crude product. The precipitated solid was collected by filtration and dried to give the product (8.6 g, 81%).

### Preparation of IM12-2

To a solution of IM12-1 (0.50 g, 1.21 mmol) and TEA (0.18 ml, 1.3 mmol) in DCM (100 ml) was added Benzenesulfonyl chloride (0.16 ml, 1.3 mmol) at 0 °C, and the mixture was stirred for 15 min at rt. The reaction mixture was quenched with water. The reaction mixture was extracted with DCM, and dried over anhydrous MgSO₄. The solvent was removed under reduced pressure and the crude product was purified by silica gel chromatography to give the product (0.57 g, 81%).

### Preparation of IM12-3

To a solution of IM12-2 (0.57 g, 0.98 mmol) in DMF (30 ml) was added conc. HCl (0.12 ml, 1.4 mmol) and Amyl Nitrite (0.20 ml, 1.5 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and the crude product was purified by silica gel chromatography to give the product (0.47 g, 79%).

### Preparation of OE12

To a solution of the IM12-3 (0.47 g, 0.77 mmol) in DCM (100 ml) was added TEA (0.11 ml, 0.78 mmol) and AcCl (0.06 ml, 0.84 mmol) at 0 °C, and the mixture was stirred for 15 min. The reaction mixture was quenched with water. The mixture was extracted with DCM, and dried over anhydrous MgSO₄. Silica gel was added to the solution. The yellow silica gel was removed by cotton filtration, and washed with DCM. The solvent was removed under reduced pressure to give the product (0.39 g, 78%).

### Example 13: Preparation of OE13

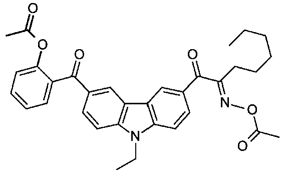

### OE13 is prepared according to the following scheme:

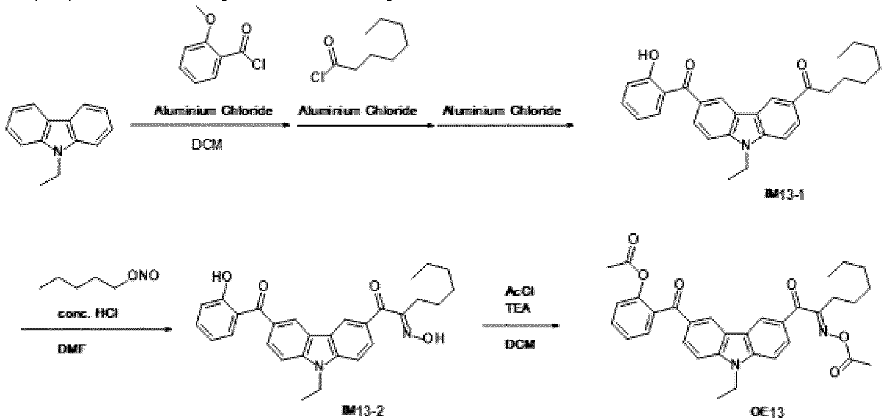

### Preparation of IM13-1

To a solution of 9-ethylcarbazole (5.00 g, 25.6 mmol) and Aluminum Chloride (3.75 g, 28.1 mmol) in DCM (250 ml) was added 2-Methoxybenzoyl chloride (4.37 g, 25.6 mmol) at 0 °C, and the mixture was stirred for 1 h. To the reaction mixture was added Aluminum Chloride (3.75 g, 28.1 mmol) and Octanoyl Chloride (4.16 g, 25.6 mmol), and the mixture was stirred for 1 h. To the reaction mixture was added Aluminum Chloride (7.5 g, 56 mmol), and the mixture was stirred for 2 h at the reflux temperature. The reaction mixture was quenched with cold water. The reaction mixture was extracted with DCM, and dried over anhydrous MgSO₄.The solvent was removed under reduced pressure and Hexane was added to the crude product. The precipitated solid was collected by filtration and dried to give the product (8.6 g, 81%).

### Preparation of IM13-2

To a solution of IM13-1 (0.55 g, 1.33 mmol) in DMF (20 ml) was added conc. HCl (0.16 ml, 1.9 mmol) and Amyl Nitrite (0.26 ml, 1.9 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and the crude product was purified by silica gel chromatography to give the product (0.42 g, 67%).

### Preparation of OE13

To a solution of the IM13-2 (0.42 g, 0.89 mmol) in DCM (100 ml) was added TEA (0.26 ml, 1.8 mmol) and AcCl (0.13 ml, 1.8 mmol) at 0 °C, and the mixture was stirred for 15 min. The reaction mixture was quenched with water. The mixture was extracted with DCM, and dried over anhydrous MgSO₄. Silica gel was added to the solution. The yellow silica gel was removed by cotton filtration, and washed with DCM. The solvent was removed under reduced pressure to give the product (0.41 g, 83%).

### Example 14: Preparation of OE14

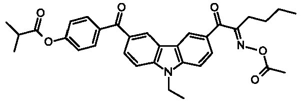

### OE14 is prepared according to the following scheme:

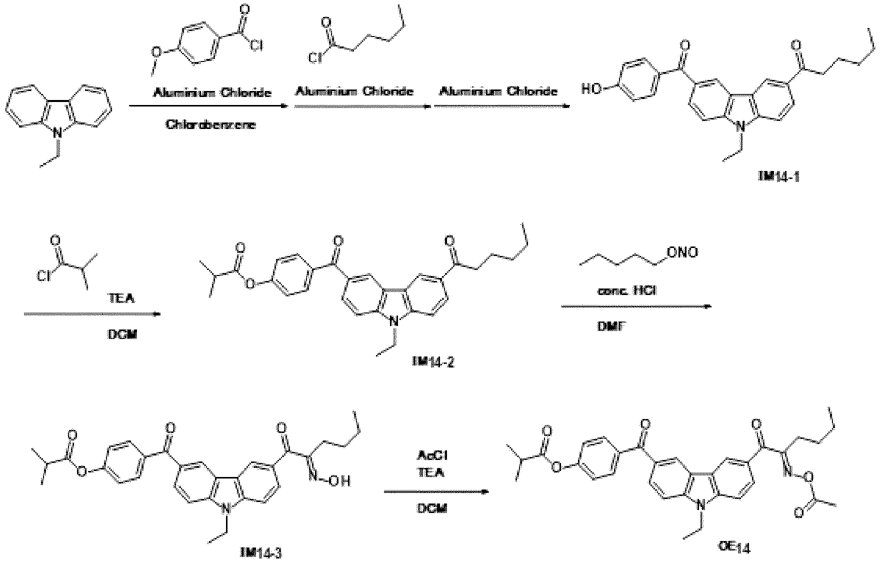

### Preparation of IM14-1

To a solution of 9-ethylcarbazole (10.0 g, 51.2 mmol) and Aluminum Chloride (7.57 g, 56.8 mmol) in Chlorobenzene (200 ml) was added 4-Methoxybenzoyl chloride (8.74 g, 51.2 mmol) at 0 °C, and the mixture was stirred for 1 h. To the reaction mixture was added Aluminum Chloride (7.57 g, 51.2 mmol) and Hexanoyl Chloride (6.89 g, 51.2 mmol), and the mixture was stirred for 1 h. To the reaction mixture was added Aluminum Chloride (7.57 g, 51.2 mmol), and the mixture was stirred for 3 h at 80 °C. The reaction mixture was quenched with cold water. The reaction mixture was extracted with DCM, and dried over anhydrous MgSO₄. The solvent was removed under reduced pressure and Hexane was added to the crude product. The precipitated solid was collected by filtration and dried to give the product (19.5 g, 92%).

### Preparation of IM14-2

To a solution of IM14-1 (4.00 g, 9.67 mmol) and TEA (1.4 ml, 10 mmol) in DCM (100 ml) was added Isobutyryl Chloride (1.1 ml, 10 mmol) at 0 °C, and the mixture was stirred for 15 min at rt. The reaction mixture was quenched with water. The reaction mixture was extracted with DCM, and dried over anhydrous MgSO₄. The solvent was removed under reduced pressure and TBME/Hexane was added to the crude product. The precipitated solid was collected by filtration and washed with TBME/Hexane to give the product (3.27 g, 70%).

### Preparation of IM14-3

To a solution of IM14-2 (3.27 g, 6.77 mmol) in DMF (30 ml) was added conc. HCl (0.85 ml, 10 mmol) and Amyl Nitrite (1.35 ml, 10 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and TBME/Hexane was added to the crude product. The precipitated solid was collected by filtration and washed with TBME/Hexane to give the product (2.60 g, 75%).

### Preparation of OE14

To a solution of the IM14-3 (2.60 g, 5.07 mmol) in DCM (50 ml) was added TEA (0.75 ml, 5.3 mmol) and AcCl (0.38 ml, 5.3 mmol) at 0 °C, and the mixture was stirred for 15 min. The reaction mixture was quenched with water. The mixture was extracted with DCM, and dried over anhydrous MgSO₄. The solvent was removed under reduced pressure and TBME/Hexane was added to the crude product. The precipitated solid was collected by filtration and washed with TBME/Hexane to give the product (1.90 g, 68%).

### Example 15: Preparation of OE15

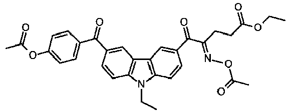

### OE15 is prepared according to the following scheme:

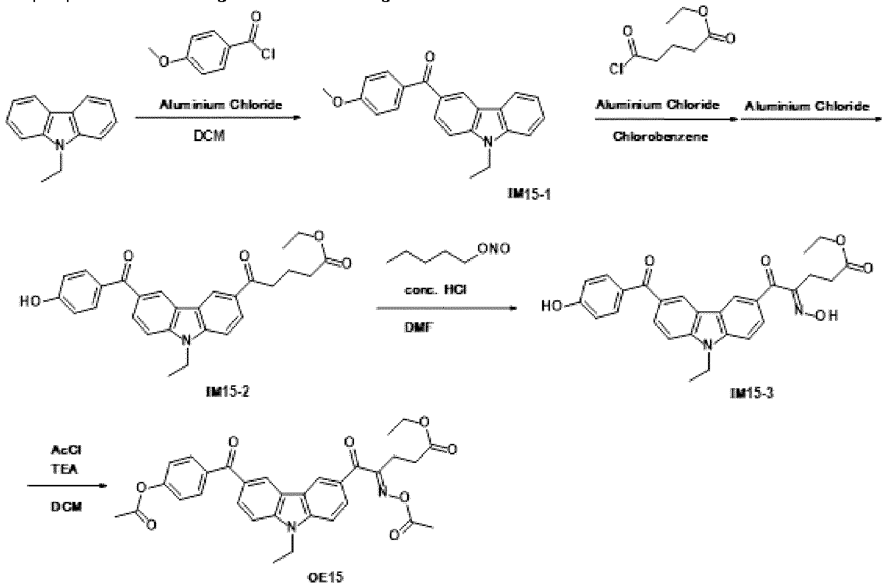

### Preparation of IM15-1

To a solution of 9-ethylcarbazole (30.0 g, 153 mmol) and Aluminum Chloride (21.5 g, 161 mmol) in DCM (750 ml) was added 4-Methoxybenzoyl chloride (4.37 g, 25.6 mmol) at 0 °C, and the mixture was stirred for 1 h. The reaction mixture was quenched with cold water. The reaction mixture was extracted with DCM, and dried over anhydrous MgSO₄. The solvent was removed under reduced pressure and Diethyl ether/Hexane was added to the crude product. The precipitated solid was collected by filtration and washed with Diethyl ether/Hexane to give the product (43.2 g, 86%).

### Preparation of IM15-2

To a solution of IM15-1 (4.00 g, 12.1 mmol) and Aluminum Chloride (4.84 g, 36.3 mmol) in Chlorobenzene (120 ml) was added Ethyl Glutaryl Chloride (1.9 ml, 12.1 mmol) at 0 °C, and the mixture was stirred for 1 h. To the reaction mixture was added Aluminum Chloride (1.61 g, 12 mmol), and the mixture was stirred for 1 h at 80 °C. The reaction mixture was quenched with cold water. The reaction mixture was extracted with DCM, and dried over anhydrous MgSO₄. The solvent was removed under reduced pressure and DCM/Hexane was added to the crude product. The precipitated solid was collected by filtration and washed with DCM/Hexane to give the product (4.81 g, 87%).

### Preparation of IM15-3

To a solution of IM15-2 (4.81 g, 10.5 mmol) in DMF (40 ml) was added conc. HCl (1.1 ml, 13 mmol) and Amyl Nitrite (1.7 ml, 13 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and TBME/Hexane was added to the crude product. The precipitated solid was collected by filtration and washed with Diethyl ether/Hexane to give the product (3.54 g, 69%).

### Preparation of OE15

To a solution of the IM15-3 (3.54 g, 7.28 mmol) in DCM (70 ml) was added TEA (2.2 ml, 15 mmol) and AcCl (1.1 ml, 15 mmol) at 0 °C, and the mixture was stirred for 15 min. The reaction mixture was quenched with water. The mixture was extracted with DCM, and dried over anhydrous MgSO₄. The solvent was removed under reduced pressure and TBME/Hexane was added to the crude product. The precipitated solid was collected by filtration and washed with Diethyl ether/Hexane to give the product (2.50 g, 60%).

### Example 16: Preparation of OE16

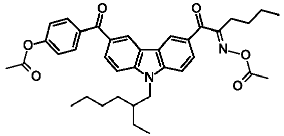

**OE16** is prepared according to the procedure described for **OE17** below using hexanoyl chloride in place of heptanoyl chloride.

### Example 17: Preparation of OE17

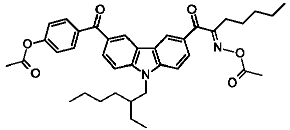

### OE17 is prepared according to the following scheme:

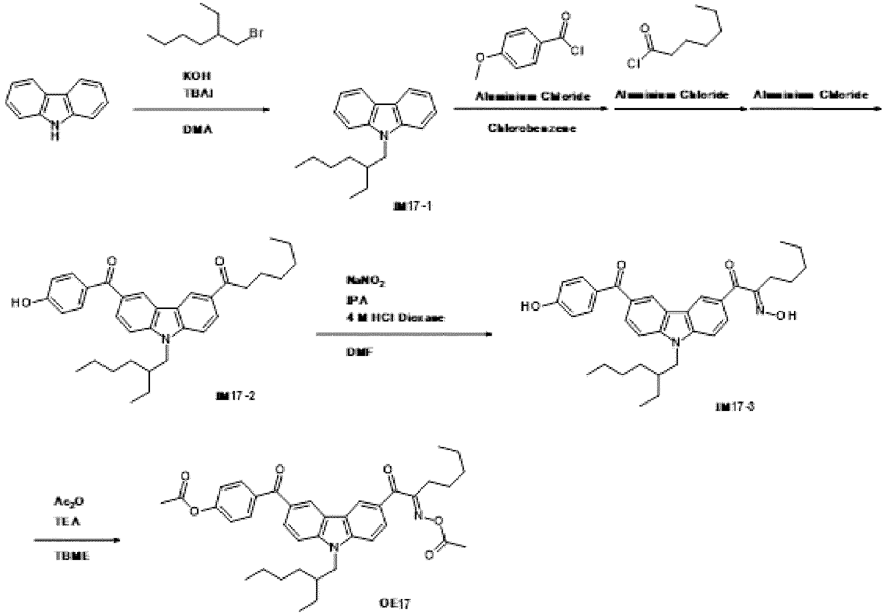

### Preparation of IM17-1

To a solution of Carbazole (50.2 g, 300 mmol) in DMA (150 ml) was added 2-Ethylhexyl Bromide (77.6 g, 450 mmol), KOH (25.3 g, 450 mmol) and Tetrabutylammonium Iodide (5.54 g, 15 mmol). The reaction mixture was stirred for overnight at 80 °C. The reaction mixture was quenched with cold water. The reaction mixture was extracted with EtOAc, and dried over anhydrous MgSO₄. The solvent was removed under reduced pressure and Hexane was added to the crude product. The precipitated solid was removed by filtration and the solvent was removed under reduced pressure to give the product (75.0 g, 90%).

### Preparation of IM17-2

To a solution of IM17-1 (27.9 g, 100 mmol) and Aluminum Chloride (14.0 g, 105 mmol) in Chlorobenzene (200 ml) was added 4-Methoxybenzoyl chloride (17 g, 100 mmol) at 0 °C, and the mixture was stirred for 30 min. To the reaction mixture was added Aluminum Chloride (28 g, 210 mmol) and Heptanoyl Chloride (15.5 g, 100 mmol), and the mixture was stirred for 1 h. To the reaction mixture was stirred for 2 h at 80 °C. The reaction mixture was quenched with cold water. The reaction mixture was extracted with EtOAc, and dried over anhydrous MgSO₄. The solvent was removed under reduced pressure and TBME/Hexane was added to the crude product. The precipitated solid was collected by filtration and washed with TBME/Hexane to give the product (34.3 g, 67%).

### Preparation of IM17-3

To a solution of IM17-2 (10.2 g, 20.0 mmol) in DMF (30 ml) was added Isopropyl alcohol (2.0 ml, 28.5 mmol), Sodium nitrite (1.66 g, 24.0 mmol) and 4 M HCl Dioxane (15 ml, 60 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and TMBE/Toluene was added to the crude product. The precipitated solid was collected by filtration and washed with Toluene to give the product (5.3 g, 49%).

### Preparation of OE17

To a solution of IM-17-3 (5.3 g, 9.7 mmol) in TBME (50 ml) was added TEA (3.2 ml, 23 mmol) and Acetic anhydride (2.1 ml, 22 mmol) at 0 °C, and the mixture was stirred for overnight at rt. The reaction mixture was quenched by water. The mixture was extracted with TBME, washed with brine, and dried over anhydrous MgSO₄. The solvent was removed under reduced pressure and TBME/Heptane was added to the crude product. The precipitated solid was collected by filtration and washed with TBME/Heptane to give the product (5.1 g, 84%).

The product OE17 was obtained mainly in E configuration. Samples of pure E isomer and pure Z isomer were prepared using conventional silica gel chromatography (DCM/Acetone). The Z isomer of OE17 (OE17-Z) can be prepared according to the following scheme:

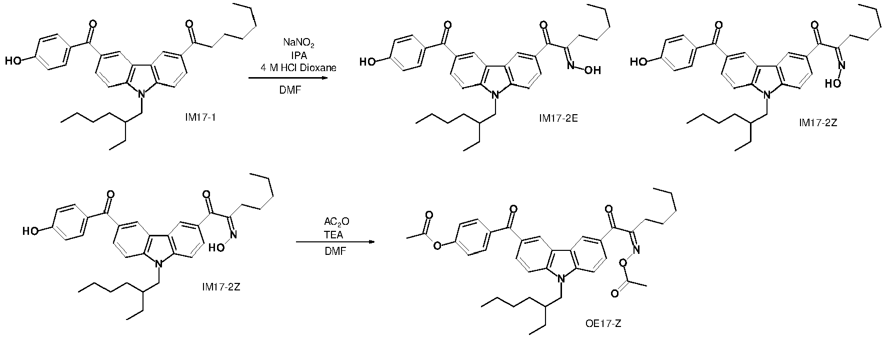

### Preparation of IM17-2Z

To a solution of IM17-1 (240.5 g, 470.0 mmol) in DMF (575 ml) was added Isopropyl alcohol (47 ml, 671 mmol), Sodium nitrite (45.4 g, 658 mmol) and 4 M HCl Dioxane (421 ml, 1.68 mol) at 0 °C, and the mixture was stirred for overnight at rt. The reaction mixture was quenched with cold water. The reaction mixture was extracted with EtOAc, washed with brine, and dried over anhydrous MgSO4. The solvent was removed under reduced pressure and the crude product was dissolved in 500 ml of toluene at 60 °C. To the solution 10 ml of butyl acetate and 50 ml of heptane were added and cooled down, leading to precipitation. The solid was isolated by filtration and washed with heptane, which was (mainly) composed of E-isomer. The filtrate was purified by silica gel chromatography (DCM/Acetone) to give 5.76 g of IM17-2Z (Z isomer).

### Preparation of OE17-Z

To a solution of IM-17-2Z (5.2 g, 9.7 mmol) in TBME (20 ml) was added TEA (2.25 g, 22 mmol) and Acetic anhydride (2.17 g, 21 mmol) at 0 °C, and the mixture was stirred for overnight at rt. The reaction mixture was quenched by water. The mixture was extracted with EtOAc, washed with brine, and dried over anhydrous MgSO₄. The solvent was removed under reduced pressure and the crude product was purified by silica gel chromatography (DCM/Acetone) to give 3.28 g (54 %) of OE17-Z (Z isomer) as a dark yellow resin.

### Example 18: Preparation of OE18

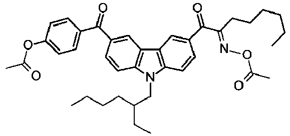

### OE18 is prepared according to the following scheme:

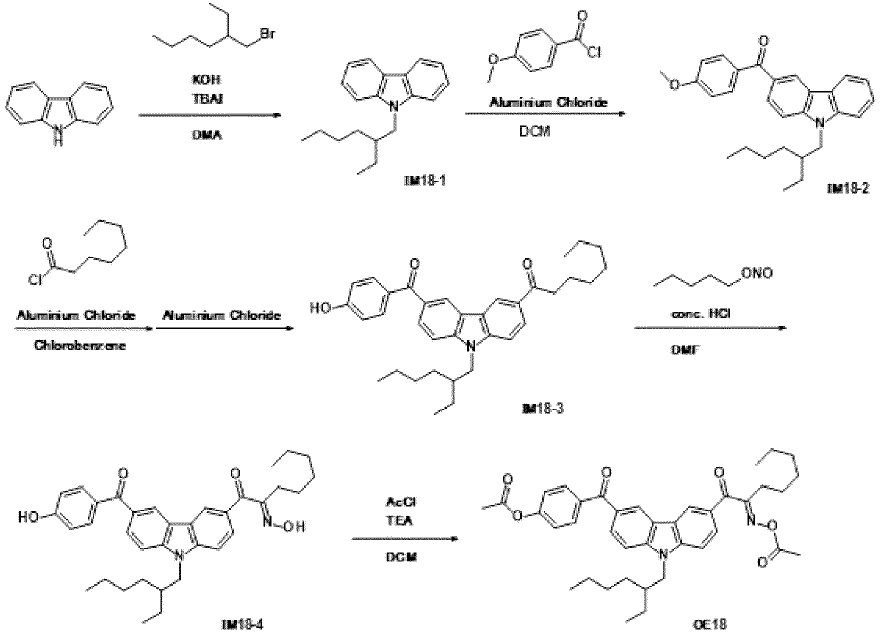

### Preparation of IM18-1

To a solution of Carbazole (50.2 g, 300 mmol) in DMA (150 ml) was added 2-Ethylhexyl Bromide (77.6 g, 450 mmol), KOH (25.3 g, 450 mmol) and Tetrabutylammonium Iodide (5.54 g, 15 mmol). The reaction mixture was stirred for overnight at 80 °C. The reaction mixture was quenched with cold water. The reaction mixture was extracted with EtOAc, and dried over anhydrous MgSO₄. The solvent was removed under reduced pressure and Hexane was added to the crude product. The precipitated solid was removed by filtration and the solvent was removed under reduced pressure to give the product (75.0 g, 90%).

### Preparation of IM18-2

To a solution of IM18-1 (23.8 g, 85.2 mmol) and Aluminum Chloride (11.9 g, 89.2 mmol) in DCM (400 ml) was added 4-Methoxybenzoyl chloride (14.5 g, 85.0 mmol) at 0 °C, and the mixture was stirred for 30 min. The reaction mixture was quenched with cold water. The reaction mixture was extracted with DCM, and dried over anhydrous MgSO₄. The solvent was removed under reduced pressure to give the product (27.4 g, 78%).

### Preparation of IM18-3

To a solution IM18-2 (12.2 g, 29.5 mmol) and Aluminum Chloride (7.86 g, 59.0 mmol) in Chlorobenzene (200 ml) was added Octanoyl chloride (5.1 ml, 29 mmol) at 0 °C, and the mixture was stirred for 30 min. To the reaction mixture was added Aluminum Chloride (3.93 g, 29.5 mmol), and the mixture was stirred for 1 h at 100 °C. The reaction mixture was quenched with cold water. The reaction mixture was extracted with DCM, and dried over anhydrous MgSO₄. The solvent was removed under reduced pressure and TBME/Hexane was added to the crude product. The precipitated solid was collected by filtration and washed with TBME/Hexane to give the product (8.96 g, 58%).

### Preparation of IM18-4

To a solution of IM18-3 (8.96 g, 17.0 mmol) in DMF (45 ml) was added conc. HCl (2.1 ml, 25 mmol) and Amyl Nitrite (3.4 ml, 25 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and the crude product was purified by silica gel chromatography to give the product (6.27 g, 67%).

### Preparation of OE18

To a solution of the IM18-4 (6.27 g, 11.3 mmol) in DCM (56 ml) was added TEA (3.3 ml, 23 mmol) and AcCl (1.7 ml, 23 mmol) at 0 °C, and the mixture was stirred for 15 min. The reaction mixture was quenched with water. The mixture was extracted with DCM, and dried over anhydrous MgSO₄. The solvent was removed under reduced pressure and Diethyl ether/Hexane was added to the crude product. The precipitated solid was collected by filtration and washed with Hexane to give the product (5.29 g, 73%).

### Example 19: Preparation of OE19

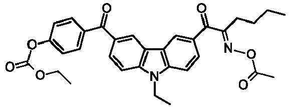

### OE19 is prepared as the following scheme:

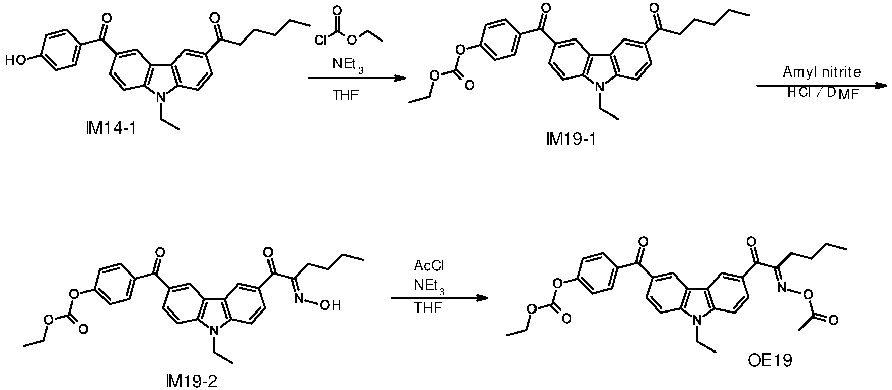

### Preparation of IM19-1

To 7.81g of IM14-1 and 2.16g of ethyl chloroformate in tetrahydrofuran (50ml), 2.11g of triethylamine was added at 0 °C and stirred for 4h. The organic layer was extracted with TBME and dried over anhydrous sodium sulfate. The solvent was removed under vacuum, and the crude was reprecipitated from TBME/hexane to give 8.44g of IM19-1 as pale orange solid.

### Preparation of IM19-2

To 8.44g of IM19-1 was added mixture of DMF (160ml), tetrahydrofuran (110ml) and hydrochloric acid (60ml) at 0 °C. Then, 4.62g of amyl nitrite was added and stirred for 4h at rt. The organic layer was extracted with TBME and washed with brine, then, dried over anhydrous sodium sulfate. The solvent was removed under vacuum, and the crude was purified via column chromatography with hexane and DCM (90:10 to 0:100 v/v) as eluent to give 7.6g of IM19-2 as brown resin.

### Preparation of OE19

To 7.6 of IM19-2 and 4.96g of acetyl chloride in tetrahydrofuran (80ml) was added 6.4g of triethylamine at 0 °C, then stirred at rt for 15h. Water was added to the reaction mixture and organic layer was extracted with ethyl acetate. The organic layer was washed with brine and dried over anhydrous sodium sulfate. The solvent was removed under vacuum, and the crude was purified via column chromatography with hexane/DCM/acetone (90:10:0 to 0:98:2 v/v/v) as eluent to give 1.02g of OE19 as pale yellow solid.

### Example 20: Preparation of OE20

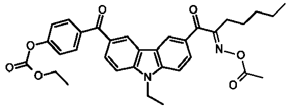

### OE20 is prepared as the following scheme:

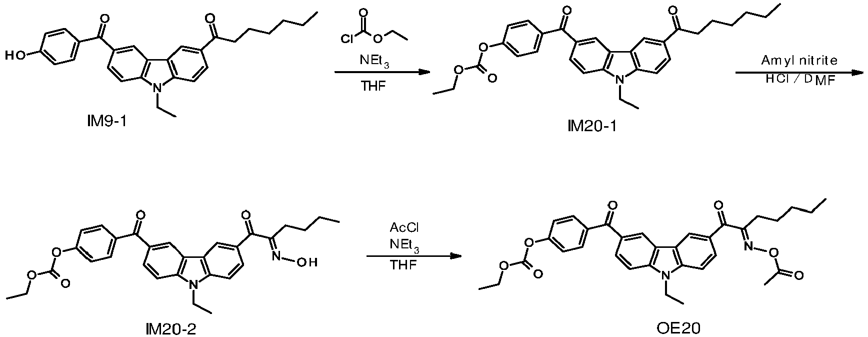

### Preparation of IM20-1

To 0.43g of IM9-1 and 0.11g of ethyl chloroformate in tetrahydrofuran (10ml), 0.12g of triethylamine was added at 0 °C and stirred for 4h. The organic layer was extracted with TBME and dried over anhydrous sodium sulfate. The solvent was removed under vacuum, and the crude was washed with TBME/hexane to give 0.50g of IM20-1 as orange powder.

### Preparation of /M20-2

To 0.50g of IM20-1 was added mixture of DMF (3ml), tetrahydrofuran (3ml) and hydrochloric acid (5ml) at 0°C. Then, 0.32g of amyl nitrite was added and stirred for 4h at rt. The organic layer was extracted with TBME and washed with brine, then, dried over anhydrous sodium sulfate. The solvent was removed under vacuum, and the crude was purified via column chromatography with hexane and acetone (100:0 to 80:20 v/v) as eluent to give 0.34g of IM20-2 as orange resin.

### Preparation of OE20

To 0.34 of IM20-2 and 0.14g of acetyl chloride in tetrahydrofuran (10ml) was added 0.24g of triethylamine at 0 °C, then stirred at rt for 2h. Water was added to the reaction mixture and organic layer was extracted with ethyl acetate. The organic layer was washed with brine and dried over anhydrous sodium sulfate. The solvent was removed under vacuum, and the crude was purified via column chromatography with hexane/DCM/acetone (90:10:0 to 0:99:1 v/v/v) as eluent to give 0.30 of OE20 as yellow resin.

### Example 21: Preparation of OE21

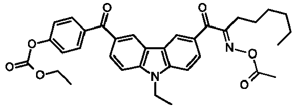

### OE21 is prepared as the following scheme:

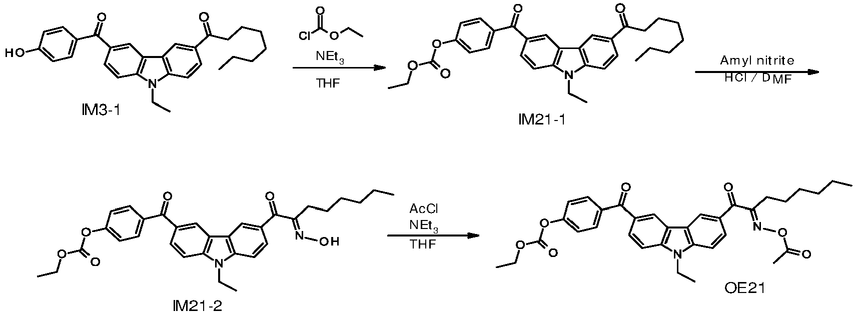

### Preparation of IM21-1

To 4.57g of IM3-1 and 1.14g of ethyl chloroformate in tetrahydrofuran (40ml), 1.14g of triethylamine was added at 0 °C and stirred for 4h. The organic layer was extracted with TBME and dried over anhydrous sodium sulfate. The solvent was removed under vacuum, and the crude was washed with TBME/hexane to give 4.88g of IM21-1 as off-white powder.

### Preparation of IM21-2

To 4.88g of IM21-1 was added mixture of DMF (100ml), tetrahydrofuran (100ml) and hydrochloric acid (30ml) at 0 °C. Then, 2.53g of amyl nitrite was added and stirred for 4h at rt. The organic layer was extracted with TBME and washed with brine, then, dried over anhydrous sodium sulfate. The solvent was removed under vacuum, and the crude was purified via column chromatography with hexane and DCM (90:10 to 0:100 v/v) as eluent to give 2.65g of IM21-2 as yellow resin.

### Preparation of OE21

To 2.65 of IM21-2 and 0.91g of acetyl chloride in tetrahydrofuran (50ml) was added 1.16g of triethylamine at 0 °C, then stirred at rt for 2h. Water was added to the reaction mixture and organic layer was extracted with ethyl acetate. The organic layer was washed with brine and dried over anhydrous sodium sulfate. The solvent was removed under vacuum, and the crude was purified via column chromatography with hexane and acetone (100: 0 to 80:20 v/v) as eluent to give 2.01 of OE21 as yellow resin.

### Example 22: Preparation of OE22

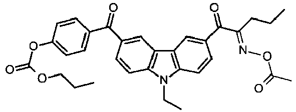

### OE22 is prepared according to the following scheme:

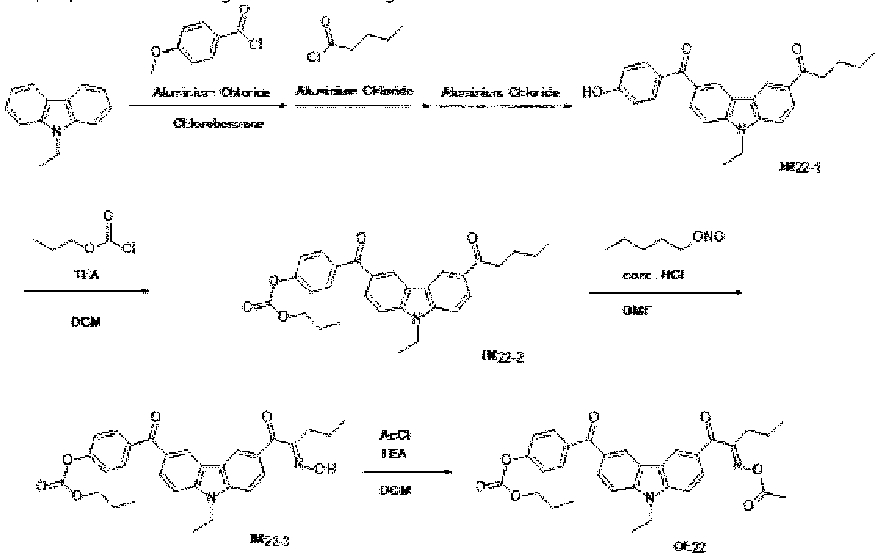

### Preparation of IM22-1

To a solution of 9-ethylcarbazole (10.0 g, 51.2 mmol) and Aluminum Chloride (7.57 g, 56.8 mmol) in Chlorobenzene (300 ml) was added 4-Methoxybenzoyl chloride (8.74 g, 51.2 mmol) at 0 °C, and the mixture was stirred for 1 h. To the reaction mixture was added Aluminum Chloride (7.57 g, 51.2 mmol) and Valeryl Chloride (6.17 g, 51.2 mmol), and the mixture was stirred for 1 h. To the reaction mixture was added Aluminum Chloride (7.57 g, 51.2 mmol), and the mixture was stirred for 3 h at 80 °C. The reaction mixture was quenched with cold water. The reaction mixture was extracted with DCM, and dried over anhydrous MgSO₄. The solvent was removed under reduced pressure to give the product (14.0 g, 68%).

### Preparation of IM22-2

To a solution of IM22-1 (10.0 g, 25.0 mmol) and TEA (3.5 ml, 25 mmol) in DCM (300 ml) was added Propyl Chloroformate (2.8 ml, 25 mmol) at 0 °C, and the mixture was stirred for 15 min at rt. The reaction mixture was quenched with water. The reaction mixture was extracted with DCM, and dried over anhydrous MgSO₄. The solvent was removed under reduced pressure and TBME/Hexane was added to the crude product. The precipitated solid was collected by filtration and washed with Hexane to give the product (11.2 g, 92%).

### Preparation of /M22-3

To a solution of IM22-2 (11.2 g, 23.1 mmol) in DMF (100 ml) was added conc. HCl (2.9 ml, 35 mmol) and Amyl Nitrite (4.6 ml, 35 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and the crude product was purified by silica gel chromatography to give the product (9.00 g, 76%).

### Preparation of OE22

To a solution of the IM22-3 (9.00 g, 17.5 mmol) in DCM (80 ml) was added TEA (2.5 ml, 18 mmol) and AcCl (1.25 ml, 18 mmol) at 0 °C, and the mixture was stirred for 15 min. The reaction mixture was quenched with water. The mixture was extracted with DCM, and dried over anhydrous MgSO₄. Silica gel was added to the solution. The yellow silica gel was removed by cotton filtration, and washed with DCM. The solvent was removed under reduced pressure to give the product (5.96 g, 61%).

### Example 23: Preparation of OE23

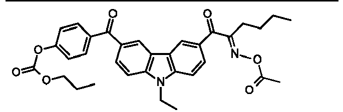

**OE23** is prepared according to the procedure described for **OE22** above using hexanoyl chloride in place of valeryl chloride.

### Example 24: Preparation of OE24

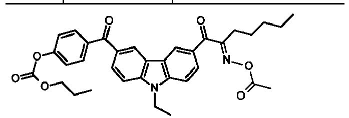

### OE24 is prepared according to the following scheme:

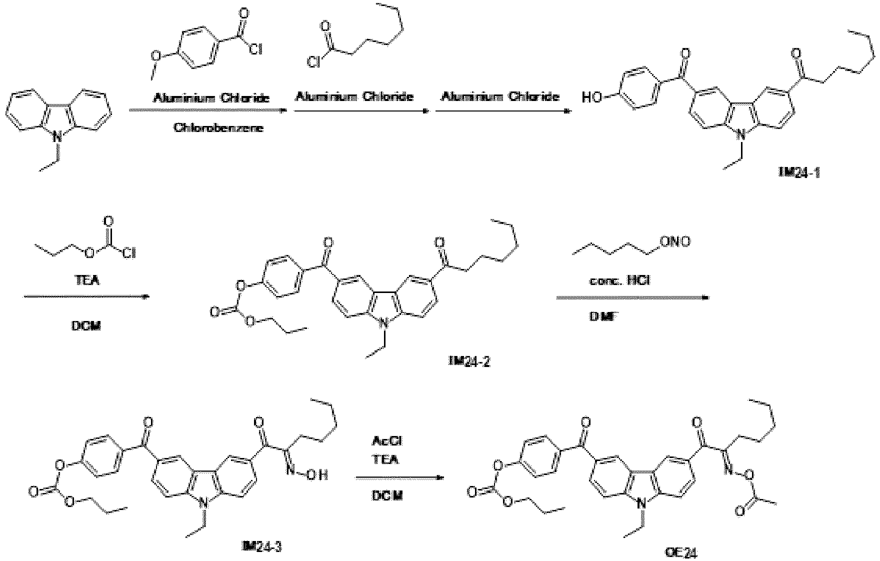

### Preparation of IM24-1

To a solution of 9-ethylcarbazole (10.0 g, 51.2 mmol) and Aluminum Chloride (7.57 g, 56.8 mmol) in Chlorobenzene (300 ml) was added 4-Methoxybenzoyl chloride (8.74 g, 51.2 mmol) at 0 °C, and the mixture was stirred for 1 h. To the reaction mixture was added Aluminum Chloride (7.57 g, 51.2 mmol) and Heptanoyl Chloride (7.61 g, 51.2 mmol), and the mixture was stirred for 1 h. To the reaction mixture was added Aluminum Chloride (7.57 g, 51.2 mmol), and the mixture was stirred for 3 h at 80 °C. The reaction mixture was quenched with cold water. The reaction mixture was extracted with DCM, and dried over anhydrous MgSO₄. The solvent was removed under reduced pressure and Hexane was added to the crude product. The precipitated solid was collected by filtration and washed with DCM/Hexane to give the product (17.8 g, 82%).

### Preparation of IM24-2

To a solution of IM24-1 (6.27 g, 14.7 mmol) and TEA (2.2 ml, 16 mmol) in DCM (70 ml) was added Propyl Chloroformate (2.2 ml, 15 mmol) at 0 °C, and the mixture was stirred for 15 min at rt. The reaction mixture was quenched with water. The reaction mixture was extracted with DCM, and dried over anhydrous MgSO₄. The solvent was removed under reduced pressure and TBME/Hexane was added to the crude product. The precipitated solid was collected by filtration and washed with Hexane to give the product (6.45 g, 85%).

### Preparation of IM24-3

To a solution of IM24-2 (6.45 g, 12.6 mmol) in DMF (120 ml) was added conc. HCl (1.6 ml, 19 mmol) and Amyl Nitrite (2.5 ml, 19 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and TBME/Hexane was added to the crude product. The precipitated solid was collected by filtration and washed with Hexane to give the product (4.40 g, 64%).

### Preparation of OE24

To a solution of the IM24-3 (4.40 g, 8.10 mmol) in DCM (80 ml) was added TEA (1.2 ml, 8.5 mmol) and AcCl (0.61 ml, 8.5 mmol) at 0 °C, and the mixture was stirred for 15 min. The reaction mixture was quenched with water. The mixture was extracted with DCM, and dried over anhydrous MgSO₄. The solvent was removed under reduced pressure and TBME/Hexane was added to the crude product. The precipitated solid was collected by filtration and washed with TBME/Hexane to give the product (2.60 g, 55%).

### Example 25: Preparation of OE25

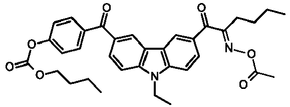

### OE25 is prepared as the following scheme:

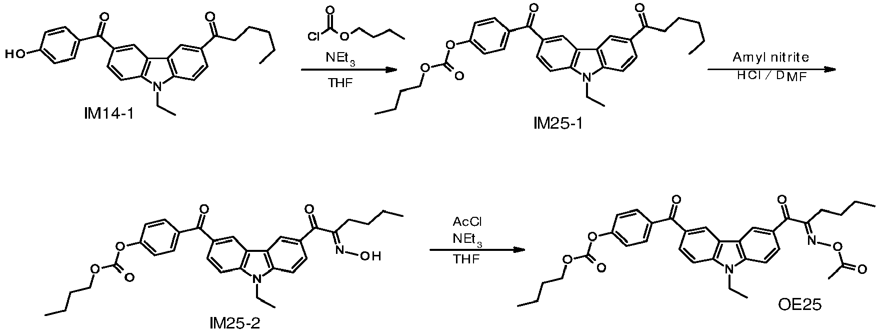

### Preparation of IM25-1

To 4.29g of IM14-1 and 1.49g of butyl chloroformate in tetrahydrofuran (30ml), 1.21g of triethylamine was added at 0 °C and stirred for 4h. The organic layer was extracted with TBME and dried over anhydrous sodium sulfate. The solvent was removed under vacuum, and the crude was washed with TBME/hexane to give 4.50g of IM25-1 as off-white powder.

### Preparation of IM25-2

To 4.47g of IM25-2 was added mixture of DMF (130ml), tetrahydrofuran (40ml) and hydrochloric acid (30ml) at 0 °C. Then, 2.49g of amyl nitrite was added and stirred for 4h at rt. The organic layer was extracted with TBME and washed with brine, then, dried over anhydrous sodium sulfate. The solvent was removed under vacuum, and the crude was purified via column chromatography with hexane and DCM (90:10 to 0:100 v/v) as eluent to give 3.20g of IM25-3 as yellow resin.

### Preparation of OE25

To 3.20 of IM25-2 and 1.30g of acetyl chloride in tetrahydrofuran (50ml) was added 1.78g of triethylamine at 0 °C, then stirred at rt for 2h. Water was added to the reaction mixture and organic layer was extracted with ethyl acetate. The organic layer was washed with brine and dried over anhydrous sodium sulfate. The solvent was removed under vacuum, and the crude was purified via column chromatography with hexane and acetone (100: 0 to 90:10 v/v) as eluent to give 2.21 of OE25 as off-white powder.

### Example 26: Preparation of OE26

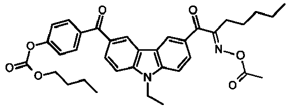

### OE26 is prepared as the following scheme:

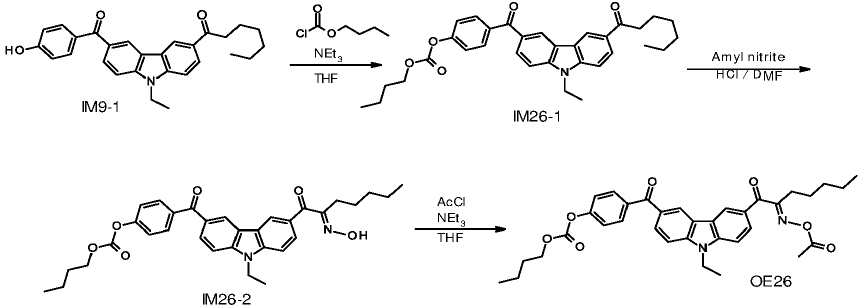

### Preparation of IM26-1

To 4.36g of IM9-1 and 1.41g of butyl chloroformate in tetrahydrofuran (50ml), 1.06g of triethylamine was added at 0 °C and stirred for 4h. The organic layer was extracted with TBME and dried over anhydrous sodium sulfate. The solvent was removed under vacuum, and the crude was washed with TBME/hexane to give 4.69g of IM26-1 as off-white powder.

### Preparation of IM26-2

To 4.69g of IM26-1 was added mixture of DMF (40ml), tetrahydrofuran (40ml) and hydrochloric acid (30ml) at 0 °C. Then, 2.02g of amyl nitrite was added and stirred for 4h at rt. The organic layer was extracted with TBME and washed with brine, then, dried over anhydrous sodium sulfate. The solvent was removed under vacuum, and the crude was purified via column chromatography with hexane and acetone (100:0 to 80:20 v/v) as eluent to give 2.35g of IM26-2 as yellow resin.

### Preparation of OE26

To 2.35 of IM26-2 and 0.31g of acetyl chloride in tetrahydrofuran (40ml) was added 0.41g of triethylamine at 0 °C, then stirred at rt for 2h. Water was added to the reaction mixture and organic layer was extracted with ethyl acetate. The organic layer was washed with brine and dried over anhydrous sodium sulfate. The solvent was removed under vacuum, and the crude was purified via column chromatography with hexane and acetone (100: 0 to 80:20 v/v) as eluent to give 1.17 of OE26 as off-white powder.

### Example 27: Preparation of OE27

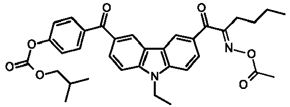

### OE27 is prepared according to the following scheme:

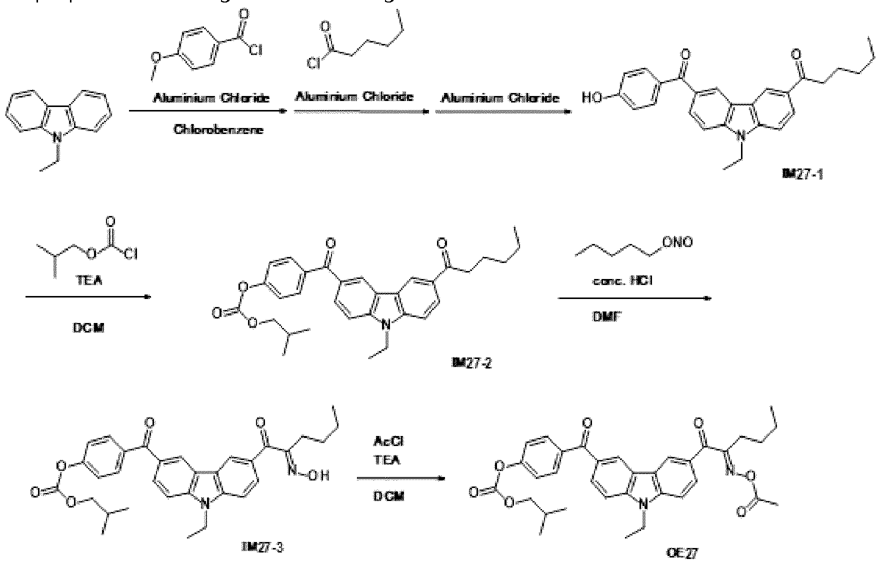

### Preparation of IM27-1

To a solution of 9-ethylcarbazole (10.0 g, 51.2 mmol) and Aluminum Chloride (7.57 g, 56.8 mmol) in Chlorobenzene (200 ml) was added 4-Methoxybenzoyl chloride (8.74 g, 51.2 mmol) at 0 °C, and the mixture was stirred for 1 h. To the reaction mixture was added Aluminum Chloride (7.57 g, 51.2 mmol) and Hexanoyl Chloride (6.89 g, 51.2 mmol), and the mixture was stirred for 1 h. To the reaction mixture was added Aluminum Chloride (7.57 g, 51.2 mmol), and the mixture was stirred for 3 h at 80 °C. The reaction mixture was quenched with cold water. The reaction mixture was extracted with DCM, and dried over anhydrous MgSO₄. The solvent was removed under reduced pressure and Hexane was added to the crude product. The precipitated solid was collected by filtration and dried to give the product (19.5 g, 92%).

### Preparation of IM27-2

To a solution of IM24-1 (4.30 g, 10.4 mmol) and TEA (1.7 ml, 12 mmol) in THF (30 ml) was added Isobutyl Chloroformate (1.4 ml, 10.7 mmol) at 0 °C, and the mixture was stirred for 15 min at rt. The reaction mixture was quenched with water. The reaction mixture was extracted with TBME, and dried over anhydrous MgSO₄. The solvent was removed under reduced pressure and TBME/Hexane was added to the crude product. The precipitated solid was collected by filtration and washed with Hexane to give the product (4.68 g, 88%).

### Preparation of IM27-3

To a solution of IM27-2 (4.63 g, 9.01 mmol) in DMF (22 ml) was added conc. HCl (1.1 ml, 13 mmol) and Amyl Nitrite (1.8 ml, 13 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and the crude product was purified by silica gel chromatography to give the product (2.83 g, 58%).

### Preparation of OE27

To a solution of the IM27-3 (2.83 g, 5.22 mmol) in DCM (26 ml) was added TEA (0.77 ml, 5.5 mmol) and AcCl (0.39 ml, 5.5 mmol) at 0 °C, and the mixture was stirred for 15 min. The reaction mixture was quenched with water. The mixture was extracted with DCM, and dried over anhydrous MgSO₄. The solvent was removed under reduced pressure and TBME/Hexane was added to the crude product. The precipitated solid was collected by filtration and washed with TBME/Hexane to give the product (2.72 g, 89%).

### Example 28: Preparation of OE28

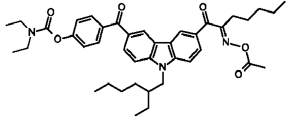

### OE28 is prepared according to the following scheme:

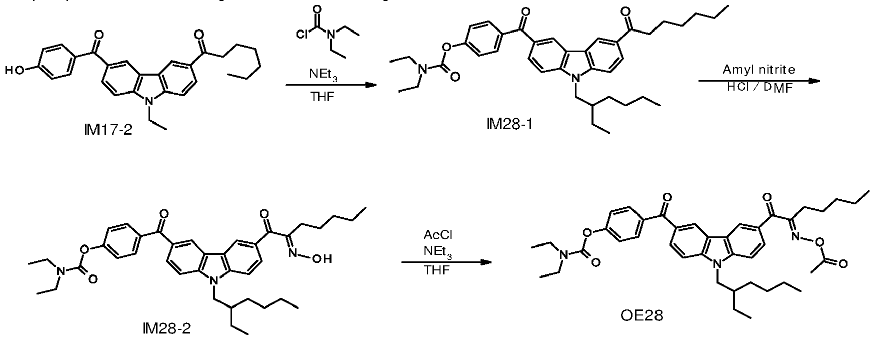

### Preparation of IM28-1

To 10.50g of IM17-2 and 2.79g of diethylcarbamoyl chloride in tetrahydrofuran (80ml), 4.44g of potassium carbonate and pyridine (3ml) was added and stirred at 70 °C for 4h. The organic layer was extracted with TBME and dried over anhydrous sodium sulfate. The solvent was removed under vacuum, and the crude was purified via column chromatography with hexane and DCM (90:10 to 50:50 v/v) as eluent to give 7.36g of IM28-1 as yellow resin.

### Preparation of IM28-2

To 7.36g of IM28-1 was added mixture of DMF (200ml), tetrahydrofuran (150ml) and hydrochloric acid (40ml) at 0 °C. Then, 4.91g of amyl nitrite was added and stirred for 4h at rt. The organic layer was extracted with TBME and washed with brine, then, dried over anhydrous sodium sulfate. The solvent was removed under vacuum, and the crude was purified via column chromatography with hexane and acetone (100:0 to 80:20 v/v) as eluent to give 1.03g of IM28-2 as yellow oil.

### Preparation of OE28

To 1.03 of IM28-2 and 0.77g of acetyl chloride in tetrahydrofuran (30ml) was added 1.02g of triethylamine at 0 °C, then stirred at rt for 2h. Water was added to the reaction mixture and organic layer was extracted with ethyl acetate. The organic layer was washed with brine and dried over anhydrous sodium sulfate. The solvent was removed under vacuum, and the crude was purified via column chromatography with hexane and DCM (100: 0 to 0:100 v/v) as eluent to give 0.81 of OE28 as yellow resin.

### Example 29: Preparation of OE29

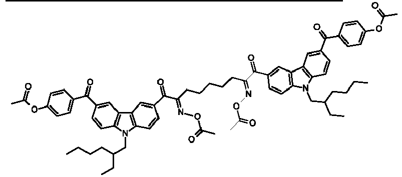

### OE29 is prepared according to the following scheme:

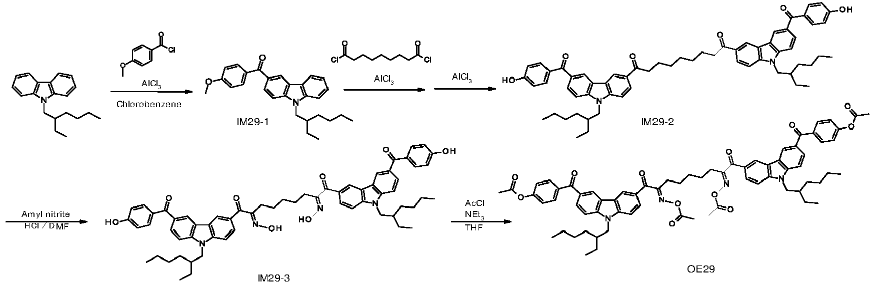

### Preparation of IM29-1

To 17.93g of 9-ethylhexylcarbazole and 8.61g of aluminum chloride in DCM (100ml) was added 10.96g of 4-methoxybenzoyl chloride and stirred at rt for 4h under N₂ flow condition. The reaction mixture was poured into ice-water and extracted with ethyl acetate, then, dried over anhydrous sodium sulfate. The crude was purified via column chromatography with hexane and DCM (100:0 to 50:50 v/v) as eluent to give 6.19g of IM29-1as yellow resin.

### Preparation of IM29-2

To 6.19g of IM29-1 and 1.68g of azelaoyl chloride in chlorobenzene (100ml), 6.09g of potassium carbonate was added and stirred at rt for 3h, then, heated at 100 °C for 4h. The organic layer was extracted with ethyl acetate and dried over anhydrous sodium sulfate. The solvent was removed under vacuum, and the crude was purified via column chromatography with hexane/DCM/acetone (90:10:0 to 0:80:20 v/v/v) as eluent to give 9.90g of IM29-2 as orange resin.

### Preparation of IM29-3

To 3.04g of IM29-3 was added mixture of DMF (100ml), tetrahydrofuran (100ml) and hydrochloric acid (40ml) at 0 °C. Then, 3.02g of amyl nitrite was added and stirred for 4h at rt. The organic layer was extracted with TBME and washed with brine, then, dried over anhydrous sodium sulfate. The solvent was removed under vacuum, and the crude was purified via column chromatography with hexane and acetone (100:0 to 80:20 v/v) as eluent to give 0.86g of IM29-3 as yellow resin.

### Preparation of OE29

To 0.86 of IM29-3 and 1.06g of acetyl chloride in tetrahydrofuran (100ml) was added 1.34g of triethylamine at 0 °C, then stirred at rt for 2h. Water was added to the reaction mixture and organic layer was extracted with ethyl acetate. The organic layer was washed with brine and dried over anhydrous sodium sulfate. The solvent was removed under vacuum, and the crude was purified via column chromatography with hexane/DCM/acetone (90:10:0 to 0:95:5 v/v/v) as eluent to give 0.30 of OE29 as yellow resin.

### Example 30: Preparation of OE30

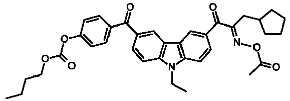

### OE30 is prepared according to following scheme:

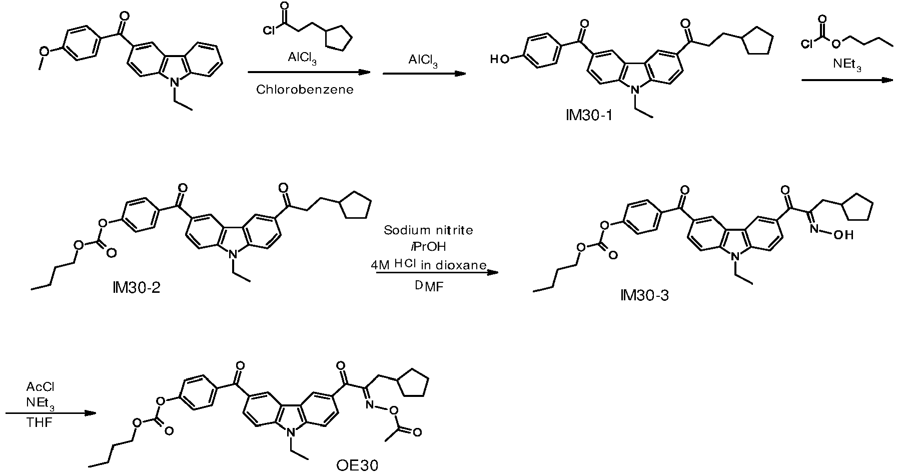

### Preparation of IM30-1

To 2.38g of (9-ethylcarbazol-3-yl)-(4-methoxyphenyl)methanone and 1.18g of 3-cyclopentylpropionyl chloride in chlorobenzene (30ml) was added 3.14g of aluminum chloride and stirred at rt for 16h under N₂ flow condition, then heated at 100 °C for 1h. The reaction mixture was poured into ice-water, and added ethyl acetate and hexane. Then, the precipitate was collected by filtration and washed with mixture of hexane and DCM. The resulting solid was dried in vacuo to give 4.26g of IM30-1 as beige solid.

### Preparation of IM30-2

To 4.26g of IM30-1 and 1.38g of butyl chloroformate in tetrahydrofuran (50ml), 1.05g of triethylamine was added at 0 °C and stirred for 16h. The organic layer was extracted with TBME and dried over anhydrous sodium sulfate. The solvent was removed under vacuum, and the crude was purified via column chromatography with hexane and DCM (90:10 to 0:100 v/v) as eluent to give 1.27g of IM30-2 as off-white resin.

### Preparation of IM30-3

To 1.27g of IM30-2 and 0.80g of sodium nitrite were added mixture of DMF (20ml) and isopropyl alcohol (10ml) at rt. Then, 4M of hydrochloric acid in dioxane solution (10ml) was dropwise and stirred for 20h. The organic layer was extracted with ethyl acetate, then, washed with brine and dried over anhydrous sodium sulfate. The solvent was removed under vacuum, and the crude was purified via column chromatography with hexane and DCM (90:10 to 0:100 v/v) as eluent to give 0.86g of IM30-3 as orange resin.

### Preparation of OE30

To 0.86 of IM30-2 and 0.31g of acetyl chloride in tetrahydrofuran (50ml) was added 0.45g of triethylamine at 0°C, then stirred at room temperature for 16h. Water was added to the reaction mixture and organic layer was extracted with ethyl acetate. The organic layer was washed with brine and dried over anhydrous sodium sulfate. The solvent was removed under vacuum, and the crude was purified via column chromatography with hexane and DCM (90:10 to 0:100 v/v) as eluent to give 0.50g of OE30 as yellow resin.

### Example 31: Preparation of OE31

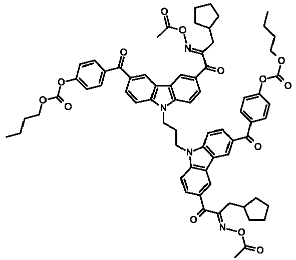

### OE31 is prepared according to the following scheme:

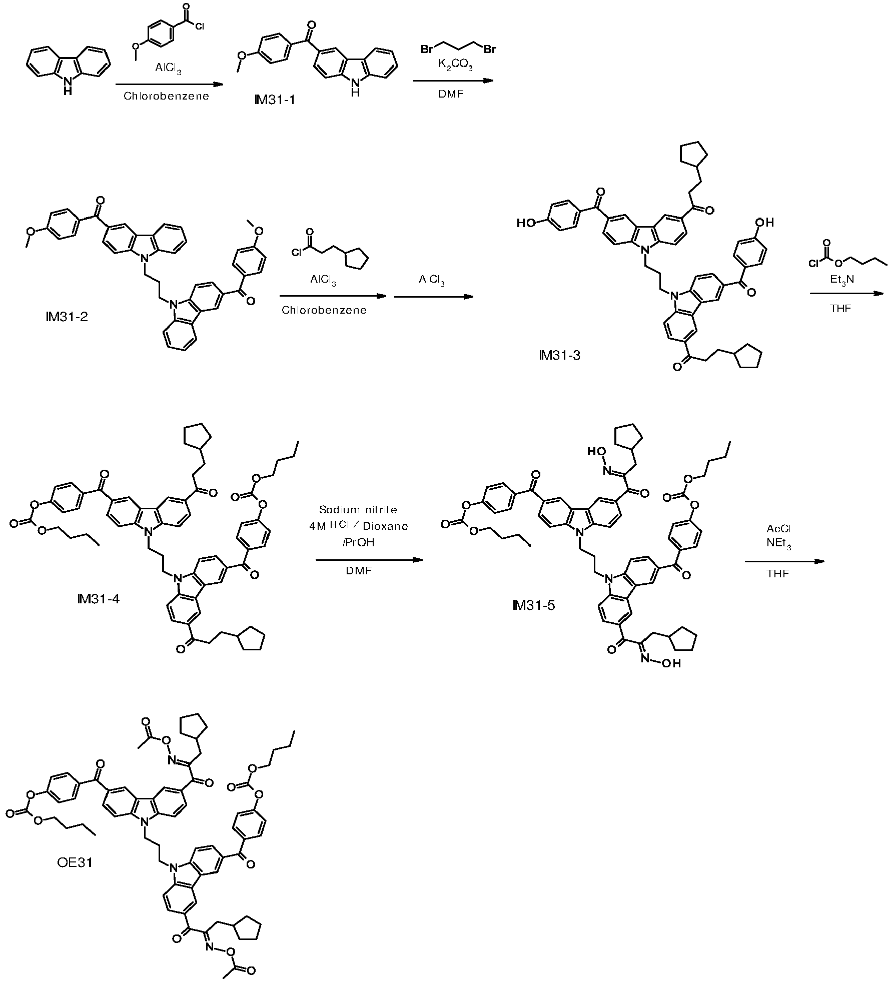

### Preparation of IM31-1

To 10.80g of carbazole and 8.64g of aluminum chloride in DCM (100ml) was added 11.04g of 4-methoxybenzoyl chloride and stirred at rt for 4h under N₂ flow condition. The reaction mixture was poured into ice-water and added hexane, then, precipitation was filtered. The resulting solid was dried in vacuo to give 11.43g of IM31-1 as beige solid.

### Preparation of IM31-2

To 6.57g of IM31-1 and 2.20g of 1,3-dibromopropane in dimethyl sulfoxide (20ml) was added 2.65g of potassium hydroxide (85% purity) at rt for 16h. The organic layer was extracted with ethyl acetate and dried over anhydrous sodium sulfate. The solvent was removed under vacuum, and the crude was purified via column chromatography with DCM and acetone (100:0 to 90:10 v/v) as eluent to give 2.65g of IM31-2 as brown resin.

### Preparation of IM31-3

To 2.65 of IM31-2 and 1.34g of 3-cyclopentylpropionyl chloride in chlorobenzene (30ml) was added 3.89g of aluminum chloride and stirred at rt for 16h under N₂ flow condition, then heated at 100°C for 1h. The reaction mixture was poured into ice-water, and added ethyl acetate and hexane. Then, the precipitate was collected by filtration and washed with the mixture of hexane and DCM. The resulting solid was dried in vacuo to give 2.01g of IM31-3 as off-white powder.

### Preparation of IM31-4

To 2.01g of IM31-3 and 0.77g of butyl chloroformate in tetrahydrofuran (50ml), 0.97g of triethylamine was added at 0 °C and stirred for 16h. The organic layer was extracted with ethyl acetate and dried over anhydrous sodium sulfate. The solvent was removed under vacuum, and the crude was purified via column chromatography with DCM and acetone (100:0 to 95:5 v/v) as eluent to give 1.01g of IM31-4 as pale brown resin.

### Preparation of IM31-5

To 1.01g of IM31-4 and 0.52g of sodium nitrite were added mixture of DMF (20ml) and isopropyl alcohol (10ml) at rt. Then, 4M of hydrochloric acid in dioxane solution (4ml) was dropwise and stirred for 20h. The organic layer was washed with brine and dried over anhydrous sodium sulfate. The solvent was removed under vacuum, and the crude was purified via column chromatography with DCM and acetone (100:0 to 95:5 v/v) as eluent to give 0.46g of IM31-5 as pale yellow resin.

### Preparation of OE31

To 0.46 of IM31-5 and 0.65g of acetyl chloride in tetrahydrofuran (30ml) was added 0.83g of triethylamine at 0 °C, then stirred at room temperature for 16h. Water was added to the reaction mixture and organic layer was extracted with ethyl acetate. The organic layer was washed with brine and dried over anhydrous sodium sulfate. The solvent was removed under vacuum, and the crude was purified via column chromatography with DCM and acetone (100:0 to 98:2 v/v) as eluent to give 0.41g of OE31 as yellow resin.

### Example 32: Preparation of OE32

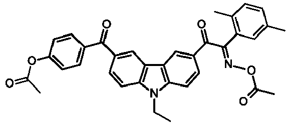

### OE32 is prepared according to the following scheme:

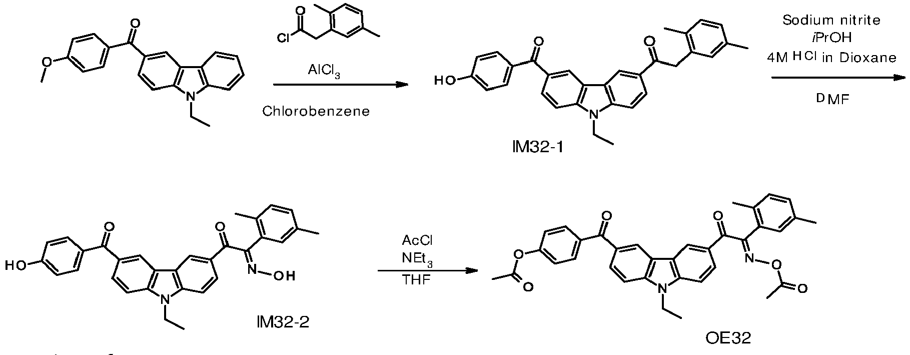

### Preparation of IM32-1

To mixture of 4.42g of (9-ethylcarbazol-3-yl)-(4-methoxyphenyl)methanone and 2.49g of 2,5-dimethylphenylacetyl chloride in chlorobenzene (50ml) was added 6.18g of aluminum chloride and stirred at rt for 16h under N₂ flow condition, then heated at 100 °C for 1h. The reaction mixture was poured into ice-water, and added DCM and hexane. Then, the precipitate was collected by filtration and washed with mixture of hexane and DCM. The resulting solid was dried in vacuo to give 7.23g of IM32-1 as off-white solid.

### Preparation of IM32-2

To 3.99g of IM32-1 and 1.90g of sodium nitrite were added mixture of DMF (50ml) and isopropyl alcohol (10ml) at rt. Then, 4M of hydrochloric acid in dioxane solution (10ml) was dropwise and stirred for 20h. The organic layer was extracted with ethyl acetate, then, washed with brine and dried over anhydrous sodium sulfate. The solvent was removed under vacuum, and the crude was purified via column chromatography with DCM and acetone (100:0 to 97:3 v/v) as eluent to give 1.64g of IM32-2 as yellow solid.

### Preparation of OE32

To 1.64 of IM32-2 and 0.61g of acetyl chloride in tetrahydrofuran (50ml) was added 0.74g of triethylamine at 0 °C, then stirred at room temperature for 16h. Water was added to the reaction mixture and organic layer was extracted with ethyl acetate. The organic layer was washed with brine and dried over anhydrous sodium sulfate. The solvent was removed under vacuum, and the crude was purified via column chromatography with hexane and DCM (90:10 to 0:100 v/v) as eluent to give 1.21g of OE32 as yellow resin.

The product OE32 was obtained mainly in E configuration. Samples of pure E isomer and pure Z isomer were prepared using conventional silica gel chromatography (DCM/Acetone). The Z isomer of OE32 (OE32-Z) can be prepared according to the following scheme:

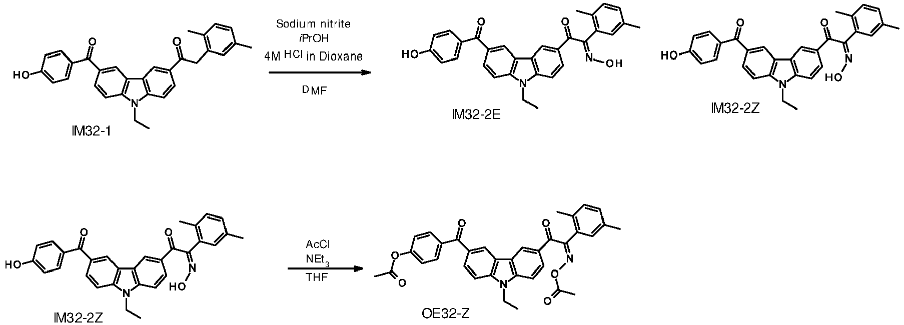

### Preparation of IM32-2Z

To 3.99g of IM32-1 and 1.90g of sodium nitrite were added mixture of DMF (50ml) and isopropyl alcohol (10ml) at rt. Then, 4M of hydrochloric acid in dioxane solution (10ml) was dropwise and stirred for 20h. The organic layer was extracted with ethyl acetate, then washed with brine and dried over anhydrous sodium sulfate. The solvent was removed under vacuum, and the crude product was purified via column chromatography with DCM and acetone (100:0 to 97:3 v/v) as eluent to give 1.64g of IM32-2 as yellow solid. The solid was isolated by filtration and washed with heptane, which was (mainly) composed of E-isomer. The filtrate was purified by silica gel chromatography (DCM/Acetone) to give 0.44 g of IM32-2Z (Z isomer).

### Preparation of OE32-Z

To 0.44g of IM32-2 and 0.61g of acetyl chloride in tetrahydrofuran (13.5ml) was added 0.20g of triethylamine at 0 °C, then stirred at room temperature for 16h. Water was added to the reaction mixture and the organic layer was extracted with ethyl acetate. The organic layer was washed with brine and dried over anhydrous sodium sulfate. The solvent was removed under vacuum, and the crude product was purified via column chromatography with hexane and DCM (90:10 to 0:100 v/v) as eluent to give 0.33g of OE32-Z as yellow resin.

### Example 34: Preparation of OE34

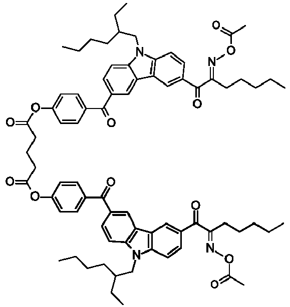

### OE34 is prepared according to the following scheme:

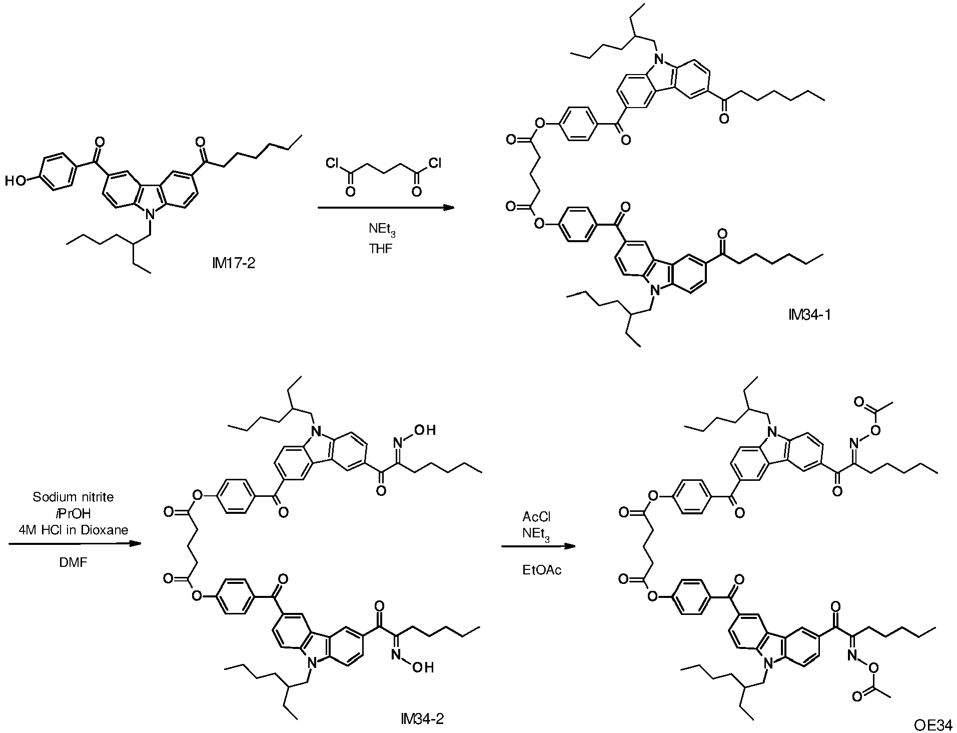

### Preparation of IM34-1

To IM17-2 (7.82g) and glutaryl chloride (1.30g) in THF (25mL) was added triethylamine (1.71g) in THF (5mL) dropwise under cooling with a water bath. After stirring for 30h at room temperature, water and then EtOAc were added to this reaction mixture. The organic layer was washed with K₂CO₃ aq. solution, water and brine, followed by drying over anhydrous MgSO₄. After filtration and concentration, the residue was applied to silica gel column chromatography with CH₂Cl₂-hexane mixture as eluent. IM34-1 was obtained as pale yellow resin (2.81g).

### Preparation of IM34-2

To IM34-1 (2.12g) and sodium nitrite (0.32g) in DMF (6mL) was added isopropyl alcohol (0.4mL). To this reaction mixture was added HCl in dioxane solution (4M, 3.0mL) dropwise at room temperature, and the mixture was stirred for 26.5h. Water and then EtOAc were added to this reaction mixture, and the organic layer was washed with water and brine, followed by drying over anhydrous MgSO₄. After filtration and concentration, the residue was applied to silica gel column chromatography with CH₂Cl₂-acetone mixture as eluent. IM34-2 was obtained as yellow resin (1.41g).

### Preparation of OE34

To IM34-2 (0.47g) and acetyl chloride (0.14g) in EtOAc (6mL) was added triethylamine (0.21g) in EtOAc (2ml) dropwise at room temperature. After stirring for 16.5h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. OE34 was obtained as yellow resin (0.35g).

### Example 35: Preparation of OE35

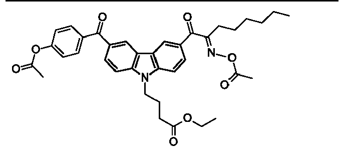

### OE35 is prepared according to the following scheme:

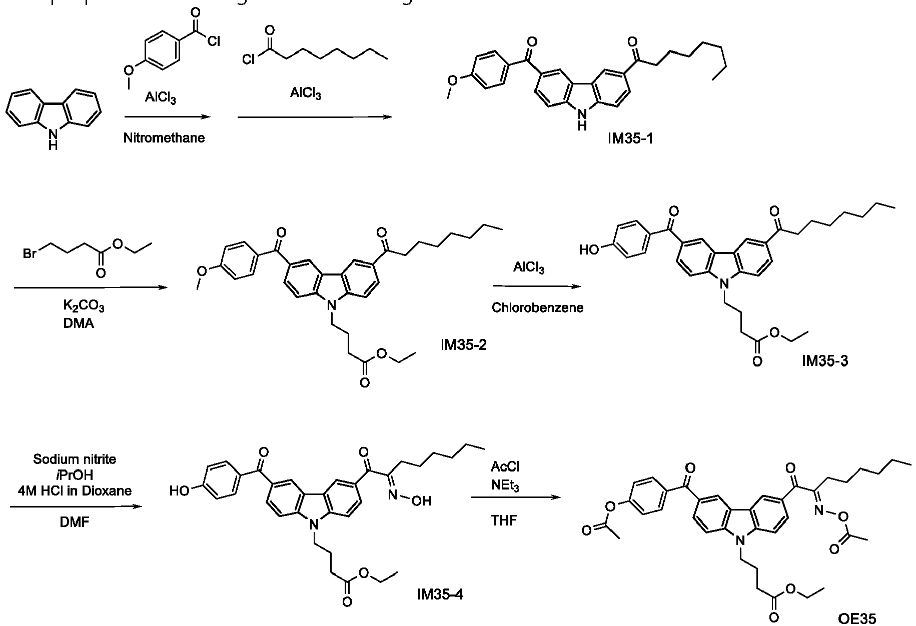

### Preparation of IM35-1

To nitromethane (10mL) were added AlCl₃ (6.69g) and then carbazole (8.37g) in nitromethane (15ml) under cooling with an ice bath, followed by adding 4-methoxybenzoyl chloride (8.54g) dropwise. After stirring for 3h at room temperature, to this reaction mixture were added n-octanoyl chloride (8.04g) in nitromethane (5ml) and then AlCl₃ (7.06g) in portions. The reaction mixture was stirred for 2.5h and then poured onto ice. The product was extracted with EtOAc, and the organic layer was washed with water and brine, followed by drying over anhydrous MgSO₄. After filtration and concentration, the residue was washed with CH₂Cl₂. IM35-1 was obtained as dark yellow solid (11.23g).

### Preparation of IM35-2

To IM35-1 (0.86g) and 4-bromobutyric acid ethyl ester (0.59g) in DMA (5mL) was added K₂CO₃ (0.56g). The reaction mixture was stirred at room temperature for 17.5h. Water was added to this reaction mixture, and the products were extracted with EtOAc. The organic layer was washed with water and brine, followed by drying over anhydrous MgSO₄. After filtration and concentration, the residue was applied to silica gel column chromatography with CH₂Cl₂-acetone mixture as eluent. IM35-2 was obtained as pale yellow resin (0.55g).

### Preparation of IM35-3

IM35-2 (0.54g) and AlCl₃ (0.61g) were combined in chlorobenzene (10mL) and heated at 80 °C for 2h. The reaction mixture was poured onto ice, and the product was extracted with EtOAc. The organic layer was washed with water and brine, followed by drying over anhydrous MgSO₄. After filtration and concentration, the crude product (0.52g) was used for the next reaction without further purification.

### Preparation of IM35-4

To the crude IM35-3 (0.52g) and sodium nitrite (0.088g) in DMF (3mL) was added isopropyl alcohol (0.15mL). To this reaction mixture was added HCl in dioxane solution (4M, 0.8mL) dropwise at room temperature, and the mixture was stirred for 15.5h. Water and then EtOAc were added to this reaction mixture, and the organic layer was washed with water and brine, followed by drying over anhydrous MgSO₄. After filtration and concentration, the residue was applied to silica gel column chromatography with CH₂Cl₂-acetone mixture as eluent. IM35-4 was obtained as yellow resin (0.38g).

### Preparation of OE35

To IM35-4 (0.33g) and acetyl chloride (0.19g) in THF (7mL) was added triethylamine (0.31g) in THF (1mL) dropwise at room temperature. After stirring for 2.5h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. OE35 was obtained as yellow resin (0.31g).

### Example 36: Preparation of OE36

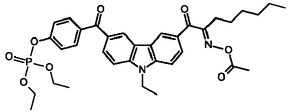

### OE36 is prepared as the following scheme.

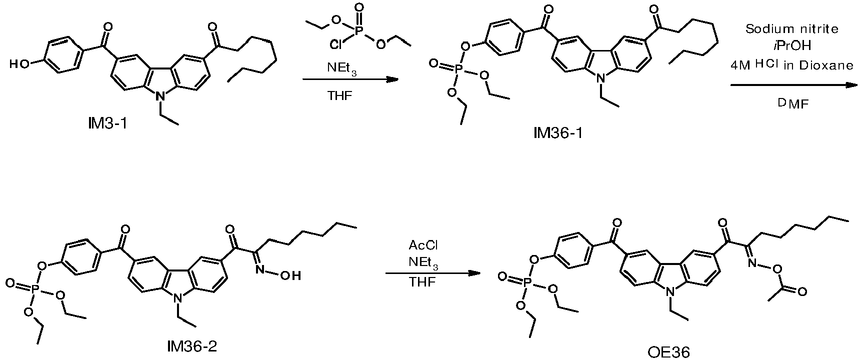

### Preparation of IM36-1

To 4.58g of IM3-1 and 1.80g of diethyl chlorophosphate in tetrahydrofuran (50ml), 1.56g of potassium carbonate and 1ml of pyridine was added and stirred at 70 °C for 16h. The organic layer was extracted with ethyl acetate and dried over anhydrous sodium sulfate. The solvent was removed under vacuum, and the crude was purified via column chromatography with DCM and acetone (100:0 to 97:3 v/v) as eluent to give 4.83g of IM36-1 as yellow resin.

### Preparation of IM36-2

To 4.83g of IM36-1 and 1.09g of sodium nitrite were added mixture of DMF (20ml) and isopropyl alcohol (10ml) at rt. Then, 4M of hydrochloric acid in dioxane solution (10ml) was dropwise and stirred for 20h. The organic layer was extracted with ethyl acetate, then, washed with brine and dried over anhydrous sodium sulfate. The solvent was removed under vacuum, and the crude was purified via column chromatography with DCM and acetone (100:0 to 90:10 v/v) as eluent to give 3.50g of IM36-2 as yellow resin.

### Preparation of OE36

To 3.50 of IM36-2 and 0.51g of acetyl chloride in tetrahydrofuran (100ml) was added 0.62g of triethylamine at 0 °C, then stirred at rt for 16h. Water was added to the reaction mixture and organic layer was extracted with ethyl acetate. The organic layer was washed with brine and dried over anhydrous sodium sulfate. The solvent was removed under vacuum, and the crude was purified via column chromatography with hexane and acetone (100: 0 to 97:3 v/v) as eluent to give 2.06 of OE36 as yellow resin.

The product OE36 was obtained mainly in E configuration. Samples of pure E isomer and pure Z isomer were prepared using conventional silica gel chromatography (DCM/Acetone). The Z isomer of OE36 (OE36-Z) can be prepared according to the following scheme:

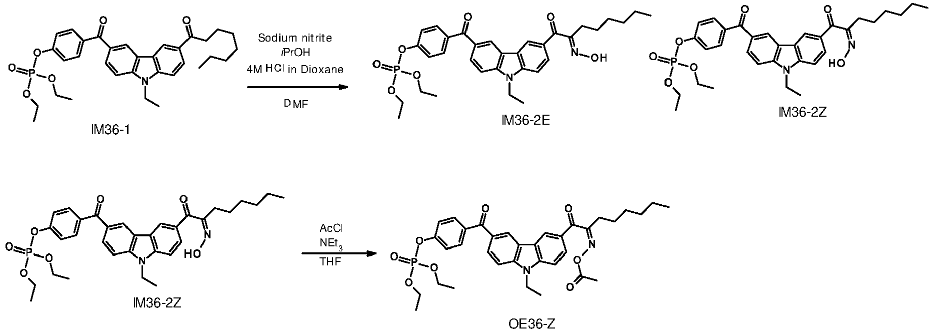

### Preparation of IM36-2Z

To 4.83g of IM36-1 and 1.09g of sodium nitrite were added mixture of DMF (20ml) and isopropyl alcohol (10ml) at rt. Then, 4M of hydrochloric acid in dioxane solution (10ml) was dropwise and stirred for 20h. The organic layer was extracted with ethyl acetate, then washed with brine and dried over anhydrous sodium sulfate. The solvent was removed under vacuum, and the crude product was purified via column chromatography with DCM and acetone (100:0 to 90:10 v/v) as eluent to give 0.50g of IM36-2Z as yellow resin.

### Preparation of OE36-Z

To 0.50g of IM36-2Z and 0.07g of acetyl chloride in tetrahydrofuran (15ml) was added 0.09g of triethylamine at 0 °C, then stirred at rt for 16h. Water was added to the reaction mixture and organic layer was extracted with ethyl acetate. The organic layer was washed with brine and dried over anhydrous sodium sulfate. The solvent was removed under vacuum, and the crude product was purified via column chromatography with hexane and acetone (100: 0 to 97:3 v/v) as eluent to give 0.50g of OE36-Z as yellow resin.

### Example 37: Preparation of OE37

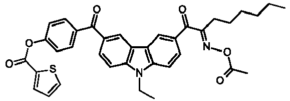

### OE37 is prepared according to the following scheme:

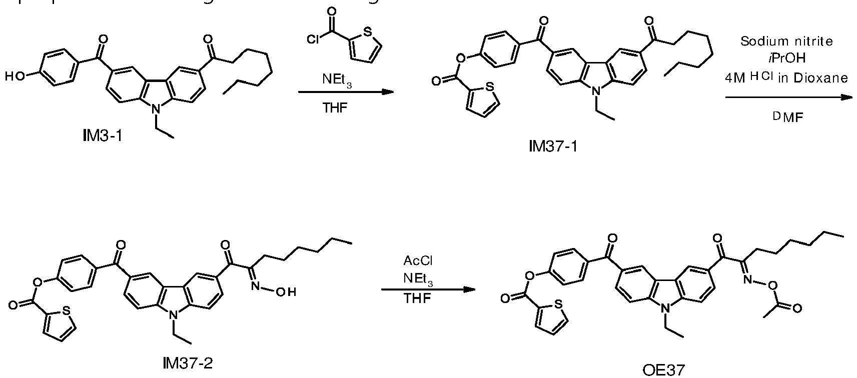

### Preparation of IM37-1

To 4.76g of IM3-1 and 1.60g of 2-thenoyl chloride in tetrahydrofuran (50ml), 1.61g of potassium carbonate and 1ml of pyridine was added and stirred at 70 °C for 4h. The organic layer was extracted with ethyl acetate and dried over anhydrous sodium sulfate. The solvent was removed under vacuum, and 5.61g of crude IM37-1 was obtained as off-white solid.

### Preparation of IM37-2

To 4.83g of IM37-1 and 1.95g of sodium nitrite were added mixture of DMF (50ml) and isopropyl alcohol (10ml) at rt. Then, 4M of hydrochloric acid in dioxane solution (20ml) was dropwise and stirred for 20h. The organic layer was extracted with ethyl acetate, then, washed with brine and dried over anhydrous sodium sulfate. The solvent was removed under vacuum, and the crude was purified via column chromatography with DCM and acetone (100:0 to 97:3 v/v) as eluent to give 2.04of IM37-2 as orange resin.

### Preparation of OE37

To 2.04 of IM37-2 and 0.34g of acetyl chloride in tetrahydrofuran (50ml) was added 0.40g of triethylamine at 0 °C, then stirred at rt for 16h. Water was added to the reaction mixture and organic layer was extracted with ethyl acetate. The organic layer was washed with brine and dried over anhydrous sodium sulfate. The solvent was removed under vacuum, and the crude was purified via column chromatography with hexane and acetone (100: 0 to 99:1 v/v) as eluent to give 1.94 of OE37 as yellow resin.

### Example 38: Preparation of OE38

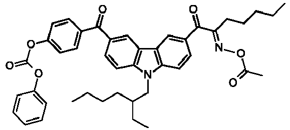

### OE38 is prepared according to the following scheme:

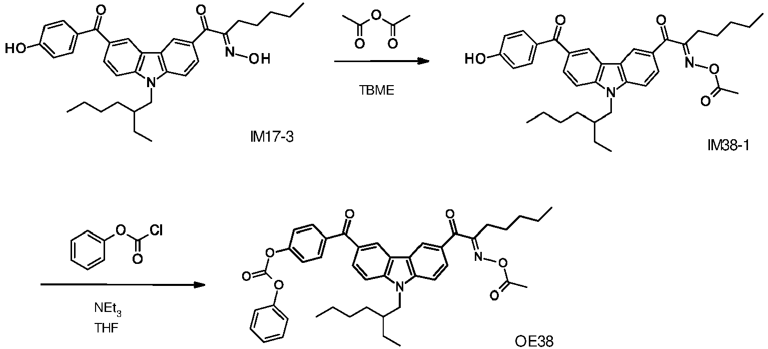

### Preparation of IM38-1

To IM17-3 (13.02g) in t-butyl methyl ether (80mL) was added acetic anhydride (4.93g) in t-butyl methyl ether (10mL) at room temperature. After stirring for 17h, the resulting precipitate was collected by filtration and washed with t-butyl methyl ether. IM38-1 was obtained as white solid (10.54g).

### Preparation of OE38

To IM38-1 (0.58g) and phenyl chloroformate (0.19g) in THF (5mL) was added triethylamine (0.14g) in THF (1mL) dropwise at room temperature. After stirring for 16h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. OE38 was obtained as yellow resin (0.31g).

### Example 39: Preparation of OE39

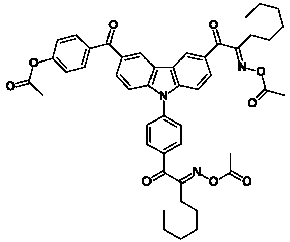

### OE39 is prepared according to the following scheme:

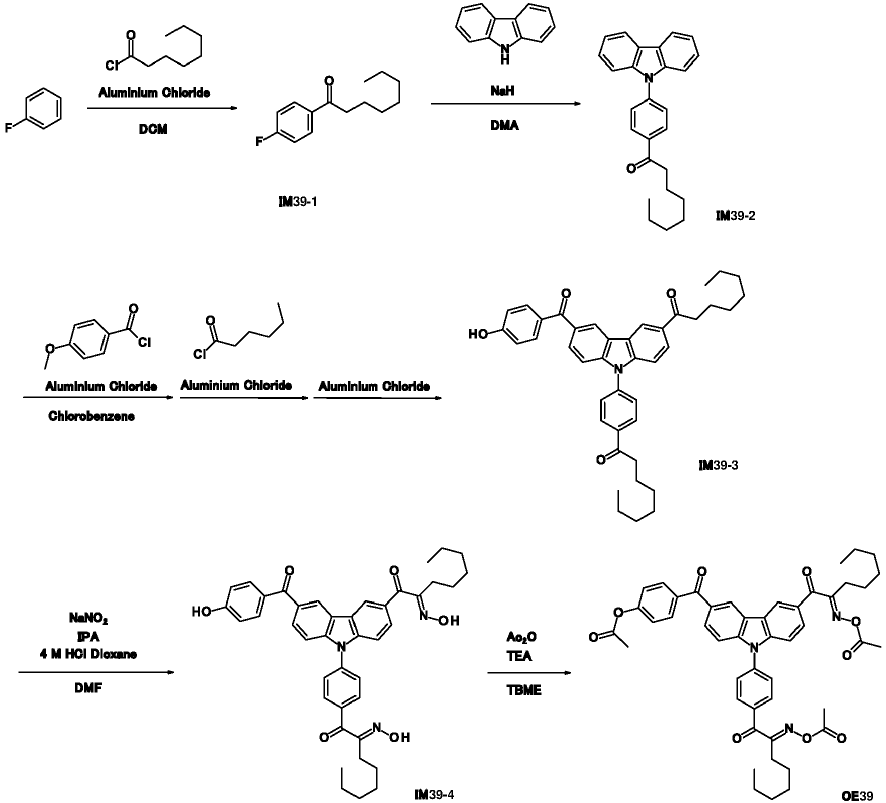

### Preparation of IM39-1

To a solution of Fluorobenzene (10.0 g, 104 mmol) and Aluminum Chloride (13.9 g, 104 mmol) in DCM (400 ml) was added Octanoyl chloride (16.9 g, 104 mmol) at 0 °C, and the mixture was stirred for 3 days at rt. The reaction mixture was quenched with cold water. The reaction mixture was extracted with DCM, and dried over anhydrous MgSO₄. Silica gel and Potassium carbonate was added to the solution. The inorganic salts were removed by cotton filtration, and washed with DCM. The solvent was removed under reduced pressure to give the product (18.0 g, 78%).

### Preparation of IM39-2

To a solution of Carbazole (4.66 g, 27.9 mmol) and IM39-1 (6.20 g, 27.9 mmol) in DMA (250 ml) was added NaH 60% in oil (1.34 g, 33.5 mmol) at 0 °C, and the mixture was stirred for overnight at 120 °C. The reaction mixture was quenched with cold water. The reaction mixture was extracted with EtOAc, washed with brine, and dried over anhydrous MgSO₄. The solvent was removed under reduced pressure and the crude product was purified by silica gel chromatography to give the product (5.66 g, 55%).

### Preparation of IM39-3

To a solution of IM39-2 (5.66 g, 15.3 mmol) and Aluminum Chloride (4.2 g, 13.6 mmol) in DCM (150 ml) was added 4-Methoxybenzoyl chloride (2.61 g, 15.3 mmol) at 0 °C, and the mixture was stirred for 30 min. To the reaction mixture was added Octanoyl Chloride (2.6 ml, 15.2 mmol) and Aluminum Chloride (2.1 g, 15.8 mmol), and the mixture was stirred for 30 min. To the reaction mixture was added Aluminum Chloride (2.1 g, 15.8 mmol), and the mixture was stirred for overnight at the reflux temperature. The reaction mixture was quenched with cold water. The reaction mixture was extracted with DCM, and dried over anhydrous MgSO₄. The solvent was removed under reduced pressure and the crude product was purified by silica gel chromatography to give the product (6.50 g, 69%).

### Preparation of IM39-4

To a solution of IM39-3 (3.03 g, 4.92 mmol) in DMF (25 ml) was added Isopropyl alcohol (1.1 ml, 14 mmol), Sodium nitrite (0.85 g, 12 mmol) and 4 M HCl Dioxane (7.4 ml, 30 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and the crude product was purified by silica gel chromatography to give the product (2.25 g, 68%).

### Preparation of OE39

To a solution of IM39-4 (2.25 g, 3.34 mmol) in TBME (100 ml) was added TEA (1.5 ml, 11 mmol) and Acetic anhydride (1.0 ml, 11 mmol) at 0 °C, and the mixture was stirred for overnight at rt. The reaction mixture was quenched by water. The mixture was extracted with TBME, washed with brine, and dried over anhydrous MgSO₄. The solvent was removed under reduced pressure and the crude product was purified by silica gel chromatography to give the product (1.72 g, 64%).

### Example 40: Preparation of OE40

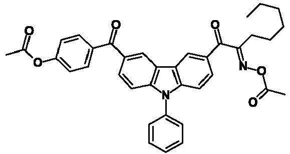

### OE40 is prepared according to the following scheme:

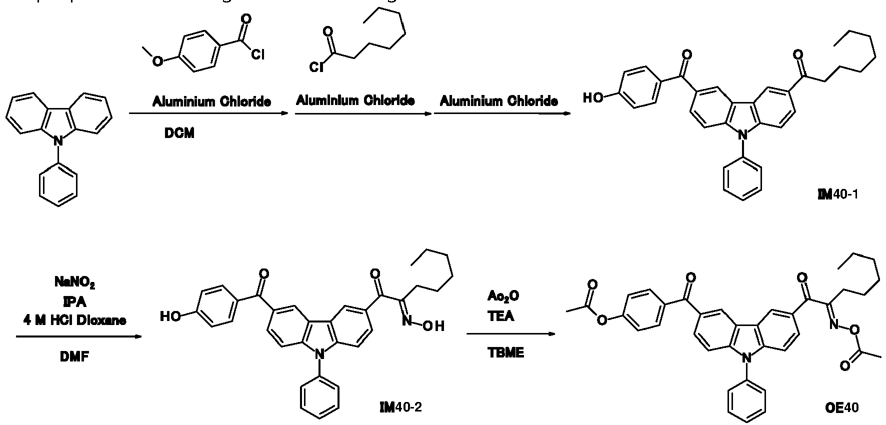

### Preparation of IM40-1

To a solution of 9-Phenylcarbazole (5.00 g, 20.5 mmol) and Aluminum Chloride (3.01 g, 22.6 mmol) in DCM (200 ml) was added 4-Methoxybenzoyl chloride (3.50 g, 20.5 mmol) at 0 °C, and the mixture was stirred for 30 min. To the reaction mixture was added Aluminum Chloride (3.01 g, 22.6 mmol) and Octanoyl Chloride (3.33 g, 20.5 mmol), and the mixture was stirred for 30 min. To the reaction mixture was added Aluminum Chloride (3.01 g, 22.6 mmol), and the mixture was stirred for overnight at the reflux temperature. The reaction mixture was quenched with cold water. The reaction mixture was extracted with DCM, and dried over anhydrous MgSO₄. The solvent was removed under reduced pressure and the crude product was purified by silica gel chromatography to give the product (4.01 g, 40%).

### Preparation of IM40-2

To a solution of IM40-1 (2.00 g, 4.09 mmol) in DMF (20 ml) was added Isopropyl alcohol (0.44 ml, 5.7 mmol), Sodium nitrite (0.34 g, 4.93 mmol) and 4 M HCl Dioxane (3.0 ml, 12 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and the crude product was purified by silica gel chromatography to give the product (1.62 g, 76%).

### Preparation of OE40

To a solution of IM40-2 (1.62 g, 3.12 mmol) in TBME (30 ml) was added TEA (0.92 ml, 6.6 mmol) and Acetic anhydride (0.62 ml, 6.6 mmol) at 0 °C, and the mixture was stirred for overnight at rt. The reaction mixture was quenched by water. The mixture was extracted with TBME, washed with brine, and dried over anhydrous MgSO₄. The solvent was removed under reduced pressure and the crude product was purified by silica gel chromatography to give the product (4.16 g, 78%).

### Example 41: Preparation of OE41

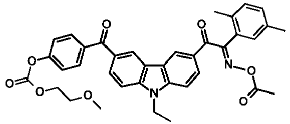

### OE41 is prepared according to the following scheme:

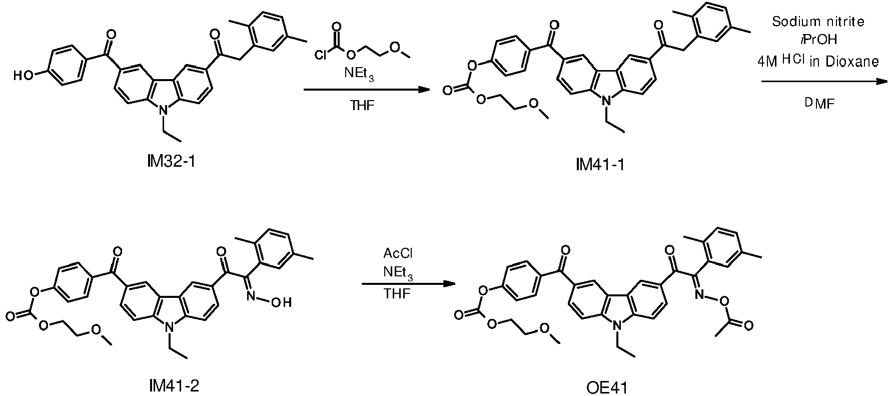

### Preparation of IM41-1

To 3.24g of IM32-1 and 0.97g of 2-methoxyethyl chloroformate in mixture of tetrahydrofuran (20ml) and DMF (10ml), 0.74g of triethylamine was added at 0 °C and stirred for 16h. The organic layer was extracted with ethyl acetate and dried over anhydrous sodium sulfate. The solvent was removed under vacuum, and 1.66g of crude IM41-1 was obtained as orange resin.

### Preparation of IM41-2

To 1.66g of crude IM41-1 and 1.01g of sodium nitrite were added mixture of DMF (20ml) and isopropyl alcohol (10ml) at rt. Then, 4M of hydrochloric acid in dioxane solution (6ml) was dropwise and stirred for 20h. The organic layer was washed with brine and dried over anhydrous sodium sulfate. The solvent was removed under vacuum, and the crude was purified via column chromatography with DCM and acetone (100:0 to 97:3 v/v) as eluent to give 1.31g of IM41-2 as yellow resin.

### Preparation of OE41

To 1.31 of IM41-2 and 0.28g of acetyl chloride in tetrahydrofuran (30ml) was added 0.35g of triethylamine at 0 °C, then stirred at room temperature for 16h. Water was added to the reaction mixture and organic layer was extracted with ethyl acetate. The organic layer was washed with brine and dried over anhydrous sodium sulfate. The solvent was removed under vacuum, and the crude was purified via column chromatography with hexane and DCM (90:10 to 0:100 v/v) as eluent to give 0.90g of OE41 as yellow resin.

### Example 42: Preparation of OE42

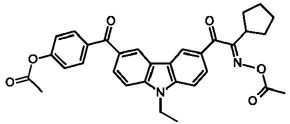

### OE42 is prepared as the following scheme.

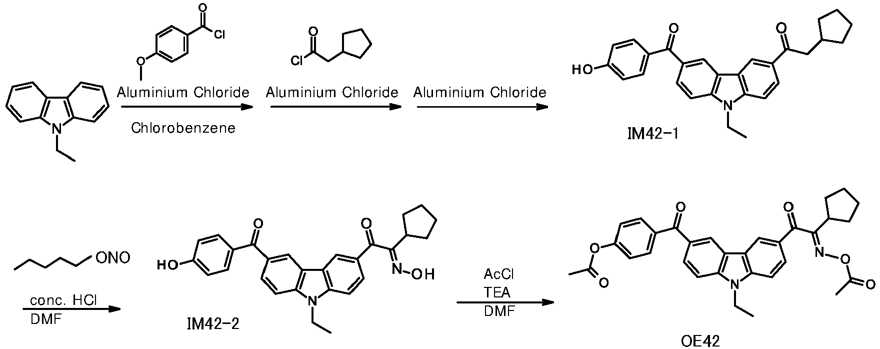

### Preparation of IM42-1

To a solution of 9-ethylcarbazole (0.98 g, 5 mmol) and Aluminum Chloride (0.73 g, 5.5 mmol) in Chlorobenzene (20 ml) was added 4-Methoxybenzoyl chloride (0.85 g, 5mmol) at 0 °C, and the mixture was stirred for 1 h. To the reaction mixture was added Aluminum Chloride (1.46 g, 11 mmol) and cyclopentylacetyl chloride (0.74 g, 5 mmol), and the mixture was stirred for 1 h. Then, the mixture was stirred for 3 h at 80 °C. The reaction mixture was quenched with cold water. The reaction mixture was extracted with DCM, and dried over anhydrous MgSO₄. The solvent was removed under reduced pressure to give the product (1.8 g, 86%).

### Preparation of IM42-2

To a solution of IM42-1 (0.9g, 2.0 mmol) in DMF (10 ml) was added conc. HCl (3.6 ml, 6 mmol) and Amyl Nitrite (0.35g, 6 mmol) at 0 °C, and the mixture was stirred for overnight at rt. The reaction mixture was quenched with cold water. The reaction mixture was extracted with EtOAc, washed with brine, and dried over anhydrous MgSO₄. The solvent was removed under reduced pressure and DCM/Hexane was added to the crude product. The precipitated solid was collected by filtration and washed with DCM/Hexane to give the product (0.92 g, 98%).

### Preparation of OE42

To a solution of the IM42-2 (0.92g, 2 mmol) in AcOEt (15ml) was added TEA (0.7g, 7 mmol) and Ac₂O (0.6g, 6 mmol) at rt, and the mixture was stirred for 2h. The reaction mixture was quenched with water. The mixture was extracted with DCM, and dried over anhydrous MgSO₄. The solvent was removed under reduced pressure and the crude product was purified by silica gel chromatography to give the product (0.6 g, 60%).

### Example 44: Preparation of OE44

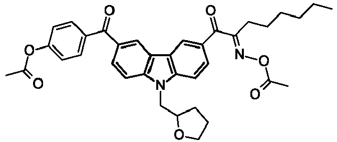

### OE44 is prepared according to the following scheme:

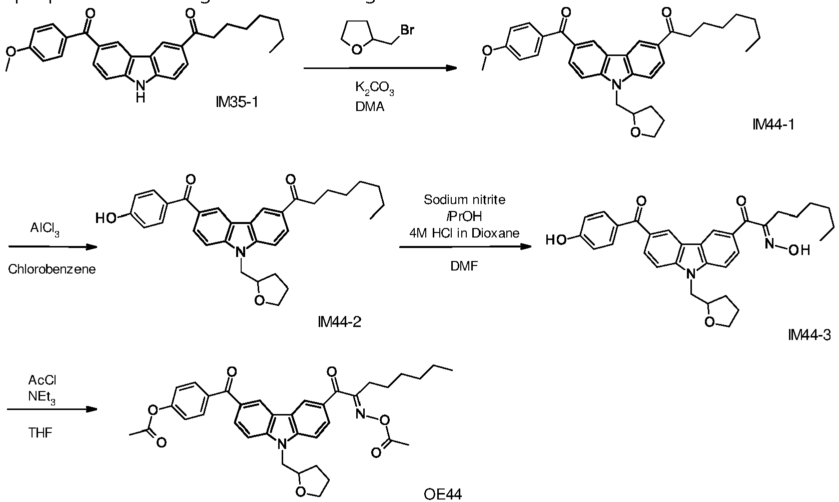

### Preparation of IM44-1

To IM35-1 (1.28g) and tetrahydrofurfuryl bromide (1.19g) in DMA (11mL) was added K₂CO₃ (0.56g). The reaction mixture was stirred at room temperature overnight and then heated at 120 °C for 4.5h. Water was added to this reaction mixture, and the products were extracted with EtOAc. The organic layer was washed with water and brine, followed by drying over anhydrous MgSO₄. After filtration and concentration, the residue was applied to silica gel column chromatography with CH₂Cl₂-acetone mixture as eluent. IM44-1 was obtained as white solid (1.27g).

### Preparation of IM44-2

IM44-1 (1.25g) and AlCl₃ (1.46g) were combined in chlorobenzene (10mL) and heated at 80°C for 2h. The reaction mixture was poured onto ice, and the product was extracted with EtOAc. The organic layer was washed with water and brine, followed by drying over anhydrous MgSO₄. After filtration and concentration, the residue was washed with TBME-hexane mixture to give IM44-2 as white solid (1.11g).

### Preparation of IM44-3

To IM44-2 (1.11g) and sodium nitrite (0.27g) in DMF (6.3mL) was added isopropyl alcohol (0.35mL). To this reaction mixture was added HCl in dioxane solution (4M, 2.5mL) dropwise at room temperature, and the mixture was stirred for 27h. Water and then EtOAc were added to this reaction mixture, and the organic layer was washed with water and brine, followed by drying over anhydrous MgSO₄. After filtration and concentration, the residue was applied to silica gel column chromatography with CH₂Cl₂-acetone mixture as eluent. IM44-3 was obtained as yellow resin (0.61g).

### Preparation of OE44

To IM44-3 (0.59g) and acetyl chloride (0.36g) in THF (9mL) was added triethylamine (0.59g) in THF (2mL) dropwise at room temperature. After stirring for 2h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. OE44 was obtained as yellow resin (0.48g).

### Example 45: Preparation of OE45

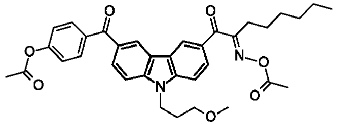

### OE45 is prepared according to the following scheme:

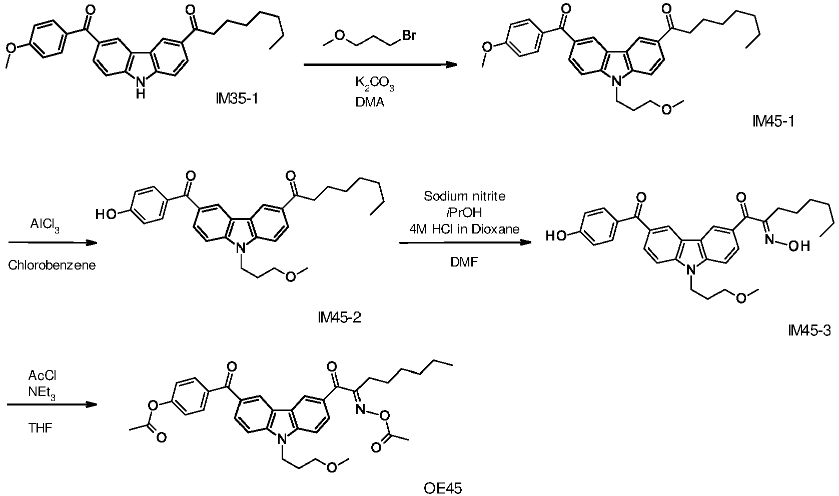

### Preparation of IM45-1

To IM35-1 (1.28g) and 1-bromo-3-methoxypropane (0.96g) in DMA (10mL) was added K₂CO₃ (0.89g). The reaction mixture was heated at 120 °C for 3h. Water was added to this reaction mixture, and the products were extracted with EtOAc. The organic layer was washed with water and brine, followed by drying over anhydrous MgSO₄. After filtration and concentration, the residue was applied to silica gel column chromatography with CH₂Cl₂-acetone mixture as eluent. IM45-1 was obtained as yellow resin (1.03g).

### Preparation of IM45-2

IM45-1 (1.00g) and AlCl₃ (1.18g) were combined in chlorobenzene (10mL) and heated at 80 °C for 2h. The reaction mixture was poured onto ice, and the product was extracted with EtOAc. The organic layer was washed with water and brine, followed by drying over anhydrous MgSO₄. After filtration and concentration, the residue was washed with TBME to give IM45-2 as pale beige solid (0.83g).

### Preparation of IM45-3

To IM45-2 (0.83g) and sodium nitrite (0.15g) in DMF (4.0mL) was added isopropyl alcohol (0.26mL). To this reaction mixture was added HCl in dioxane solution (4M, 1.3mL) dropwise at room temperature, and the mixture was stirred for 23h. Water and then EtOAc were added to this reaction mixture, and the organic layer was washed with water and brine, followed by drying over anhydrous MgSO₄. After filtration and concentration, the residue was applied to silica gel column chromatography with CH₂Cl₂-acetone mixture as eluent. IM45-3 was obtained as yellow resin (0.64g).

### Preparation of OE45

To IM45-3 (0.63g) and acetyl chloride (0.39g) in THF (9mL) was added triethylamine (0.63g) in THF (3mL) dropwise at room temperature. After stirring for 2h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. OE45 was obtained as yellow resin (0.56g).

### Example 46: Preparation of OE46

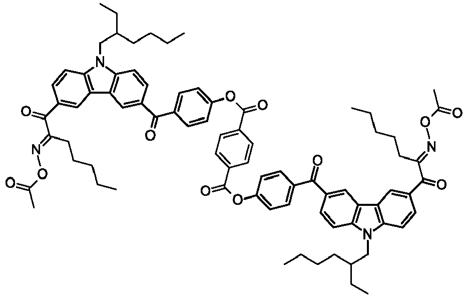

### OE46 is prepared according to the following scheme:

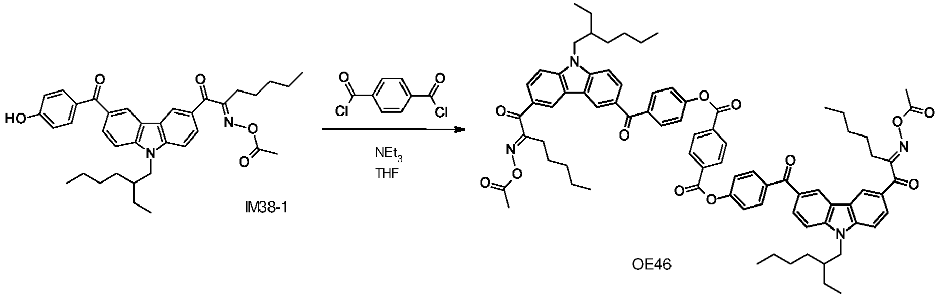

### Preparation of OE46

To IM38-1 (0.71g) and terephthaloyl chloride (0.13g) in THF (5mL) was added triethylamine (0.15g) in THF (1mL) dropwise at room temperature. After stirring for 22.5h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. OE46 was obtained as yellow resin (0.61g).

### Example 47: Preparation of OE47

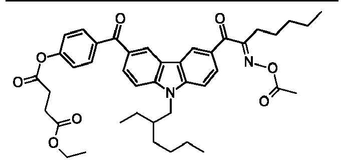

### OE47 is prepared according to the following scheme:

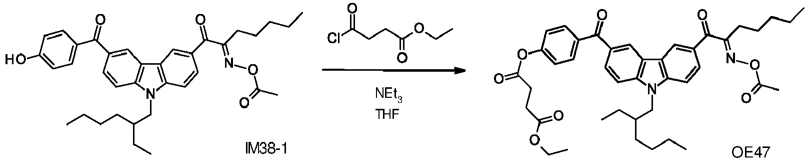

### Preparation of OE47

To IM38-1 (0.59g) and ethyl succinyl chloride (0.21g) in THF (5mL) was added triethylamine (0.14g) in THF (1mL) dropwise at room temperature. After stirring for 26.5h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. OE47 was obtained as yellow resin (0.53g).

### Example 48: Preparation of OE48

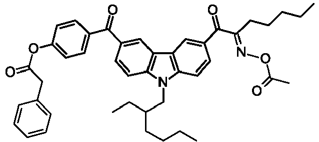

### OE48 is prepared according to the following scheme:

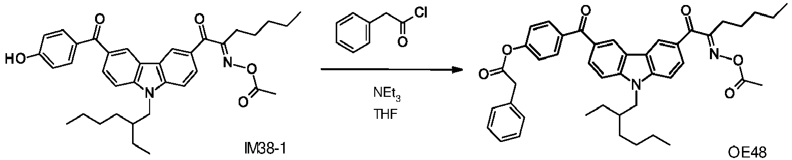

### Preparation of OE48

To IM38-1 (0.58g) and phenylacetyl chloride (0.19g) in THF (5mL) was added triethylamine (0.13g) in THF (1mL) dropwise at room temperature. After stirring for 26h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. OE48 was obtained as yellow resin (0.57g).

### Example 49: Preparation of OE49

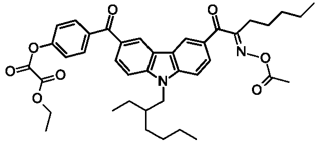

### OE49 is prepared according to the following scheme:

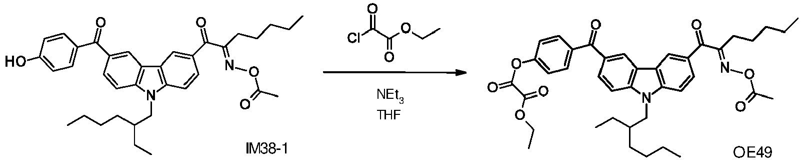

### Preparation of OE49

To IM38-1 (0.58g) and ethyl chloroglyoxylate (0.17g) in THF (5mL) was added triethylamine (0.14g) in THF (1mL) dropwise at room temperature. After stirring for 26h, water and then EtOAc were added to this reaction mixture. The organic layer was washed with water and brine, followed by drying over anhydrous MgSO₄. After filtration and concentration, the residue was solidified from TBME-hexane mixture under cooling with a dry ice-acetone bath. The resulting solid was washed with hexane to give OE48 as white solid (0.34g).

### Example 50: Preparation of OE50

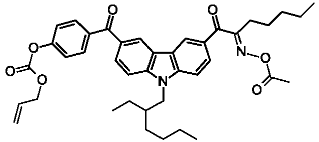

### OE50 is prepared according to the following scheme:

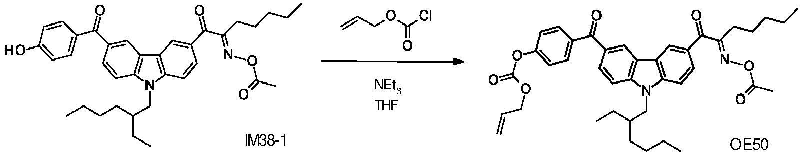

### Preparation of OE50

To IM38-1 (0.59g) and allyl chloroformate (0.15g) in THF (5mL) was added triethylamine (0.14g) in THF (1mL) dropwise at room temperature. After stirring for 16h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. OE50 was obtained as yellow resin (0.57g).

### Example 51: Preparation of OE51

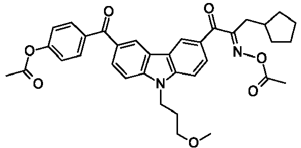

### OE51 is prepared according to the following scheme.

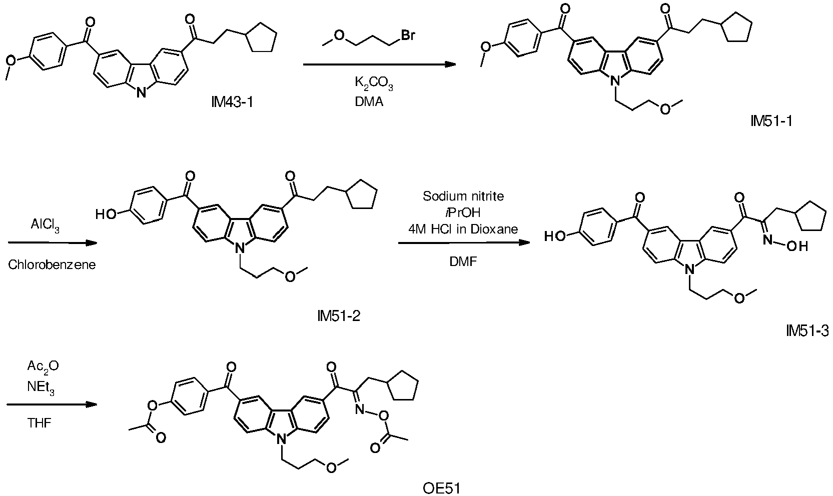

### Preparation of IM51-1

To IM43-1 (4.27g) and 1-bromo-3-methoxypropane (2.30g) in DMA (20mL) was added K₂CO₃ (2.22g). The reaction mixture was heated at 120 °C for 21h. Water was added to this reaction mixture, and the products were extracted with EtOAc. The organic layer was washed with water and brine, followed by drying over anhydrous MgSO₄. After filtration and concentration, the residue was applied to silica gel column chromatography with CH₂Cl₂-acetone mixture as eluent. IM51-1 was obtained as white solid (2.58g).

### Preparation of IM51-2

IM51-1 (2.58g) and AlCl₃ (3.05g) were combined in chlorobenzene (20mL) and heated at 80 °C for 2h. The reaction mixture was poured onto ice, and the product was extracted with EtOAc. The organic layer was washed with water and brine, followed by drying over anhydrous MgSO₄. After filtration and concentration, the residue was applied to silica gel column chromatography with CH₂Cl₂-acetone mixture as eluent. IM51-2 was obtained as white solid (2.39g).

### Preparation of IM51-3

To IM51-2 (2.38g) and sodium nitrite (0.41g) in DMF (9.8mL) was added isopropyl alcohol (0.75mL). To this reaction mixture was added HCl in dioxane solution (4M, 4.0mL) dropwise at room temperature, and the mixture was stirred for 4.5h. Water and then EtOAc were added to this reaction mixture, and the organic layer was washed with water and brine, followed by drying over anhydrous MgSO₄. After filtration and concentration, the residue was applied to silica gel column chromatography with CH₂Cl₂-acetone mixture as eluent. The collected solid was further washed with TBME-hexane mixture to give IM51-3 as pale yellow solid (1.72g).

### Preparation of OE51

To IM51-3 (0.44g) in THF (4mL) were added acetic anhydride (0.20g) in THF (1mL) and then triethylamine (0.21g) in THF (1mL) dropwise at room temperature. After stirring for 21.5h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. OE51 was obtained as pale yellow solid (0.42g).

### Example 52: Preparation of OE52

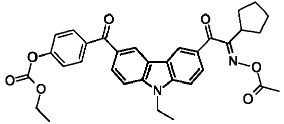

### OE52 is prepared according to the following scheme:

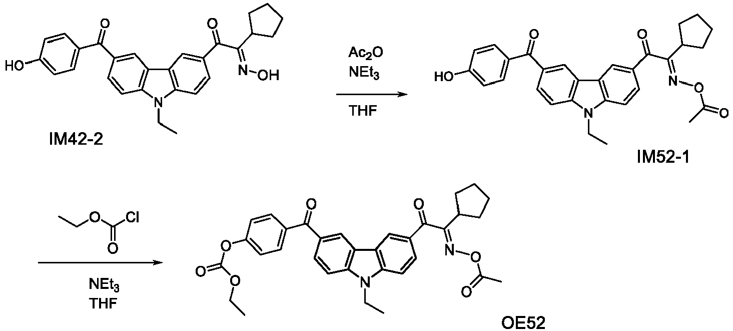

### Preparation of IM52-1

To IM42-2 (0.52g) in THF (5mL) was added acetic anhydride (0.24g) in THF (1mL) at room temperature. After stirring for 21h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. IM52-1 was obtained as yellow resin (0.48g).

### Preparation of OE52

To IM52-1 (0.47g) and ethyl chloroformate (0.16g) in THF (6mL) was added triethylamine (0.17g) in THF (1mL) dropwise at room temperature. After stirring for 20h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. OE52 was obtained as pale yellow solid (0.46g).

The product OE52 was obtained mainly in E configuration. Samples of pure E isomer and pure Z isomer were prepared using conventional silica gel chromatography (DCM/Acetone). The Z isomer of OE52 (OE52-Z) can be prepared according to the following scheme:

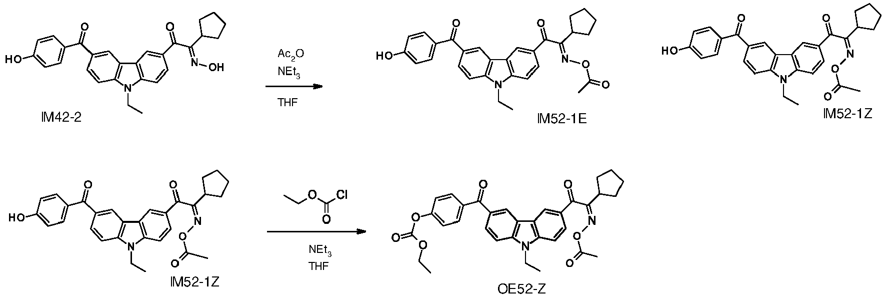

### Preparation of IM52-1Z

To IM42-2 (0.52g), which was (mainly) composed of E-isomer, in THF (5mL) was added acetic anhydride (0.24g) in THF (1mL) at room temperature. After stirring for 21h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. IM52-1Z was obtained as yellow resin (0.11g).

### Preparation of OE52-Z

To IM52-1Z (0.11g) and ethyl chloroformate (0.04g) in THF (1.5mL) was added triethylamine (0.04g) in THF (1mL) dropwise at room temperature. After stirring for 20h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. OE52-Z (Z isomer) was obtained as pale yellow solid (0.11g)

### Example 53: Preparation of OE53

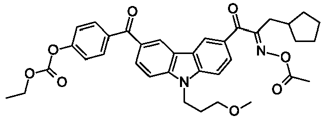

### OE53 is prepared according to the following scheme:

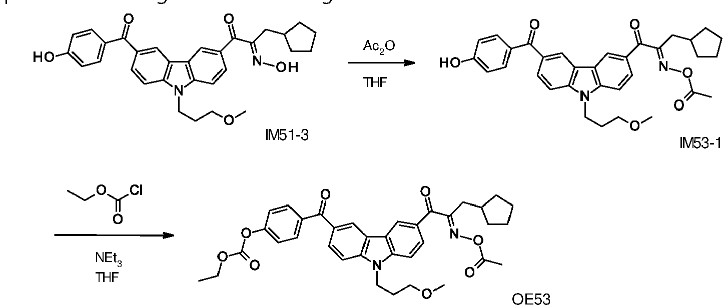

### Preparation of IM53-1

To IM51-3 (1.28g) in THF (15mL) was added acetic anhydride (0.78g) in THF (2mL) at room temperature. After stirring for 41h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. IM53-1 was obtained as yellow solid (1.13g).

### Preparation of OE53

To IM53-1 (0.33g) and ethyl chloroformate (0.099g) in THF (4mL) was added triethylamine (0.10g) in THF (1mL) dropwise at room temperature. After stirring for 16.5h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. OE53 was obtained as pale yellow resin (0.31g).

### Example 54: Preparation of OE54

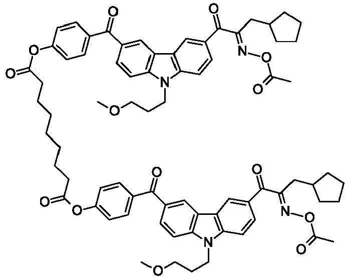

### OE54 is prepared according to the following scheme:

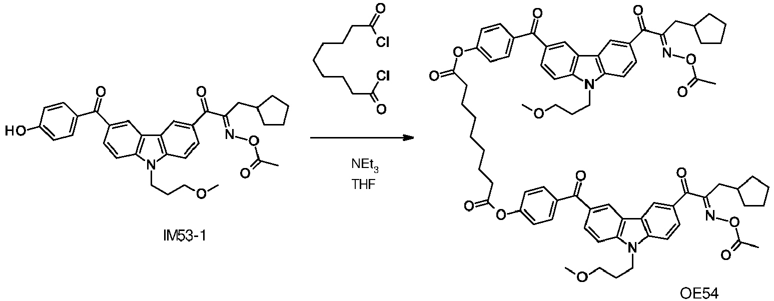

### Preparation of OE54

To IM53-1 (0.81g) and azelaoyl chloride (0.17g) in THF (5mL) was added triethylamine (0.19g) in THF (1mL) dropwise at room temperature. After stirring for 16.5h, water and then EtOAc were added to this reaction mixture. The organic layer was washed with water and brine, followed by drying over anhydrous MgSO₄. After filtration and concentration, the residue was applied to silica gel column chromatography with CH₂Cl₂-acetone mixture as eluent. OE53 was obtained as yellow resin (0.45g).

### Example 56: Preparation of OE56

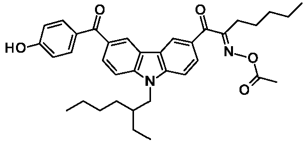

**OE56** is the same as IM36-1.

### Compound list and physical data thereof

| Oxime ester (OE) | Structure | ¹H NMR in CDCl₃ |
|---|---|---|
| OE1 | 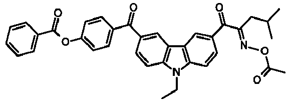 | 1.00 (d, 6H), 1.50 (t, 3H), 2.02-2.12 (m, 1H), 2.28 (s, 3H), 2.80 (d, 2H), 4.45 (q, 2H), 7.40 (d, 2H), 7.48-7.58, (m, 4H), 7.68 (t, 1H), 7.96 (d, 2H), 8.08 (d, 1H), 8.25 (d, 2H), 8.34 (d, 1H), 8.70 (s, 1H), 8.94 (s, 1H) |
| OE2 | 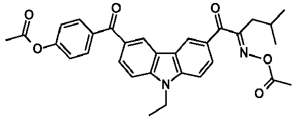 | 0.98 (d, 3H), 1.48 (t, 3H), 2.28 (s, 3H), 2.35 (s, 3H), 2.80 (d, 2H), 4.45 (q, 2H), 7.28 (d, 2H), 7.50, (d, 2H), 7.90 (d, 2H), 8.05 (d, 1H), 8.34 (d, 1H), 8.68 (s, 1H), 8.90 (s, 1H) |
| OE3 | 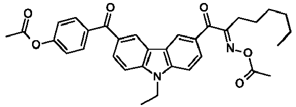 | 0.86 (t, 3H), 1.26-1.68 (m, 11H), 2.29 (s, 3H), 2.37 (s, 3H), 2.85 (t, 2H), 4.46 (q, 2H), 7.28 (d, 2H), 7.51, (dd, 2H), 7.92 (d, 2H), 8.06 (dd, 1H), 8.31 (dd, 1H), 8.67 (s, 1H), 8.87 (s, 1H) |
| OE4 | 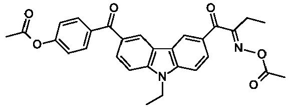 | 0.88 (t, 3H), 1.50 (t, 3H), 2.29 (s, 3H), 2.37 (s, 3H), 2.87 (q, 2H), 4.46 (q, 2H), 7.28 (d, 2H), 7.51, (dd, 2H), 7.92 (d, 2H), 8.06 (dd, 1H), 8.30 (dd, 1H), 8.66 (s, 1H), 8.87 (s, 1H) |
| OE5 | 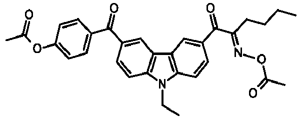 | 0.90 (t, 3H), 1.36-1.65 (m, 8H), 2.28 (s, 3H), 2.35 (s, 3H), 2.86 (t, 2H), 4.45 (q, 2H), 7.28 (d, 2H), 7.50, (d, 2H), 7.90 (d, 2H), 8.05 (d, 1H), 8.30 (d, 1H), 8.65 (s, 1H), 8.88 (s, 1H) |
| OE6 | 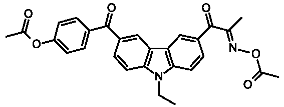 | 1.50 (t, 3H), 2.28 (s, 3H), 2.35 (s, 3H), 2.37 (s, 3H), 4.50 (q, 2H), 7.28 (d, 2H), 7.50, (d, 2H), 7.90 (d, 2H), 8.05 (d, 1H), 8.30 (d, 1H), 8.65 (s, 1H), 8.88 (s, 1H) |
| OE7 | 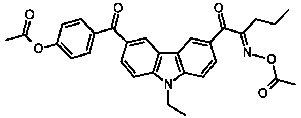 | 1.00 (t, 3H), 1.48 (t, 3H), 1.60-1.72 (m, 2H), 2.28 (s, 3H), 2.35 (s, 3H), 2.84 (t, 2H), 4.45 (q, 2H), 7.28 (d, 2H), 7.50, (d, 2H), 7.90 (d, 2H), 8.05 (d, 1H), 8.30 (d, 1H), 8.65 (s, 1H), 8.88 (s, 1H) |
| OE8 | 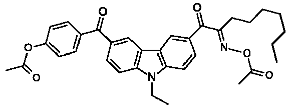 | 0.85 (t, 3H), 1.25-1.68 (m, 13H), 2.29 (s, 3H), 2.37 (s, 3H), 2.85 (t, 2H), 4.46 (q, 2H), 7.28 (d, 2H), 7.51, (dd, 2H), 7.92 (d, 2H), 8.06 (dd, 1H), 8.31 (dd, 1H), 8.67 (s, 1H), 8.87 (s, 1H) |
| OE9 | 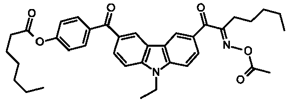 | 0.85 (t, 3H), 0.92 (t, 3H), 1.30-1.50 (m, 10H), 1.50 (t, 3H), 1.55-1.65 (m, 2H), 1.75-1.82 (m, 2H), 2.28 (s, 3H), 2.60 (t, 2H), 2.85 (t, 2H), 4.45 (q, 2H), 7.28 (d, 2H), 7.50, (d, 2H), 7.90 (d, 2H), 8.05 (d, 1H), 8.30 (d, 1H), 8.65 (s, 1H), 8.86 (s, 1H) |
| OE10 | 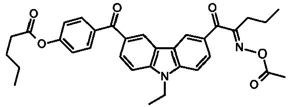 | 0.98 (t, 3H), 1.02 (t, 3H), 1.45-1.54 (m, 5H), 1.60-1.70 (m, 2H), 1.75-1.82 (m, 2H), 2.28 (s, 3H), 2.60 (t, 2H), 2.85 (t, 2H), 4.50 (q, 2H), 7.28 (d, 2H), 7.50, (d, 2H), 7.90 (d, 2H), 8.05 (d, 1H), 8.30 (d, 1H), 8.65 (s, 1H), 8.86 (s, 1H) |
| OE11 | 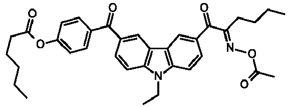 | 0.90 (t, 3H), 0.95 (t, 3H), 1.45-1.52 (m, 9H), 1.55-1.62 (m, 2H), 1.75-1.85 (m, 2H), 2.28 (s, 3H), 2.60 (t, 2H), 2.85 (t, 2H), 4.50 (q, 2H), 7.28 (d, 2H), 7.50, (d, 2H), 7.90 (d, 2H), 8.05 (d, 1H), 8.30 (d, 1H), 8.65 (s, 1H), 8.86 (s, 1H) |
| OE12 | 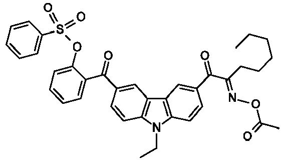 | 0.85 (t, 3H), 1.25-1.30 (m, 4H), 1.35-1.45 (m, 2H), 1.50 (t, 3H), 1.60-1.70 (m, 2H), 2.30 (s, 3H), 2.85 (t, 2H), 4.42 (q, 2H), 7.28 (t, 2H), 7.38-7.52 (m, 6H), 7.54-7.60 (m, 3H), 7.87 (dd, 1H), 8.30 (dd, 1H), 8.35 (d, 1H), 8.82 (d, 1H) |
| OE13 | 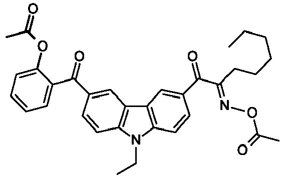 | 0.85 (t, 3H), 1.25-1.30 (m, 4H), 1.35-1.45 (m, 2H), 1.50 (t, 3H), 1.65-1.75 (m, 2H), 1.96 (s, 3H), 2.30 (s, 3H), 2.85 (t, 2H), 4.42 (q, 2H), 7.28 (d, 1H), 7.38 (t, 1H), 7.45 (dd, 2H), 7.56 (dd, 2H), 8.00 (d, 1H), 8.28 (d, 1H), 8.64 (s, 1H), 8.84 (s,1H) |
| OE14 | 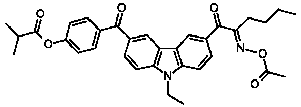 | 0.92 (t, 3H), 1.35 (d, 6H), 1.35-1.45 (m, 2H), 1.50 (t, 3H), 1.65-1.75 (m, 2H), 2.28 (s, 3H), 2.80-2.90 (m, 3H), 4.50 (q, 2H), 7.28 (d, 2H), 7.50, (d, 2H), 7.90 (d, 2H), 8.05 (d, 1H), 8.30 (d, 1H), 8.65 (s, 1H), 8.86 (s, 1H) |
| OE15 | 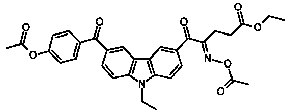 | 1.15 (t, 3H), 1.48 (t, 3H), 2.26 (s, 3H), 2.35 (s, 3H), 2.72 (t, 2H), 3.14 (t, 2H), 4.05 (q, 2H), 4.50 (q, 2H), 7.28 (d, 2H), 7.50, (d, 2H), 7.90 (d, 2H), 8.05 (d, 1H), 8.30 (d, 1H), 8.65 (s, 1H), 8.88 (s, 1H) |
| OE16 | 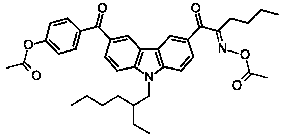 | 0.87 (t, 3H), 0.94 (t, 6H), 1.20-1.50 (m, 10H), 1.56-1.67 (m, 2H), 2.01-2.12 (m, 1H), 2.29 (s, 3H), 2.37 (s, 3H), 2.86 (dd, 2H), 4.25 (d, 2H), 7.27 (d, 2H), 7.48, (d, 2H), 7.92 (d, 2H), 8.05 (dd, 1H), 8.30 (dd, 1H), 8.66 (d, 1H), 8.87 (d, 1H) |
| OE17 (E) | 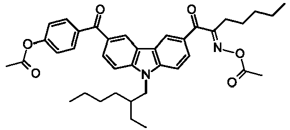 | 0.85 (t, 3H), 0.88 (t, 3H), 0.94 (t, 3H), 1.20-1.45 (m, 12H), 1.60-1.68 (m, 2H), 2.02-2.10 (m, 1H), 2.28 (s, 3H), 2.36 (s, 3H), 2.84 (t, 2H), 4.50 (d, 2H), 7.28 (d, 2H), 7.48, (d, 2H), 7.90 (d, 2H), 8.05 (d, 1H), 8.30 (d, 1H), 8.65 (s, 1H), 8.86 (s, 1H) |
| OE17 (Z) | 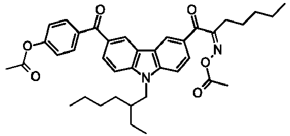 | 0.86 (t, 3H), 0.87 (t, 3H), 0.95 (t, 3H), 1.20-1.45 (m, 12H), 1.60-1.70 (m, 2H), 2.02-2.10 (m, 1H), 2.36 (s, 3H), 2.38 (s, 3H), 2.70 (t, 2H), 4.27 (d, 2H), 7.28 (d, 2H), 7.52, (d, 2H), 7.92 (d, 2H), 8.02 (d, 1H), 8.09 (d, 1H), 8.63 (s, 1H), 8.64 (s, 1H) |
| OE18 | 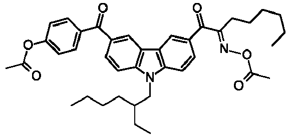 | 0.85 (t, 3H), 0.86 (t, 3H), 0.92 (t, 3H), 1.20-1.45 (m, 16H), 1.58-1.65 (m, 2H), 2.02-2.10 (m, 1H), 2.26 (s, 3H), 2.36 (s, 3H), 2.84 (t, 2H), 4.50 (d, 2H), 7.28 (d, 2H), 7.48, (d, 2H), 7.90 (d, 2H), 8.04 (d, 1H), 8.30 (d, 1H), 8.65 (s, 1H), 8.86 (s, 1H) |
| OE19 | 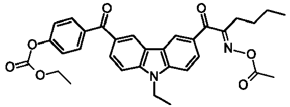 | 0.95 (t, 3H), 1.43 -1.61 (m, 10H), 2.29 (s, 3H), 2.86 (t, 2H), 4.38 (q, 2H), 4.46 (q, 2H), 7.36 (d, 2H), 7.51, (dd, 2H), 7.92 (d, 2H), 8.06 (dd, 1H), 8.32 (dd, 1H), 8.67 (s, 1H), 8.88 (s, 1H) |
| OE20 | 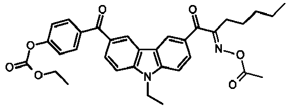 | 0.88 (t, 3H), 1.25-1.60 (m, 15H), 2.29 (s, 3H), 2.37 (t, 3H), 2.84 (t, 2H), 4.37 (q, 2H), 4.45 (q, 2H), 7.37 (d, 2H), 7.50, (dd, 2H), 7.91 (d, 2H), 8.04 (dd, 1H), 8.29 (dd, 1H), 8.66 (s, 1H), 8.87 (s, 1H) |
| OE21 | 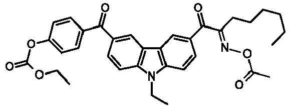 | 0.85 (t, 3H), 1.25-1.68 (m, 17H), 2.29 (s, 3H), 2.85 (t, 2H), 4.38 (q, 2H), 4.48 (q, 2H), 7.36 (d, 2H), 7.51, (dd, 2H), 7.92 (d, 2H), 8.06 (dd, 1H), 8.30 (dd, 1H), 8.66 (s, 1H), 8.88 (s, 1H) |
| OE22 | 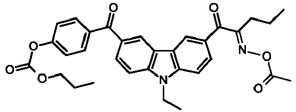 | 1.00 (t, 3H), 1.02 (t, 3H), 1.48 (t, 3H), 1.60-1.70 (m, 2H), 1.75-1.85 (m, 2H), 2.26 (s, 3H), 3.84 (t, 2H), 4.25 (t, 2H), 4.50 (q, 2H), 7.35 (d, 2H), 7.50 (d, 2H), 7.90 (d, 2H), 8.05 (d, 1H), 8.30 (d, 1H), 8.65 (s, 1H), 8.88 (s, 1H) |
| OE23 | 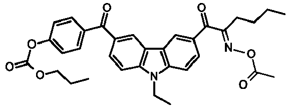 | 0.93 (t, 3H), 1.05 (t, 3H), 1.36-1.48 (m, 2H), 1.51 (t, 3H), 1.54-1.66 (m, 2H), 1.76-1.87 (m, 2H), 2.29 (s, 3H), 2.86 (dd, 2H), 4.27 (t, 2H), 4.46 (q, 2H), 7.37 (d, 2H), 7.51 (d, 2H), 7.92 (d, 2H), 8.06 (dd, 1H), 8.31 (dd, 1H), 8.67 (d, 1H), 8.88 (d, 1H) |
| OE24 | 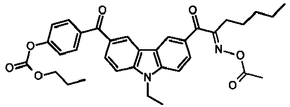 | 0.86 (t, 3H), 1.04 (t, 3H), 1.30-1.40 (m, 4H), 1.50 (t, 3H), 1.56-1.65 (m, 2H), 1.76-1.85 (m, 2H), 2.26 (s, 3H), 2.84 (t, 2H), 4.25 (t, 2H), 4.50 (q, 2H), 7.35 (d, 2H), 7.50, (d, 2H), 7.90 (d, 2H), 8.05 (d, 1H), 8.30 (d, 1H), 8.65 (s, 1H), 8.88 (s, 1H) |
| OE25 | 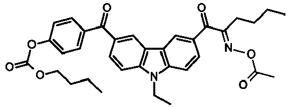 | 0.91 (t, 3H), 1.00 (t, 3H), 1.25-1.81 (m, 11H), 2.29 (s, 3H), 2.86 (t, 2H), 4.31 (t, 2H), 4.46 (q, 2H), 7.37 (d, 2H), 7.51, (dd, 2H), 7.92 (d, 2H), 8.06 (dd, 1H), 8.31 (dd, 1H), 8.66 (s, 1H), 8.88 (s, 1H) |
| OE26 | 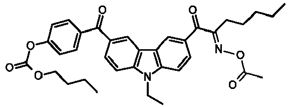 | 0.88 (t, 3H), 1.00 (t, 3H), 1.29-1.81 (m, 13H), 2.29 (s, 3H), 2.85 (t, 2H), 4.32 (t, 2H), 4.46 (q, 2H), 7.37 (d, 2H), 7.51, (dd, 2H), 7.92 (d, 2H), 8.06 (dd, 1H), 8.31 (dd, 1H), 8.66 (s, 1H), 8.88 (s, 1H) |
| OE27 | 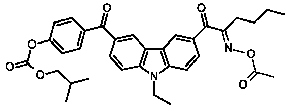 | 0.92 (t, 3H), 1.04 (d, 6H), 1.38-1.65 (m, 9H), 2.05-2.14 (m, 1H), 2.28 (s, 3H), 2.85 (t, 2H), 4.20 (d, 2H), 4.45 (q, 2H), 7.35 (d, 2H), 7.50 (d, 2H), 7.90 (d, 2H), 8.05 (d, 1H), 8.30 (d, 1H), 8.65 (s, 1H), 8.88 (s, 1H) |
| OE28 | 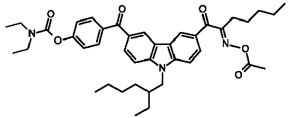 | 0.86-0.95 (m, 9H), 1.24-1.68 (m, 20H), 2.03-2.10 (m, 1H), 2.29 (s, 3H), 2.85 (t, 2H), 3.42-3.50 (m, 4H), 4.20-4.30 (m, 2H), 7.30 (d, 2H), 7.48 (d, 2H), 7.90 (d, 2H), 8.05 (d, 1H), 8.29 (d, 2H), 8.65 (s, 1H), 8.85 (s, 1H) |
| OE29 | 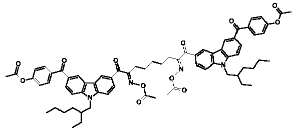 | 0.86 (t, 6H), 0.92 (t, 6H), 1.26-2.09 (m, 24H), 2.26 (s, 6H), 2.36 (s, 6H), 2.84 (t, 4H), 4.22-4.24 (m, 4H), 7.26 (d, 4H), 7.91 (4H), 7.44-7.49 (m, 4H), 8.04 (dd, 2H), 8.26 (dd, 2H), 8.64 (s, 2H), 8.85 (s, 2H) |
| OE30 | 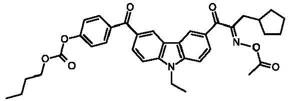 | 0.99 (t, 3H), 1.22-1.27 (m, 2H), 1.49-1.79 (m, 12H), 2.10-2.18 (m, 1H), 2.29 (s, 3H), 2.94 (d, 2H), 4.32 t, 2H), 4.46 (q, 2H), 7.36 (d, 2H), 7.51 (d, 2H), 7.92 (d, 2H), 8.05 (dd, 1H), 8.32 (dd, 1H), 8.68 (s, 1H), 8.91 s, 1H) |
| OE31 | 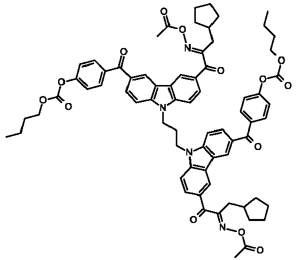 | 1.00 (t, 6H), 1.20-1.26 (m, 4H), 1.49-1.80 (m, 14H), 2.10-2.18 (m, 2H), 2.28 (s, 6H), 2.92 (d, 4H), 4.31 (t, 6H), 4.51 (t, 4H), 7.30-7.38 (m, 8H), 7.90 (d, 4H), 7.98 (dd, 2H), 8.26 (dd, 2H), 8.68 (s, 2H), 8.92 (s, 2H) |
| OE32 (E) | 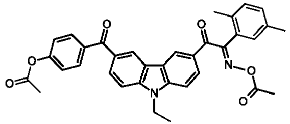 | 1.52 (t, 3H), 2.16 (s, 3H), 2.17 (s, 3H), 2.32 (s, 3H), 2.36 (s, 3H), 4.47 (q, 2H), 7.08 (s, 1H), 7.16-7.29 (m, 4H), 7.54 (t, 2H), 7.92 (d, 2H), 8.07 (dd, 1H), 8.42 (dd, 1H), 8.70 (s, 1H), 9.04 (s, 1H) |
| OE32 (Z) | 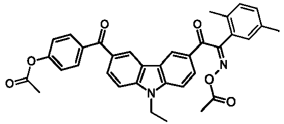 | 1.52 (t, 3H), 1.98 (s, 3H), 2.20 (s, 3H), 2.36 (s, 3H), 2.68 (s, 3H), 4.47 (q, 2H), 7.08 (s, 1H), 7.16-7.29 (m, 4H), 7.54 (t, 2H), 7.88 (d, 2H), 8.10 (dd, 1H), 8.41 (dd, 1H), 8.62 (s, 1H), 8.75 (s, 1H) |
| OE34 | 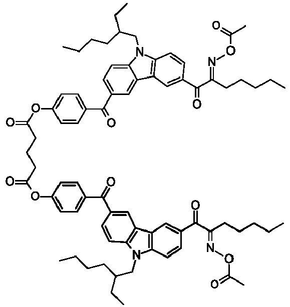 | 0.86-0.90 (m, 12H), 0.93 (t, 6H), 1.20-1.48 (m, 24H), 1.57-1.68 (m, 4H), 2.00-2.13 (m, 2H), 2.23-2.36 (m, 2H), 2.30 (s, 6H), 2.80-2.90 (m, 8H), 4.25 (d, 4H), 7.30 (d, 4H), 7.48 (d, 4H), 7.94 (d, 4H), 8.05 (dd, 2H), 8.29 (dd, 2H), 8.66 (d, 2H), 8.87 (d, 2H) |
| OE35 | 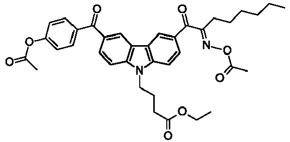 | 0.86 (t, 3H), 1.22-1.35 (m, 7H), 1.35-1.45 (m, 2H), 1.56-1.68 (m, 2H), 2.19-2.28 (m, 2H), 2.29 (s, 3H), 2.34-2.40 (m, 2H), 2.37 (s, 3H), 2.85 (dd, 2H), 4.16 (q, 2H), 4.49 (t, 2H), 7.28 (d, 2H), 7.54 (d, 1H), 7.55 (d, 1H), 7.92 (d, 2H), 8.06 (dd, 1H), 8.30 (dd, 1H), 8.66 (d, 1H), 8.87 (d, 1H) |
| OE36 (E) | 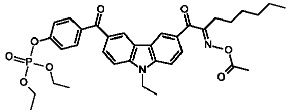 | 0.86 (t, 3H), 1.24-1.69 (m, 17H), 2.30 (s, 3H), 2.85 (t, 2H), 4.25-4.30 (m, 4H), 4.38 (q, 2H), 7.38 (d, 2H), 7.51 (dd, 2H), 7.88 (d, 2H), 8.03 (dd, 1H), 8.30 (dd, 1H), 8.65 (s. 1H), 8.87 (s, 1H) |
| OE36 (Z) | 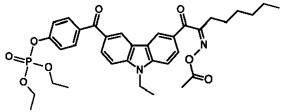 | 0.86 (t, 3H), 1.24-1.69 (m, 17H), 1.88 (s, 3H), 2.69 (t, 2H), 4.25-4.30 (m, 4H), 4.38 (q, 2H), 7.39 (d, 2H), 7.53 (dd, 2H), 7.87 (d, 2H), 8.07 (dd, 1H), 8.30 (dd, 1H), 8.62 (s. 1H), 8.87 (s, 1H) |
| OE37 | 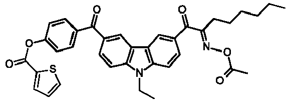 | 0.86 (t, 3H), 1.25-1.71 (m, 11H), 2.29 (s, 3H), 2.86 (t, 2H), 4.46 (q, 2H), 7.22 (dd, 1H), 7.41 (d, 2H), 7.51 (dd, 2H), 7.72 (dd, 1H), 7.96 (d, 2H), 8.04 (dd, 1H), 8.08 (dd, 1H), 8.31 (dd, 1H), 8.68 (s, 1H), 8.89 (s, 1H) |
| OE38 | 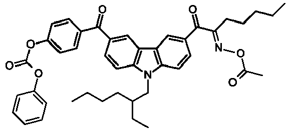 | 0.87 (t, 3H), 0.88 (t, 3H), 0.94 (t, 3H), 1.20-1.48 (m, 12H), 1.57-1.68 (m, 2H), 2.00-2.14 (m, 1H), 2.28 (s, 3H), 2.85 (dd, 2H), 4.26 (d, 2H), 7.28-7.35 (m, 3H), 7.42-7.53 (m, 6H), 7.96 (d, 2H), 8.06 (dd, 1H), 8.30 (dd, 1H), 8.66 (d, 1H), 8.88 (d, 1H) |
| OE39 | 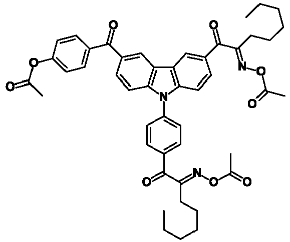 | 0.86 (t, 3H), 0.88 (t, 3H), 1.24-1.46 (m, 12H), 1.58-1.68 (m, 4 H), 2.28 (s, 3H), 2.90 (s, 3H), 2.36 (s, 3H), 2.84 (t, 2H), 2.86 (t, 2H), 7.28 (d, 2H), 7.52 (dd, 2H), 7.72 (d, 2H), 7.92 (d, 2H), 7.98 (dd, 1H), 8.24 (dd, 1H), 2.41 (d, 2H), 8.70 (d, 1H), 8.92 (d, 1H) |
| OE40 | 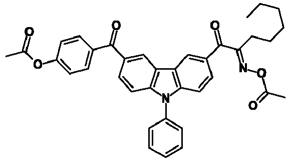 | 0.86 (t, 3H), 1.24-1.32 (m, 4H), 1.35-1.45 (m, 2 H), 1.56-1.66 (m, 2H), 2.28 (s, 3H), 2.36 (s, 3H), 2.84 (t, 2H), 7.26 (d, 2H), 7.42 (dd, 2H), 7.56 (dt, 3H), 7.66 (t, 2H), 7.92 (d, 2H), 7.97 (dd, 1H), 8.22 (dd, 1H), 8.70 (d, 1H), 8.92 (d, 1H) |
| OE41 | 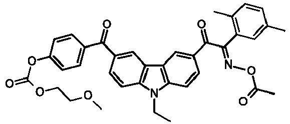 | 1.52 (t, 3H), 2.16 (s, 3H), 2.32 (s, 3H), 3.46 (s, 3H), 3.72 (t, 2H), 4.43-4.50 (m, 4H), 7.08-7.23 (m, 3H), 7.37 (d, 2H), 7.51-7.56 (m, 2H), 7.91 (d, 2H), 8.07 (dd, 1H), 8.42 (dd, 1H), 8.69 (s, 1H), 9.04 (s, 1H) |
| OE42 | 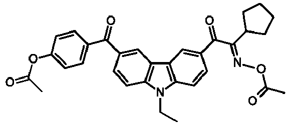 | 1.45-2.11 (m, 12H), 2.28 (s, 3H), 2.38 (s, 3H), 4.46 (q, 2H), 7.37 (d, 2H), 7.51, (dd, 2H), 7.92 (d, 2H), 8.06 (dd, 1H), 8.31 (dd, 1H), 8.64 (s, 1H), 8.75 (s, 1H) |
| OE44 | 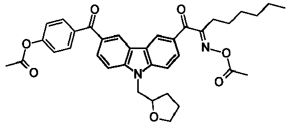 | 0.86 (t, 3H), 1.21-1.35 (m, 4H), 1.35-1.45 (m, 2H), 1.56-1.73 (m, 3H), 1.74-1.95 (m, 2H), 1.99-2.11 (m, 1H), 2.29 (s, 3H), 2.37 (s, 3H), 2.85 (t, 2H), 3.68-3.85 (m, 2H), 4.35-4.57 (m, 3H), 7.27 (d, 2H), 7.58 (d, 1H), 7.59 (d, 1H), 7.92 (d, 2H), 8.05 (dd, 1H), 8.29 (dd, 1H), 8.65 (d, 1H), 8.85 (d, 1H) |
| OE45 | 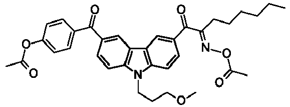 | 0.86 (t, 3H), 1.22-1.46 (m, 6H), 1.56-1.69 (m, 2H), 2.11-2.21 (m, 2H), 2.29 (s, 3H), 2.37 (s, 3H), 2.86 (t, 2H), 3.26 (t, 2H), 3.13 (s, 3H), 4.53 (t, 2H), 7.27 (d, 2H), 7.56 (d, 2H), 7.92 (d, 2H), 8.06 (dd, 1H), 8.30 (dd, 1H), 8.66 (d, 1H), 8.86 (d, 1H) |
| OE46 | 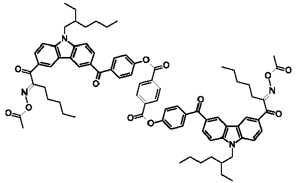 | 0.88 (t, 6H), 0.89 (t, 6H), 0.95 (t, 6H), 1.21-1.50 (m, 24H), 1.59-1.69 (m, 4H), 2.02-2.15 (m, 2H), 2.30 (s, 6H), 2.86 (dd, 4H), 4.27 (d, 4H), 7.46 (d, 4H), 7.49 (d, 2H), 7.52 (d, 2H), 8.01 (d, 4H), 8.10 (dd, 2H), 8.31 (dd, 2H), 8.42 (s, 4H), 8.70 (d, 2H), 8.90 (d, 2H) |
| OE47 | 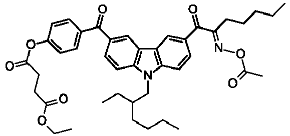 | 0.83-0.91 (m, 6H), 0.94 (t, 3H), 1.20-1.48 (m, 12H), 1.30 (t, 3H), 1.58-1.68 (m, 2H), 2.00-2.12 (m, 1H), 2.29 (s, 3H), 2.78 (t, 2H), 2.85 (dd, 2H), 2.95 (t, 2H), 4.21 (q, 2H), 4.25 (d, 2H), 7.28 (d, 2H), 7.48 (d, 2H), 7.92 (d, 2H), 8.04 (dd, 1H), 8.29 (dd, 1H), 8.66 (d, 1H), 8.87 (d, 1H) |
| OE48 | 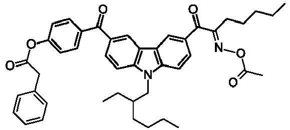 | 0.86 (t, 3H), 0.88 (t, 3H), 0.93 (t, 3H), 1.20-1.48 (m, 12H), 1.57-1.67 (m, 2H), 2.00-2.12 (m, 1H), 2.26 (s, 3H), 2.84 (dd, 2H), 3.93 (s, 2H), 4.24 (d, 2H), 7.25 (d, 2H), 7.31-7.45 (m, 5H), 7.48 (d, 2H), 7.90 (d, 2H), 8.04 (dd, 1H), 8.29 (dd, 1H), 8.64 (d, 1H), 8.86 (d, 1H) |
| OE49 | 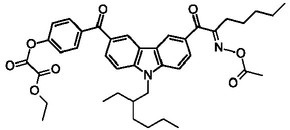 | 0.87 (t, 3H), 0.88 (t, 3H), 0.94 (t, 3H), 1.20-1.70 (m, 14H), 1.47 (t, 3H), 2.00-2.12 (m, 1H), 2.29 (s, 3H), 2.85 (dd, 2H), 4.26 (d, 2H), 4.49 (q, 2H), 7.39 (d, 2H), 7.48 (d, 1H), 7.50 (d, 1H), 7.96 (d, 2H), 8.06 (dd, 1H), 8.30 (dd, 1H), 8.66 (d, 1H), 8.89 (d, 1H) |
| OE50 | 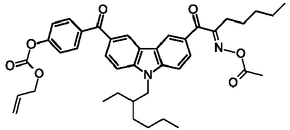 | 0.83-0.91 (m, 6H), 0.94 (t, 3H), 1.20-1.50 (m, 12H), 1.58-1.69 (m, 2H), 2.00-2.12 (m, 1H), 2.30 (s, 3H), 2.85 (dd, 2H), 4.25 (d, 2H), 4.79 (d, 2H), 5.38 (d, 1H), 5.47 (d, 1H), 6.04 (ddt, 1H), 7.37 (d, 2H), 7.48 (d, 1H), 7.49 (d, 1H), 7.93 (d, 2H), 8.05 (dd, 1H), 8.30 (dd, 1H), 8.66 (d, 1H), 8.88 (d, 1H) |
| OE51 | 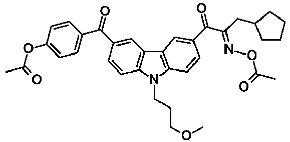 | 1.18-1.31 (m, 2H), 1.44-1.72 (m, 4H), 1.73-1.84 (m, 2H), 2.11-2.22 (m, 3H), 2.29 (s, 3H), 2.37 (s, 3H), 2.93 (d, 2H), 3.27 (t, 2H), 3.32 (s, 3H), 4.53 (t, 2H), 7.27 (d, 2H), 7.56 (d, 2H), 7.92 (d, 2H), 8.05 (dd, 1H), 8.32 (dd, 1H), 8.68 (d, 1H), 8.90 (d, 1H) |
| OE52 (E) | 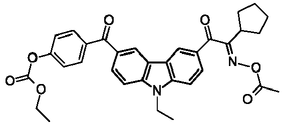 | 1.43 (t, 3H), 1.51 (t, 3H), 1.58-1.82 (m, 6H), 1.93-2.10 (m, 2H), 2.29 (s, 3H), 3.48-3.61 (m, 1H), 4.38 (q, 2H), 4.42-4.52 (m, 2H), 7.34-7.40 (m, 2H), 7.48-7.56 (m, 2H), 7.92 (d, 2H), 8.06 (dd, 1H), 8.24 (dd, 1H), 8.63-8.68 (m, 1H), 8.78 (d, 1H) |
| OE52 (Z) | 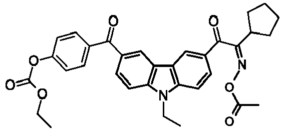 | 1.43 (t, 3H), 1.53 (t, 3H), 1.58-1.82 (m, 6H), 1.88 (s, 3H), 1.93-2.10 (m, 2H), 3.07-3.18 (m, 1H), 4.38 (q, 2H), 4.42-4.52 (m, 2H), 7.34-7.40 (m, 2H), 7.48-7.56 (m, 2H), 7.91 (d, 2H), 8.02 (dd, 1H), 8.09 (dd, 1H), 8.63-8.68 (m, 2H) |
| OE53 | 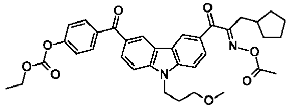 | 1.18-1.32 (m, 2H), 1.43 (t, 3H), 1.47-1.56 (m, 2H), 1.58-1.72 (m, 2H), 1.72-1.85 (m, 2H), 2.10-2.23 (m, 3H), 2.29 (s, 3H), 2.93 (d, 2H), 3.27 (t, 2H), 3.32 (s, 3H), 4.38 (q, 2H), 4.53 (t, 2H), 7.37 (d, 2H), 7.56 (d, 2H), 7.92 (d, 2H), 8.05 (dd, 1H), 8.32 (dd, 1H), 8.67 (d, 1H), 8.90 (d, 1H) |
| OE54 | 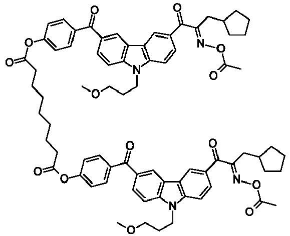 | 1.18-1.33 (m, 4H), 1.42-1.72 (m, 14H), 1.72-1.90 (m, 8H), 2.10-2.23 (m, 6H), 2.29 (s, 6H), 2.65 (t, 4H), 2.93 (d, 4H), 3.26 (t, 4H), 3.32 (s, 6H), 4.52 (t, 4H), 7.27 (d, 4H), 7.55 (d, 4H), 7.91 (d, 4H), 8.04 (dd, 2H), 8.31 (dd, 2H), 8.67 (d, 2H), 8.90 (d, 2H) |
| OE56 | 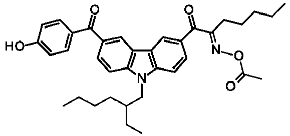 | 0.82-0.90 (m, 6H), 0.93 (t, 3H), 1.20-1.50 (m, 12H), 1.58-1.68 (m, 2H), 2.00-2.13 (m, 1H), 2.30 (s, 3H), 2.85 (dd, 2H), 4.24 (d, 2H), 5.68 (s, 1H), 6.95 (d, 2H), 7.47 (d, 1H), 7.48 (d, 1H), 7.83 (d, 2H), 8.02 (dd, 1H), 8.28 (dd, 1H), 8.59 (d, 1H), 8.85 (d, 1H) |

### Application Examples

### Sensitivity test: A

### Preparation of color filter resist (blue)

Blue pigment dispersion is prepared by mixing the following components and dispersing them by using a Paint conditioner (SKANDEX).

### Blue dispersion

| | |
|---|---|
| 5.6 parts by weight | blue pigment (PB15:6, Blue E provided by Toyo Ink) |
| 0.6 parts by weight | violet pigment (PV23, Cromophtal Violet GA provided by BASF) |
| 2.6 parts by weight | dispersant (EFKA6745 provided by BASF) |
| 0.4 parts by weight | synergist (Solsperse S5000 provided by Lubrizol) |
| 19.6 parts by weight | solvent (PGMEA) |

Color filter resist (blue) are prepared by further adding the following components to the above dispersion prepared.

| | |
|---|---|
| 6.0 parts by weight | alkaline developable binder, 37.8 % solution (Ripoxy SPC-2000, |
| | provided by Shoko Highpolymer) |
| 41.9 parts by weight | solvent (PGMEA) |
| 5.0 parts by weight | multifunctional acrylate (DPHA, provided by Sigma-Aldrich) |
| 0.7 parts by weight | Photoinitiator |

All operations are carried out under yellow light. The compositions are applied to a glass plate using a spincoater. The solvent is removed by heating at 80°C for 10 minutes in a convection oven. Exposure is carried out using a 250W super high pressure mercury lamp (USHIO, USH-250BY) at a distance of 15 cm. A total exposure dose measured by an optical power meter (ORC UV Light Measure Model UV-M02 with UV-35 detector) on the glass filter is 50mJ/cm². After exposure, the exposed film is baked at 230°C for 30 minutes in a forced convention oven. The film is measured transmittance from 350nm to 780 nm after drying, exposure and baking using UV-VIS spectrometer (Shimazu, UV-2550). Y value at 0.094 of y is calculated from transmittance of the film. Y value means brightness of the film after baking 230°C for 30 minutes. Larger Y value indicates higher brightness. The results are listed in Table 1.

**Table 1: Brightness**

| Example No. | PI | Y |
|---|---|---|
| Example A1 | OE1 | 10.47 |
| Example A2 | OE2 | 10.47 |
| Example A3 | OE3 | 10.48 |
| Example A4 | OE4 | 10.49 |
| Example A5 | OE5 | 10.50 |
| Example A6 | OE6 | 10.51 |
| Example A7 | OE7 | 10.50 |
| Example A8 | OE8 | 10.50 |
| Example A9 | OE9 | 10.48 |
| Example A10 | OE10 | 10.48 |
| Example A11 | OE11 | 10.48 |
| Example A12 | OE12 | 10.46 |
| Example A13 | OE13 | 10.46 |
| Example A14 | OE14 | 10.53 |
| Example A15 | OE15 | 10.48 |
| Example A17 | OE17 | 10.5* |
| Example A18 | OE18 | 10.47 |
| Example A19 | OE19 | 10.48 |
| Example A20 | OE20 | 10.5 |
| Example A21 | OE21 | 10.49 |
| Example A22 | OE22 | 10.51 |
| Example A23 | OE23 | 10.5 |
| Example A24 | OE24 | 10.53 |
| Example A25 | OE25 | 10.52 |
| Example A26 | OE26 | 10.53 |
| Example A27 | OE27 | 10.48 |
| Example A28 | OE28 | 10.44 |
| Example A29 | OE29 | 10.49 |
| Example A30 | OE30 | 10.49 |
| Example A31 | OE31 | 10.51 |
| Example A32 | OE32 | 10.51* |
| Example A34 | OE34 | 10.47 |
| Example A35 | OE35 | 10.52 |
| Example A36 | OE36 | 10.44* |
| Example A37 | OE37 | 10.46 |
| Example A38 | OE38 | 10.49 |
| Example A39 | OE39 | 10.49 |
| Example A40 | OE40 | 10.53 |
| Example A41 | OE41 | 10.45 |
| Example A42 | OE42 | 10.49 |
| Example A44 | OE44 | 10.50 |
| Example A45 | OE45 | 10.50 |
| Example A46 | OE46 | 10.50 |
| Example A47 | OE47 | 10.50 |
| Example A48 | OE48 | 10.51 |
| Example A49 | OE49 | 10.51 |
| Example A50 | OE50 | 10.49 |
| Example A51 | OE51 | 10.51 |
| Example A52 | OE52 | 10.50* |
| Example A53 | OE53 | 10.53 |
| Example A54 | OE54 | 10.54 |
| Example A56 | OE56 | 10.51 |
| Comparative example CE1 | | 10.37 |
| Comparative example CE2 | | 10.03 |
| Comparative example CE3 | | 10.43 |
| Comparative example CE4 | | 10.43 |
| Comparative example CE5 | | 10.40 |

| | | |
|---|---|---|
| * The obtained brightness value Y did not depend on the use of E or Z isomers or mixtures thereof. | | |

The following photoinitiators were used in the comparative examples:

| | | | | |
|---|---|---|---|---|
| CE1 | 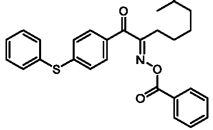 | | CE4 | 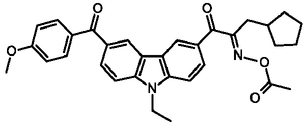 |
| CE2 | 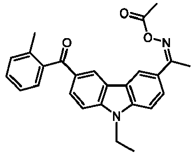 | | CE5 | 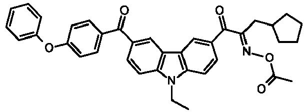 |
| CE3 | 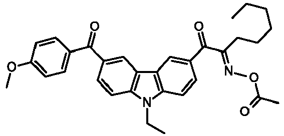 | | | |

## Claims

1. Compounds of the formula I, II, III and IV 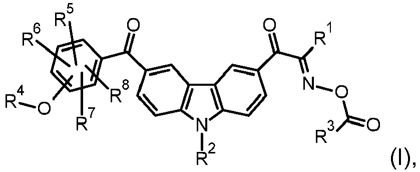 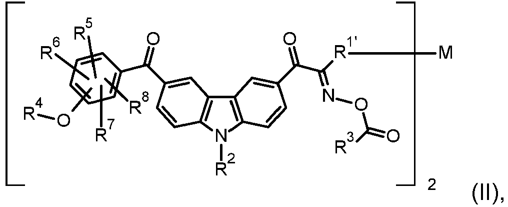 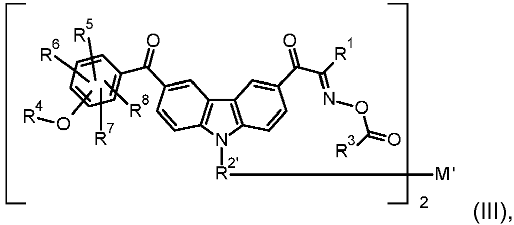 and 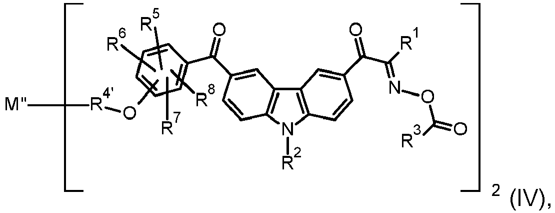 wherein
**R¹** is hydrogen, C₁-C₂₀alkyl, C₁-C₆alkyl-C₃-C₆cycloalkyl, C₃-C₂₀cycloalkyl, C₂-C₁₂alkenyl, wherein C₃-C₂₀cycloalkyl or C₂-C₁₂alkenyl is uninterrupted or interrupted by one or more O, S, CO, NR¹⁰ or COOR⁴; or
**R¹** is C₁-C₂₀alkyl which is unsubstituted or substituted by one or more halogen, OR⁴, SR⁹, NR¹⁰R¹¹, COOR⁴ CONR¹⁰R¹¹, CN, PO(OR^{3a})₂, S(O)ₘ-R^{3a}, 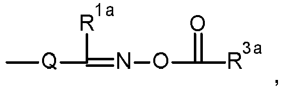 C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹, NR¹⁰R¹¹, PO(OR^{3a})₂ or S(O)ₘ-R^{3a}; or
**R¹** is C₂-C₂₀alkyl which is interrupted by one or more O, CO, S, C(O)O, OC(O), SO, SO₂, phenylene, naphthylene or NR¹⁰, wherein the interrupted C₂-C₂₀alkyl is unsubstituted or substituted by one or more halogen, C₃-C₈cycloalkyl, OH, SH, OR⁴, SR⁹, COOR⁴, O(CO)-R^{3a}, CONR¹⁰R¹¹, NR¹⁰R¹¹, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR⁴, SR⁹ or NR¹⁰R¹¹; or
**R¹** is C₆-C₂₀aryl or C₃-C₂₀heteroaryl each of which is unsubstituted or substituted by one or more C₁-C₂₀alkyl, phenyl, halogen, C₁-C₄haloalkyl, CN, NO₂, OR⁴, SR⁹, NR¹⁰R¹¹, COOR⁴, (CO)-R^{3a}, (CO)NR¹⁰R¹¹, PO(OR^{3a})₂, S(O)ₘ-R^{3a} or group 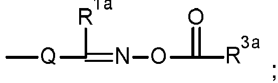 or by one or more C₂-C₂₀alkyl which is interrupted by one or more O, S, or NR¹⁰; or by one or more C₁-C₂₀alkyl which is unsubstituted or substituted by one or more halogen, COOR⁴, CONR¹⁰R¹¹, phenyl, C₃-C₈cycloalkyl, C₃-C₂₀heteroaryl, C₆-C₂₀aryloxycarbonyl, C₃-C₂₀heteroaryloxycarbonyl, OR⁴, SR⁹ or NR¹⁰R¹¹; or by one or more phenyl, naphthyl, benzoyl or naphthoyl, each of which is unsubstituted or substituted by OR⁴, SR⁹ or NR¹⁰R¹¹;
**R¹** is C₂-C₂₀alkanoyl or benzoyl which is unsubstituted or substituted by one or more C₁-C₆alkyl, phenyl, OR⁴, SR⁹ or NR¹⁰R¹¹; or
**R¹** is C₂-C₁₂alkoxycarbonyl optionally interrupted by one or more -O- and/or optionally substituted by one or more hydroxyl groups; or
**R¹** is phenoxycarbonyl which is unsubstituted or substituted by C₁-C₆alkyl, halogen, phenyl, OR⁴, SR⁹ or NR¹⁰R¹¹; or
**R¹** is CN, (CO)-R^{3a}, COOR⁴, CONR¹⁰R¹¹, NO₂, PO(OR^{3a})₂ or S(O)ₘ-R^{3a};
**R^{1'}** is C₁-C₂₀alkylene, C₁-C₂₀alkylene which is interrupted by one or more O, S, (CO)O, O(CO), phenylene, naphthylene or NR⁸, wherein the C₁-C₂₀alkylene and interrupted C₁-C₂₀alkylene is unsubstituted or substituted by halogen or OR⁴, or
**R^{1'}** is C₂-C₂₀alkenylene, C₂-C₂₀alkenylene which is interrupted by one or more O, S, (CO)O, O(CO), phenylene, naphthylene or by NR¹⁰, wherein the C₂-C₂₀alkenylene and interrupted C₂-C₂₀alkenylene is unsubstituted or substituted by halogen or OR⁴, or 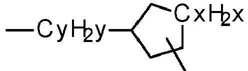
**R^{1'}** is C₅-C₈cycloalkylene, C₅-C₈cycloalkenylene, 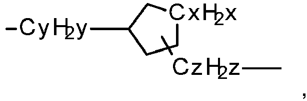 wherein the groups C_{y}H_{2y} and C_{z}H_{2z} are uninterrupted or interrupted by one or more O, S or by NR¹⁰;
**R^{1a}** is hydrogen, C₁-C₂₀alkyl, CN, (CO)-R^{3a}, COOR^{4a}, CONR^{10a}R^{11a}, NO₂, PO(OR^{3a})₂ or S(O)ₘ-R^{3a}; or
**R^{1a}** is C₁-C₂₀alkyl substituted by one or more halogen, OR^{4a}, SR^{9a}, NR^{10a}R^{11a}, CN, COOR^{4a}, CONR^{10a}R^{11a}, PO(OR^{3a})₂, S(O)ₘ-R^{3a}, C₃-C₈cycloalkyl which is uninterrupted or interrupted by one or more O, S, CO or NR^{10a}; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen phenyl, C₁-C₂₀alkylphenyl, C₁-C₈alkoxyphenyl, C₁-C₄haloalkyl, CN, NO₂, OR^{4a}, SR^{9a}, NR^{10a}R^{11a}, PO(OR^{3a})₂ or S(O)ₘ-R^{3a}; or
**R^{1a}** is C₁-C₂₀alkyl interrupted by one or more O, S, NR^{10a}, CO, SO or SO₂, which is unsubstituted or substituted by C₃-C₈cycloalkyl, OH, SH, O(CO)R^{3a}, COOR^{4a}, CONR^{10a}R^{11a}, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR^{4a}, SR^{9a} or NR^{10a}R^{11a}; or
**R^{1a}** is C₂-C₁₂alkenyl or C₃-C₂₀cycloalkyl, each of which is uninterrupted or interrupted by one or more O, S, CO, NR^{10a} or COOR^{4a}; or
**R^{1a}** is C₆-C₂₀aryl or C₃-C₂₀heteroaryl, each of which is unsubstituted or substituted by one or more halogen, CN, NO₂, OR^{4a}, SR^{9a}, NR^{10a}R^{11a}, COOR^{4a}, (CO)-R^{3a} CONR^{10a}R^{11a}, PO(OR^{3a})₂ or S(O)ₘ-R^{3a}; or by one or more C₁-C₂₀alkyl which is unsubstituted or substituted by one or more halogen, COOR^{4a}, CONR^{10a}R^{11a}, phenyl, C₃-C₈cycloalkyl, C₃-C₂₀heteroaryl, OR^{4a}, SR^{9a} or NR^{10a}R^{11a}; or by one or more C₂-C₂₀alkyl which is interrupted by one or more O, S or NR^{10a}; or by one or more phenyl, naphthyl, benzoyl or naphthoyl, each of which is unsubstituted or substituted by OR^{4a}, SR^{9a} or NR^{10a}R^{11a};
**R²** is hydrogen, C₁-C₂₀alkyl or C₁-C₆alkyl-C₃-C₆-cycloalkyl which is unsubstituted or substituted by one or more halogen, OR⁴, SR⁹, COOR⁴ CONR¹⁰R¹¹, NR¹⁰R¹¹, PO(OR^{3a})₂; COR^{3a}, or
**R²** is C₂-C₂₀alkyl or C₁-C₆alkyl-C₃-C₆-cycloalkyl which is interrupted by one or more O, CO, S, C(O)O, OC(O), SO, SO₂, phenylene, naphthylene or NR¹⁰, wherein the interrupted C₂-C₂₀alkyl is unsubstituted or substituted by one or more halogen, OR⁴, SR⁹, COOR⁴, CONR¹⁰R¹¹, NR¹⁰R¹¹; or
**R²** is C₂-C₄hydroxyalkyl, C₂-C₁₀alkoxyalkyl, C₃-C₅alkenyl, C₃-C₈cycloalkyl, phenyl-C₁-C₃alkyl, C₂-C₈alkanoyl, C₃-C₁₂alkenoyl, benzoyl; or
**R²** is C₆-C₂₀aryl or C₃-C₂₀heteroaryl each of which is unsubstituted or substituted by one or more C₁-C₁₂alkyl, C₁-C₄haloalkyl, phenyl, halogen, CN, NO₂, OR⁴, SR⁹, NR¹⁰R¹¹, (CO)-R^{3a}, or by C₂-C₂₀alkyl which is interrupted by one or more O, S, or NR¹⁰, or each of which is substituted by one or more C₁₋C₂₀alkyl which is unsubstituted or substituted by one or more halogen, COOR⁴, CONR¹⁰R¹¹, phenyl, C₃-C₈cycloalkyl, C₃-C₂₀heteroaryl, C₆-C₂₀aryloxycarbonyl, C₃-C₂₀heteroaryloxycarbonyl, OR^{4,} SR⁹ or NR¹⁰R¹¹; or
**R²** is a group 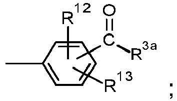
**R^{2'}** is C₁-C₂₀alkylene, C₁-C₂₀alkylene which is interrupted by one or more O, S, (CO)O, O(CO), phenylene, naphthylene or NR⁸, wherein the C₁-C₂₀alkylene and interrupted C₁-C₂₀alkylene is unsubstituted or substituted by halogen or OR⁴, or
**R^{2'}** is C₂-C₂₀alkenylene, C₂-C₂₀alkenylene which is interrupted by one or more O, S, (CO)O, O(CO), phenylene, naphthylene or by NR¹⁰, wherein the C₂-C₂₀alkenylene and interrupted C₂-C₂₀alkenylene is unsubstituted or substituted by halogen or OR⁴, or
**R^{2'}** is C₅-C₈cycloalkylene, C₅-C₈cycloalkenylene, 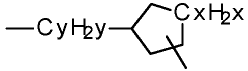 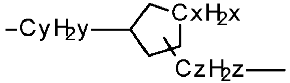 , wherein the groups C_{y}H_{2y} and C_{z}H_{2z} are uninterrupted or interrupted by one or more O, S or by NR¹⁰;
**R³** is hydrogen or C₁-C₂₀alkyl; or
**R³** is C₁-C₂₀alkyl substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹ or NR¹⁰R¹¹; or
**R³** is C₁-C₂₀alkyl interrupted by one or more O, S, NR¹⁰, CO, SO or SO₂, which is unsubstituted or substituted by C₃-C₈cycloalkyl, OH, SH, O(CO)-R^{3a}, COOR⁴, CONR¹⁰R¹¹ C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR⁴, SR⁹ or NR¹⁰R¹¹; or
**R³** is C₂-C₁₂alkenyl or C₃-C₂₀cycloalkyl, each of which is uninterrupted or interrupted by one or more O, S, CO, NR¹⁰ or COOR⁴; or
**R³** is C₆-C₂₀aryl or C₃-C₂₀heteroaryl, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR⁴, SR⁹, NR¹⁰R¹¹, COOR⁴, (CO)-R^{3a} or SO₂-R^{3a}; or
**R³** is C₁-C₂₀alkoxy, which is unsubstituted or substituted by one or more C₁-C₁₀alkyl, C₁-C₄haloalkyl, halogen, phenyl, C₁-C₂₀alkylphenyl or C₁-C₈alkoxyphenyl; or
**R³** is C₁-C₂₀alkoxy, which is interrupted by one or more O, S, NR¹⁰, CO, SO or SO₂; or
**R³** is C₆-C₂₀aryloxy or C₃-C₂₀heteroaryloxy, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR⁴, SR⁹, NR¹⁰R¹¹, COOR⁴, (CO)-R^{3a} or SO₂-R^{3a};
**R^{3a}** is hydrogen or C₁-C₂₀alkyl; or
**R^{3a}** is C₁-C₂₀alkyl substituted by one or more halogen, OR^{4a}, SR^{9a}, NR^{10a}R^{11a}, CN, COOR^{4a}, CONR^{10a}R^{11a}, C₃-C₈cycloalkyl which is uninterrupted or interrupted by one or more O, S, CO or NR^{10a}; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR^{4a}, SR^{9a} or NR^{10a}R^{11a}; or
**R^{3a}** is C₁-C₂₀alkyl interrupted by one or more O, S, NR^{10a}, CO, SO or SO₂, which is unsubstituted or substituted by C₃-C₈cycloalkyl, OH, SH, O(CO)-(C₁-C₈alkyl), COOR^{4a}, CONR^{10a}R^{11a}, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR^{4a}, SR^{9a} or NR^{10a}R^{11a}; or
**R^{3a}** is C₂-C₁₂alkenyl or C₃-C₂₀cycloalkyl, each of which is uninterrupted or interrupted by one or more O, S, CO, NR^{10a} or COOR^{4a}; or
**R^{3a}** is C₆-C₂₀aryl or C₃-C₂₀heteroaryl, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR^{4a}, SR^{9a}, NR^{10a}R^{11a}, COOR^{4a}, (CO)-(C₁-C₈alkyl) or SO₂-(C₁-C₄haloalkyl); or
**R^{3a}** is C₁-C₂₀alkoxy, which is unsubstituted or substituted by one or more C₁-C₁₀alkyl, C₁-C₄haloalkyl, halogen, phenyl, C₁-C₂₀alkylphenyl or C₁-C₈alkoxyphenyl; or
**R^{3a}** is C₁-C₂₀alkoxy, which is interrupted by one or more O, S, NR^{10a}, CO, SO or SO₂; or
**R^{3a}** is C₆-C₂₀aryloxy or C₃-C₂₀heteroaryloxy, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR^{4a}, SR^{9a}, NR^{10a}R^{11a}, COOR^{4a}, (CO)-(C₁-C₈alkyl) or SO₂-(C₁-C₄haloalkyl); or
**R^{3a}** is a group 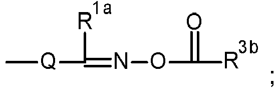
**R^{3b}** is hydrogen or C₁-C₂₀alkyl; or
**R^{3b}** is C₁-C₂₀alkyl substituted by one or more halogen, OR^{4a}, SR^{9a}, NR^{10a}R^{11a}, CN, COOR^{4a}, CONR^{10a}R^{11a}, C₃-C₈cycloalkyl which is uninterrupted or interrupted by one or more O, S, CO or NR^{10a}; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR^{4a}, SR^{9a} or NR^{10a}R^{11a}; or
**R^{3b}** is C₁-C₂₀alkyl interrupted by one or more O, S, NR^{10a}, CO, SO or SO₂, which is unsubstituted or substituted by C₃-C₈cycloalkyl, OH, SH, O(CO)-(C₁-C₈alkyl), COOR^{4a}, CONR^{10a}R^{11a}, C₆-C₂₀aryl, C₃-C₂₀heteroaryl, C₆-C₂₀aroyl or C₃-C₂₀heteroarylcarbonyl, wherein C₆ C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR^{4a}, SR^{9a} or NR^{10a}R^{11a}; or
**R^{3b}** is C₂-C₁₂alkenyl or C₃-C₂₀cycloalkyl, each of which is uninterrupted or interrupted by one or more O, S, CO, NR^{10a} or COOR^{4a}; or
**R^{3b}** is C₆-C₂₀aryl or C₃-C₂₀heteroaryl, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR^{4a}, SR^{9a}, NR^{10a}R^{11a}, COOR^{4a}, (CO)-(C₁-C₈alkyl) or SO₂-(C₁-C₄haloalkyl); or
**R^{3b}** is C₁-C₂₀alkoxy, which is unsubstituted or substituted by one or more C₁-C₁₀alkyl, C₁-C₄haloalkyl, halogen, phenyl, C₁-C₂₀alkylphenyl or C₁-C₈alkoxyphenyl; or
**R^{3b}** is C₁-C₂₀alkoxy, which is interrupted by one or more O, S, NR^{10a}, CO, SO or SO₂; or
**R^{3b}** is C₆-C₂₀aryloxy or C₃-C₂₀heteroaryloxy, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR^{4a}, SR^{9a}, NR^{10a}R^{11a}, COOR^{4a}, (CO)-(C₁-C₈alkyl) or SO₂-(C₁-C₄haloalkyl);
**R⁴** is hydrogen, (CO)-R^{3a}, COOR^{4a}, CONR¹⁰R¹¹, S(O)ₘ-R^{3a} or PO(OR^{3a})₂; or
**R⁴** is C₁-C₂₀alkyl, which is substituted by one or more halogen, OR^{4a}, SR^{9a}, NR^{10a}R^{11a}, CN, COOR^{4a}, CONR^{10a}R^{11a}, PO(OR^{3a})₂, S(O)ₘ-R^{3a}, C₃-C₈cycloalkyl which is uninterrupted or interrupted by one or more O, S, CO or NR^{10a}; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR^{4a}, SR^{9a}, NR^{10a}R^{11a}, PO(OR^{3a})₂ or S(O)ₘ-R^{3a}; or
**R⁴** is C₁-C₂₀alkyl interrupted by one or more O, S, NR^{10a}, CO, SO or SO₂, which is unsubstituted or substituted by C₃-C₈cycloalkyl, OH, SH, O(CO)-R^{3a}, COOR^{4a}, CONR^{10a}R^{11a}, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR^{4a}, SR^{9a} or NR^{10a}R^{11a}; or
**R⁴** is C₂-C₁₂alkenyl or C₃-C₂₀cycloalkyl, each of which is uninterrupted or interrupted by one or more O, S, CO, NR^{10a} or COOR^{4a}; or
**R⁴** is C₆-C₂₀aryl, which is substituted by one or more halogen, CN, NO₂, OR^{4a}, SR^{9a}, NR^{10a}R^{11a}, COOR^{4a}, (CO)-R^{3a}, CONR^{10a}R^{11a}, PO(OR^{3a})₂, S(O)ₘ-R^{3a} or group 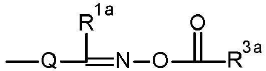 ; or by one or more C₁-C₂₀alkyl which is unsubstituted or substituted by one or more halogen, COOR^{4a}, CONR^{10a}R^{11a}, phenyl, C₃-C₈cycloalkyl, C₃-C₂₀heteroaryl, OR^{4a}, SR^{9a} or NR^{10a}R^{11a}; or by one or more C₂-C₂₀alkyl which is interrupted by one or more O, S or NR^{10a}; or by one or more phenyl, naphthyl, benzoyl or naphthoyl, each of which is unsubstituted or substituted by OR^{4a}, SR^{9a} or NR^{10a}R^{11a}; or
**R⁴** is C₃-C₂₀heteroaryl, each of which is unsubstituted or substituted by one or more halogen, CN, NO₂, OR^{4a}, SR^{9a}, NR^{10a}R^{11a}, COOR^{4a}, (CO)-R^{3a}, CONR^{10a}R^{11a}, PO(OR^{3a})₂, S(O)ₘ-R^{3a} or group 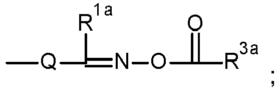 or by one or more C₁-C₂₀alkyl which is unsubstituted or substituted by one or more halogen, COOR^{4a}, CONR^{10a}R^{11a}, phenyl, C₃-C₈cycloalkyl, C₃-C₂₀heteroaryl, OR^{4a}, SR^{9a} or NR^{10a}R^{11a}; or by one or more C₂-C₂₀alkyl which is interrupted by one or more O, S or NR^{10a}; or by one or more phenyl, naphthyl, benzoyl or naphthoyl, each of which is unsubstituted or substituted by OR^{4a}, SR^{9a} or NR^{10a}R^{11a}; or
**R⁴** together with one of the carbon atom of R¹ forms a 5- or 6-membered saturated or unsaturated ring which is uninterrupted or interrupted by O, S or NR^{10a}, and which 5- or 6-membered saturated or unsaturated ring is unsubstituted or substituted by one or more C₁-C₂₀alkyl, OR^{4a}, SR^{9a}, NR^{10a}R^{11a}, (CO)-R^{3a}, NO₂, halogen, C₁-C₄haloalkyl, CN, phenyl, C₁-C₂₀alkylphenyl, C₁-C₈alkoxyphenyl, 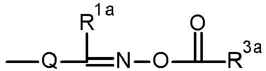 or C₃-C₂₀cyclalkyl which is uninterrupted or interrupted by one or more O, S, CO or NR^{10a};
**R^{4'}** is -(CO)-, -(CO)O-, -(CO)N-, -S(O)ₘ-, -PO(O)₂-, or -OP(O)₂;
**R^{4a}** is hydrogen, C₁-C₂₀alkyl, (CO)O(C₁-C₈alkyl) or CON(C₁-C₈alkyl)₂; or
**R^{4a}** is C₁-C₂₀alkyl substituted by one or more halogen, OH, SH, CN, C₃-C₈alkenoxy, OCH₂CH₂CN, OCH₂CH₂(CO)O(C₁-C₈alkyl), O(CO)-(C₁-C₈alkyl), O(CO)-(C₂-C₄)alkenyl, O(CO)-phenyl, (CO)OH, (CO)O(C₁-C₈alkyl), C₃-C₈cycloalkyl, SO₂-(C₁-C₄haloalkyl), O(C₁-C₄haloalkyl), phenyl, C₁-C₈alkylphenyl, C₁-C₈alkoxyphenyl or C₃-C₈cycloalkyl which is interrupted by one or more O; or
**R^{4a}** is C₂-C₂₀alkyl interrupted by one or more O, S, N(C₁-C₈alkyl), CO, SO or SO₂, which is unsubstituted or substituted by C₃-C₈cycloalkyl, OH, SH, O(CO)(C₁-C₈alkyl), (CO)O(C₁-C₈alkyl), (CO)N(C₁-C₈alkyl)₂, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, C₁-C₈alkoxy, C₁-C₈alkylsulfanyl or N(C₁-C₈alkyl)₂; or
**R^{4a}** is C₂-C₁₂alkenyl, (CO)O(C₁-C₈alkenyl) or C₃-C₈cycloalkyl, each of which is uninterrupted or interrupted by one or more O, S, CO, N(C₁-C₈alkyl) or COO(C₁-C₈alkyl); or
**R^{4a}** is C₆-C₂₀aryl, C₃-C₂₀heteroaryl, C₆-C₂₀aroyl or C₃-C₂₀heteroarylcarbonyl, each of which is unsubstituted or substituted by one or more halogen, CN, NO₂, OH, C₁-C₈alkyl, C₁-C₄haloalkyl, C₁-C₈alkoxy, phenyl-C₁-C₃alkyloxy, phenoxy, C₁-C₈alkylsulfanyl, phenylsulfanyl, N(C₁-C₈alkyl)₂, diphenylamino, (CO)O(C₁-C₈alkyl), (CO)-C₁-C₈alkyl or (CO)N(C₁-C₈)₂, phenyl or benzoyl; or
**R^{4a}** is C₁-C₂₀alkanoyl, C₃-C₁₂alkenoyl, each of which is unsubstituted or substituted by one or more halogen, phenyl, C₁-C₈alkylphenyl, C₁-C₈alkoxyphenyl, OH, C₁₋C₈alkoxy, phenoxy, C₁-C₈alkylsulfanyl, phenylsulfanyl, N(C₁-C₈alkyl)₂ or diphenylamino;
**R⁵, R⁶**, **R⁷** and **R⁸** independently of each other are hydrogen, C₁-C₂₀alkyl, C₆-C₂₀aryl, C₁-C₂₀alkoxy, C₆-C₂₀arylC₁-C₂₀alkyl, hydroxyC₁-C₂₀alkyl, hydroxyC₁-C₂₀alkoxyC₁-C₂₀alkyl, C₃-C₁₀cycloalkyl, amino, CN, NO₂, hydroxy, 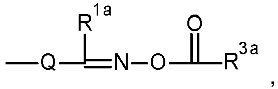 (CO)-R^{3a}, OR^{4a} or COOR⁴
**R⁹** is hydrogen or C₁-C₂₀alkyl; or
**R⁹** is C₁-C₂₀alkyl substituted by one or more halogen, OR^{4a}, SR^{9a}, NR^{10a}R^{11a}, CN, COOR^{4a}, CONR^{10a}R^{11a}, PO(OR^{3a})₂, S(O)ₘ-R^{3a}, C₃-C₈cycloalkyl which is uninterrupted or interrupted by one or more O, S, CO or NR^{10a}; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen phenyl, C₁-C₂₀alkylphenyl, C₁-C₈alkoxyphenyl, C₁-C₄haloalkyl, CN, NO₂, OR^{4a}, SR^{9a}, NR^{10a}R^{11a}, PO(OR^{3a})₂ or S(O)ₘ-R^{3a}; or
**R⁹** is C₁-C₂₀alkyl interrupted by one or more O, S, NR^{10a}, CO, SO or SO₂, which is unsubstituted or substituted by C₃-C₈cycloalkyl, OH, SH, O(CO)-R^{3a}, COOR^{4a}, CONR^{10a}R^{11a}, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR^{4a}, SR^{9a} or NR^{10a}R^{11a}; or
**R⁹** is C₂-C₁₂alkenyl or C₃-C₂₀cycloalkyl, each of which is uninterrupted or interrupted by one or more O, S, CO, NR^{10a} or COOR^{4a}; or
**R⁹** is C₆-C₂₀aryl or C₃-C₂₀heteroaryl, each of which is unsubstituted or substituted by one or more halogen, CN, NO₂, OR^{4a}, SR^{9a}, NR^{10a}R^{11a}, COOR^{4a}, (CO)-R^{3a} CONR^{10a}R^{11a}, PO(OR^{3a})₂, S(O)ₘ-R^{3a} or group 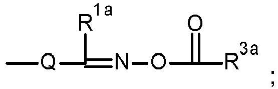 or by one or more C₁-C₂₀alkyl which is unsubstituted or substituted by one or more halogen, COOR^{4a}, CONR^{10a}R^{11a}, phenyl, C₃-C₈cycloalkyl, C₃-C₂₀heteroaryl, OR^{4a}, SR^{9a} or NR^{10a}R^{11a}; or by one or more C₂-C₂₀alkyl which is interrupted by one or more O, S or NR^{10a}; or by one or more phenyl, naphthyl, benzoyl or naphthoyl, each of which is unsubstituted or substituted by OR^{4a}, SR^{9a} or NR^{10a}R^{11a}; or
**R⁹** together with one of the carbon atom of R¹ forms a 5- or 6-membered saturated or unsaturated ring which is uninterrupted or interrupted by O, S or NR^{10a}, and which 5- or 6-membered saturated or unsaturated ring is unsubstituted or substituted by one or more C₁-C₂₀alkyl, OR^{4a}, SR^{9a}, NR^{10a}R^{11a}, (CO)-R^{3a}, NO₂, halogen, C₁-C₄haloalkyl, CN, phenyl, C₁-C₂₀alkylphenyl, C₁-C₈alkoxyphenyl, 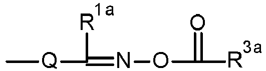 or C₃-C₂₀cyclalkyl which is uninterrupted or interrupted by one or more O, S, CO or NR^{10a};
**R^{9a}** is hydrogen or C₁-C₂₀alkyl; or
**R^{9a}** is C₁-C₂₀alkyl substituted by one or more halogen, OH, SH, CN, C₃-C₈alkenoxy, OCH₂CH₂CN, OCH₂CH₂(CO)O(C₁-C₈alkyl), O(CO)-(C₁-C₈alkyl), O(CO)-(C₂-C₄)alkenyl, O(CO)-phenyl, (CO)OH, (CO)O(C₁-C₈alkyl), C₃-C₈cycloalkyl, SO₂-(C₁-C₄haloalkyl), O(C₁-C₄haloalkyl), phenyl, C₁-C₈alkylphenyl, C₁-C₈alkoxyphenyl or C₃-C₈cycloalkyl which is interrupted by one or more O; or
**R^{9a}** is C₂-C₂₀alkyl interrupted by one or more O, S, N(C₁-C₈alkyl), CO, SO or SO₂, which is unsubstituted or substituted by C₃-C₈cycloalkyl, OH, SH, O(CO)(C₁-C₈alkyl), (CO)O(C₁-C₈alkyl), (CO)N(C₁-C₈alkyl)₂, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, C₁-C₈alkoxy, C₁-C₈alkylsulfanyl or N(C₁-C₈alkyl)₂; or
**R^{9a}** is C₂-C₁₂alkenyl or C₃-C₈cycloalkyl, each of which is uninterrupted or interrupted by one or more O, S, CO, N(C₁-C₈alkyl) or COO(C₁-C₈alkyl); or
**R^{9a}** is C₆-C₂₀aryl, C₃-C₂₀heteroaryl, C₆-C₂₀aroyl or C₃-C₂₀heteroarylcarbonyl, each of which is unsubstituted or substituted by one or more halogen, CN, NO₂, OH, C₁-C₈alkyl, C₁-C₄haloalkyl, C₁-C₈alkoxy, phenyl-C₁-C₃alkyloxy, phenoxy, C₁-C₈alkylsulfanyl, phenylsulfanyl, N(C₁-C₈alkyl)₂, diphenylamino, (CO)O(C₁-C₈alkyl), (CO)-C₁-C₈alkyl or (CO)N(C₁-C₈)₂, phenyl or benzoyl; or
**R^{9a}** is C₁-C₂₀alkanoyl, C₃-C₁₂alkenoyl, each of which is unsubstituted or substituted by one or more halogen, phenyl, C₁-C₈alkylphenyl, C₁-C₈alkoxyphenyl, OH, C₁₋C₈alkoxy, phenoxy, C₁-C₈alkylsulfanyl, phenylsulfanyl, N(C₁-C₈alkyl)₂ or diphenylamino;
**R¹⁰** and **R¹¹** independently of each other are hydrogen, C₁-C₂₀alkyl, S(O)ₘ-R^{3a}, O(CO)-R^{3a} (CO)-R^{3a} or CONR^{10a}R^{11a}; or
**R¹⁰** and **R¹¹** independently of each other are C₁-C₂₀alkyl substituted by one or more halogen, OR^{4a}, SR^{9a}, NR^{10a}R^{11a}, CN, COOR^{4a}, CONR^{10a}R^{11a}, PO(OR^{3a})₂, S(O)ₘ-R^{3a}, C₃-C₈cycloalkyl which is uninterrupted or interrupted by one or more O, S, CO or NR^{10a}; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR^{4a}, SR^{9a}, NR^{10a}R^{11a}, PO(OR^{3a})₂ or S(O)ₘ-R^{3a}; or
**R¹⁰** and **R¹¹** independently of each other are C₁-C₂₀alkyl interrupted by one or more O, S, NR^{10a}, CO, SO or SO₂, which is unsubstituted or substituted by C₃-C₈cycloalkyl, OH, SH, O(CO)-R^{3a}, COOR^{4a}, CONR^{10a}R^{11a}, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR^{4a}, SR^{9a} or NR^{10a}R^{11a}; or
**R¹⁰** and **R¹¹** independently of each other are C₂-C₁₂alkenyl or C₃-C₂₀cycloalkyl, each of which is uninterrupted or interrupted by one or more O, S, CO, NR^{10a} or COOR^{4a}; or
**R¹⁰** and **R¹¹** independently of each other are C₆-C₂₀aryl or C₃-C₂₀heteroaryl, each of which is unsubstituted or substituted by one or more halogen, CN, NO₂, OR^{4a}, SR^{9a}, NR^{10a}R^{11a}, COOR^{4a} (CO)-R^{3a} CONR^{10a}R^{11a}, PO(OR^{3a})₂, S(O)ₘ-R^{3a} or group 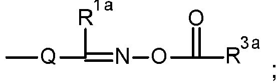 or by one or more C₁-C₂₀alkyl which is unsubstituted or substituted by one or more halogen, COOR^{4a}, CONR^{10a}R^{11a}, phenyl, C₃-C₈cycloalkyl, C₃-C₂₀heteroaryl, OR^{4a}, SR^{9a} or NR^{10a}R^{11a}; or by one or more C₂-C₂₀alkyl which is interrupted by one or more O, S or N R^{10a}; or by one or more phenyl, naphthyl, benzoyl or naphthoyl, each of which is unsubstituted or substituted by OR^{4a}, SR^{9a} or NR^{10a}R^{11a}; or
**R¹⁰** and **R¹¹** independently of each other are C₁-C₂₀alkoxy, which is unsubstituted or substituted by one or more halogen, phenyl, C₁-C₈alkylphenyl or C₁-C₈alkoxyphenyl; or
**R¹⁰** and **R¹¹** independently of each other are C₁-C₂₀alkoxy, which is interrupted by one or more O, S, NR^{10a}, CO, SO or SO₂; or
**R¹⁰** and **R¹¹** independently of each other are C₆-C₂₀aryloxy or C₃-C₂₀heteroaryloxy, each of which is unsubstituted or substituted by one or more halogen, C₁-C₈alkyl, C₁-C₄haloalkyl, phenyl, C₁-C₈alkylphenyl, C₁-C₈alkoxyphenyl, CN, NO₂, OR^{4a}, SR^{9a}, NR^{10a}R^{11a}, COOR^{4a}, (CO)-R^{3a} or SO₂-R^{3a}; or
**R¹⁰** together with one of the carbon atom of R¹ forms a 5- or 6-membered saturated or unsaturated ring which is uninterrupted or interrupted by O, S or NR^{10a}, and which 5- or 6-membered saturated or unsaturated ring is unsubstituted or substituted by one or more C₁-C₂₀alkyl, OR^{4a}, SR^{9a}, NR^{10a}R^{11a}, (CO)-R^{3a}, NO₂, halogen, C₁-C₄-haloalkyl, CN, phenyl, C₁-C₂₀alkylphenyl, C₁-C₈alkoxyphenyl, 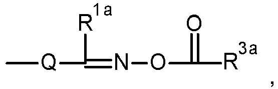 or C₃-C₂₀cyclalkyl which is uninterrupted or interrupted by one or more O, S, CO or NR^{10a}; or
**R¹⁰** and **R¹¹** together with the N-atom to which they are attached form a 5- or 6-membered saturated or unsaturated ring which is uninterrupted or interrupted by O, S or NR^{10a}, and which 5- or 6-membered saturated or unsaturated ring is unsubstituted or substituted by one or more C₁-C₂₀alkyl, OR^{4a}, SR^{9a}, NR^{10a}R^{11a}, (CO)-R^{3a}, NO₂, halogen, C₁-C₄-haloalkyl, CN, phenyl, 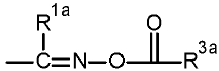 or C₃-C₂₀cyclalkyl which is uninterrupted or interrupted by one or more O, S, CO or NR^{10a};
**R^{10a}** and **R^{11a}** independently of each other are hydrogen, C₁-C₂₀alkyl, S(O)ₘ-(C₁-C₈alkyl), O(CO)(C₁-C₈alkyl), (CO)(C₁-C₈alkyl), (CO)O(C₁-C₈alkyl) or CON(C₁-C₈alkyl)₂; or
**R^{10a}** and **R^{11a}** independently of each other are C₁-C₂₀alkyl substituted by one or more halogen, OH, SH, CN, C₃-C₈alkenoxy, OCH₂CH₂CN, OCH₂CH₂(CO)O(C₁-C₈alkyl), O(CO)-(C₁-C₈alkyl), O(CO)-(C₂-C₄)alkenyl, O(CO)-phenyl, (CO)OH, (CO)O(C₁-C₈alkyl), C₃-C₈cycloalkyl, SO₂-(C₁-C₄haloalkyl), O(C₁-C₄haloalkyl), phenyl, C₁-C₈alkylphenyl, C₁-C₈alkoxyphenyl or C₃-C₈cycloalkyl which is interrupted by one or more O; or
**R^{10a}** and **R^{11a}** independently of each other are C₂-C₂₀alkyl interrupted by one or more O, S, N(C₁-C₈alkyl), CO, SO or SO₂, which is unsubstituted or substituted by C₃-C₈cycloalkyl, OH, SH O(CO)(C₁-C₈alkyl), (CO)O(C₁-C₈alkyl), (CO)N(C₁-C₈alkyl)₂, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, C₁-C₈alkoxy, C₁-C₈alkylsulfanyl or N(C₁-C₈alkyl)₂; or
**R^{10a}** and **R^{11a}** independently of each other are C₂-C₁₂alkenyl or C₃-C₈cycloalkyl, each of which is uninterrupted or interrupted by one or more O, S, CO, N(C₁-C₈alkyl) or COO(C₁-C₈alkyl); or
**R^{10a}** and **R^{11a}** independently of each other are C₆-C₂₀aryl, C₃-C₂₀heteroaryl, C₆-C₂₀aroyl or C₃-C₂₀heteroarylcarbonyl, each of which is unsubstituted or substituted by one or more halogen, CN, NO₂, OH, C₁-C₈alkyl, C₁-C₄haloalkyl, C₁-C₈alkoxy, phenyl-C₁-C₃alkyloxy, phenoxy, C₁-C₈alkylsulfanyl, phenylsulfanyl, N(C₁-C₈alkyl)₂, diphenylamino, (CO)O(C₁-C₈alkyl), (CO)-C₁-C₈alkyl or (CO)N(C₁-C₈alkyl)₂, phenyl or benzoyl; or
**R^{10a}** and **R^{11a}** independently of each other are C₁-C₂₀alkanoyl, C₃-C₁₂alkenoyl, each of which is unsubstituted or substituted by one or more halogen, phenyl, C₁-C₈alkylphenyl, C₁-C₈alkoxyphenyl, OH, C₁-C₈alkoxy, phenoxy, C₁-C₈alkylsulfanyl, phenylsulfanyl, N(C₁-C₈alkyl)₂ or diphenylamino; or
**R^{10a}** and **R^{11a}** independently of each other are C₁-C₂₀alkoxy, which is unsubstituted or substituted by one or more halogen, phenyl, C₁-C₈alkylphenyl or C₁-C₈alkoxyphenyl; or
**R^{10a}** and **R^{11a}** independently of each other are C₁-C₂₀alkoxy, which is interrupted by one or more O, S, N(C₁-C₈alkyl), CO, SO or SO₂; or
**R^{10a}** and **R^{11a}** independently of each other are C₆-C₂₀aryloxy or C₃-C₂₀heteroaryloxy, each of which is unsubstituted or substituted by one or more halogen, C₁-C₈alkyl, C₁-C₄haloalkyl, phenyl, C₁-C₈alkylphenyl, C₁-C₈alkoxyphenyl, CN, NO₂, C₁-C₈alkoxy, C₁-C₈alkylsulfanyl, N(C₁-C₈alkyl)₂, CO(OC₁-C₈alkyl), (CO)-(C₁-C₈alkyl) or SO₂-(C₁C₈alkyl); or
**R^{10a}** and **R^{11a}** together with the N-atom to which they are attached form a 5- or 6-membered saturated or unsaturated ring which is uninterrupted or interrupted by O, S or N(C₁-C₈alkyl), and which 5- or 6-membered saturated or unsaturated ring is unsubstituted or substituted by one or more C₁-C₈alkyl, C₁-C₈alkoxy, C₁-C₈alkylsulfanyl, N(C₁-C₈alkyl)₂, NO₂, halogen, C₁-C₄haloalkyl, CN, phenyl or C₃-C₂₀cyclalkyl which is uninterrupted or interrupted by one or more O, S, CO or N(C₁-C₈alkyl);
**R¹²** and **R¹³** independently of each other are hydrogen, C₁-C₁₂alkyl optionally substituted by one or more halogen, phenyl, CN, -OH, -SH, C₁-C₄alkoxy, (CO)OH or (CO)O(C₁-C₄alkyl); or
**R¹²** and **R¹³** are phenyl optionally substituted by one or more C₁-C₆alkyl, halogen, CN, OR⁴, SR⁹ or NR¹⁰R¹¹; or
**R¹²** and **R¹³** are halogen, CN, OR⁴, SR⁹, SOR⁹, SO₂R⁹ or NR¹⁰R¹¹, wherein the substituents OR⁴, SR⁹ or NR¹⁰R¹¹ optionally form 5- or 6-membered rings via the radicals R⁴, R⁹, R¹⁰ and/or R¹¹ with one of the carbon atoms of the phenyl, naphthyl, benzoyl or naphthoyl group or that of the substituent R^{3a}; or
**R¹²** and **R¹³** together are a group 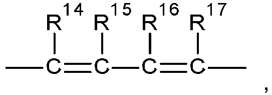 wherein R¹⁴, R¹⁵, R¹⁶ and R¹⁷ independently of one another are hydrogen, C₁-C₁₂alkyl optionally substituted by one or more halogen, phenyl, CN, -OH, -SH, C₁-C₄alkoxy, (CO)OH or (CO)O(C₁-C₄alkyl); or R¹⁴, R¹⁵, R¹⁶ and R¹⁷ are phenyl optionally substituted by one or more C₁-C₆alkyl, halogen, CN, OR⁴, SR⁹ or NR¹⁰R¹¹; or R¹⁴, R¹⁵, R¹⁶ and R¹⁷ are halogen, CN, OR⁴, SR⁹ or NR¹⁰R¹¹; or
**R¹²** and **R¹³** together are a group 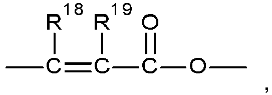 wherein R¹⁸ and R¹⁹ independently of each other are hydrogen, C₁-C₁₂alkyl optionally substituted by one or more halogen, phenyl, CN, -OH, -SH, C₁-C₄-alkoxy, (CO)OH or (CO)O(C₁-C₄alkyl); or R¹⁸ and R¹⁹ are phenyl optionally substituted by one or more C₁-C₆alkyl, halogen, CN, OR⁴, SR⁹ or NR¹⁰R¹¹;
**M** is a direct bond or a divalent linking group C₁-C₂₀alkylene or C₁-C₂₀alkylene substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹ or NR¹⁰R¹¹; or
**M** is C₁-C₂₀alkylene interrupted by one or more O, S, NR¹⁰, CO, SO or SO₂, which is unsubstituted or substituted by C₃-C₈cycloalkyl, OH, SH, O(CO)-R^{3a}, COOR⁴, CONR¹⁰R¹¹, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR⁴, SR⁹ or NR¹⁰R¹¹; or
**M** is C₂-C₁₂alkenylene or C₃-C₂₀cycloalkylene, each of which is uninterrupted or interrupted by one or more O, S, CO, NR¹⁰ or COOR⁴; or
**M** is C₆-C₂₀arylene or C₃-C₂₀heteroarylene, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR⁴, SR⁹, NR¹⁰R¹¹, COOR⁴, (CO)-R^{3a} or SO₂-R^{3a};
**M'** is a direct bond or a divalent linking group selected from C₁-C₂₀alkylene or C₁-C₂₀alkylene substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹ or NR¹⁰R¹¹; or
**M'** is C₁-C₂₀alkylene interrupted by one or more O, S, NR¹⁰, CO, SO or SO₂, which is unsubstituted or substituted by C₃-C₈cycloalkyl, OH, SH, O(CO)-R^{3a}, COOR⁴, CONR¹⁰R¹¹, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR⁴, SR⁹ or NR¹⁰R¹¹; or
**M'** is C₂-C₁₂alkenylene or C₃-C₂₀cycloalkylene, each of which is uninterrupted or interrupted by one or more O, S, CO, NR¹⁰ or COOR⁴; or
**M'** is C₆-C₂₀arylene or C₃-C₂₀heteroarylene, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR⁴, SR⁹, NR¹⁰R¹¹, COOR⁴, (CO)-R^{3a} or SO₂-R^{3a};
**M"** is a direct bond or a divalent linking group selected from C₁-C₂₀alkylene or C₁-C₂₀alkylene substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹ or NR¹⁰R¹¹; or
**M"** is C₁-C₂₀alkylene interrupted by one or more O, S, NR¹⁰, CO, SO or SO₂, which is unsubstituted or substituted by C₃-C₈cycloalkyl, OH, SH, O(CO)-R^{3a}, COOR⁴, CONR¹⁰R¹¹, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR⁴, SR⁹ or NR¹⁰R¹¹; or
**M"** is C₂-C₁₂alkenylene or C₃-C₂₀cycloalkylene, each of which is uninterrupted or interrupted by one or more O, S, CO, NR¹⁰ or COOR⁴; or
**M"** is C₆-C₂₀arylene or C₃-C₂₀heteroarylene, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR⁴, SR⁹, NR¹⁰R¹¹, COOR⁴, (CO)-R^{3a} or SO₂-R^{3a};
**m** is 1 or 2;
**Q** is CO or a direct bond; and
**x, y** and **z** independently of each other are an integer 1, 2, 3 or 4.

2. Compounds of the formula I, II, III and IV according to claim 1, wherein the compounds are compounds of the formula la, Ila, Illa or IVa: 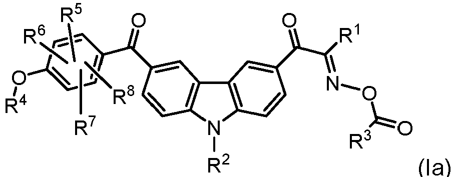 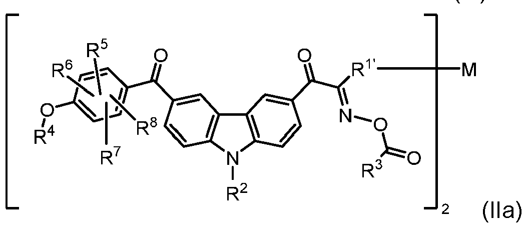 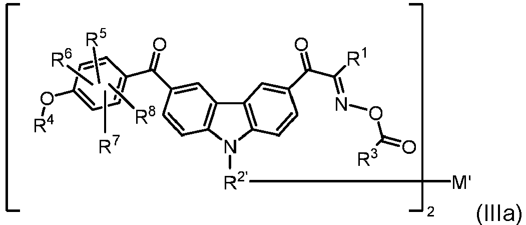 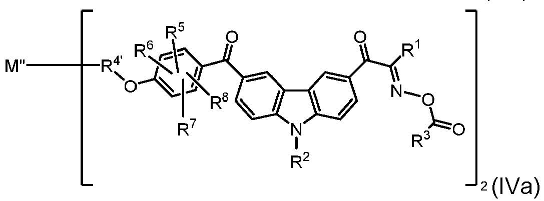 wherein
**R¹** to **R⁸, R^{1'}, R^{2'}, R^{4'}, M, M'** and **M"** are defined as in claim 1.

3. Compounds of the formula I, II, III and IV according to claim 1 or 2, wherein the compounds are compounds of the formula Ib, lib, IIIb or IVb: 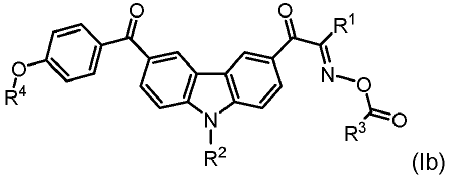 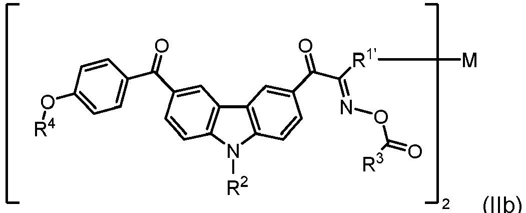 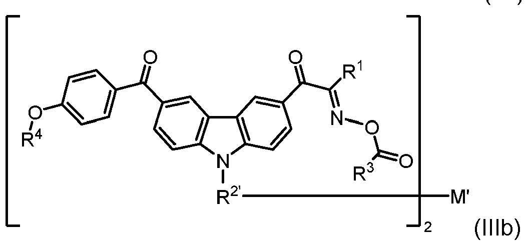 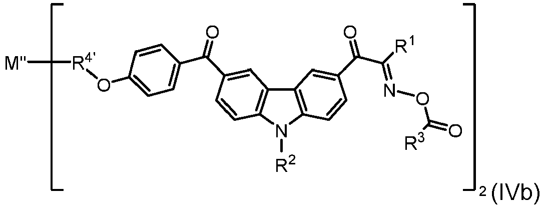 wherein
**R¹** is hydrogen, C₁-C₂₀alkyl, C₁-C₆alkyl-C₃-C₆cycloalkyl, C₃-C₂₀cycloalkyl, C₂-C₁₂alkenyl, wherein C₃-C₂₀cycloalkyl or C₂-C₁₂alkenyl is uninterrupted or interrupted by one or more O, S, CO, NR¹⁰ or COOR⁴; or
**R¹** is C₂-C₂₀alkyl which is interrupted by one or more O, CO, S, C(O)O, OC(O), SO, SO₂, phenylene, naphthylene or NR¹⁰, wherein the interrupted C₂-C₂₀alkyl is unsubstituted or substituted by one or more halogen, C₃-C₈cycloalkyl, OH, SH, OR⁴, SR⁹, COOR⁴, O(CO)-R^{3a}, CONR¹⁰R¹¹, NR¹⁰R¹¹, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR⁴, SR⁹ or NR¹⁰R¹¹; or
**R¹** is C₆-C₂₀aryl or C₃-C₂₀heteroaryl each of which is unsubstituted or substituted by one or more C₁-C₂₀alkyl;
**R^{1'}** is C₁-C₂₀alkylene, C₁-C₂₀alkylene which is interrupted by one or more O, S, (CO)O, O(CO), phenylene, naphthylene or NR⁸, wherein the C₁-C₂₀alkylene and interrupted C₁-C₂₀alkylene is unsubstituted or substituted by halogen or OR⁴,
**R²** is hydrogen, C₁-C₂₀alkyl which is unsubstituted or substituted by one or more halogen, OR⁴, SR⁹, COOR⁴ CONR¹⁰R¹¹, NR¹⁰R¹¹, PO(OR^{3a})₂; (CO)-R^{3a}, or
**R²** is C₂-C₂₀alkyl or C₁-C₆alkyl-C₃-C₆-cycloalkyl which is interrupted by one or more O, CO, S, C(O)O, OC(O), SO, SO₂, phenylene, naphthylene or NR¹⁰; or
**R²** is C₆-C₂₀aryl or C₃-C₂₀heteroaryl each of which is unsubstituted or substituted by one or more C₁-C₁₂alkyl, (CO)-R^{3a};
**R^{2'}** is C₁-C₂₀alkylene, C₁-C₂₀alkylene which is interrupted by one or more O, S, (CO)O, O(CO), phenylene, naphthylene or NR⁸, wherein the C₁-C₂₀alkylene and interrupted C₁-C₂₀alkylene is unsubstituted or substituted by halogen or OR⁴,
**R³ is** hydrogen or C₁-C₂₀alkyl; or
**R³** is C₁-C₂₀alkyl substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹ or NR¹⁰R¹¹;
**R^{3a}** is hydrogen or C₁-C₂₀alkyl; or
**R^{3a}** is C₁-C₂₀alkyl interrupted by one or more O, S, NR^{10a}, CO, SO or SO₂, which is unsubstituted or substituted by C₃-C₈cycloalkyl, OH, SH, O(CO)-(C₁-C₈alkyl), COOR^{4a}, CONR^{10a}R^{11a}, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, OR^{4a}, SR^{9a} or NR^{10a}R^{11a}; or
**R^{3a}** is C₆-C₂₀aryl or C₃-C₂₀heteroaryl, each of which is unsubstituted or substituted by one or more halogen, C₁-C₂₀alkyl, C₁-C₄haloalkyl, phenyl, C₁-C₂₀alkylphenyl, C₁-C₈alkoxyphenyl, CN, NO₂, OR^{4a}, SR^{9a}, NR^{10a}R^{11a}, COOR^{4a}, (CO)-(C₁-C₈alkyl) or SO₂-(C₁-C₄haloalkyl);
**R⁴** is hydrogen, (CO)-R^{3a}, COOR^{4a}, CONR¹⁰R¹¹, S(O)ₘ-R^{3a} or PO(OR^{3a})₂;
**R^{4'}** is -(CO)-, -(CO)O-, -(CO)N-, -S(O)ₘ-, -PO(O)₂-, or-OP(O)₂;
**R^{4a}** is hydrogen, C₁-C₂₀alkyl, (CO)O(C₁-C₈alkyl) or CON(C₁-C₈alkyl)₂; or
**R^{4a}** is C₂-C₂₀alkyl interrupted by one or more O, S, N(C₁-C₈alkyl), CO, SO or SO₂, which is unsubstituted or substituted by C₃-C₈cycloalkyl, OH, SH, O(CO)(C₁-C₈alkyl), (CO)O(C₁-C₈alkyl), (CO)N(C₁-C₈alkyl)₂, C₆-C₂₀aryl, C₃-C₂₀heteroaryl, C₆-C₂₀aroyl or C₃-C₂₀heteroarylcarbonyl, wherein C₆-C₂₀aryl, C₃-C₂₀heteroaryl, C₆-C₂₀aroyl or C₃-C₂₀heteroarylcarbonyl is unsubstituted or substituted by one or more halogen, C₁-C₈alkyl, C₁-C₈alkoxy, C₁-C₈alkylsulfanyl or N(C₁-C₈alkyl)₂; or
**R^{4a}** is C₆-C₂₀aryl, C₃-C₂₀heteroaryl, C₆-C₂₀aroyl or C₃-C₂₀heteroarylcarbonyl, each of which is unsubstituted or substituted by one or more halogen, CN, NO₂, OH, C₁-C₈alkyl, C₁-C₄haloalkyl, C₁-C₈alkoxy, phenyl-C₁-C₃alkyloxy, phenoxy, C₁-C₈alkylsulfanyl, phenylsulfanyl, N(C₁-C₈alkyl)₂, diphenylamino, (CO)O(C₁-C₈alkyl), (CO)-C₁-C₈alkyl or (CO)N(C₁-C₈)₂, phenyl or benzoyl;
**R⁸** is hydrogen, C₁-C₂₀alkyl or C₆-C₂₀aryl;
**R⁹** is hydrogen or C₁-C₂₀alkyl;
**R^{9a}** is hydrogen or C₁-C₂₀alkyl;
**R¹⁰** and **R¹¹** independently of each other are hydrogen, C₁-C₂₀alkyl, S(O)ₘ-R^{3a}, O(CO)-R^{3a} (CO)-R^{3a} or CONR^{10a}R^{11a};
**R^{10a}** and **R^{11a}** independently of each other are hydrogen, C₁-C₂₀alkyl, S(O)ₘ-(C₁-C₈alkyl), O(CO)(C₁-C₈alkyl), (CO)(C₁-C₈alkyl), (CO)O(C₁-C₈alkyl) or CON(C₁-C₈alkyl)₂;
**M** is a direct bond or a divalent linking group C₁-C₂₀alkylene or C₁-C₂₀alkylene substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹ or NR¹⁰R¹¹;
**M'** is a direct bond or a divalent linking group selected from C₁-C₂₀alkylene or C₁-C₂₀alkylene substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹ or NR¹⁰R¹¹;
**M"** is a direct bond or a divalent linking group selected from C₁-C₂₀alkylene or C₁-C₂₀alkylene substituted by one or more halogen, OR⁴, SR⁹, NR¹⁰R¹¹, CN, COOR⁴, CONR¹⁰R¹¹, C₃-C₈cycloalkyl which is uninterrupted or interrupted by one or more O, S, CO or NR¹⁰; or by one or more C₆-C₂₀aryl, C₃-C₂₀heteroaryl, C₆-C₂₀aroyl or C₃-C₂₀heteroarylcarbonyl, each of which is unsubstituted or substituted by one or more halogen, phenyl, C₁-C₂₀alkylphenyl, C₁-C₈alkoxyphenyl, C₁-C₄haloalkyl, CN, NO₂, OR⁴, SR⁹ or NR¹⁰R¹¹; and
**m** is 1 or 2.

4. Compounds of the formula I, II, III and IV according to claim 3, wherein the compounds are compounds of the formula Ib, wherein
**R¹** is C₁-C₂₀alkyl, C₁-C₆alkyl-C₃-C₆cycloalkyl, C₃-C₂₀cycloalkyl; or
**R¹** is C₂-C₂₀alkyl which is interrupted by one or more O, CO, S, C(O)O, OC(O), SO, or SO₂; or
**R¹** is C₆-C₂₀aryl which is unsubstituted or substituted by one or more C₁-C₂₀alkyl;
**R²** is C₁-C₂₀alkyl; or
**R²** is C₂-C₂₀alkyl or C₁₋C₆alkyl-C₃-C₆-cycloalkyl which is interrupted by one or more O, CO, S, C(O)O, OC(O), SO or SO₂; or
**R²** is C₆-C₂₀aryl which is unsubstituted or substituted by one or more C₁-C₁₂alkyl or (CO)-R^{3a};
**R³** is C₁-C₂₀alkyl;
R^{3a} is C₁-C₂₀alkyl, C₆-C₂₀aryl or C₃-C₂₀heteroaryl;
**R⁴** is (CO)-R^{3a}, COOR^{4a}, CONR¹⁰R¹¹, S(O)ₘ-R^{3a} or PO(OR^{3a})₂;
**R^{4a}** is C₁-C₂₀alkyl, C₆-C₂₀aryl or C₃-C₂₀heteroaryl;
**R¹⁰** and **R¹¹** are C₁-C₂₀alkyl; and
**m** is 1 or 2.

5. Compounds of the formula I, II, III and IV according to any one of claims 1 to 4, wherein
(i) more than 50%, preferably 55% or more, more preferably 75% or more, still more preferably 90% or more, such as 95% or more, of the C=N double bonds present in oxime ester groups in the molecule are in Z configuration; or
(ii) more than 50%, preferably 55% or more, more preferably 75% or more, still more preferably 90% or more, such as 95% or more, of the C=N double bonds present in oxime ester groups in the molecule are in E configuration.

6. A photopolymerizable composition comprising
(a) at least one ethylenically unsaturated photopolymerizable compound and
(b) as photoinitiator, at least one compound of the formula I, II, III or IV as defined in any one of claims 1 to 5.

7. A photopolymerizable composition according to claim 6, wherein the component (a) is a resin obtained by the reaction of a saturated or unsaturated polybasic acid anhydride with a product of the reaction of an epoxy resin and an unsaturated monocarboxylic acid; and/or
wherein additionally to the photoinitiator (b) the photopolymerizable composition comprises at least one further photoinitiator (c), and/or other additives (d).

8. A photopolymerizable composition according to claim 6 as further additive (d) comprising a pigment, or a mixture of pigments, or a dye, or a mixture of dyes, or a mixture of one or more pigments with one or more dyes; *preferably as* further additive (d) comprising a dispersant or a mixture of dispersants.

9. A photopolymerizable composition according to any one of claims 6 to 8, comprising 0.05 to 25 % by weight of the photoinitiator (b), or the photoinitiators (b) and (c), based on the weight of the composition; and/or
as further additive (d) comprising a photosensitizer, in particular a compound selected from the group consisting of benzophenone, benzophenone derivatives, thioxanthone, thioxanthone derivatives, anthraquinone, anthraquinone derivatives, coumarin and coumarine derivatives; and/or
additionally comprising a binder polymer (e), in particular a copolymer of methacrylate and methacrylic acid.

10. A process for the preparation of a compound of the formula I, II, III or IV according to claim 1 by reacting the corresponding oxime compound with an acyl halide of the formula I' or an anhydride of the formula I" 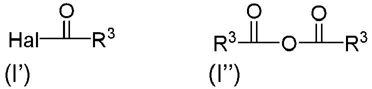 wherein Hal is a halogen, in particular Cl, and R³ is as defined in claim 1, in the presence of a base or a mixture of bases.

11. A process for the photopolymerization of compounds containing ethylenically unsaturated double bonds, which comprises irradiating a composition according to any one of claims 6 to 9 with electromagnetic radiation in the range from 150 to 600 nm, or with electron beam or with X-rays.

12. A process according to claim 11 for producing pigmented and nonpigmented paints and varnishes, powder coatings, printing inks, printing plates, adhesives, pressure sensitive adhesives, dental compositions, gel coats, photoresists for electronics, electroplating resists, etch resists, both liquid and dry films, solder resists, resists to manufacture color filters for a variety of display applications, resists to generate structures in the manufacturing processes of plasma-display panels, electroluminescence displays and LCD, spacers for LCD, for holographic data storage (HDS), as composition for encapsulating electrical and electronic components, for producing magnetic recording materials, micromechanical parts, waveguides, optical switches, plating masks, etch masks, colour proofing systems, glass fibre cable coatings, screen printing stencils, for producing three-dimensional objects by means of stereolithography, as image recording material, for holographic recordings, microelectronic circuits, decolorizing materials, decolorizing materials for image recording materials, for image recording materials using microcapsules, as a photoresist material for a UV and visible laser direct imaging system, as a photoresist material used for forming dielectric layers in a sequential build-up layer of a printed circuit board.

13. Coated substrate which is coated on at least one surface with a composition according to any one of claims 6 to 9.

14. Process for the photographic production of relief images, in which a coated substrate according to claim 13 is subjected to imagewise exposure and then the unexposed portions are removed with a developer.

15. A color filter prepared by providing red, green and blue picture elements and a black matrix, all comprising a photosensitive resin and a pigment on a transparent substrate and providing a transparent electrode either on the surface of the substrate or on the surface of the color filter layer, wherein said photosensitive resin comprises a polyfunctional acrylate monomer, an organic polymer binder and a photopolymerization initiator of formula I, II, III or IV as defined in claim 1.

16. Use of a compound of the formula I, II, III or IV as defined in claim 1 for the photopolymerization of a composition comprising at least one ethylenically unsaturated photopolymerizable compound.

17. Compounds of the formula IA, IIA, IIIA and IVA 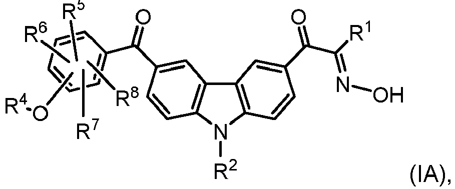 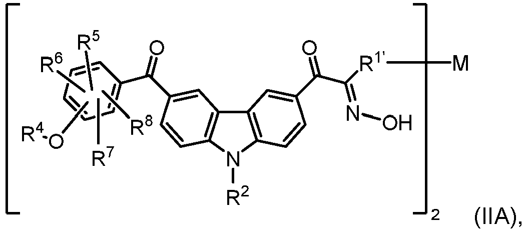 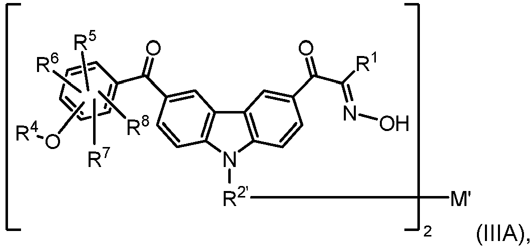 and 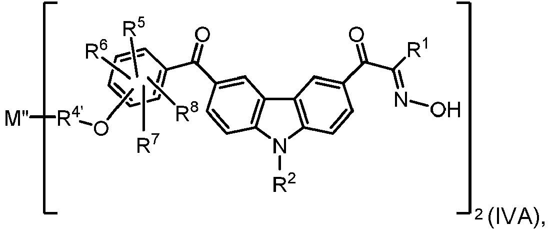 wherein
**R¹, R², R⁴** to **R⁸, R^{1'}, R^{2'}, R^{4'}, M, M'** and **M"** are defined as in any one of claims 1 to 4.

## Patentansprüche

1. Verbindungen der Formel I, II, III und IV 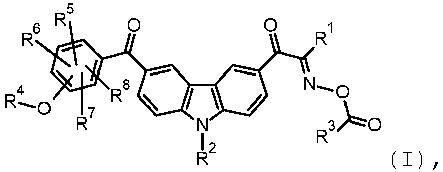 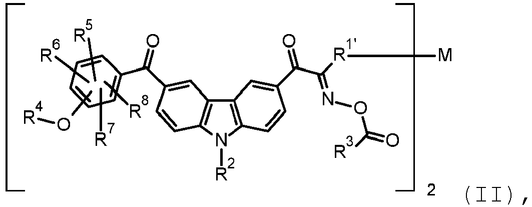 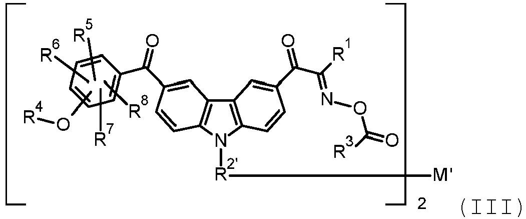 und 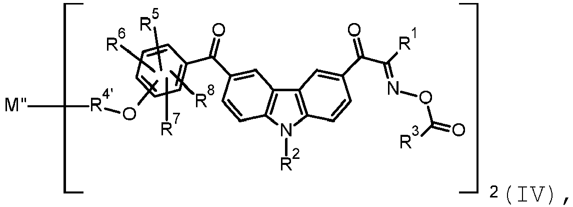 wobei
R¹ für Wasserstoff, C₁-C₂₀-Alkyl, C₁-C₆-Alkyl-C₃-C₆-cycloalkyl, C₃-C₂₀-Cycloalkyl oder C₂-C₁₂-Alkenyl steht, wobei C₃-C₂₀-Cycloalkyl oder C₂-C₁₂-Alkenyl ununterbrochen oder durch ein oder mehrere 0, S, CO, NR¹⁰ oder COOR⁴ unterbrochen ist; oder
R¹ für C₁-C₂₀-Alkyl steht, das unsubstituiert oder durch ein oder mehrere Halogen, OR⁴, SR⁹, NR¹⁰R¹¹, COOR⁴ CONR¹⁰R¹¹, CN, PO(OR^{3a})₂, S(O)ₘ-R^{3a}, 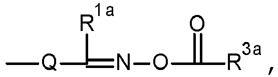 C₃-C₈-Cycloalkyl, das ununterbrochen oder durch ein oder mehrere 0, S, CO oder NR¹⁰ unterbrochen ist, oder durch ein oder mehrere C₆-C₂₀-Aryl, C₃-C₂₀-Heteroaryl, C₆-C₂₀-Aroyl oder C₃-C₂₀-Heteroarylcarbonyl, wobei jede dieser Gruppen unsubstituiert oder durch ein oder mehrere Halogen, Phenyl, C₁-C₂₀-Alkylphenyl, C₁-C₈-Alkoxyphenyl, C₁-C₄-Halogenalkyl, CN, NO₂, OR⁴, SR⁹, NR¹⁰R¹¹, PO(OR^{3a})₂ oder S(O)ₘ-R^{3a} substituiert ist, substituiert ist; oder
R¹ für C₂-C₂₀-Alkyl steht, das durch ein oder mehrere 0, CO, S, C(O)O, OC(O), SO, SO₂, Phenylen, Naphthylen oder NR¹⁰ unterbrochen ist, wobei das unterbrochene C₂-C₂₀-Alkyl unsubstituiert oder durch ein oder mehrere Halogen, C₃-C₈-Cycloalkyl, OH, SH, OR⁴, SR⁹, COOR⁴, O(CO)-R^{3a}, CONR¹⁰R¹¹, NR¹⁰R¹¹, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, OR⁴, SR⁹ oder NR¹⁰R¹¹ substituiert ist; oder
R¹ für C₆-C₂₀-Aryl oder C₃-C₂₀-Heteroaryl steht, wobei jede dieser Gruppen unsubstituiert oder durch ein(e) oder mehrere C₁-C₂₀-Alkyl, Phenyl, Halogen, C₁-C₄-Halogenalkyl, ON, NO₂, OR⁴, SR⁹, NR¹⁰R¹¹, COOR⁴, (CO)-R^{3a}, (CO)NR¹⁰R¹¹, PO(OR^{3a})₂, S(O)ₘ-R^{3a} oder Gruppen 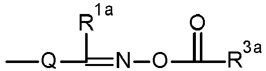 oder durch ein oder mehrere C₂-C₂₀-Alkyl, das durch ein oder mehrere 0, S oder NR¹⁰ unterbrochen ist, oder durch ein oder mehrere C₁-C₂₀-Alkyl, das unsubstituiert oder durch ein oder mehrere Halogen, COOR⁴, CONR¹⁰R¹¹, Phenyl, C₃-C₈-Cycloalkyl, C₃-C₂₀-Heteroaryl, C₆-C₂₀-Aryloxycarbonyl, C₃-C₂₀-Heteroaryloxycarbonyl, OR⁴, SR⁹ oder NR¹⁰R¹¹ oder durch ein oder mehrere Phenyl, Naphthyl, Benzoyl oder Naphthoyl, wobei jede dieser Gruppen unsubstituiert oder durch OR⁴, SR⁹ oder NR¹⁰R¹¹ substituiert ist, substituiert ist, substituiert ist;
R¹ für C₂-C₂₀-Alkanoyl oder Benzoyl, das unsubstituiert oder durch ein oder mehrere C₁-C₆-Alkyl, Phenyl, OR⁴, SR⁹ oder NR¹⁰R¹¹ substituiert ist, steht; oder
R¹ für C₂-C₁₂-Alkoxycarbonyl, das gegebenenfalls durch ein oder mehrere -0- unterbrochen und/oder gegebenenfalls durch ein oder mehrere Hydroxylgruppen substituiert ist, steht; oder
R¹ für Phenoxycarbonyl, das unsubstituiert oder durch C₁-C₆-Alkyl, Halogen, Phenyl, OR⁴, SR⁹ oder NR¹⁰R¹¹ substituiert ist, steht; oder
R¹ für CN, (CO) -R^{3a}, COOR⁴, CONR¹⁰R¹¹, NO₂, PO(OR^{3a})₂ oder S(O)ₘ-R^{3a} steht;
R¹' für C₁-C₂₀-Alkylen, C₁-C₂₀-Alkylen, das durch ein oder mehrere 0, S, (C0)0, O(CO), Phenylen, Naphthylen oder NR⁸ unterbrochen ist, steht, wobei das C₁-C₂₀-Alkylen und unterbrochene C₁-C₂₀-Alkylen unsubstituiert oder durch Halogen oder OR⁴ substituiert sind, oder
R¹' für C₂-C₂₀-Alkenylen, C₂-C₂₀-Alkenylen, das durch ein oder mehrere 0, S, (C0)0, 0(CO), Phenylen, Naphthylen oder durch NR¹⁰ unterbrochen ist, steht, wobei das C₂-C₂₀-Alkenylen und unterbrochene C₂-C₂₀-Alkenylen unsubstituiert oder durch Halogen oder OR⁴ substituiert sind, oder
R¹' für C₅-C₈-Cycloalkylen, C₅-C₈-Cycloalkenylen, 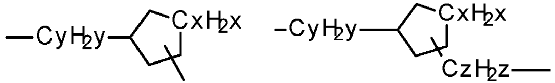 steht, wobei die Gruppen C_{y}H_{2y} und C_{z}H_{2z} ununterbrochen oder durch ein oder mehrere 0, S oder durch NR¹⁰ unterbrochen sind;
R^{1a} für Wasserstoff, C₁-C₂₀-Alkyl, CN, (CO) -R^{3a}, COOR^{4a}, CONR^{10a}R^{11a}, NO₂, PO(OR^{3a})₂ oder S(O)ₘ-R^{3a} steht; oder
R^{1a} für C₁-C₂₀-Alkyl steht, das durch ein oder mehrere Halogen, OR^{4a}, SR^{9a}, NR^{10a}R^{11a}, CN, COOR^{4a}, CONR^{10a}R^{11a}, PO(OR^{3a})₂, S(O)ₘ-R^{3a}, C₃-C₈-Cycloalkyl, das ununterbrochen oder durch ein oder mehrere 0, S, CO oder NR^{10a} unterbrochen ist, oder durch ein oder mehrere C₆-C₂₀-Aryl, C₃-C₂₀-Heteroaryl, C₆-C₂₀-Aroyl oder C₃-C₂₀-Heteroarylcarbonyl, wobei jede dieser Gruppen unsubstituiert oder durch ein oder mehrere Halogen, Phenyl, C₁-C₂₀-Alkylphenyl, C₁-C₈-Alkoxyphenyl, C₁-C₄-Halogenalkyl, CN, NO₂, OR^{4a}, SR^{9a}, NR^{10a}R^{11a}, PO(OR^{3a})₂ oder S(O)ₘ-R^{3a} substituiert ist, substituiert ist; oder
R^{1a} für C₁-C₂₀-Alkyl steht, das durch ein oder mehrere 0, S, NR^{10a}, CO, SO oder SO₂ unterbrochen ist, das unsubstituiert oder durch C₃-C₈-Cycloalkyl, OH, SH, O(CO)R^{3a}, COOR^{4a}, CONR^{10a}R^{11a}, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, OR^{4a}, SR^{9a} oder NR^{10a}R^{11a} substituiert ist; oder
R^{1a} für C₂-C₁₂-Alkenyl oder C₃-C₂₀-Cycloalkyl steht, wobei jede dieser Gruppen ununterbrochen oder durch ein oder mehrere 0, S, CO, NR^{10a} oder COOR^{4a} unterbrochen ist; oder R^{1a} für C₆-C₂₀-Aryl oder C₃-C₂₀-Heteroaryl steht, wobei jede dieser Gruppen unsubstituiert oder durch ein oder mehrere Halogen, CN, N02, OR^{4a}, SR^{9a}, NR^{10a}R^{11a}, COOR^{4a}, (CO)-R^{3a} CONR^{10a}R^{11a}, PO(OR^{3a})₂ oder S(O)ₘ-R^{3a} oder durch ein oder mehrere C₁-C₂₀-Alkyl, das unsubstituiert oder durch ein oder mehrere Halogen, COOR^{4a}, CONR^{10a}R^{11a}, Phenyl, C₃-C₈-Cycloalkyl, C₃-C₂₀-Heteroaryl, OR^{4a}, SR^{9a} oder NR^{10a}R^{11a} substituiert ist, oder durch ein oder mehrere C₂-C₂₀-Alkyl, das durch ein oder mehrere 0, S oder NR^{10a} unterbrochen ist, oder durch ein oder mehrere Phenyl, Naphthyl, Benzoyl oder Naphthoyl, wobei jede dieser Gruppen unsubstituiert oder durch OR^{4a}, SR^{9a} oder NR^{10a}R^{11a} substituiert ist, substituiert ist;
R² für Wasserstoff, C₁-C₂₀-Alkyl oder C₁-C₆-Alkyl-C₃-C₆-cycloalkyl, das unsubstituiert oder durch ein oder mehrere Halogen, OR⁴, SR⁹, COOR⁴ CONR¹⁰R¹¹, NR¹⁰R¹¹, PO(OR^{3a})₂ oder COR^{3a} substituiert ist, steht oder
R² für C₂-C₂₀-Alkyl oder C₁-C₆-Alkyl-C₃-C₆-cycloalkyl, das durch ein oder mehrere 0, CO, S, C(0)0, OC(O), SO, SO₂, Phenylen, Naphthylen oder NR¹⁰ unterbrochen ist, steht, wobei das unterbrochene C₂-C₂₀-Alkyl unsubstituiert oder durch ein oder mehrere Halogen, OR⁴, SR⁹, COOR⁴, CONR¹⁰R¹¹, NR¹⁰R¹¹ substituiert ist; oder
R² für C₂-C₄-Hydroxyalkyl, C₂-C₁₀-Alkoxyalkyl, C₃-C₅-Alkenyl, C₃-C₈-Cycloalkyl, Phenyl-C₁-C₃-alkyl, C₂-C₈-Alkanoyl, C₃-C₁₂-Alkenoyl oder Benzoyl steht; oder
R² für C₆-C₂₀-Aryl oder C₃-C₂₀-Heteroaryl steht, wobei jede dieser Gruppen unsubstituiert oder durch ein oder mehrere C₁-C₁₂-Alkyl, C₁-C₄-Halogenalkyl, Phenyl, Halogen, CN, NO₂, OR⁴, SR⁹, NR¹⁰R¹¹, (CO)-R^{3a} oder durch C₂-C₂₀-Alkyl, das durch ein oder mehrere 0, S oder NR¹⁰ unterbrochen ist, substituiert ist oder jede dieser Gruppen durch ein oder mehrere C₁-C₂₀-Alkyl, das unsubstituiert oder durch ein oder mehrere Halogen, COOR⁴, CONR¹⁰R¹¹, Phenyl, C₃-C₈-Cycloalkyl, C₃-C₂₀-Heteroaryl, C₆-C₂₀-Aryloxycarbonyl, C₃-C₂₀-Heteroaryloxycarbonyl, OR⁴, SR⁹ oder NR¹⁰R¹¹ substituiert ist, substituiert ist; oder
R² für eine Gruppe 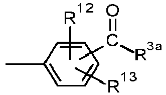 steht;
R^{2'} für C₁-C₂₀-Alkylen, C₁-C₂₀-Alkylen, das durch ein oder mehrere 0, S, (C0)0, 0(CO), Phenylen, Naphthylen oder NR⁸ unterbrochen ist, steht, wobei das C₁-C₂₀-Alkylen und unterbrochene C₁-C₂₀-Alkylen unsubstituiert oder durch Halogen oder OR⁴ substituiert sind, oder
R^{2'} für C₂-C₂₀-Alkenylen, C₂-C₂₀-Alkenylen, das durch ein oder mehrere 0, S, (CO)O, 0(CO), Phenylen, Naphthylen oder durch NR¹⁰ unterbrochen ist, steht, wobei das C₂-C₂₀-Alkenylen und unterbrochene C₂-C₂₀-Alkenylen unsubstituiert oder durch Halogen oder OR⁴ substituiert sind, oder
R^{2'} für C₅-C₈-Cycloalkylen, C₅-C₈-Cycloalkenylen, 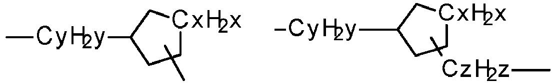 steht, wobei die Gruppen C_{y}H_{2y} und C_{z}H_{2z} ununterbrochen oder durch ein oder mehrere 0, S oder durch NR¹⁰ unterbrochen sind;
R³ für Wasserstoff oder C₁-C₂₀-Alkyl steht; oder
R³ für C₁-C₂₀-Alkyl steht, das durch ein oder mehrere Halogen, OR⁴, SR⁹, NR¹⁰R¹¹, CN, COOR⁴, CONR¹⁰R¹¹, C₃-C₈-Cycloalkyl, das ununterbrochen oder durch ein oder mehrere 0, S, CO oder NR¹⁰ unterbrochen ist, oder durch ein oder mehrere C₆-C₂₀-Aryl, C₃-C₂₀-Heteroaryl, C₆-C₂₀-Aroyl oder C₃-C₂₀-Heteroarylcarbonyl, wobei jede dieser Gruppen unsubstituiert oder durch ein oder mehrere Halogen, Phenyl, C₁-C₂₀-Alkylphenyl, C₁-C₈-Alkoxyphenyl, C₁-C₄-Halogenalkyl, CN, NO₂, OR⁴, SR⁹ oder NR¹⁰R¹¹ substituiert ist, substituiert ist; oder
R³ für C₁-C₂₀-Alkyl steht, das durch ein oder mehrere 0, S, NR¹⁰, CO, SO oder SO₂ unterbrochen ist, das unsubstituiert oder durch C₃-C₈-Cycloalkyl, OH, SH, O(CO)R^{3a}, COOR⁴, CONR¹⁰R¹¹, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, OR⁴, SR⁹ oder NR¹⁰R¹¹ substituiert ist; oder
R³ für C₂-C₁₂-Alkenyl oder C₃-C₂₀-Cycloalkyl steht, wobei jede dieser Gruppen ununterbrochen oder durch ein oder mehrere 0, S, CO, NR¹⁰ oder COOR⁴ unterbrochen ist; oder R³ für C₆-C₂₀-Aryl oder C₃-C₂₀-Heteroaryl steht, wobei jede dieser Gruppen unsubstituiert oder durch ein oder mehrere Halogen, C₁-C₂₀-Alkyl, C₁-C₄-Halogenalkyl, Phenyl, C₁-C₂₀-Alkylphenyl, C₁-C₈-Alkoxyphenyl, CN, NO₂, OR⁴, SR⁹, NR¹⁰R¹¹, COOR⁴, (CO)-R^{3a} oder SO₂-R^{3a} substituiert ist; oder
R³ für C₁-C₂₀-Alkoxy steht, das unsubstituiert oder durch ein oder mehrere C₁-C₁₀-Alkyl, C₁-C₄-Halogenalkyl, Halogen, Phenyl, C₁-C₂₀-Alkylphenyl oder C₂-C₈-Alkoxyphenyl substituiert ist; oder
R³ für C₁-C₂₀-Alkoxy steht, das durch ein oder mehrere 0, S, NR¹⁰, CO, SO oder SO₂ unterbrochen ist; oder
R³ für C₆-C₂₀-Aryloxy oder C₃-C₂₀-Heteroaryloxy steht, wobei jede dieser Gruppen unsubstituiert oder durch ein oder mehrere Halogen, C₁-C₂₀-Alkyl, C₁-C₄-Halogenalkyl, Phenyl, C₁-C₂₀-Alkylphenyl, C₁-C₈-Alkoxyphenyl, CN, NO₂, OR⁴, SR⁹, NR¹⁰R¹¹, COOR⁴, (CO)-R^{3a} oder SO₂-R^{3a} substituiert ist;
R^{3a} für Wasserstoff oder C₁-C₂₀-Alkyl steht; oder
R^{3a} für C₁-C₂₀-Alkyl steht, das durch ein oder mehrere Halogen, OR^{4a}, SR^{9a}, NR^{10a}R^{11a}, CN, COOR^{4a}, CONR^{10a}R^{11a}, C₃-C₈-Cycloalkyl, das ununterbrochen oder durch ein oder mehrere 0, S, CO oder NR¹⁰ unterbrochen ist, oder durch ein oder mehrere C₆-C₂₀-Aryl, C₃-C₂₀-Heteroaryl, C₆-C₂₀-Aroyl oder C₃-C₂₀-Heteroarylcarbonyl, wobei jede dieser Gruppen unsubstituiert oder durch ein oder mehrere Halogen, Phenyl, C₁-C₂₀-Alkylphenyl, C₁-C₈-Alkoxyphenyl, C₁-C₄-Halogenalkyl, CN, NO₂, OR^{4a}, SR^{9a} oder NR^{10a}R^{11a} substituiert ist, substituiert ist; oder
R^{3a} für C₁-C₂₀-Alkyl steht, das durch ein oder mehrere 0, S, NR^{10a}, CO, SO oder SO₂ unterbrochen ist, das unsubstituiert oder durch C₃-C₈-Cycloalkyl, OH, SH, O(CO)-(C₁-C₈-Alkyl) , COOR^{4a}, CONR^{10a}R^{11a}, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, OR^{4a}, SR^{9a} oder NR^{10a}R^{11a} substituiert ist; oder
R^{3a} für C₂-C₁₂-Alkenyl oder C₃-C₂₀-Cycloalkyl steht, wobei jede dieser Gruppen ununterbrochen oder durch ein oder mehrere 0, S, CO, NR^{10a} oder COOR^{4a} unterbrochen ist; oder R^{3a} für C₆-C₂₀-Aryl oder C₃-C₂₀-Heteroaryl steht, wobei jede dieser Gruppen unsubstituiert oder durch ein oder mehrere Halogen, C₁-C₂₀-Alkyl, C₁-C₄-Halogenalkyl, Phenyl, C₁-C₂₀-Alkylphenyl, C₁-C₈-Alkoxyphenyl, CN, NO₂, OR^{4a}, SR^{9a}, NR^{10a}R^{11a}, COOR^{4a}, (CO) - (C₁-C₈-Alkyl) oder SO₂- (C₁-C₄-Halogenalkyl) substituiert ist; oder
R^{3a} für C₁-C₂₀-Alkoxy steht, das unsubstituiert oder durch ein oder mehrere C₁-C₁₀-Alkyl, C₁-C₄-Halogenalkyl, Halogen, Phenyl, C₁-C₂₀-Alkylphenyl oder C₁-C₈-Alkoxyphenyl substituiert ist; oder
R^{3a} für C₁-C₂₀-Alkoxy steht, das durch ein oder mehrere 0, S, NR^{10a}, CO, SO oder SO₂ unterbrochen ist; oder
R^{3a} für C₆-C₂₀-Aryloxy oder C₃-C₂₀-Heteroaryloxy steht, wobei jede dieser Gruppen unsubstituiert oder durch ein oder mehrere Halogen, C₁-C₂₀-Alkyl, C₁-C₄-Halogenalkyl, Phenyl, C₁-C₂₀-Alkylphenyl, C₁-C₈-Alkoxyphenyl, CN, NO₂, OR^{4a}, SR^{9a}, NR^{10a}R^{11a}, COOR^{4a}, (CO) - (C₁-C₈-Alkyl) oder SO₂-(C₁-C₄-Halogenalkyl) substituiert ist; oder
R^{3a} für eine Gruppe 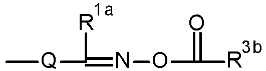 steht;
R^{3b} für Wasserstoff oder C₁-C₂₀-Alkyl steht; oder
R^{3b} für C₁-C₂₀-Alkyl steht, das durch ein oder mehrere Halogen, OR^{4a}, SR^{9a}, NR^{10a}R^{11a}, CN, COOR^{4a}, CONR^{10a}R^{11a}, C₃-C₈-Cycloalkyl, das ununterbrochen oder durch ein oder mehrere 0, S, CO oder NR¹⁰ unterbrochen ist, oder durch ein oder mehrere C₆-C₂₀-Aryl, C₃-C₂₀-Heteroaryl, C₆-C₂₀-Aroyl oder C₃-C₂₀-Heteroarylcarbonyl, wobei jede dieser Gruppen unsubstituiert oder durch ein oder mehrere Halogen, Phenyl, C₁-C₂₀-Alkylphenyl, C₁-C₈-Alkoxyphenyl, C₁-C₄-Halogenalkyl, CN, NO₂, OR^{4a}, SR^{9a} oder NR^{10a}R^{11a} substituiert ist, substituiert ist; oder
R^{3b} für C₁-C₂₀-Alkyl steht, das durch ein oder mehrere 0, S, NR^{10a}, CO, SO oder SO₂ unterbrochen ist, das unsubstituiert oder durch C₃-C₈-Cycloalkyl, OH, SH, O(CO)-(C₁-C₈-Alkyl) , COOR^{4a}, CONR^{10a}R^{11a}, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, OR^{4a}, SR^{9a} oder NR^{10a}R^{11a} substituiert ist; oder
R^{3b} für C₂-C₁₂-Alkenyl oder C₃-C₂₀-Cycloalkyl steht, wobei jede dieser Gruppen ununterbrochen oder durch ein oder mehrere 0, S, CO, NR^{10a} oder COOR^{4a} unterbrochen ist; oder R^{3b} für C₆-C₂₀-Aryl oder C₃-C₂₀-Heteroaryl steht, wobei jede dieser Gruppen unsubstituiert oder durch ein oder mehrere Halogen, C₁-C₂₀-Alkyl, C₁-C₄-Halogenalkyl, Phenyl, C₁-C₂₀-Alkylphenyl, C₁-C₈-Alkoxyphenyl, CN, NO₂, OR^{4a}, SR^{9a}, NR^{10a}R^{11a}, COOR^{4a}, (CO) - (C₁-C₈-Alkyl) oder SO₂-(C₁-C₄-Halogenalkyl) substituiert ist; oder
R^{3b} für C₁-C₂₀-Alkoxy steht, das unsubstituiert oder durch ein oder mehrere C₁-C₁₀-Alkyl, C₁-C₄-Halogenalkyl, Halogen, Phenyl, C₁-C₂₀-Alkylphenyl oder C₁-C₈-Alkoxyphenyl substituiert ist; oder
R^{3b} für C₁-C₂₀-Alkoxy steht, das durch ein oder mehrere 0, S, NR^{10a}, CO, SO oder SO₂ unterbrochen ist; oder
R^{3b} für C₆-C₂₀-Aryloxy oder C₃-C₂₀-Heteroaryloxy steht, wobei jede dieser Gruppen unsubstituiert oder durch ein oder mehrere Halogen, C₁-C₂₀-Alkyl, C₁-C₄-Halogenalkyl, Phenyl, C₁-C₂₀-Alkylphenyl, C₁-C₈-Alkoxyphenyl, CN, NO₂, OR^{4a}, SR^{9a}, NR^{10a}R^{11a}, COOR^{4a}, (CO) - (C₁-C₈-Alkyl) oder SO₂-(C₁-C₄-Halogenalkyl) substituiert ist;
R⁴ für Wasserstoff, (CO) -R^{3a}, COOR^{4a}, CONR¹⁰R¹¹, S(O)ₘ-R^{3a} oder PO(OR^{3a})₂ steht; oder
R⁴ für C₁-C₂₀-Alkyl steht, das durch ein oder mehrere Halogen, OR^{4a}, SR^{9a}, NR^{10a}R^{11a}, CN, COOR^{4a}, CONR^{10a}R^{11a}, PO(OR^{3a})₂, S(O)ₘ-R^{3a}, C₃-C₈-Cycloalkyl, das ununterbrochen oder durch ein oder mehrere 0, S, CO oder NR^{10a} unterbrochen ist, oder durch ein oder mehrere C₆-C₂₀-Aryl, C₃-C₂₀-Heteroaryl, C₆-C₂₀-Aroyl oder C₃-C₂₀-Heteroarylcarbonyl, wobei jede dieser Gruppen unsubstituiert oder durch ein oder mehrere Halogen, Phenyl, C₁-C₂₀-Alkylphenyl, C₁-C₈-Alkoxyphenyl, C₁-C₄-Halogenalkyl, CN, NO₂, OR^{4a}, SR^{9a}, NR^{10a}R^{11a}, PO(OR^{3a})₂ oder S(O)ₘ-R^{3a} substituiert ist, substituiert ist; oder
R⁴ für C₁-C₂₀-Alkyl steht, das durch ein oder mehrere 0, S, NR^{10a}, CO, SO oder SO₂ unterbrochen ist, das unsubstituiert oder durch C₃-C₈-Cycloalkyl, OH, SH, O(CO)R^{3a}, COOR^{4a}, CONR^{10a}R^{11a}, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, OR^{4a}, SR^{9a} oder NR^{10a}R^{11a} substituiert ist; oder
R⁴ für C₂-C₁₂-Alkenyl oder C₃-C₂₀-Cycloalkyl steht, wobei jede dieser Gruppen ununterbrochen oder durch ein oder mehrere 0, S, C0, NR^{10a} oder COOR^{4a} unterbrochen ist; oder R⁴ für C₆-C₂₀-Aryl steht, das durch ein oder mehrere Halogen, CN, NO₂, OR^{4a}, SR^{9a}, NR^{10a}R^{11a}, COOR^{4a}, (CO) -R^{3a}, CONR^{10a}R^{11a}, PO(OR^{3a})₂, S(O)ₘ-R^{3a} oder Gruppen 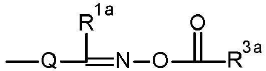 oder durch ein oder mehrere C₁-C₂₀-Alkyl, das unsubstituiert oder durch ein(e) oder mehrere Halogen, COOR^{4a}, CONR^{10a}R^{11a}, Phenyl, C₃-C₈-Cycloalkyl, C₃-C₂₀-Heteroaryl, OR^{4a}, SR^{9a} oder NR^{10a}R^{11a} substituiert ist, oder durch ein oder mehrere C₂-C₂₀-Alkyl, das durch ein oder mehrere 0, S oder NR^{10a} unterbrochen ist, oder durch ein oder mehrere Phenyl, Naphthyl, Benzoyl oder Naphthoyl, wobei jede dieser Gruppen unsubstituiert oder durch OR^{4a}, SR^{9a} oder NR^{10a}R^{11a} substituiert ist, substituiert ist; oder
R⁴ für C₃-C₂₀-Heteroaryl steht, wobei jede dieser Gruppen unsubstituiert oder durch ein(e) oder mehrere Halogen, CN, NO₂, OR^{4a}, SR^{9a}, NR^{10a}R^{11a}, COOR^{4a}, (CO) -R^{3a}, CONR^{10a}R^{11a}, PO(OR^{3a})₂, S(O)ₘ-R^{3a} oder Gruppen 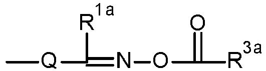 oder durch ein oder mehrere C₁-C₂₀-Alkyl, das unsubstituiert oder durch ein oder mehrere Halogen, COOR^{4a}, CONR^{10a}R^{11a}, Phenyl, C₃-C₈-Cycloalkyl, C₃-C₂₀-Heteroaryl, OR^{4a}, SR^{9a} oder NR^{10a}R^{11a} substituiert ist, oder durch ein oder mehrere C₂-C₂₀-Alkyl, das durch ein oder mehrere 0, S oder NR^{10a} unterbrochen ist, oder durch ein oder mehrere Phenyl, Naphthyl, Benzoyl oder Naphthoyl, wobei jede dieser Gruppen unsubstituiert oder durch OR^{4a}, SR^{9a} oder NR^{10a}R^{11a} substituiert ist, substituiert ist; oder
R⁴ zusammen mit einem der Kohlenstoffatome von R¹ einen 5- oder 6-gliedrigen gesättigten oder ungesättigten Ring bildet, der ununterbrochen oder durch 0, S oder NR^{10a} unterbrochen ist, und wobei der 5- oder 6-gliedrige gesättigte oder ungesättigte Ring unsubstituiert oder durch ein oder mehrere C₁-C₂₀-Alkyl, OR^{4a}, SR^{9a}, NR^{10a}R^{11a}, (CO)-R^{3a}, NO₂, Halogen, C₁-C₄-Halogenalkyl, CN, Phenyl, C₁-C₂₀-Alkylphenyl, C₁-C₈-Alkoxyphenyl, 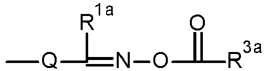 oder C₃-C₂₀-Cyclalkyl, das ununterbrochen oder durch ein oder mehrere 0, S, CO oder NR^{10a} unterbrochen ist, substituiert ist;
R^{4'} für -(CO)-, -(CO)O-, -(CO)N-, -S(O)ₘ-, -PO(O)₂- oder -OP(O)₂ steht;
R^{4a} für Wasserstoff, C₁-C₂₀-Alkyl, (CO)O(C₁-C₈-Alkyl) oder CON(C₁-C₈-Alkyl)₂ steht; oder
R^{4a} für C₁-C₂₀-Alkyl steht, das durch ein oder mehrere Halogen, OH, SH, CN, C₃-C₈-Alkenoxy, OCH₂CH₂CN, OCH₂CH₂(CO)O(C₁-C₈-Alkyl) , O(CO)-(C₂-C₈-Alkyl), O(CO)-(C₂-C₄)-Alkenyl, 0 (CO) -Phenyl, (CO) OH, (CO)O(C₁-C₈-Alkyl), C₃-C₈-Cycloalkyl, SO₂-(C₁-C₄-Halogenalkyl) , O(C₁-C₄-Halogenalkyl), Phenyl, C₁-C₈-Akylphenyl, C₁-C₈-Alkoxyphenyl oder C₃-C₈-Cycloalkyl, das durch ein oder mehrere 0 unterbrochen ist, substituiert ist; oder
R^{4a} für C₂-C₂₀-Alkyl steht, das durch ein oder mehrere 0, S, N(C₁-C₈-Alkyl), CO, SO oder SO₂ unterbrochen ist, das unsubstituiert oder durch C₃-C₈-Cycloalkyl, OH, SH, O(CO)(C₁-C₈-Alkyl), (CO)O(C₁-C₈-Alkyl), (CO)N(C₁-C₈-Alkyl)₂, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, C₁-C₈-Alkoxy, C₁-C₈-Alkylsulfanyl oder N(C₁-C₈-Alkyl)₂ substituiert ist; oder R^{4a} für C₂-C₁₂-Alkenyl, (CO)O(C₁-C₈-Alkenyl) oder C₃-C₈-Cycloalkyl steht, wobei jede dieser Gruppen ununterbrochen oder durch ein oder mehrere 0, S, CO, N(C₁-C₈-Alkyl) oder COO(C₁-C₈-Alkyl) unterbrochen ist; oder
R^{4a} für C₆-C₂₀-Aryl, C₃-C₂₀-Heteroaryl, C₆-C₂₀-Aroyl oder C₃-C₂₀-Heteroarylcarbonyl steht, wobei jede dieser Gruppen unsubstituiert oder durch ein oder mehrere Halogen, CN, NO₂, OH, C₁-C₈-Alkyl, C₁-C₄-Halogenalkyl, C₁-C₈-Alkoxy, Phenyl-C₁-C₃-alkyloxy, Phenoxy, C₁-C₈-Alkylsulfanyl, Phenylsulfanyl, N(C₁-C₈-Alkyl) ₂, Diphenylamino, (CO)O(C₁-C₈-Alkyl), (CO)-C₁-C₈-Alkyl oder (CO)N(C₁-C₈)₂, Phenyl oder Benzoyl substituiert ist; oder
R^{4a} für C₁-C₂₀-Alkanoyl oder C₃-C₁₂-Alkenoyl steht, wobei jede dieser Gruppen unsubstituiert oder durch ein oder mehrere Halogen, Phenyl, C₁-C₈-Alkylphenyl, C₁-C₈-Alkoxyphenyl, OH, C₁-C₈-Alkoxy, Phenoxy, C₁-C₈-Alkylsulfanyl, Phenylsulfanyl, N(C₁-C₈-Alkyl)₂ oder Diphenylamino substituiert ist;
R⁵, R⁶, R⁷ und R⁸ unabhängig voneinander für Wasserstoff, C₁-C₂₀-Alkyl, C₆-C₂₀-Aryl, C₁-C₂₀-Alkoxy, C₆-C₂₀-Aryl-C₁-C₂₀-alkyl, Hydroxy-C₁-C₂₀-alkyl, Hydroxy-C₁-C₂₀-alkoxy-C₁-C₂₀-alkyl, C₃-C₁₀-Cycloalkyl, Amino, CN, NO₂, Hydroxy, 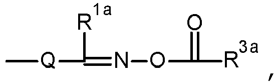 (CO) -R^{3a}, OR^{4a} oder COOR⁴ stehen;
R⁹ für Wasserstoff oder C₁-C₂₀-Alkyl steht; oder
R⁹ für C₁-C₂₀-Alkyl steht, das durch ein oder mehrere Halogen, OR^{4a}, SR^{9a}, NR^{10a}R^{11a}, CN, COOR^{4a}, CONR^{10a}R^{11a}, PO(OR^{3a})₂, S(O)ₘ-R^{3a}, C₃-C₈-Cycloalkyl, das ununterbrochen oder durch ein oder mehrere 0, S, CO oder NR^{10a} unterbrochen ist, oder durch ein oder mehrere C₆-C₂₀-Aryl, C₃-C₂₀-Heteroaryl, C₆-C₂₀-Aroyl oder C₃-C₂₀-Heteroarylcarbonyl, wobei jede dieser Gruppen unsubstituiert oder durch ein oder mehrere Halogen, Phenyl, C₁-C₂₀-Alkylphenyl, C₁-C₈-Alkoxyphenyl, C₁-C₄-Halogenalkyl, CN, NO₂, OR^{4a}, SR^{9a}, NR^{10a}R^{11a}, PO(OR^{3a})₂ oder S(O)ₘ-R^{3a} substituiert ist, substituiert ist; oder
R⁹ für C₁-C₂₀-Alkyl steht, das durch ein oder mehrere 0, S, NR^{10a}, CO, SO oder SO₂ unterbrochen ist, das unsubstituiert oder durch C₃-C₈-Cycloalkyl, OH, SH, O(CO)R^{3a}, COOR^{4a}, CONR^{10a}R^{11a}, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, OR^{4a}, SR^{9a} oder NR^{10a}R^{11a} substituiert ist; oder
R⁹ für C₂-C₁₂-Alkenyl oder C₃-C₂₀-Cycloalkyl steht, wobei jede dieser Gruppen ununterbrochen oder durch ein oder mehrere 0, S, CO, NR^{10a} oder COOR^{4a} unterbrochen ist; oder R⁹ für C₆-C₂₀-Aryl oder C₃-C₂₀-Heteroaryl steht, wobei jede dieser Gruppen unsubstituiert oder durch ein(e) oder mehrere Halogen, CN, NO₂, OR^{4a}, SR^{9a}, NR^{10a}R^{11a}, COOR^{4a}, (CO)-R^{3a} CONR^{10a}R^{11a}, PO(OR^{3a})₂, S(O)ₘ-R^{3a} der Gruppen 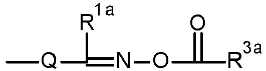 oder durch ein oder mehrere C₁-C₂₀-Alkyl, das unsubstituiert oder durch ein oder mehrere Halogen, COOR^{4a}, CONR^{10a}R^{11a}, Phenyl, C₃-C₈-Cycloalkyl, C₃-C₂₀-Heteroaryl, OR^{4a}, SR^{9a} oder NR^{10a}R^{11a} substituiert ist, oder durch ein oder mehrere C₂-C₂₀-Alkyl, das durch ein oder mehrere 0, S oder NR^{10a} unterbrochen ist, oder durch ein oder mehrere Phenyl, Naphthyl, Benzoyl oder Naphthoyl, wobei jede dieser Gruppen unsubstituiert oder durch OR^{4a}, SR^{9a} oder NR^{10a}R^{11a} substituiert ist, substituiert ist; oder
R⁹ zusammen mit einem der Kohlenstoffatome von R¹ einen 5- oder 6-gliedrigen gesättigten oder ungesättigten Ring bildet, der ununterbrochen oder durch 0, S oder NR^{10a} unterbrochen ist, und wobei der 5- oder 6-gliedrige gesättigte oder ungesättigte Ring unsubstituiert oder durch ein oder mehrere C₁-C₂₀-Alkyl, OR^{4a}, SR^{9a}, NR^{10a}R^{11a}, (CO)-R^{3a}, NO₂, Halogen, C₁-C₄-Halogenalkyl, CN, Phenyl, C₁-C₂₀-Alkylphenyl, C₁-C₈-Alkoxyphenyl, 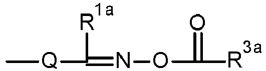 oder C₃-C₂₀-Cyclalkyl, das ununterbrochen oder durch ein oder mehrere 0, S, CO oder NR^{10a} unterbrochen ist, substituiert ist;
R^{9a} für Wasserstoff oder C₁-C₂₀-Alkyl steht; oder
R^{9a} für C₁-C₂₀-Alkyl steht, das durch ein oder mehrere Halogen, OH, SH, CN, C₃-C₈-Alkenoxy, OCH₂CH₂CN, OCH₂CH₂(CO)O(C₁-C₈-Alkyl), O(CO)-(C₁-C₈-Alkyl), O(CO)-(C₂-C₄)-Alkenyl, O(CO)-Phenyl, (CO) OH, (CO)O(C₁-C₈-Alkyl), C₃-C₈-Cycloalkyl, SO₂-(C₁-C₄-Halogenalkyl), O(C₁-C₄-Halogenalkyl), Phenyl, C₁-C₈-Akylphenyl, C₁-C₈-Alkoxyphenyl oder C₃-C₈-Cycloalkyl, das durch ein oder mehrere 0 unterbrochen ist, substituiert ist; oder
R^{9a} für C₂-C₂₀-Alkyl steht, das durch ein oder mehrere 0, S, N(C₁-C₈-Alkyl), CO, SO oder SO₂ unterbrochen ist, das unsubstituiert oder durch C₃-C₈-Cycloalkyl, OH, SH, O(CO)(C₁-C₈-Alkyl), (CO)O(C₁-C₈-Alkyl), (CO)N(C₁-C₈-Alkyl)₂, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, C₁-C₈-Alkoxy, C₁-C₈-Alkylsulfanyl oder N(C₁-C₈-Alkyl)₂ substituiert ist; oder R^{9a} für C₂-C₁₂-Alkenyl oder C₃-C₈-Cycloalkyl steht, wobei jede dieser Gruppen ununterbrochen oder durch ein oder mehrere 0, S, CO, N(C₁-C₈-Alkyl) oder COO(C₁-C₈-Alkyl) unterbrochen ist; oder
R^{9a} für C₆-C₂₀-Aryl, C₃-C₂₀-Heteroaryl, C₆-C₂₀-Aroyl oder C₃-C₂₀-Heteroarylcarbonyl steht, wobei jede dieser Gruppen unsubstituiert oder durch ein oder mehrere Halogen, CN, NO₂, OH, C₁-C₈-Alkyl, C₁-C₄-Halogenalkyl, C₁-C₈-Alkoxy, Phenyl-C₁-C₃-alkyloxy, Phenoxy, C₁-C₈-Alkylsulfanyl, Phenylsulfanyl, N(C₁-C₈-Alkyl)₂, Diphenylamino, (CO)O(C₁-C₈-Alkyl), (CO)-C₁-C₈-Alkyl oder (CO)N(C₁-C₈)₂, Phenyl oder Benzoyl substituiert ist; oder
R^{9a} für C₁-C₂₀-Alkanoyl oder C₃-C₁₂-Alkenoyl steht, wobei jede dieser Gruppen unsubstituiert oder durch ein oder mehrere Halogen, Phenyl, C₁-C₈-Alkylphenyl, C₁-C₈-Alkoxyphenyl, OH, C₁-C₈-Alkoxy, Phenoxy, C₁-C₈-Alkylsulfanyl, Phenylsulfanyl, N(C₁-C₈-Alkyl)₂ oder Diphenylamino substituiert ist;
R¹⁰ und R¹¹ unabhängig voneinander für Wasserstoff, C₁-C₂₀-Alkyl, S(O)ₘ-R^{3a}, O(CO)-R^{3a} (CO)-R^{3a} oder CONR^{10a}R^{11a} stehen; oder
R¹⁰ und R¹¹ unabhängig voneinander für C₁-C₂₀-Alkyl stehen, das durch ein oder mehrere Halogen, OR^{4a}, SR^{9a}, NR^{10a}R^{11a}, CN, COOR^{4a}, CONR^{10a}R^{11a}, PO(OR^{3a})₂, S(O)ₘ-R^{3a}, C₃-C₈-Cycloalkyl, das ununterbrochen oder durch ein oder mehrere 0, S, CO oder NR^{10a} unterbrochen ist, oder durch ein oder mehrere C₆-C₂₀-Aryl, C₃-C₂₀-Heteroaryl, C₆-C₂₀-Aroyl oder C₃-C₂₀-Heteroarylcarbonyl, wobei jede dieser Gruppen unsubstituiert oder durch ein oder mehrere Halogen, Phenyl, C₁-C₂₀-Alkylphenyl, C₁-C₈-Alkoxyphenyl, C₁-C₄-Halogenalkyl, CN, NO₂, OR^{4a}, SR^{9a}, NR^{10a}R^{11a}, PO(OR^{3a})₂ oder S(O)ₘ-R^{3a} substituiert ist, substituiert ist; oder
R¹⁰ und R¹¹ unabhängig voneinander für C₁-C₂₀-Alkyl stehen, das durch ein oder mehrere 0, S, NR^{10a}, CO, SO oder SO₂ unterbrochen ist, das unsubstituiert oder durch C₃-C₈-Cycloalkyl, OH, SH, O(CO)R^{3a}, COOR^{4a}, CONR^{10a}R^{11a}, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, OR^{4a}, SR^{9a} oder NR^{10a}R^{11a} substituiert ist; oder
R¹⁰ und R¹¹ unabhängig voneinander für C₂-C₁₂-Alkenyl oder C₃-C₂₀-Cycloalkyl stehen, wobei jede dieser Gruppen ununterbrochen oder durch ein oder mehrere 0, S, CO, NR^{10a} oder COOR^{4a} unterbrochen ist; oder
R¹⁰ und R¹¹ unabhängig voneinander für C₆-C₂₀-Aryl oder C₃-C₂₀-Heteroaryl stehen, wobei jede dieser Gruppen unsubstituiert oder durch ein(e) oder mehrere Halogen, CN, NO₂, OR^{4a}, SR^{9a}, NR^{10a}R^{11a}, COOR^{4a}, (CO) -R^{3a}CONR^{10a}R^{11a}, PO(OR^{3a})₂, S(O)ₘ-R^{3a} oder Gruppen 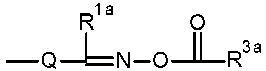 oder durch ein oder mehrere C₁-C₂₀-Alkyl, das unsubstituiert oder durch ein oder mehrere Halogen, COOR^{4a}, CONR^{10a}R^{11a}, Phenyl, C₃-C₈-Cycloalkyl, C₃-C₂₀-Heteroaryl, OR^{4a}, SR^{9a} oder NR^{10a}R^{11a} substituiert ist, oder durch ein oder mehrere C₂-C₂₀-Alkyl, das durch ein oder mehrere 0, S oder NR^{10a} unterbrochen ist, oder durch ein oder mehrere Phenyl, Naphthyl, Benzoyl oder Naphthoyl, wobei jede dieser Gruppen unsubstituiert oder durch OR^{4a}, SR^{9a} oder NR^{10a}R^{11a} substituiert ist, substituiert ist; oder
R¹⁰ und R¹¹ unabhängig voneinander für C₁-C₂₀-Alkoxy stehen, das unsubstituiert oder durch ein oder mehrere Halogen, Phenyl, C₁-C₈-Alkylphenyl oder C₁-C₈-Alkoxyphenyl substituiert ist; oder
R¹⁰ und R¹¹ unabhängig voneinander für C₁-C₂₀-Alkoxy stehen, das durch ein oder mehrere 0, S, NR^{10a}, CO, SO oder SO₂ unterbrochen ist; oder
R¹⁰ und R¹¹ unabhängig voneinander für C₆-C₂₀-Aryloxy oder C₃-C₂₀-Heteroaryloxy stehen, wobei jede dieser Gruppen unsubstituiert oder durch ein oder mehrere Halogen, C₁-C₈-Alkyl, C₁-C₄-Halogenalkyl, Phenyl, C₁-C₈-Alkylphenyl, C₁-C₈-Alkoxyphenyl, CN, NO₂, OR^{4a}, SR^{9a}, NR^{10a}R^{11a}, COOR^{4a}, (CO)-R^{3a} oder SO₂-R^{3a} substituiert ist; oder
R¹⁰ zusammen mit einem der Kohlenstoffatome von R¹ einen 5- oder 6-gliedrigen gesättigten oder ungesättigten Ring bildet, der ununterbrochen oder durch 0, S oder NR^{10a} unterbrochen ist, und wobei der 5- oder 6-gliedrige gesättigte oder ungesättigte Ring unsubstituiert oder durch ein oder mehrere C₁-C₂₀-Alkyl, OR^{4a}, SR^{9a}, NR^{10a}R^{11a}, (CO)-R^{3a}, NO₂, Halogen, C₁-C₄-Halogenalkyl, CN, Phenyl, C₁-C₂₀-Alkylphenyl, C₁-C₈-Alkoxyphenyl, 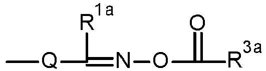 oder C₃-C₂₀-Cyclalkyl, das ununterbrochen oder durch ein oder mehrere 0, S, CO oder NR^{10a} unterbrochen ist, substituiert ist; oder
R¹⁰ und R¹¹ zusammen mit dem N-Atom, an das sie gebunden sind, einen 5- oder 6-gliedrigen gesättigten oder ungesättigten Ring bilden, der ununterbrochen oder durch 0, S oder NR^{10a} unterbrochen ist, und wobei der 5- oder 6-gliedrige gesättigte oder ungesättigte Ring unsubstituiert oder durch ein oder mehrere C₁-C₂₀-Alkyl, OR^{4a}, SR^{9a}, NR^{10a}R^{11a}, (CO) -R^{3a}, NO₂, Halogen, C₁-C₄-Halogenalkyl, CN, Phenyl, 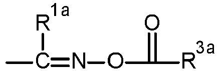 oder C₃-C₂₀-Cyclalkyl, das ununterbrochen oder durch ein oder mehrere 0, S, CO oder NR^{10a} unterbrochen ist, substituiert ist;
R^{10a} und R^{11a} unabhängig voneinander für Wasserstoff, C₁-C₂₀-Alkyl, S(O)ₘ-(C₁-C₈-Alkyl), O(CO)(C₁-C₈-Alkyl), (CO)(C₁-C₈-Alkyl), (CO)O(C₁-C₈-Alkyl) oder CON(C₁-C₈-Alkyl)₂ stehen; oder
R^{10a} und R^{11a} unabhängig voneinander für C₁-C₂₀-Alkyl stehen, das durch ein oder mehrere Halogen, OH, SH, CN, C₃-C₈-Alkenoxy, OCH₂CH₂CN, OCH₂CH₂(CO)O(C₁-C₈-Alkyl), O(CO)-(C₁-C₈-Alkyl), O(CO)-(C₂-C₄)-Alkenyl, O(CO)-Phenyl, (CO)OH, (CO)O(C₁-C₈-Alkyl), C₃-C₈-Cycloalkyl, SO₂-(C₁-C₄-Halogenalkyl), O(C₁-C₄-Halogenalkyl), Phenyl, C₁-C₈-Akylphenyl, C₁-C₈-Alkoxyphenyl oder C₃-C₈-Cycloalkyl, das durch ein oder mehrere 0 unterbrochen ist, substituiert ist; oder
R^{10a} und R^{11a} unabhängig voneinander für C₂-C₂₀-Alkyl stehen, das durch ein oder mehrere 0, S, N(C₁-C₈-Alkyl), CO, SO oder SO₂ unterbrochen ist, das unsubstituiert oder durch C₃-C₈-Cycloalkyl, OH, SH, O(CO)(C₁-C₈-Alkyl), (CO)O(C₁-C₈-Alkyl), (CO)N(C₁-C₈-Alkyl)₂, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, C₁-C₈-Alkoxy, C₁-C₈-Alkylsulfanyl oder N(C₁-C₈-Alkyl)₂ substituiert ist; oder
R^{10a} und R^{11a} unabhängig voneinander für C₂-C₁₂-Alkenyl oder C₃-C₈-Cycloalkyl stehen, wobei jede dieser Gruppen ununterbrochen oder durch ein oder mehrere 0, S, CO, N(C₁-C₈-Alkyl) oder COO(C₁-C₈-Alkyl) unterbrochen ist; oder
R^{10a} und R^{11a} unabhängig voneinander für C₆-C₂₀-Aryl, C₃-C₂₀-Heteroaryl, C₆-C₂₀-Aroyl oder C₃-C₂₀-Heteroarylcarbonyl stehen, wobei jede dieser Gruppen unsubstituiert oder durch ein oder mehrere Halogen, CN, NO₂, OH, C₁-C₈-Alkyl, C₁-C₄-Halogenalkyl, C₁-C₈-Alkoxy, Phenyl-C₁-C₃-alkyloxy, Phenoxy, C₁-C₈-Alkylsulfanyl, Phenylsulfanyl, N(C₁-C₈-Alkyl)₂, Diphenylamino, (CO)O(C₁-C₈-Alkyl), (CO) -C₁-C₈-Alkyl oder (CO)N(C₁-C₈-Alkyl)₂, Phenyl oder Benzoyl substituiert ist; oder
R^{10a} und R^{11a} unabhängig voneinander für C₁-C₂₀-Alkanoyl oder C₃-C₁₂-Alkenoyl stehen, wobei jede dieser Gruppen unsubstituiert oder durch ein oder mehrere Halogen, Phenyl, C₁-C₈-Alkylphenyl, C₁-C₈-Alkoxyphenyl, OH, C₁-C₈-Alkoxy, Phenoxy, C₁-C₈-Alkylsulfanyl, Phenylsulfanyl, N(C₁-C₈-Alkyl)₂ oder Diphenylamino substituiert ist; oder R^{10a} und R^{11a} unabhängig voneinander für C₁-C₂₀-Alkoxy stehen, das unsubstituiert oder durch ein oder mehrere Halogen, Phenyl, C₁-C₈-Alkylphenyl oder C₁-C₈-Alkoxyphenyl substituiert ist; oder
R^{10a} und R^{11a} unabhängig voneinander für C₁-C₂₀-Alkoxy stehen, das durch ein oder mehrere 0, S, N(C₁-C₈-Alkyl), CO, SO oder SO₂ unterbrochen ist; oder
R^{10a} und R^{11a} unabhängig voneinander für C₆-C₂₀-Aryloxy oder C₃-C₂₀-Heteroaryloxy stehen, wobei jede dieser Gruppen unsubstituiert oder durch ein oder mehrere Halogen, C₁-C₈-Alkyl, C₁-C₄-Halogenalkyl, Phenyl, C₁-C₈-Alkylphenyl, C₁-C₈-Alkoxyphenyl, CN, NO₂, C₁-C₈-Alkoxy, C₁-C₈-Alkylsulfanyl, N(C₁-C₈-Alkyl)₂, CO(OC₁-C₈-Alkyl), (CO)-(C₁-C₈-Alkyl) oder SO₂-(C₁-C₈-Alkyl) substituiert ist; oder
R^{10a} und R^{11a} zusammen mit dem N-Atom, an das sie gebunden sind, einen 5- oder 6-gliedrigen gesättigten oder ungesättigten Ring bilden, der ununterbrochen oder durch 0, S oder N(C₁-C₈-Alkyl) unterbrochen ist, und wobei der 5- oder 6-gliedrige gesättigte oder ungesättigte Ring unsubstituiert oder durch ein oder mehrere C₁-C₈-Alkyl, C₁-C₈-Alkoxy, C₁-C₈-Alkylsulfanyl, N(C₁-C₈-Alkyl) ₂, NO₂, Halogen, C₁-C₄-Halogenalkyl, CN, Phenyl oder C₃-C₂₀-Cycloalkyl, das ununterbrochen oder durch ein oder mehrere 0, S, CO oder N(C₁-C₈-Alkyl) unterbrochen ist, substituiert ist;
R¹² und R¹³ unabhängig voneinander für Wasserstoff oder C₁-C₁₂-Alkyl, das gegebenenfalls durch ein oder mehrere Halogen substituiert ist, Phenyl, CN, -OH, -SH, C₁-C₄-Alkoxy, (CO)OH oder (CO)O(C₁-C₄alkyl) substituiert ist, stehen; oder
R¹² und R¹³ für Phenyl stehen, das gegebenenfalls durch ein oder mehrere C₁-C₆-Alkyl, Halogen, CN, OR⁴, SR⁹ oder NR¹⁰R¹¹ substituiert ist; oder
R¹² und R¹³ für Halogen, CN, OR⁴, SR⁹, SOR⁹, SO₂R⁹ oder NR¹⁰R¹¹ stehen, wobei die Substituenten OR⁴, SR⁹ oder NR¹⁰R¹¹ gegebenenfalls über die Reste R⁴, R⁹, R¹⁰ und/oder R¹¹ mit einem der Kohlenstoffatome der Phenyl-, Naphthyl-, Benzoyl- oder Naphthoylgruppe oder dem des Substituenten R^{3a} einen 5- bis 6-gliedrigen Ring bilden; oder
R¹² und R¹³ zusammen für eine Gruppe 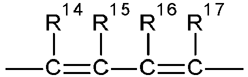 stehen, wobei R¹⁴, R¹⁵, R¹⁶ und R¹⁷ unabhängig voneinander für Wasserstoff, C₁-C₁₂-Alkyl, das gegebenenfalls durch ein oder mehrere Halogen, Phenyl, CN, -OH, -SH, C₁-C₄-Alkoxy, (CO)OH oder (CO) 0 (C₁-C₄-Alkyl) substituiert ist, stehen; oder R¹⁴, R¹⁵, R¹⁶ und R¹⁷ für Phenyl, das gegebenenfalls durch ein oder mehrere C₁-C₆-Alkyl, Halogen, CN, OR⁴, SR⁹ oder NR¹⁰R¹¹ substituiert ist, stehen; oder R¹⁴, R¹⁵, R¹⁶ und R¹⁷ für Halogen, CN, OR⁴, SR⁹ oder NR¹⁰R¹¹ stehen; oder
R¹² und R¹³ zusammen für eine Gruppe 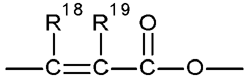 stehen, wobei R¹⁸ und R¹⁹ unabhängig voneinander für Wasserstoff, C₁-C₁₂-Alkyl, das gegebenenfalls durch ein oder mehrere Halogen, Phenyl, CN, -OH, -SH, C₁-C₄-Alkoxy, (CO)OH oder (CO)O(C₁-C₄Alkyl) substituiert ist, stehen; oder R¹⁸ und R¹⁹ für Phenyl, das gegebenenfalls durch ein oder mehrere C₁-C₆-Alkyl, Halogen, CN, OR⁴, SR⁹ oder NR¹⁰R¹¹ substituiert ist, stehen;
M für eine direkte Bindung oder eine zweiwertige Verknüpfungsgruppe C₁-C₂₀-Alkylen oder C₁-C₂₀-Alkylen, das durch ein oder mehrere Halogen, OR⁴, SR⁹, NR¹⁰R¹¹, CN, COOR⁴, CONR¹⁰R¹¹, C₃-C₈-Cycloalkyl, das ununterbrochen oder durch ein oder mehrere 0, S, CO oder NR¹⁰ unterbrochen ist, oder durch ein oder mehrere C₆-C₂₀-Aryl, C₃-C₂₀-Heteroaryl, C₆-C₂₀-Aroyl oder C₃-C₂₀-Heteroarylcarbonyl, wobei jede dieser Gruppen unsubstituiert oder durch ein oder mehrere Halogen, Phenyl, C₁-C₂₀-Alkylphenyl, C₁-C₈-Alkoxyphenyl, C₁-C₄-Halogenalkyl, CN, NO₂, OR⁴, SR⁹ oder NR¹⁰R¹¹ substituiert ist, substituiert ist, steht; oder
M für C₁-C₂₀-Alkylen steht, das durch ein oder mehrere 0, S, NR¹⁰, CO, SO oder SO₂ unterbrochen ist, das unsubstituiert oder durch C₃-C₈-Cycloalkyl, OH, SH, O(CO)R^{3a}, COOR⁴, CONR¹⁰R¹¹, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, OR⁴, SR⁹ oder NR¹⁰R¹¹ substituiert ist; oder
M für C₂-C₁₂-Alkenylen oder C₃-C₂₀-Cycloalkylen steht, wobei jede dieser Gruppen ununterbrochen oder durch ein oder mehrere 0, S, C0, NR¹⁰ oder COOR⁴ unterbrochen ist; oder
M für C₆-C₂₀-Arylen oder C₃-C₂₀-Heteroarylen steht, wobei jede dieser Gruppen unsubstituiert oder durch ein oder mehrere Halogen, C₁-C₂₀-Alkyl, C₁-C₄-Halogenalkyl, Phenyl, C₁-C₂₀-Alkylphenyl, C₁-C₈-Alkoxyphenyl, CN, NO₂, OR⁴, SR⁹, NR¹⁰R¹¹, COOR⁴, (CO)-R^{3a} oder SO₃-R^{3a} substituiert ist;
M' für eine direkte Bindung oder eine zweiwertige Verknüpfungsgruppe, die aus C₁-C₂₀-Alkylen oder C₁-C₂₀-Alkylen, das durch ein oder mehrere Halogen, OR⁴, SR⁹, NR¹⁰R¹¹, CN, COOR⁴, CONR¹⁰R¹¹, C₃-C₈-Cycloalkyl, das ununterbrochen oder durch ein oder mehrere 0, S, CO oder NR¹⁰ unterbrochen ist, oder durch ein oder mehrere C₆-C₂₀-Aryl, C₃-C₂₀-Heteroaryl, C₆-C₂₀-Aroyl oder C₃-C₂₀-Heteroarylcarbonyl, wobei jede dieser Gruppen unsubstituiert oder durch ein oder mehrere Halogen, Phenyl, C₁-C₂₀-Alkylphenyl, C₁-C₈-Alkoxyphenyl, C₁-C₄-Halogenalkyl, CN, NO₂, OR⁴, SR⁹ oder NR¹⁰R¹¹ substituiert ist, substituiert ist, ausgewählt ist, steht; oder
M' für C₁-C₂₀-Alkylen steht, das durch ein oder mehrere 0, S, NR¹⁰, CO, SO oder SO₂ unterbrochen ist, das unsubstituiert oder durch C₃-C₈-Cycloalkyl, OH, SH, O(CO)R^{3a}, COOR⁴, CONR¹⁰R¹¹, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, OR⁴, SR⁹ oder NR¹⁰R¹¹ substituiert ist; oder
M' für C₂-C₁₂-Alkenylen oder C₃-C₂₀-Cycloalkylen steht, wobei jede dieser Gruppen ununterbrochen oder durch ein oder mehrere 0, S, C0, NR¹⁰ oder COOR⁴ unterbrochen ist; oder
M' für C₆-C₂₀-Arylen oder C₃-C₂₀-Heteroarylen steht, wobei jede dieser Gruppen unsubstituiert oder durch ein oder mehrere Halogen, C₁-C₂₀-Alkyl, C₁-C₄-Halogenalkyl, Phenyl, C₁-C₂₀-Alkylphenyl, C₁-C₈-Alkoxyphenyl, CN, NO₂, OR⁴, SR⁹, NR¹⁰R¹¹, COOR⁴, (CO)-R^{3a} oder SO₃-R^{3a} substituiert ist;
M'' für eine direkte Bindung oder eine zweiwertige Verknüpfungsgruppe, die aus C₁-C₂₀-Alkylen oder C₁-C₂₀-Alkylen, das durch ein oder mehrere Halogen, OR⁴, SR⁹, NR¹⁰R¹¹, CN, COOR⁴, CONR¹⁰R¹¹, C₃-C₈-Cycloalkyl, das ununterbrochen oder durch ein oder mehrere 0, S, CO oder NR¹⁰ unterbrochen ist, oder durch ein oder mehrere C₆-C₂₀-Aryl, C₃-C₂₀-Heteroaryl, C₆-C₂₀-Aroyl oder C₃-C₂₀-Heteroarylcarbonyl, wobei jede dieser Gruppen unsubstituiert oder durch ein oder mehrere Halogen, Phenyl, C₁-C₂₀-Alkylphenyl, C₁-C₈-Alkoxyphenyl, C₁-C₄-Halogenalkyl, CN, NO₂, OR⁴, SR⁹ oder NR¹⁰R¹¹ substituiert ist, substituiert ist, ausgewählt ist, steht; oder
M'' für C₁-C₂₀-Alkylen steht, das durch ein oder mehrere 0, S, NR¹⁰, CO, SO oder SO₂ unterbrochen ist, das unsubstituiert oder durch C₃-C₈-Cycloalkyl, OH, SH, O(CO)R^{3a}, COOR⁴, CONR¹⁰R¹¹, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, OR⁴, SR⁹ oder NR¹⁰R¹¹ substituiert ist; oder
M" für C₂-C₁₂-Alkenylen oder C₃-C₂₀-Cycloalkylen steht, wobei jede dieser Gruppen ununterbrochen oder durch ein oder mehrere 0, S, C0, NR¹⁰ oder COOR⁴ unterbrochen ist; oder
M" für C₆-C₂₀-Arylen oder C₃-C₂₀-Heteroarylen steht, wobei jede dieser Gruppen unsubstituiert oder durch ein oder mehrere Halogen, C₁-C₂₀-Alkyl, C₁-C₄-Halogenalkyl, Phenyl, C₁-C₂₀-Alkylphenyl, C₁-C₈-Alkoxyphenyl, CN, NO₂, OR⁴, SR⁹, NR¹⁰R¹¹, COOR⁴, (CO)-R^{3a} oder SO₂-R^{3a} substituiert ist;
m für 1 oder 2 steht;
Q für CO oder eine direkte Bindung steht; und
x, y und z unabhängig voneinander für eine ganze Zahl mit einem Wert von 1, 2, 3 oder 4 stehen.

2. Verbindungen der Formel I, II, III und IV nach Anspruch 1, wobei es sich bei den Verbindungen um Verbindungen der Formel Ia, IIa, IIIa oder IVa handelt: 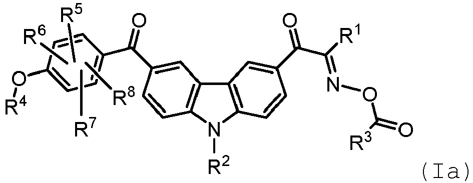 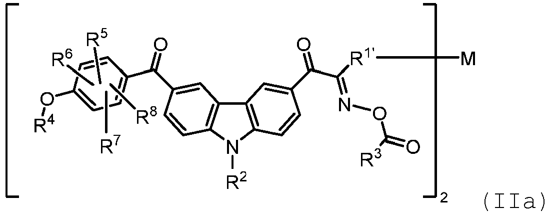 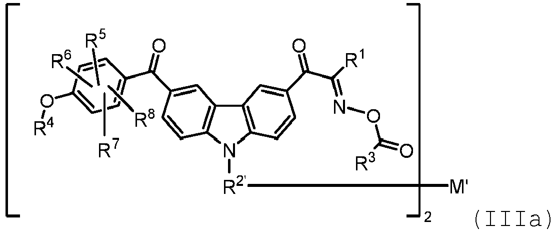 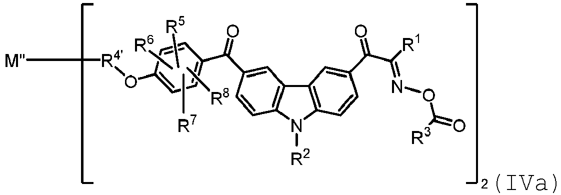 wobei
R¹ bis R⁸, R¹' , R²', R⁴', M, M' und M" wie in Anspruch 1 definiert sind.

3. Verbindungen der Formel I, II, III und IV nach Anspruch 1 oder 2, wobei es sich bei den Verbindungen um Verbindungen der Formel Ib, IIb, IIIb oder IVb handelt: 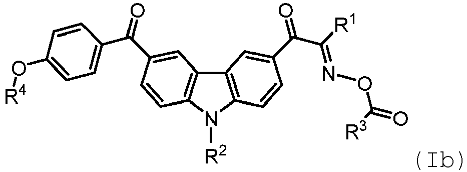 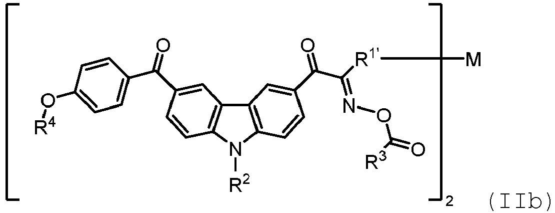 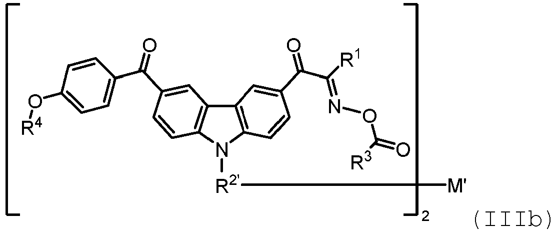 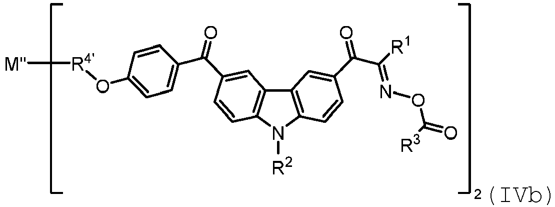 wobei
R¹ für Wasserstoff, C₁-C₂₀-Alkyl, C₁-C₆-Alkyl-C₃-C₆-cycloalkyl, C₃-C₂₀-Cycloalkyl oder C₂-C₁₂-Alkenyl steht, wobei C₃-C₂₀-Cycloalkyl oder C₂-C₁₂-Alkenyl ununterbrochen oder durch ein oder mehrere 0, S, CO, NR¹⁰ oder COOR⁴ unterbrochen ist; oder
R¹ für C₂-C₂₀-Alkyl steht, das durch ein oder mehrere 0, CO, S, C (0) 0, OC(O), SO, SO₂, Phenylen, Naphthylen oder NR¹⁰ unterbrochen ist, wobei das unterbrochene C₂-C₂₀-Alkyl unsubstituiert oder durch ein oder mehrere Halogen, C₃-C₈-Cycloalkyl, OH, SH, OR⁴, SR⁹, COOR⁴, O(CO)-R^{3a}, CONR¹⁰R¹¹, NR¹⁰R¹¹, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, OR⁴, SR⁹ oder NR¹⁰R¹¹ substituiert ist; oder
R¹ für C₆-C₂₀-Aryl oder C₃-C₂₀-Heteroaryl steht, wobei jede dieser Gruppen unsubstituiert oder durch ein oder mehrere C₁-C₂₀-Alkyl substituiert ist;
R^{1'} für C₁-C₂₀-Alkylen, C₁-C₂₀-Alkylen, das durch ein oder mehrere 0, S, (C0)0, O(CO), Phenylen, Naphthylen oder NR⁸ unterbrochen ist, steht, wobei das C₁-C₂₀-Alkylen und unterbrochene C₁-C₂₀-Alkylen unsubstituiert oder durch Halogen oder OR⁴ substituiert sind,
R² für Wasserstoff oder C₁-C₂₀-Alkyl, das unsubstituiert oder durch ein oder mehrere Halogen, OR⁴, SR⁹, COOR⁴ CONR¹⁰R¹¹, NR¹⁰R¹¹, PO(OR^{3a})₂ oder (CO) -R^{3a} substituiert ist, steht oder
R² für C₂-C₂₀-Alkyl oder C₁-C₆-Alkyl-C₃C₆-cycloalkyl, das durch ein oder mehrere 0, CO, S, C(0)0, OC(O), SO, SO₂, Phenylen, Naphthylen oder NR¹⁰ unterbrochen ist, steht; oder
R² für C₆-C₂₀-Aryl oder C₃-C₂₀-Heteroaryl steht, wobei jede dieser Gruppen unsubstituiert oder durch ein oder mehrere C₁-C₁₂-Alkyl oder (CO) -R^{3a} substituiert ist;
R^{2'} für C₁-C₂₀-Alkylen, C₁-C₂₀-Alkylen, das durch ein oder mehrere 0, S, (C0)0, O(CO), Phenylen, Naphthylen oder NR⁸ unterbrochen ist, steht, wobei das C₁-C₂₀-Alkylen und unterbrochene C₁-C₂₀-Alkylen unsubstituiert oder durch Halogen oder OR⁴ substituiert sind,
R³ für Wasserstoff oder C₁-C₂₀-Alkyl steht; oder
R³ für C₁-C₂₀-Alkyl steht, das durch ein oder mehrere Halogen, OR⁴, SR⁹, NR¹⁰R¹¹, CN, COOR⁴, CONR¹⁰R¹¹, C₃-C₈-Cycloalkyl, das ununterbrochen oder durch ein oder mehrere 0, S, CO oder NR¹⁰ unterbrochen ist, oder durch ein oder mehrere C₆-C₂₀-Aryl, C₃-C₂₀-Heteroaryl, C₆-C₂₀-Aroyl oder C₃-C₂₀-Heteroarylcarbonyl, wobei jede dieser Gruppen unsubstituiert oder durch ein oder mehrere Halogen, Phenyl, C₁-C₂₀-Alkylphenyl, C₁-C₈-Alkoxyphenyl, C₁-C₄-Halogenalkyl, CN, NO₂, OR⁴, SR⁹ oder NR¹⁰R¹¹ substituiert ist, substituiert ist;
R^{3a} für Wasserstoff oder C₁-C₂₀-Alkyl steht; oder
R^{3a} für C₁-C₂₀-Alkyl steht, das durch ein oder mehrere 0, S, NR^{10a}, CO, SO oder SO₂ unterbrochen ist, das unsubstituiert oder durch C₃-C₈-Cycloalkyl, OH, SH, O(CO) - (C₁-C₈-Alkyl), COOR^{4a}, CONR^{10a}R^{11a}, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, OR^{4a}, SR^{9a} oder NR^{10a}R^{11a} substituiert ist; oder
R^{3a} für C₆-C₂₀-Aryl oder C₃-C₂₀-Heteroaryl steht, wobei jede dieser Gruppen unsubstituiert oder durch ein oder mehrere Halogen, C₁-C₂₀-Alkyl, C₁-C₄-Halogenalkyl, Phenyl, C₁-C₂₀-Alkylphenyl, C₁-C₈-Alkoxyphenyl, CN, NO₂, OR^{4a}, SR^{9a}, NR^{10a}R^{11a}, COOR^{4a}, (CO) - (C₁-C₈-Alkyl)oder SO₂-(C₁-C₄-Halogenalkyl) substituiert ist;
R⁴ für Wasserstoff, (CO) -R^{3a}, COOR^{4a}, CONR¹⁰R¹¹, S(O)ₘ-R^{3a} oder PO(OR^{3a})₂ steht;
R^{4'} für -(CO)-, -(CO)O-, -(CO)N-, -S(O)ₘ-, -PO(O)₂- oder -OP(O)₂ steht;
R^{4a} für Wasserstoff, C₁-C₂₀-Alkyl, (CO) O(C₁-C₈-Alkyl)oder CON(C₁-C₈-Alkyl)₂ steht; oder
R^{4a} für C₂-C₂₀-Alkyl steht, das durch ein oder mehrere 0, S, N(C₁-C₈-Alkyl), CO, SO oder SO₂ unterbrochen ist, das unsubstituiert oder durch C₃-C₈-Cycloalkyl, OH, SH, O(CO)(C₁-C₈-Alkyl), (CO)O(C₁-C₈-Alkyl), (CO)N(C₁-C₈-Alkyl)₂, C₆-C₂₀-Aryl, C₃-C₂₀-Heteroaryl, C₆-C₂₀-Aroyl oder C₃-C₂₀-Heteroarylcarbonyl substituiert ist, wobei C₆-C₂₀-Aryl, C₃-C₂₀-Heteroaryl, C₆-C₂₀-Aroyl oder C₃-C₂₀-Heteroarylcarbonyl unsubstituiert oder durch ein oder mehrere Halogen, C₁-C₈-Alkyl, C₁-C₈-Alkoxy, C₁-C₈-Alkylsulfanyl oder N(C₁-C₈-Alkyl)₂ substituiert ist; oder R^{4a} für C₆-C₂₀-Aryl, C₃-C₂₀-Heteroaryl, C₆-C₂₀-Aroyl oder C₃-C₂₀-Heteroarylcarbonyl steht, wobei jede dieser Gruppen unsubstituiert oder durch ein oder mehrere Halogen, CN, NO₂, OH, C₁-C₈-Alkyl, C₁-C₄-Halogenalkyl, C₁-C₈-Alkoxy, Phenyl-C₁-C₃-alkyloxy, Phenoxy, C₁-C₈-Alkylsulfanyl, Phenylsulfanyl, N(C₁-C₈-Alkyl)₂, Diphenylamino, (CO)O(C₁-C₈-Alkyl), (CO)-C₁-C₈-Alkyl oder (CO)N(C₁-C₈)₂, Phenyl oder Benzoyl substituiert ist;
R⁸ für Wasserstoff, C₁-C₂₀-Alkyl oder C₆-C₂₀-Aryl steht;
R⁹ für Wasserstoff oder C₁-C₂₀-Alkyl steht;
R^{9a} für Wasserstoff oder C₁-C₂₀-Alkyl steht;
R¹⁰ und R¹¹ unabhängig voneinander für Wasserstoff, C₁-C₂₀-Alkyl, S(O)ₘ-R^{3a}, O(CO)-R^{3a} (CO)-R^{3a} oder CONR^{10a}R^{11a} stehen; R^{10a} und R^{11a} unabhängig voneinander für Wasserstoff, C₁-C₂₀-Alkyl, S(O)ₘ-(C₁-C₈-Alkyl), O(CO)(C₁-C₈-Alkyl), (CO)(C₁-C₈-Alkyl)(CO)O(C₁-C₈-Alkyl) oder CON(C₁-C₈-Alkyl)₂ stehen;
M für eine direkte Bindung oder eine zweiwertige Verknüpfungsgruppe C₁-C₂₀-Alkylen oder C₁-C₂₀-Alkylen, das durch ein oder mehrere Halogen, OR⁴, SR⁹, NR¹⁰R¹¹, CN, COOR⁴, CONR¹⁰R¹¹, C₃-C₈-Cycloalkyl, das ununterbrochen oder durch ein oder mehrere 0, S, CO oder NR¹⁰ unterbrochen ist, oder durch ein oder mehrere C₆-C₂₀-Aryl, C₃-C₂₀-Heteroaryl, C₆-C₂₀-Aroyl oder C₃-C₂₀-Heteroarylcarbonyl, wobei jede dieser Gruppen unsubstituiert oder durch ein oder mehrere Halogen, Phenyl, C₁-C₂₀-Alkylphenyl, C₁-C₈-Alkoxyphenyl, C₁-C₄-Halogenalkyl, CN, NO₂, OR⁴, SR⁹ oder NR¹⁰R¹¹ substituiert ist, substituiert ist, steht;
M' für eine direkte Bindung oder eine zweiwertige Verknüpfungsgruppe, die aus C₁-C₂₀-Alkylen oder C₁-C₂₀-Alkylen, das durch ein oder mehrere Halogen, OR⁴, SR⁹, NR¹⁰R¹¹, CN, COOR⁴, CONR¹⁰R¹¹, C₃-C₈-Cycloalkyl, das ununterbrochen oder durch ein oder mehrere O, S, CO oder NR¹⁰ unterbrochen ist, oder durch ein oder mehrere C₆-C₂₀-Aryl, C₃-C₂₀-Heteroaryl, C₆-C₂₀-Aroyl oder C₃-C₂₀-Heteroarylcarbonyl, wobei jede dieser Gruppen unsubstituiert oder durch ein oder mehrere Halogen, Phenyl, C₁-C₂₀-Alkylphenyl, C₁-C₈-Alkoxyphenyl, C₁-C₄-Halogenalkyl, CN, NO₂, OR⁴, SR⁹ oder NR¹⁰R¹¹ substituiert ist, substituiert ist, ausgewählt ist, steht;
M'' für eine direkte Bindung oder eine zweiwertige Verknüpfungsgruppe, die aus C₁-C₂₀-Alkylen oder C₁-C₂₀-Alkylen, das durch ein oder mehrere Halogen, OR⁴, SR⁹, NR¹⁰R¹¹, CN, COOR⁴, CONR¹⁰R¹¹, C₃-C₈-Cycloalkyl, das ununterbrochen oder durch ein oder mehrere O, S, CO oder NR¹⁰ unterbrochen ist, oder durch ein oder mehrere C₆-C₂₀-Aryl, C₃-C₂₀-Heteroaryl, C₆-C₂₀-Aroyl oder C₃-C₂₀-Heteroarylcarbonyl, wobei jede dieser Gruppen unsubstituiert oder durch ein oder mehrere Halogen, Phenyl, C₁-C₂₀-Alkylphenyl, C₁-C₈-Alkoxyphenyl, C₁-C₄-Halogenalkyl, CN, NO₂, OR⁴, SR⁹ oder NR¹⁰R¹¹ substituiert ist, substituiert ist, ausgewählt ist, steht; und
m für 1 oder 2 steht.

4. Verbindungen der Formel I, II, III und IV nach Anspruch 3, wobei es sich bei den Verbindungen um Verbindungen der Formel Ib handelt, wobei
R¹ für C₁-C₂₀-Alkyl, C₁-C₆-Alkyl-C₃C₆-cycloalkyl oder C₃-C₂₀-Cycloalkyl steht; oder
R¹ für C₂-C₂₀-Alkyl steht, das durch ein oder mehrere 0, CO, S, C(0)0, OC(O), SO, oder SO₂ unterbrochen ist; oder
R¹ für C₆-C₂₀-Aryl steht, das unsubstituiert oder durch ein oder mehrere C₁-C₂₀-Alkyl substituiert ist;
R² für C₁-C₂₀-Alkyl steht; oder
R² für C₂-C₂₀-Alkyl oder C₁-C₆-Alkyl-C₃C₆-cycloalkyl, das durch ein oder mehrere 0, C0, S, C(O)O, OC(O), SO oder SO₂ unterbrochen ist, steht; oder
R² für C₆-C₂₀-Aryl steht, das unsubstituiert oder durch ein oder mehrere C₁-C₁₂-Alkyl oder (CO)-R^{3a} substituiert ist;
R³ für C₁-C₂₀-Alkyl steht;
R^{3a} für C₁-C₂₀-Alkyl, C₆-C₂₀-Aryl oder C₃-C₂₀-Heteroaryl steht;
R⁴ für (CO) -R^{3a}, COOR^{4a}, CONR¹⁰R¹¹, S(O)ₘ-R^{3a} oder PO(OR^{3a})₂ steht;
R^{4a} für C₁-C₂₀-Alkyl, C₆-C₂₀-Aryl oder C₃-C₂₀-Heteroaryl steht;
R¹⁰ und R¹¹ für C₁-C₂₀-Alkyl stehen; und
m für 1 oder 2 steht.

5. Verbindungen der Formel I, II, III und IV nach einem der Ansprüche 1 bis 4, wobei
(i) mehr als 50 %, vorzugsweise 55 % oder mehr, weiter bevorzugt 75 % oder mehr, noch weiter bevorzugt 90 % oder mehr, wie 95 % oder mehr der in Oximestergruppen in dem Molekül vorliegenden C=N-Doppelbindungen in Z-Konfiguration vorliegen; oder
(ii) mehr als 50 %, vorzugsweise 55 % oder mehr, weiter bevorzugt 75 % oder mehr, noch weiter bevorzugt 90 % oder mehr, wie 95 % oder mehr der in Oximestergruppen in dem Molekül vorliegenden C=N-Doppelbindungen in E-Konfiguration vorliegen.

6. Photopolymerisierbare Zusammensetzung, umfassend
(a) mindestens eine ethylenisch ungesättigte photopolymerisierbare Verbindung und
(b) als Photoinitiator mindestens eine Verbindung der Formel I, II, III oder IV gemäß einem der Ansprüche 1 bis 5.

7. Photopolymerisierbare Zusammensetzung nach Anspruch 6, wobei es sich bei der Komponente (a) um ein durch Umsetzung eines Anhydrids einer gesättigten oder ungesättigten mehrbasigen Säure mit einem Produkt der Umsetzung eines Epoxidharzes und einer ungesättigten Monocarbonsäure erhaltenes Harz handelt; und/oder wobei die photopolymerisierbare Zusammensetzung zusätzlich zu dem Photoinitiator (b) mindestens einen weiteren Photoinitiator (c) und/oder weitere Zusatzstoffe (d) umfasst.

8. Photopolymerisierbare Zusammensetzung nach Anspruch 6, umfassend als weiteren Zusatzstoff (d) ein Pigment oder eine Mischung von Pigmenten oder einen Farbstoff oder eine Mischung von Farbstoffen oder eine Mischung von einem oder mehreren Pigmenten mit einem oder mehreren Farbstoffen; vorzugsweise umfassend als weiteren Zusatzstoff (d) ein Dispergiermittel oder eine Mischung von Dispergiermitteln.

9. Photopolymerisierbare Zusammensetzung nach einem der Ansprüche 6 bis 8, umfassend 0,05 bis 25 Gew.-% des Photoinitiators (b) bzw. der Photoinitiatoren (b) und (c), bezogen auf das Gewicht der Zusammensetzung; und/oder
umfassend als weiteren Zusatzstoff (d) einen Photosensibilisator, insbesondere eine Verbindung ausgewählt aus der Gruppe bestehend aus Benzophenon, Benzophenonderivaten, Thioxanthon, Thioxanthonderivaten, Anthrachinon, Anthrachinonderivaten, Cumarin und Cumarinderivaten; und/oder
zusätzlich umfassend ein Bindemittelpolymer (e), insbesondere ein Copolymer von Methacrylat und Methacrylsäure.

10. Verfahren zur Herstellung einer Verbindung der Formel I, II, III oder IV nach Anspruch 1 durch Umsetzen der entsprechenden Oximverbindung mit einem Acylhalogenid der Formel I' oder einem Anyhdrid der Formel I" 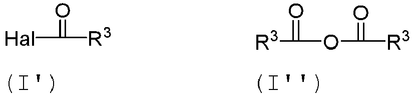 wobei Hal für ein Halogen, insbesondere Cl, steht und R³ wie in Anspruch 1 definiert ist, in Gegenwart einer Base oder einer Mischung von Basen.

11. Verfahren zur Photopolymerisation von Verbindungen mit ethylenisch ungesättigten Doppelbindungen, bei dem man eine Zusammensetzung nach einem der Ansprüche 6 bis 9 mit elektromagnetischer Strahlung im Bereich von 150 bis 600 nm oder mit Elektronenstrahlung oder mit Röntgenstrahlen bestrahlt.

12. Verfahren nach Anspruch 11 zur Herstellung von pigmentierten und nichtpigmentierten Farben und Lacken, Pulverbeschichtungen, Druckfarben, Druckplatten, Klebstoffen, Haftklebstoffen, Dentalzusammensetzungen, Gelcoats, Photoresists für die Elektronik, Galvanisierungsresists, Ätzresists, sowohl flüssigen als auch trockenen Filmen, Lötresists, Resists zur Herstellung von Farbfiltern für verschiedene Anzeigeanwendungen, Resists zur Erzeugung von Strukturen bei Herstellungsverfahren für Plasmaanzeigefelder, Elektrolumineszenzanzeigen und LCD, Abstandshaltern für LCD, für die holographische Datenspeicherung (HDS), als Zusammensetzung zum Verkapseln von elektrischen und elektronischen Bauteilen, zur Herstellung von magnetischen Aufzeichnungsmaterialien, mikromechanischen Teilen, Wellenleitern, optischen Schaltern, Plattierungsmasken, Ätzmasken, Farbprüfsystemen, Überzügen für Glasfaserkabel, Siebdruckschablonen, zur Herstellung von dreidimensionalen Objekten mittels Stereolithographie, als Bildaufzeichnungsmaterial, für holographische Aufzeichnungen, mikroelektronische Schaltkreise, Entfärbungsmaterialien, Entfärbungsmaterialien für Bildaufzeichnungsmaterialien, für Bildaufzeichnungsmaterialien unter Verwendung von Mikrokapseln, als Photoresistmaterial für ein UV- und VIS-Laser-Direct-Imaging-System, als Photoresistmaterial zur Bildung dielektrischer Schichten in einer sequenziellen Aufbauschicht einer Leiterplatte.

13. Beschichtetes Substrat, das auf mindestens einer Oberfläche mit einer Zusammensetzung nach einem der Ansprüche 6 bis 9 beschichtet ist.

14. Verfahren zur photographischen Herstellung von Reliefbildern, bei dem man ein beschichtetes Substrat nach Anspruch 13 bildmäßig belichtet und dann die unbelichteten Teile mit einem Entwickler entfernt.

15. Farbfilter, hergestellt durch Bereitstellen von roten, grünen und blauen Bildelementen und einer schwarzen Matrix, die alle ein lichtempfindliches Harz und ein Pigment auf einem transparenten Substrat umfassen, und Bereitstellen einer transparenten Elektrode entweder auf der Oberfläche des Substrats oder auf der Oberfläche der Farbfilterschicht, wobei das lichtempfindliche Harz ein polyfunktionelles Acrylatmonomer, ein organisches Polymerbindemittel und einen Photoinitiator der Formel I, II, III oder IV gemäß Anspruch 1 umfasst.

16. Verwendung einer Verbindung der Formel I, II, III oder IV gemäß Anspruch 1 zur Photopolymerisation einer Zusammensetzung, die mindestens eine ethylenisch ungesättigte photopolymerisierbare Verbindung umfasst.

17. Verbindungen der Formel IA, IIA, IIIA und IVA 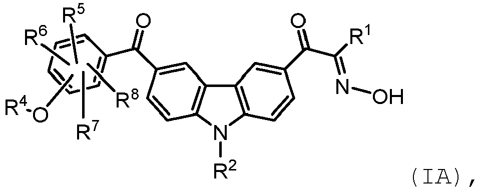 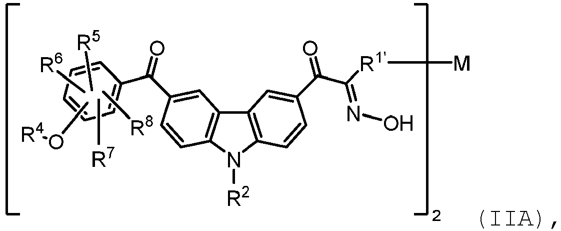 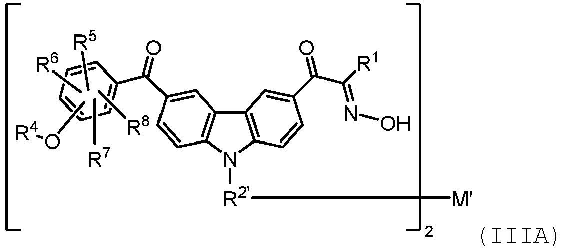 und 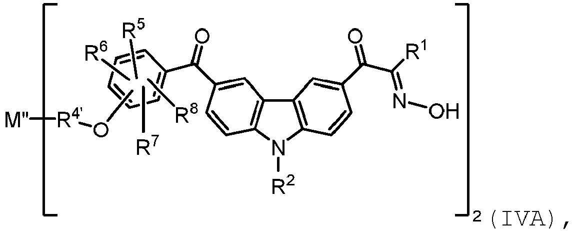 wobei
R¹, R², R⁴ bis R⁸, R¹' , R²', R⁴', M, M' und M" wie in einem der Ansprüche 1 bis 4 definiert sind.

## Revendications

1. Composés des formules I, II, III et IV 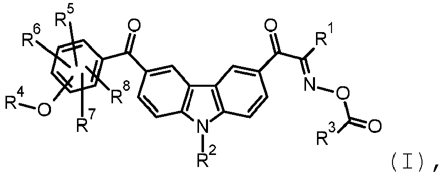 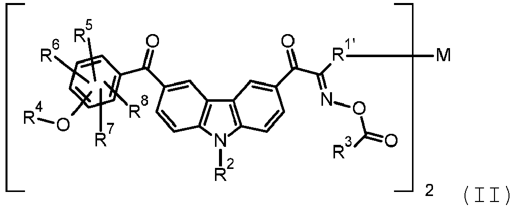 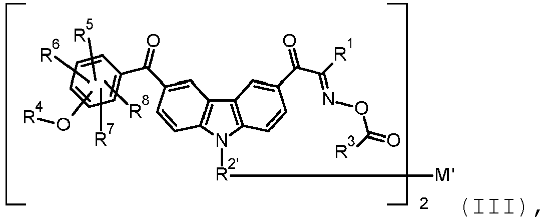 et 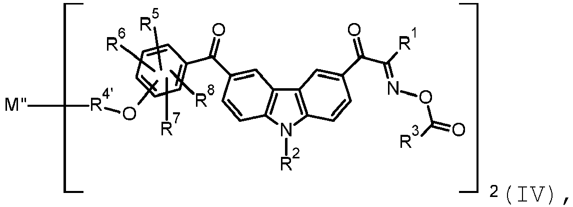 dans lesquels
R¹ est hydrogène, alkyle en C₁-C₂₀, (alkyle en C₁-C₆)-(cycloalkyle en C₃-C₆), cycloalkyle en C₃-C₂₀, alcényle en C₂-C₁₂, dans lequel cycloalkyle en C₃-C₂₀ ou alcényle en C₂-C₁₂ est ininterrompu ou interrompu par un ou plusieurs groupes O, S, CO, NR¹⁰ ou COOR⁴ ; ou
R¹ est alkyle en C₁-C₂₀ qui est non substitué ou substitué par un ou plusieurs groupes halogène, OR⁴, SR⁹, NR¹⁰R¹¹, COOR⁴ CONR¹⁰R¹¹, CN, PO (OR^{3a})₂, S(O)ₘ-R^{3a}, 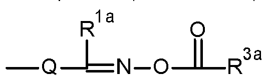 , cycloalkyle en C₃-C₈ qui est ininterrompu ou interrompu par un ou plusieurs groupes O, S, CO ou NR¹⁰ ; ou par un ou plusieurs groupes aryle en C₆-C₂₀, hétéroaryle en C₃-C₂₀, aroyle en C₆-C₂₀ ou (hétéroaryle en C₃-C₂₀) carbonyle, dont chacun est non substitué ou substitué par un ou plusieurs groupes halogène, phényle, (alkyle en C₁-C₂₀) phényle, (alcoxy en C₁-C₈) phényle, halogénoalkyle en C₁-C₄, CN, NO₂, OR⁴, SR⁹, NR¹⁰R¹¹, PO(OR^{3a})₂ ou S(O)ₘ-R^{3a} ; ou
R¹ est alkyle en C₂-C₂₀ qui est interrompu par un ou plusieurs groupes O, CO, S, C(O)O, OC(O), SO, SO₂, phénylène, naphtylène ou NR¹⁰, dans lequel l'alkyle en C₂-C₂₀ interrompu est non substitué ou substitué par un ou plusieurs groupes halogène, cycloalkyle en C₃-C₈, OH, SH, OR⁴, SR⁹, COOR⁴, O(CO)-R^{3a}, CONR¹⁰R¹¹, NR¹⁰R¹¹, aryle en C₆-C₂₀, hétéroaryle en C₃-C₂₀, aroyle en C₆-C₂₀ ou (hétéroaryle en C₃-C₂₀) carbonyle, dans lequel aryle en C₆-C₂₀, hétéroaryle en C₃-C₂₀, aroyle en C₆-C₂₀ ou (hétéroaryle en C₃-C₂₀)carbonyle est non substitué ou substitué par un ou plusieurs groupes halogène, alkyle en C₁-C₈, OR⁴, SR⁹ ou NR¹⁰R¹¹ ; ou
R¹ est aryle en C₆-C₂₀ ou hétéroaryle en C₃-C₂₀ dont chacun est non substitué ou substitué par un ou plusieurs groupes alkyle en C₁-C₂₀, phényle, halogène, halogénoalkyle en C₁-C₄, ON, NO₂, OR⁴, SR⁹, NR¹⁰R¹¹, COOR⁴, (CO) -R^{3a}, (CO) NR¹⁰R¹¹, PO(OR^{3a})₂, S(O)ₘ-R^{3a} ou un groupe 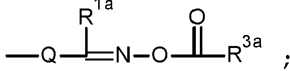 ou par un ou plusieurs groupes alkyle en C₂-C₂₀ qui sont interrompus par un ou plusieurs groupes O, S, ou NR¹⁰ ; ou par un ou plusieurs groupes alkyle en C₁-C₂₀ qui sont non substitués ou substitués par un ou plusieurs groupes halogène, COOR⁴, CONR¹⁰R¹¹, phényle, cycloalkyle en C₃-C₈, hétéroaryle en C₃-C₂₀, (aryloxy en C₆-C₂₀) carbonyle, (hétéroaryloxy en C₃-C₂₀) carbonyle, OR⁴, SR⁹ ou NR¹⁰R¹¹ ; ou par un ou plusieurs groupes phényle, naphtyle, benzoyle ou naphtoyle, dont chacun est non substitué ou substitué par OR⁴, SR⁹ ou NR¹⁰R¹¹ ;
R¹ est alcanoyle en C₂-C₂₀ ou benzoyle qui est non substitué ou substitué par un ou plusieurs groupes alkyle en C₁-C₆, phényle, OR⁴, SR⁹ ou NR¹⁰R¹¹ ; ou
R¹ est (alcoxy en C₂-C₁₂) carbonyle éventuellement interrompu par un ou plusieurs groupes -O- et/ou éventuellement substitué par un ou plusieurs groupes hydroxyle ; ou
R¹ est phénoxycarbonyle qui est non substitué ou substitué par alkyle en C₁-C₆, halogène, phényle, OR⁴, SR⁹ ou NR¹⁰R¹¹ ; ou
R¹ est CN, (CO)-R^{3a}, COOR⁴, CONR¹⁰R¹¹, NO₂, PO(OR^{3a})₂ ou S(O)ₘ-R^{3a} ;
R¹' est alkylène en C₁-C₂₀, alkylène en C₁-C₂₀ qui est interrompu par un ou plusieurs groupes O, S, (CO)O, O(CO), phénylène, naphtylène ou NR⁸, dans lequel l'alkylène en C₁-C₂₀ et l'alkylène en C₁-C₂₀ interrompu sont non substitués ou substitués par halogène ou OR⁴, ou R¹' est alcénylène en C₂-C₂₀, alcénylène en C₂-C₂₀ qui est interrompu par un ou plusieurs groupes O, S, (CO)O, O(CO), phénylène, naphtylène ou par NR¹⁰, dans lequel l'alcénylène en C₂-C₂₀ et l'alcénylène en C₂-C₂₀ interrompu sont non substitués ou substitués par halogène ou OR⁴, ou R¹' est cycloalkylène en C₅-C₈, cycloalcénylène en C₅-C₈, 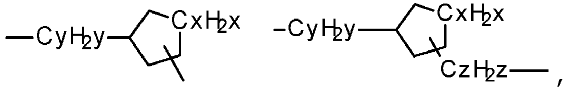 dans lequel les groupes C_{y}H_{2y} et C_{z}H_{2z} sont ininterrompus ou interrompus par un ou plusieurs groupes O, S ou par NR¹⁰ ;
R^{1a} est hydrogène, alkyle en C₁-C₂₀, CN, (CO) -R^{3a}, COOR^{4a}, CONR^{10a}R^{11a}, NO₂, PO(OR^{3a})₂ ou S(O)ₘ-R^{3a} ; ou
R^{1a} est alkyle en C₁-C₂₀ substitué par un ou plusieurs groupes halogène, OR^{4a}, SR^{9a}, NR^{10a}R^{11a}, CN, COOR^{4a}, CONR^{10a}R^{11a}, PO(OR^{3a})₂, S(O)ₘ-R^{3a}, cycloalkyle en C₃-C₈ qui est ininterrompu ou interrompu par un ou plusieurs groupes O, S, CO ou NR^{10a} ; ou par un ou plusieurs groupes aryle en C₆-C₂₀, hétéroaryle en C₃-C₂₀, aroyle en C₆-C₂₀ ou (hétéroaryle en C₃-C₂₀) carbonyle, dont chacun est non substitué ou substitué par un ou plusieurs groupes halogène, phényle, (alkyle en C₁-C₂₀)phényle, (alcoxy en C₂-C₈) phényle, halogénoalkyle en C₁-C₄, CN, NO₂, OR^{4a}, SR^{9a}, NR^{10a}R^{11a}, PO(OR^{3a})₂ ou S (O)ₘ-R^{3a} ; ou
R^{1a} est alkyle en C₁-C₂₀ interrompu par un ou plusieurs groupes O, S, NR^{10a}, CO, SO ou SO₂, qui est non substitué ou substitué par cycloalkyle en C₃-C₈, OH, SH, O(CO)R^{3a}, COOR^{4a}, CONR^{10a}R^{11a}, aryle en C₆-C₂₀, hétéroaryle en C₃-C₂₀, aroyle en C₆-C₂₀ ou (hétéroaryle en C₃-C₂₀) carbonyle, dans lequel aryle en C₆-C₂₀, hétéroaryle en C₃-C₂₀, aroyle en C₆-C₂₀ ou (hétéroaryle en C₃-C₂₀) carbonyle est non substitué ou substitué par un ou plusieurs groupes halogène, alkyle en C₁-C₈, OR^{4a}, SR^{9a} ou NR^{10a}R^{11a} ; ou
R^{1a} est alcényle en C₂-C₁₂ ou cycloalkyle en C₃-C₂₀, dont chacun est ininterrompu ou interrompu par un ou plusieurs groupes O, S, CO, NR^{10a} ou COOR^{4a} ; ou
R^{1a} est aryle en C₆-C₂₀ ou hétéroaryle en C₃-C₂₀, dont chacun est non substitué ou substitué par un ou plusieurs groupes halogène, CN, NO₂, OR^{4a}, SR^{9a}, NR^{10a}R^{11a}, COOR^{4a}, (CO)-R^{3a} CONR^{10a}R^{11a}, PO(OR^{3a})₂ ou S(O)ₘ-R^{3a} ; ou par un ou plusieurs groupes alkyle en C₁-C₂₀ qui sont non substitués ou substitués par un ou plusieurs groupes halogène, COOR^{4a}, CONR^{10a}R^{11a}, phényle, cycloalkyle en C₃-C₈, hétéroaryle en C₃-C₂₀, OR^{4a}, SR^{9a} ou NR^{10a}R^{11a} ; ou par un ou plusieurs groupes alkyle en C₂-C₂₀ qui sont interrompus par un ou plusieurs groupes O, S ou NR^{10a} ; ou par un ou plusieurs groupes phényle, naphtyle, benzoyle ou naphtoyle, dont chacun est non substitué ou substitué par OR^{4a}, SR^{9a} ou NR^{10a}R^{11a} ;
R² est hydrogène, alkyle en C₁-C₂₀ ou (alkyle en C₁-C₆)-(cycloalkyle en C₃-C₆) qui est non substitué ou substitué par un ou plusieurs groupes halogène, OR⁴, SR⁹, COOR⁴ CONR¹⁰R¹¹, NR¹⁰R²¹, PO(OR^{3a})₂, COR^{3a}, ou
R² est alkyle en C₂-C₂₀ ou (alkyle en C₁-C₆)-(cycloalkyle en C₃-C₆) qui est interrompu par un ou plusieurs groupes O, CO, S, C(O)O, OC(O), SO, SO₂, phénylène, naphtylène ou NR¹⁰, dans lequel l'alkyle en C₂-C₂₀ interrompu est non substitué ou substitué par un ou plusieurs groupes halogène, OR⁴, SR⁹, COOR⁴, CONR¹⁰R¹¹, NR¹⁰R¹¹ ; ou
R² est hydroxyalkyle en C₂-C₄, alcoxyalkyle en C₂-C₁₀, alcényle en C₃-C₅, cycloalkyle en C₃-C₈, phényl- (alkyle en C₁-C₃), alcanoyle en C₂-C₈, alcénoyle en C₃-C₁₂, benzoyle ; ou
R² est aryle en C₆-C₂₀ ou hétéroaryle en C₃-C₂₀ dont chacun est non substitué ou substitué par un ou plusieurs groupes alkyle en C₁-C₁₂, halogénoalkyle en C₁-C₄, phényle, halogène, CN, NO₂, OR⁴, SR⁹, NR¹⁰R¹¹, (CO) -R^{3a}, ou par alkyle en C₂-C₂₀ qui est interrompu par un ou plusieurs groupes O, S ou NR¹⁰, ou dont chacun est substitué par un ou plusieurs groupes alkyle en C₁-C₂₀ qui sont non substitués ou substitués par un ou plusieurs groupes halogène, COOR⁴, CONR¹⁰R¹¹, phényle, cycloalkyle en C₈-C₈, hétéroaryle en C₃-C₂₀, (aryloxy en C₆-C₂₀) carbonyle, hétéroaryloxy en C₃-C₂₀carbonyle, OR⁴, SR⁹ ou NR¹⁰R¹¹ ; ou
R² est un groupe 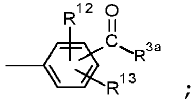
R^{2'} est alkylène en C₁-C₂₀, alkylène en C₁-C₂₀ qui est interrompu par un ou plusieurs groupes O, S, (CO)O, O(CO), phénylène, naphtylène ou NR⁸, dans lequel l'alkylène en C₁-C₂₀ et l'alkylène en C₁-C₂₀ interrompu sont non substitués ou substitués par halogène ou OR⁴, ou R^{2'} est alcénylène en C₂-C₂₀, alcénylène en C₂-C₂₀ qui est interrompu par un ou plusieurs groupes O, S, (CO)O, O(CO), phénylène, naphtylène ou par NR¹⁰, dans lequel l'alcénylène en C₂-C₂₀ et l'alcénylène en C₂-C₂₀ interrompu sont non substitués ou substitués par halogène ou OR⁴, ou R^{2'} est cycloalkylène en C₅-C₈, cycloalcénylène en C₅-C₈, 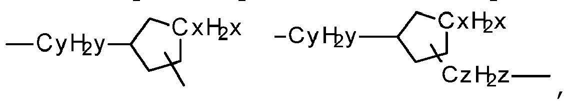 dans lequel les groupes C_{y}H_{2y} et C_{z}H_{2z} sont ininterrompus ou interrompus par un ou plusieurs groupes O, S ou par NR¹⁰ ;
R³ est hydrogène ou alkyle en C₁-C₂₀ ; ou
R³ est alkyle en C₁-C₂₀ substitué par un ou plusieurs groupes halogène, OR⁴, SR⁹, NR¹⁰R¹¹, CN, COOR⁴, CONR¹⁰R¹¹, cycloalkyle en C₃-C₈ qui est ininterrompu ou interrompu par un ou plusieurs groupes O, S, CO ou NR¹⁰ ; ou par un ou plusieurs groupes aryle en C₆-C₂₀, hétéroaryle en C₃-C₂₀, aroyle en C₆-C₂₀ ou (hétéroaryle en C₃-C₂₀) carbonyle, dont chacun est non substitué ou substitué par un ou plusieurs groupes halogène, phényle, (alkyle en C₁-C₂₀) phényle, (alcoxy en C₁-C₈) phényle, halogénoalkyle en C₁-C₄, CN, NO₂, OR⁴, SR⁹ ou NR¹⁰R¹¹ ; ou
R³ est alkyle en C₁-C₂₀ interrompu par un ou plusieurs groupes O, S, NR¹⁰, CO, SO ou SO₂, qui est non substitué ou substitué par cycloalkyle en C₃-C₈, OH, SH, O(CO)-R^{3a}, COOR⁴, CONR¹⁰R¹¹, aryle en C₆-C₂₀, hétéroaryle en C₃-C₂₀, aroyle en C₆-C₂₀ ou (hétéroaryle en C₃-C₂₀) carbonyle, dans lequel aryle en C₆-C₂₀, hétéroaryle en C₃-C₂₀, aroyle en C₆-C₂₀ ou (hétéroaryle en C₃-C₂₀) carbonyle est non substitué ou substitué par un ou plusieurs groupes halogène, alkyle en C₁-C₈, OR⁴, SR⁹ ou NR¹⁰R¹¹ ; ou
R³ est alcényle en C₂-C₁₂ ou cycloalkyle en C₃-C₂₀, dont chacun est ininterrompu ou interrompu par un ou plusieurs groupes O, S, CO, NR¹⁰ ou COOR⁴ ; ou
R³ est aryle en C₆-C₂₀ ou hétéroaryle en C₃-C₂₀, dont chacun est non substitué ou substitué par un ou plusieurs groupes halogène, alkyle en C₁-C₂₀, halogénoalkyle en C₁-C₄, phényle, (alkyle en C₁-C₂₀) phényle, (alcoxy en C₁-C₈) phényle, CN, NO₂, OR⁴, SR⁹, NR¹⁰R¹¹, COOR⁴, (CO)-R^{3a} ou SO₂-R^{3a} ; ou
R³ est alcoxy en C₁-C₂₀, qui est non substitué ou substitué par un ou plusieurs groupes alkyle en C₁-C₁₀, halogénoalkyle en C₁-C₄, halogène, phényle, (alkyle en C₁-C₂₀) phényle ou (alcoxy en C₁-C₈)phényle ; ou
R³ est alcoxy en C₁-C₂₀, qui est interrompu par un ou plusieurs groupes O, S, NR¹⁰, CO, SO ou SO₂ ; ou
R³ est aryloxy en C₆-C₂₀ ou hétéroaryloxy en C₃-C₂₀, dont chacun est non substitué ou substitué par un ou plusieurs groupes halogène, alkyle en C₁-C₂₀, halogénoalkyle en C₁-C₄, phényle, (alkyle en C₁-C₂₀) phényle, (alcoxy en C₁-C₈)phényle, CN, NO₂, OR⁴, SR⁹, NR¹⁰R¹¹, COOR⁴, (CO)-R^{3a} ou SO₂-R^{3a} ;
R^{3a} est hydrogène ou alkyle en C₁-C₂₀ ; ou
R^{3a} est alkyle en C₁-C₂₀ substitué par un ou plusieurs groupes halogène, OR^{4a}, SR^{9a}, NR^{10a}R^{11a}, CN, COOR^{4a}, CONR^{10a}R^{11a}, cycloalkyle en C₃-C₈ qui est ininterrompu ou interrompu par un ou plusieurs groupes O, S, CO ou NR^{10a} ; ou par un ou plusieurs groupes aryle en C₆-C₂₀, hétéroaryle en C₃-C₂₀, aroyle en C₆-C₂₀ ou (hétéroaryle en C₃-C₂₀)carbonyle, dont chacun est non substitué ou substitué par un ou plusieurs groupes halogène, phényle, (alkyle en C₁-C₂₀)phényle, (alcoxy en C₁-C₈)phényle, halogénoalkyle en C₁-C₄, CN, NO₂, OR^{4a}, SR^{9a} ou NR^{10a}R^{11a} ; ou
R^{3a} est alkyle en C₁-C₂₀ interrompu par un ou plusieurs groupes O, S, NR^{10a}, CO, SO ou SO₂, qui est non substitué ou substitué par cycloalkyle en C₃-C₈, OH, SH, O(CO)-(alkyle en C₁-C₈), COOR^{4a}, CONR^{10a}R^{11a}, aryle en C₆-C₂₀, hétéroaryle en C₃-C₂₀, aroyle en C₆-C₂₀ ou (hétéroaryle en C₃-C₂₀)carbonyle, dans lequel aryle en C₆-C₂₀, hétéroaryle en C₃-C₂₀, aroyle en C₆-C₂₀ ou (hétéroaryle en C₃-C₂₀)carbonyle est non substitué ou substitué par un ou plusieurs groupes halogène, alkyle en C₂-C₈, OR^{4a}, SR^{9a} ou NR^{10a}R^{11a} ; ou
R^{3a} est alcényle en C₂-C₁₂ ou cycloalkyle en C₃-C₂₀, dont chacun est ininterrompu ou interrompu par un ou plusieurs groupes O, S, CO, NR^{10a} ou COOR^{4a} ; ou
R^{3a} est aryle en C₆-C₂₀ ou hétéroaryle en C₃-C₂₀, dont chacun est non substitué ou substitué par un ou plusieurs groupes halogène, alkyle en C₁-C₂₀, halogénoalkyle en C₁-C₄, phényle, (alkyle en C₁-C₂₀) phényle, (alcoxy en C₁-C₈) phényle, CN, NO₂, OR^{4a}, SR^{9a}, NR^{10a}R^{11a}, COOR^{4a}, (CO)-(alkyle en C₂-C₈) ou SO₂-(halogénoalkyle en C₁-C₄) ; ou
R^{3a} est alcoxy en C₁-C₂₀, qui est non substitué ou substitué par un ou plusieurs groupes alkyle en C₂-C₁₀, halogénoalkyle en C₁-C₄, halogène, phényle, (alkyle en C₁-C₂₀) phényle ou (alcoxy en C₂-C₈) phényle ; ou
R^{3a} est alcoxy en C₁-C₂₀, qui est interrompu par un ou plusieurs groupes O, S, NR^{10a}, CO, SO ou SO₂ ; ou
R^{3a} est aryloxy en C₆-C₂₀ ou hétéroaryloxy en C₃-C₂₀, dont chacun est non substitué ou substitué par un ou plusieurs groupes halogène, alkyle en C₁-C₂₀, halogénoalkyle en C₁-C₄, phényle, (alkyle en C₁-C₂₀) phényle, (alcoxy en C₁-C₈) phényle, CN, NO₂, OR^{4a}, SR^{9a}, NR^{10a}R^{11a,} COOR^{4a}, (CO) - (alkyle en C₂-C₈) ou SO₂- (halogénoalkyle en C₁-C₄) ; ou
R^{3a} est un groupe 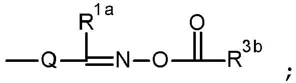
R^{3b} est hydrogène ou alkyle en C₁-C₂₀ ; ou
R^{3b} est alkyle en C₁-C₂₀ substitué par un ou plusieurs groupes halogène, OR^{4a}, SR^{9a}, NR^{10a}R^{11a}, CN, COOR^{4a}, CONR^{10a}R^{11a}, cycloalkyle en C₃-C₈ qui est ininterrompu ou interrompu par un ou plusieurs groupes O, S, CO ou NR^{10a} ; ou par un ou plusieurs groupes aryle en C₆-C₂₀, hétéroaryle en C₃-C₂₀, aroyle en C₆-C₂₀ ou (hétéroaryle en C₃-C₂₀) carbonyle, dont chacun est non substitué ou substitué par un ou plusieurs groupes halogène, phényle, (alkyle en C₁-C₂₀) phényle, (alcoxy en C₁-C₈) phényle, halogénoalkyle en C₁-C₄, CN, NO₂, OR^{4a}, SR^{9a} ou NR^{10a}R^{11a} ; ou
R^{3b} est alkyle en C₁-C₂₀ interrompu par un ou plusieurs groupes O, S, NR^{10a}, CO, SO ou SO₂, qui est non substitué ou substitué par cycloalkyle en C₃-C₈, OH, SH, O(CO)-(alkyle en C₁-C₈), COOR^{4a}, CONR^{10a}R^{11a}, aryle en C₆-C₂₀, hétéroaryle en C₃-C₂₀, aroyle en C₆-C₂₀ ou (hétéroaryle en C₃-C₂₀) carbonyle, dans lequel aryle en C₆-C₂₀, hétéroaryle en C₃-C₂₀, aroyle en C₆-C₂₀ ou (hétéroaryle en C₃-C₂₀) carbonyle est non substitué ou substitué par un ou plusieurs groupes halogène, alkyle en C₁-C₈, OR^{4a}, SR^{9a} ou NR^{10a}R^{11a} ; ou
R^{3b} est alcényle en C₂-C₁₂ ou cycloalkyle en C₃-C₂₀, dont chacun est ininterrompu ou interrompu par un ou plusieurs groupes O, S, CO, NR^{10a} ou COOR^{4a} ; ou
R^{3b} est aryle en C₆-C₂₀ ou hétéroaryle en C₃-C₂₀, dont chacun est non substitué ou substitué par un ou plusieurs groupes halogène, alkyle en C₁-C₂₀, halogénoalkyle en C₁-C₄, phényle, (alkyle en C₁-C₂₀) phényle, (alcoxy en C₁-C₈) phényle, CN, NO₂, OR^{4a}, SR^{9a}, NR^{10a}R^{11a}, COOR^{4a}, (CO) - (alkyle en C₁-C₈) ou SO₂- (halogénoalkyle en C₁-C₄) ; ou
R^{3b} est alcoxy en C₁-C₂₀, qui est non substitué ou substitué par un ou plusieurs groupes alkyle en C₁-C₁₀, halogénoalkyle en C₁-C₄, halogène, phényle, (alkyle en C₁-C₂₀) phényle ou (alcoxy en C₁-C₈) phényle ; ou
R^{3b} est alcoxy en C₁-C₂₀, qui est interrompu par un ou plusieurs groupes O, S, NR^{10a}, CO, SO ou SO₂ ; ou
R^{3b} est aryloxy en C₆-C₂₀ ou hétéroaryloxy en C₃-C₂₀, dont chacun est non substitué ou substitué par un ou plusieurs groupes halogène, alkyle en C₁-C₂₀, halogénoalkyle en C₁-C₄, phényle, (alkyle en C₁-C₂₀) phényle, (alcoxy en C₁-C₈) phényle, CN, NO₂, OR^{4a}, SR^{9a}, NR^{10a}R^{11a}, COOR^{4a}, (CO) - (alkyle en C₁-C₈) ou SO₂- (halogénoalkyle en C₁-C₄) ;
R⁴ est hydrogène, (CO)-R^{3a}, COOR^{4a}, CONR¹⁰R¹¹, S (O) ₘ-R^{3a} ou PO (OR^{3a}) ₂ ; ou
R⁴ est alkyle en C₁-C₂₀ substitué par un ou plusieurs groupes halogène, OR^{4a}, SR^{9a}, NR^{10a}R^{11a}, CN, COOR^{4a}, CONR^{10a}R^{11a}, PO(OR^{3a}) ₂, S(O)ₘ-R^{3a}, cycloalkyle en C₃-C₈ qui est ininterrompu ou interrompu par un ou plusieurs groupes O, S, CO ou NR^{10a} ; ou par un ou plusieurs groupes aryle en C₆-C₂₀, hétéroaryle en C₃-C₂₀, aroyle en C₆-C₂₀ ou (hétéroaryle en C₃-C₂₀) carbonyle, dont chacun est non substitué ou substitué par un ou plusieurs groupes halogène, phényle, (alkyle en C₁-C₂₀)phényle, (alcoxy en C₁-C₈) phényle, halogénoalkyle en C₁-C₄, CN, NO₂, OR^{4a}, SR^{9a}, NR^{10a}R^{11a}, PO(OR^{3a})₂ ou S(O)ₘ-R^{3a} ; ou
R⁴ est alkyle en C₁-C₂₀ interrompu par un ou plusieurs groupes O, S, NR^{10a}, CO, SO ou SO₂, qui est non substitué ou substitué par cycloalkyle en C₃-C₈, OH, SH, O(CO)R^{3a}, COOR^{4a}, CONR^{10a}R^{11a}, aryle en C₆-C₂₀, hétéroaryle en C₃-C₂₀, aroyle en C₆-C₂₀ ou (hétéroaryle en C₃-C₂₀) carbonyle, dans lequel aryle en C₆-C₂₀, hétéroaryle en C₃-C₂₀, aroyle en C₆-C₂₀ ou (hétéroaryle en C₃-C₂₀) carbonyle est non substitué ou substitué par un ou plusieurs groupes halogène, alkyle en C₁-C₈, OR^{4a}, SR^{9a} ou NR^{10a}R^{11a} ; ou
R⁴ est alcényle en C₂-C₁₂ ou cycloalkyle en C₃-C₂₀, dont chacun est ininterrompu ou interrompu par un ou plusieurs groupes O, S, CO, NR^{10a} ou COOR^{4a} ; ou
R⁴ est aryle en C₆-C₂₀, qui est substitué par un ou plusieurs groupes halogène, CN, NO₂, OR^{4a}, SR^{9a}, NR^{10a}R^{11a}, COOR^{4a}, (CO)-R^{3a}, CONR^{10a}R^{11a}, PO(OR^{3a})₂, S(O)ₘ-R^{3a}ou un groupe 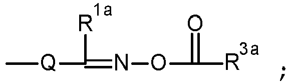 ou par un ou plusieurs groupes alkyle en C₁-C₂₀ qui sont non substitués ou substitués par un ou plusieurs groupes halogène, COOR^{4a}, CONR^{10a}R^{11a}, phényle, cycloalkyle en C₃-C₈, hétéroaryle en C₃-C₂₀, OR^{4a}, SR^{9a} ou NR^{10a}R^{11a} ; ou par un ou plusieurs groupes alkyle en C₂-C₂₀ qui sont interrompus par un ou plusieurs groupes O, S ou NR^{10a} ; ou par un ou plusieurs groupes phényle, naphtyle, benzoyle ou naphtoyle, dont chacun est non substitué ou substitué par OR^{4a}, SR^{9a} ou NR^{10a}R^{11a} ; ou
R⁴ est hétéroaryle en C₃-C₂₀, dont chacun est non substitué ou substitué par un ou plusieurs groupes halogène, CN, NO₂, OR^{4a}, SR^{9a}, NR^{10a}R^{11a}, COOR^{4a}, (CO) -R^{3a}, CONR^{10a}R^{11a}, PO(OR^{3a})₂, S(O)ₘ-R^{3a} ou groupe 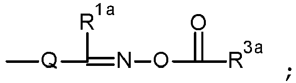 ou par un ou plusieurs groupes alkyle en C₁-C₂₀ qui sont non substitués ou substitués par un ou plusieurs groupes halogène, COOR^{4a}, CONR^{10a}R^{11a}, phényle, cycloalkyle en C₃-C₈, hétéroaryle en C₃-C₂₀, OR^{4a}, SR^{9a} ou NR^{10a}R^{11a} ; ou par un ou plusieurs groupes alkyle en C₂-C₂₀ qui sont interrompus par un ou plusieurs groupes O, S ou NR^{10a} ; ou par un ou plusieurs groupes phényle, naphtyle, benzoyle ou naphtoyle, dont chacun est non substitué ou substitué par OR^{4a}, SR^{9a} ou NR^{10a}R^{11a} ; ou
R⁴, conjointement avec l'un des atomes de carbone de R¹, forme un cycle saturé ou insaturé de 5 ou 6 chaînons qui est ininterrompu ou interrompu par O, S ou NR^{10a}, et ledit cycle saturé ou insaturé de 5 ou 6 chaînons est non substitué ou substitué par un ou plusieurs groupes alkyle en C₁-C₂₀, OR^{4a}, SR^{9a}, NR^{10a}R^{11a}, (CO)-R^{3a}, NO₂, halogène, halogénoalkyle en C₁-C₄, CN, phényle, (alkyle en C₁-C₂₀) phényle, (alcoxy en C₁-C₈) phényle, 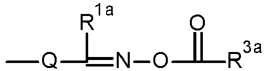 ou cycloalkyle en C₃-C₂₀ qui est ininterrompu ou interrompu par un ou plusieurs groupes O, S, CO ou NR^{10a} ;
R⁴' est -(CO)-, -(CO)O-, -(CO)N-, -S(O)ₘ-, -PO(O)₂-, ou - OP(O)₂ ;
R^{4a} est hydrogène, alkyle en C₁-C₂₀, (CO)O(alkyle en C₁-C₈) ou CON (alkyle en C₁-C₈)₂ ; ou
R^{4a} est alkyle en C₁-C₂₀ substitué par un ou plusieurs groupes halogène, OH, SH, CN, alcénoxy en C₃-C₈, OCH₂CH₂CN, OCH₂CH₂(CO)O (alkyle en C₁-C₈), O(CO)-(alkyle en C₁-C₈), O(CO)-(alcényle en C₂-C₄), O(CO)-phényle, (CO)OH, (CO) O (alkyle en C₁-C₈), cycloalkyle en C₃-C₈, SO₂-(halogénoalkyle en C₁-C₄), O(C₁-C₄haloalkyl), phényle, (alkyle en C₁-C₈) phényle, (alcoxy en C₁-C₈) phényle ou cycloalkyle en C₃-C₈ qui est interrompu par un ou plusieurs groupes O ; ou
R^{4a} est alkyle en C₂-C₂₀ interrompu par un ou plusieurs groupes O, S, N(alkyle en C₁-C₈), CO, SO ou SO₂, qui est non substitué ou substitué par cycloalkyle en C₃-C₈, OH, SH, O(CO) (alkyle en C₁-C₈), (CO) O (alkyle en C₁-C₈), (CO) N (alkyle en C₁-C₈)₂, aryle en C₆-C₂₀, hétéroaryle en C₃-C₂₀, aroyle en C₆-C₂₀ ou (hétéroaryle en C₃-C₂₀)carbonyle, dans lequel aryle en C₆-C₂₀, hétéroaryle en C₃-C₂₀, aroyle en C₆-C₂₀ ou (hétéroaryle en C₃-C₂₀)carbonyle est non substitué ou substitué par un ou plusieurs groupes halogène, alkyle en C₁-C₈, alcoxy en C₁-C₈, (alkyle en C₁-C₈)sulfanyle ou N(alkyle en C₁-C₈)₂ ; ou
R^{4a} est alcényle en C₂-C₁₂, (CO) O(alcényle en C₂-C₈) ou cycloalkyle en C₃-C₈, dont chacun est ininterrompu ou interrompu par un ou plusieurs groupes O, S, CO, N(alkyle en C₁-C₈) ou COO (alkyle en C₂-C₈) ; ou
R^{4a} est aryle en C₆-C₂₀, hétéroaryle en C₃-C₂₀, aroyle en C₆-C₂₀ ou (hétéroaryle en C₃-C₂₀)carbonyle, dont chacun est non substitué ou substitué par un ou plusieurs groupes halogène, CN, NO₂, OH, alkyle en C₁-C₈, halogénoalkyle en C₁-C₄, alcoxy en C₁-C₈, phényl- (alkyloxy en C₁-C₃), phénoxy, (alkyle en C₁-C₈)sulfanyle, phénylsulfanyle, N(alkyle en C₁-C₈)₂, diphénylamino, (CO)O(alkyle en C₁-C₈), (CO)-(alkyle en C₁-C₈) ou (CO)N(C₁-C₈)₂, phényle ou benzoyle ; ou
R^{4a} est alcanoyle en C₁-C₂₀, alcénoyle en C₃-C₁₂, dont chacun est non substitué ou substitué par un ou plusieurs groupes halogène, phényle, (alkyle en C₁-C₈)phényle, (alcoxy en C₁-C₈) phényle, OH, alcoxy en C₁-C₈, phénoxy, (alkyle en C₁-C₈)sulfanyle, phénylsulfanyle, N(alkyle en C₁-C₈)₂ ou diphénylamino ;
R⁵, R⁶, R⁷ et R⁸, indépendamment les uns des autres, sont hydrogène, alkyle en C₁-C₂₀, aryle en C₆-C₂₀, alcoxy en C₁-C₂₀, (aryle en C₆-C₂₀) (alkyle en C₁-C₂₀) , hydroxy(alkyle en C₁-C₂₀), hydroxy(alcoxy en C₁-C₂₀) (alkyle en C₁-C₂₀), cycloalkyle en C₃-C₁₀, amino, CN, NO₂, hydroxy, 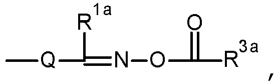 (CO) -R^{3a}, OR^{4a} ou COOR⁴ ;
R⁹ est hydrogène ou alkyle en C₁-C₂₀ ; ou
R⁹ est alkyle en C₁-C₂₀ substitué par un ou plusieurs groupes halogène, OR^{4a}, SR^{9a}, NR^{10a}R^{11a}, CN, COOR^{4a}, CONR^{10a}R^{11a}, PO(OR^{3a})₂, S(O)ₘ-R^{3a}, cycloalkyle en C₃-C₈ qui est ininterrompu ou interrompu par un ou plusieurs groupes O, S, CO ou NR^{10a} ; ou par un ou plusieurs groupes aryle en C₆-C₂₀, hétéroaryle en C₃-C₂₀, aroyle en C₆-C₂₀ ou (hétéroaryle en C₃-C₂₀) carbonyle, dont chacun est non substitué ou substitué par un ou plusieurs groupes halogène, phényle, (alkyle en C₁-C₂₀)phényle, (alcoxy en C₁-C₈) phényle, halogénoalkyle en C₁-C₄, CN, NO₂, OR^{4a}, SR^{9a}, NR^{10a}R^{11a}, PO(OR^{3a})₂ ou S(O)ₘ-R^{3a} ; ou
R⁹ est alkyle en C₁-C₂₀ interrompu par un ou plusieurs groupes O, S, NR^{10a}, CO, SO ou SO₂, qui est non substitué ou substitué par cycloalkyle en C₃-C₈, OH, SH, O(CO)R^{3a}, COOR^{4a}, CONR^{10a}R^{11a}, aryle en C₆-C₂₀, hétéroaryle en C₃-C₂₀, aroyle en C₆-C₂₀ ou (hétéroaryle en C₃-C₂₀) carbonyle, dans lequel aryle en C₆-C₂₀, hétéroaryle en C₃-C₂₀, aroyle en C₆-C₂₀ ou (hétéroaryle en C3-C20) carbonyle est non substitué ou substitué par un ou plusieurs groupes halogène, alkyle en C₁-C₈, OR^{4a}, SR^{9a} ou NR^{10a}R^{11a} ; ou
R⁹ est alcényle en C₂-C₁₂ ou cycloalkyle en C₃-C₂₀, dont chacun est ininterrompu ou interrompu par un ou plusieurs groupes O, S, CO, NR^{10a} ou COOR^{4a} ; ou
R⁹ est aryle en C₆-C₂₀ ou hétéroaryle en C₃-C₂₀, dont chacun est non substitué ou substitué par un ou plusieurs groupes halogène, CN, NO₂, OR^{4a}, SR^{9a}, NR^{10a}R^{11a}, COOR^{4a}, (CO)-R^{3a} CONR^{10a}R^{11a}, PO(OR^{3a})₂, S(O)ₘ-R^{3a} ou groupe 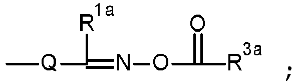 ou par un ou plusieurs groupes alkyle en C₁-C₂₀ qui est non substitué ou substitué par un ou plusieurs groupes halogène, COOR^{4a}, CONR^{10a}R^{11a}, phényle, cycloalkyle en C₃-C₈, hétéroaryle en C₃-C₂₀, OR^{4a}, SR^{9a} ou NR^{10a}R^{11a} ; ou par un ou plusieurs groupes alkyle en C₂-C₂₀ qui est interrompu par un ou plusieurs groupes O, S ou NR^{10a} ; ou par un ou plusieurs groupes phényle, naphtyle, benzoyle ou naphtoyle, dont chacun est non substitué ou substitué par OR^{4a}, SR^{9a} ou NR^{10a}R^{11a} ; ou
R⁹, conjointement avec l'un des atomes de carbone de R¹, forme un cycle saturé ou insaturé de 5 ou 6 chaînons qui est ininterrompu ou interrompu par O, S ou NR^{10a}, et ledit cycle saturé ou insaturé de 5 ou 6 chaînons est non substitué ou substitué par un ou plusieurs groupes alkyle en C₁-C₂₀, OR^{4a}, SR^{9a}, NR^{10a}R^{11a}, (CO)-R^{3a}, NO₂, halogène, halogénoalkyle en C₁-C₄, CN, phényle, (alkyle en C₁-C₂₀) phényle, (alcoxy en C₂-C₈) phényle, 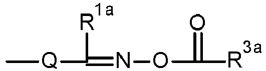 ou cycloalkyle en C₃-C₂₀ qui est ininterrompu ou interrompu par un ou plusieurs groupes O, S, CO ou NR^{10a} ;
R^{9a} est hydrogène ou alkyle en C₁-C₂₀ ; ou
R^{9a} est alkyle en C₁-C₂₀ substitué par un ou plusieurs groupes halogène, OH, SH, CN, alcénoxy en C₃-C₈, OCH₂CH₂CN, OCH₂CH₂ (CO) O (alkyle en C₁-C₈), O(CO)-(alkyle en C₁-C₈), O(CO)-(alcényle en C₂-C₄), O(CO)-phényle, (CO)OH, (CO) O (alkyle en C₁-C₈), cycloalkyle en C₃-C₈, SO₂-(halogénoalkyle en C₁-C₄), O(C₁-C₄haloalkyl), phényle, (alkyle en C₁-C₈) phényle, (alcoxy en C₁-C₈) phényle ou cycloalkyle en C₃-C₈ qui est interrompu par un ou plusieurs groupes O ; ou
R^{9a} est alkyle en C₂-C₂₀ interrompu par un ou plusieurs groupes O, S, N(alkyle en C₁-C₈), CO, SO ou SO₂, qui est non substitué ou substitué par cycloalkyle en C₃-C₈, OH, SH, O(CO) (alkyle en C₁-C₈), (CO) O (alkyle en C₁-C₈), (CO)N(alkyle en C₁-C₈)₂, aryle en C₆-C₂₀, hétéroaryle en C₃-C₂₀, aroyle en C₆-C₂₀ ou (hétéroaryle en C₃-C₂₀)carbonyle, dans lequel aryle en C₆-C₂₀, hétéroaryle en C₃-C₂₀, aroyle en C₆-C₂₀ ou (hétéroaryle en C₃-C₂₀)carbonyle est non substitué ou substitué par un ou plusieurs groupes halogène, alkyle en C₁-C₈, alcoxy en C1-C8, (alkyle en C₁-C₈)sulfanyle ou N (alkyle en C₁-C₈)₂ ; ou
R^{9a} est alcényle en C₂-C₁₂ ou cycloalkyle en C₃-C₈, dont chacun est ininterrompu ou interrompu par un ou plusieurs groupes O, S, CO, N(alkyle en C₁-C₈) ou COO(alkyle en C₁-C₈) ; ou
R^{9a} est aryle en C₆-C₂₀, hétéroaryle en C₃-C₂₀, aroyle en C₆-C₂₀ ou (hétéroaryle en C₃-C₂₀)carbonyle, dont chacun est non substitué ou substitué par un ou plusieurs groupes halogène, CN, NO₂, OH, alkyle en C₁-C₈, halogénoalkyle en C₁-C₄, alcoxy en C1-C8, phényl-(alkyloxy en C₁-C₃), phénoxy, (alkyle en C₁-C₈)sulfanyle, phénylsulfanyle, N (alkyle en C₁-C₈)₂, diphénylamino, (CO) O (alkyle en C₁-C₈), (CO) - (alkyle en C₁-C₈) ou (CO)N(C₁-C₈)₂, phényle ou benzoyle ; ou
R^{9a} est alcanoyle en C₁-C₂₀, alcénoyle en C₃-C₁₂, dont chacun est non substitué ou substitué par un ou plusieurs groupes halogène, phényle, (alkyle en C₁-C₈)phényle, (alcoxy en C₁-C₈) phényle, OH, alcoxy en C1-C8, phénoxy, (alkyle en C₁-C₈)sulfanyle, phénylsulfanyle, N(alkyle en C₁-C₈)₂ ou diphénylamino ;
R¹⁰ et R¹¹, indépendamment l'un de l'autre, sont hydrogène, alkyle en C₁-C₂₀, S(O)ₘ-R^{3a}, O(CO) -R^{3a}, (CO)-R^{3a} ou CONR^{10a}R^{11a} ; ou
R¹⁰ et R¹¹, indépendamment l'un de l'autre, sont alkyle en C₁-C₂₀ substitué par un ou plusieurs groupes halogène, OR^{4a}, SR^{9a}, NR^{10a}R^{11a}, CN, COOR^{4a}, CONR^{10a}R^{11a}, PO(OR^{3a})₂, S(O) ₘ-R^{3a}, cycloalkyle en C₃-C₈ qui est ininterrompu ou interrompu par un ou plusieurs groupes O, S, CO ou NR^{10a} ; ou par un ou plusieurs groupes aryle en C₆-C₂₀, hétéroaryle en C₃-C₂₀, aroyle en C₆-C₂₀ ou (hétéroaryle en C₃-C₂₀) carbonyle, dont chacun est non substitué ou substitué par un ou plusieurs groupes halogène, phényle, (alkyle en C₁-C₂₀) phényle, (alcoxy en C₁-C₈) phényle, halogénoalkyle en C₁-C₄, CN, NO₂, OR^{4a}, SR^{9a}, NR^{10a}R^{11a}, PO(OR^{3a})₂ ou S(O)ₘ-R^{3a} ; ou
R¹⁰ et R¹¹, indépendamment l'un de l'autre, sont alkyle en C₁-C₂₀ interrompu par un ou plusieurs groupes O, S, NR^{10a}, CO, SO ou SO₂, qui est non substitué ou substitué par cycloalkyle en C₃-C₈, OH, SH, O (CO) -R^{3a}, COOR^{4a}, CONR^{10a}R^{11a}, aryle en C₆-C₂₀, hétéroaryle en C₃-C₂₀, aroyle en C₆-C₂₀ ou (hétéroaryle en C₃-C₂₀)carbonyle, dans lesquels aryle en C₆-C₂₀, hétéroaryle en C₃-C₂₀, aroyle en C₆-C₂₀ ou (hétéroaryle en C₃-C₂₀) carbonyle est non substitué ou substitué par un ou plusieurs groupes halogène, alkyle en C₁-C₈, OR^{4a}, SR^{9a} ou NR^{10a}R^{11a} ; ou
R¹⁰ et R¹¹, indépendamment l'un de l'autre, sont alcényle en C₂-C₁₂ ou cycloalkyle en C₃-C₂₀, dont chacun est ininterrompu ou interrompu par un ou plusieurs groupes O, S, CO, NR^{10a} ou COOR^{4a} ; ou
R¹⁰ et R¹¹, indépendamment l'un de l'autre, sont aryle en C₆-C₂₀ ou hétéroaryle en C₃-C₂₀, dont chacun est non substitué ou substitué par un ou plusieurs groupes halogène, CN, NO₂, OR^{4a}, SR^{9a}, NR^{10a}R^{11a}, COOR^{4a}, (CO) - R^{3a}CONR^{10a}R^{11a}, PO(OR^{3a})₂, S(O)ₘ-R^{3a} ou un groupe 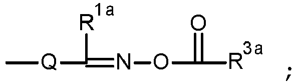 ou par un ou plusieurs groupes alkyle en C₁-C₂₀ qui est non substitué ou substitué par un ou plusieurs groupes halogène, COOR^{4a}, CONR^{10a}R^{11a}, phényle, cycloalkyle en C₃-C₈, hétéroaryle en C₃-C₂₀, OR^{4a}, SR^{9a} ou NR^{10a}R^{11a} ; ou par un ou plusieurs groupes alkyle en C₂-C₂₀ qui est interrompu par un ou plusieurs groupes O, S ou NR^{10a} ; ou par un ou plusieurs groupes phényle, naphtyle, benzoyle ou naphtoyle, dont chacun est non substitué ou substitué par OR^{4a}, SR^{9a} ou NR^{10a}R^{11a} ; ou
R¹⁰ et R¹¹, indépendamment l'un de l'autre, sont alcoxy en C₁-C₂₀, qui est non substitué ou substitué par un ou plusieurs groupes halogène, phényle, (alkyle en C₁-C₈)phényle ou (alcoxy en C₁-C₈) phényle ; ou
R¹⁰ et R¹¹, indépendamment l'un de l'autre, sont alcoxy en C₁-C₂₀, qui est interrompu par un ou plusieurs groupes O, S, NR^{10a}, CO, SO ou SO₂ ; ou
R¹⁰ et R¹¹, indépendamment l'un de l'autre, sont aryloxy en C₆-C₂₀ ou hétéroaryloxy en C₃-C₂₀, dont chacun est non substitué ou substitué par un ou plusieurs groupes halogène, alkyle en C₁-C₈, halogénoalkyle en C₁-C₄, phényle, (alkyle en C₁-C₈) phényle, (alcoxy en C₁-C₈) phényle, CN, NO₂, OR^{4a}, SR^{9a}, NR^{10a}R^{11a}, COOR^{4a}, (CO) -R^{3a} ou SO₂-R^{3a} ; ou
R¹⁰, conjointement avec l'un des atomes de carbone de R¹, forme un cycle saturé ou insaturé de 5 ou 6 chaînons qui est ininterrompu ou interrompu par O, S ou NR^{10a}, et ledit cycle saturé ou insaturé de 5 ou 6 chaînons est non substitué ou substitué par un ou plusieurs groupes alkyle en C₁-C₂₀, OR^{4a}, SR^{9a}, NR^{10a}R^{11a}, (CO) -R^{3a}, NO₂, halogène, halogénoalkyle en C₁-C₄, CN, phényle, (alkyle en C₁-C₂₀) phényle, (alcoxy en C₁-C₈) phényle, 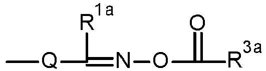 ou cycloalkyle en C₃-C₂₀ qui est ininterrompu ou interrompu par un ou plusieurs groupes O, S, CO ou NR^{10a} ; ou
R¹⁰ et R¹¹, conjointement avec l'atome N auquel ils sont liés, forment un cycle saturé ou insaturé de 5 ou 6 chaînons qui est ininterrompu ou interrompu par O, S ou NR^{10a}, et ledit cycle saturé ou insaturé de 5 ou 6 chaînons est non substitué ou substitué par un ou plusieurs groupes alkyle en C₁-C₂₀, OR^{4a}, SR^{9a}, NR^{10a}R^{11a}, (CO) -R^{3a}, NO₂, halogène, halogénoalkyle en C₁-C₄, CN, phényle, 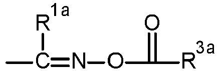 ou cycloalkyle en C₃-C₂₀ qui est ininterrompu ou interrompu par un ou plusieurs groupes O, S, CO ou NR^{10a} ;
R^{10a} et R^{11a}, indépendamment l'un de l'autre, sont hydrogène, alkyle en C₁-C₂₀, S(O)ₘ-(alkyle en C₁-C₈), O(CO) (alkyle en C₁-C₈), (CO)(alkyle en C₁-C₈), (CO)O(alkyle en C₁-C₈) ou CON (alkyle en C₁-C₈)₂ ; ou
R^{10a} et R^{11a}, indépendamment l'un de l'autre, sont alkyle en C₁-C₂₀ substitué par un ou plusieurs groupes halogène, OH, SH, CN, alcénoxy en C₃-C₈, OCH₂CH₂CN, OCH₂CH₂(CO)O(alkyle en C₁-C₈), O(CO)-(alkyle en C₁-C₈), O(CO) - (C₂-C₄) alcényle, O(CO)-phényle, (CO)OH, (CO)O(alkyle en C₁-C₈), cycloalkyle en C₃-C₈, SO₂- (C₁-C₄haloalkyl), O(halogénoalkyle en C₁-C₄), phényle, (alkyle en C₁-C₈)phényle, (alcoxy en C₁-C₈)phényle ou cycloalkyle en C₃-C₈ qui est interrompu par un ou plusieurs groupes O ; ou
R^{10a} et R^{11a}, indépendamment l'un de l'autre, sont alkyle en C₂-C₂₀ interrompu par un ou plusieurs groupes O, S, N(alkyle en C₁-C₈), CO, SO ou SO₂, qui est non substitué ou substitué par cycloalkyle en C₃-C₈, OH, SH, O(CO) (alkyle en C₁-C₈), (CO) O (alkyle en C₁C₈), (CO) N (alkyle en C₁C₈)₂, aryle en C₆-C₂₀, hétéroaryle en C₃-C₂₀, aroyle en C₆-C₂₀ ou (hétéroaryle en C₃-C₂₀)carbonyle, dans lesquels aryle en C₆-C₂₀, hétéroaryle en C₃-C₂₀, aroyle en C₆-C₂₀ ou (hétéroaryle en C₃-C₂₀)carbonyle est non substitué ou substitué par un ou plusieurs groupes halogène, alkyle en C₁-C₈, alcoxy en C₁-C₈, (alkyle en C₁-C₈)sulfanyle ou N(alkyle en C₁-C₈)₂ ; ou
R^{10a} et R^{11a}, indépendamment l'un de l'autre, sont alcényle en C₂-C₁₂ ou cycloalkyle en C₃-C₈, dont chacun est ininterrompu ou interrompu par un ou plusieurs groupes O, S, CO, N(alkyle en C₁-C₈) ou COO(alkyle en C₁-C₈) ; ou
R^{10a} et R^{11a}, indépendamment l'un de l'autre, sont aryle en C₆-C₂₀, hétéroaryle en C₃-C₂₀, aroyle en C₆-C₂₀ ou (hétéroaryle en C₃-C₂₀)carbonyle, dont chacun est non substitué ou substitué par un ou plusieurs groupes halogène, CN, NO₂, OH, alkyle en C₁-C₈, halogénoalkyle en C₁-C₄, alcoxy en C₁-C₈, phényl- (alkyloxy en C₁-C₃), phénoxy, (alkyle en C₁-C₈)sulfanyle, phénylsulfanyle, N(alkyle en C₁-C₈)₂, diphénylamino, (CO) O (alkyle en C₁-C₈), (CO)-(alkyle en C₁-C₈) ou (CO) N (alkyle en C₁-C₈)₂, phényle ou benzoyle ; ou
R^{10a} et R^{11a}, indépendamment l'un de l'autre, sont alcanoyle en C₁-C₂₀, alcénoyle en C₃-C₁₂, dont chacun est non substitué ou substitué par un ou plusieurs groupes halogène, phényle, (alkyle en C₁-C₈)phényle, (alcoxy en C₁-C₈) phényle, OH, alcoxy en C₁-C₈, phénoxy, (alkyle en C₁-C₈) sulfanyle, phénylsulfanyle, N(alkyle en C₁-C₈)₂ ou diphénylamino ; ou
R^{10a} et R^{11a}, indépendamment l'un de l'autre, sont alcoxy en C₁-C₂₀, qui est non substitué ou substitué par un ou plusieurs groupes halogène, phényle, (alkyle en C₁-C₈) phényle ou (alcoxy en C₁-C₈)phényle ; ou
R^{10a} et R^{11a}, indépendamment l'un de l'autre, sont alcoxy en C₁-C₂₀, qui est interrompu par un ou plusieurs groupes O, S, N(alkyle en C₁-C₈), CO, SO ou SO₂ ; ou
R^{10a} et R^{11a}, indépendamment l'un de l'autre, sont aryloxy en C₆-C₂₀ ou hétéroaryloxy en C₃-C₂₀, dont chacun est non substitué ou substitué par un ou plusieurs groupes halogène, alkyle en C₁-C₈, halogénoalkyle en C₁-C₄, phényle, (alkyle en C₁-C₈)phényle, (alcoxy en C₁-C₈) phényle, CN, NO₂, alcoxy en C₁-C₈, (alkyle en C₁-C₈) sulfanyle, N (alkyle en C₁-C₈)₂, CO(O)(alkyle en C₁-C₈), (CO) - (alkyle en C₁-C₈) ou SO₂- (alkyle en C₁-C₈) ; ou
R^{10a} et R^{11a}, conjointement avec l'atome N auquel ils sont liés, forment un cycle saturé ou insaturé de 5 ou 6 chaînons qui est ininterrompu ou interrompu par O, S ou N(alkyle en C₁-C₈), et ledit cycle saturé ou insaturé de 5 ou 6 chaînons est non substitué ou substitué par un ou plusieurs groupes alkyle en C₁-C₈, alcoxy en C₁-C₈, (alkyle en C₁-C₈) sulfanyle, N (alkyle en C₁-C₈)₂, NO₂, halogène, halogénoalkyle en C₁-C₄, CN, phényle ou cycloalkyle en C₃-C₂₀ qui est ininterrompu ou interrompu par un ou plusieurs groupes O, S, CO ou N(alkyle en C₁-C₈) ;
R¹² et R¹³, indépendamment l'un de l'autre, sont hydrogène, alkyle en C₁-C₁₂ éventuellement substitué par un ou plusieurs groupes halogène, phényle, CN, -OH, -SH, alcoxy en C₁-C₄, (CO) OH ou (CO)O(alkyle en C₁-C₄) ; ou
R¹² et R¹³ sont phényle éventuellement substitué par un ou plusieurs groupes alkyle en C₁-C₆, halogène, CN, OR⁴, SR⁹ ou NR¹⁰R¹¹ ; ou
R¹² et R¹³ sont halogène, CN, OR⁴, SR⁹, SOR⁹, SO₂R⁹ ou NR¹⁰R¹¹, dans lesquels les substituants OR⁴, SR⁹ ou NR¹⁰R¹¹ forment éventuellement des cycles de 5 ou 6 chaînons par l'intermédiaire des radicaux R⁴, R⁹, R¹⁰ et/ou R¹¹ avec l'un des atomes de carbone du groupe phényle, naphtyle, benzoyle ou naphtoyle ou celui du substituant R^{3a} ; ou
R¹² et R¹³ sont conjointement un groupe 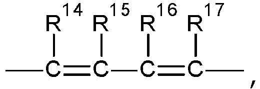 dans lequel R¹⁴, R¹⁵, R¹⁶ et R¹⁷, indépendamment les uns des autres sont hydrogène, alkyle en C₁-C₁₂ éventuellement substitué par un ou plusieurs groupes halogène, phényle, CN, -OH, -SH, alcoxy en C₁-C₄, (CO)OH ou (CO)O(alkyle en C₁-C₄) ; ou R¹⁴, R¹⁵, R¹⁶ et R¹⁷ sont phényle éventuellement substitué par un ou plusieurs groupes alkyle en C₁-C₈, halogène, CN, OR⁴, SR⁹ ou NR¹⁰R¹¹ ; ou R¹⁴, R¹⁵, R¹⁶ et R¹⁷ sont halogène, CN, OR⁴, SR⁹ ou NR¹⁰R¹¹ ; ou
R¹² et R¹³ sont conjointement un groupe 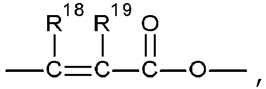 dans lequel R¹⁸ et R¹⁹, indépendamment l'un de l'autre, sont hydrogène, alkyle en C₁-C₁₂ éventuellement substitué par un ou plusieurs groupes halogène, phényle, CN, -OH, -SH, alcoxy en C₁-C₄, (CO)OH ou (CO)O(alkyle en C₁-C₄) ; ou R¹⁸ et R¹⁹ sont phényle éventuellement substitué par un ou plusieurs groupes alkyle en C₁-C₆, halogène, CN, OR⁴, SR⁹ ou NR¹⁰R¹¹ ;
M est une liaison directe ou un groupe de liaison divalent alkylène en C₁-C₂₀ ou alkylène en C₁-C₂₀ substitué par un ou plusieurs groupes halogène, OR⁴, SR⁹, NR¹⁰R¹¹, CN, COOR⁴, CONR¹⁰R¹¹, cycloalkyle en C₃-C₈ qui est ininterrompu ou interrompu par un ou plusieurs groupes O, S, CO ou NR¹⁰ ; ou par un ou plusieurs groupes aryle en C₆-C₂₀, hétéroaryle en C₃-C₂₀, aroyle en C₆-C₂₀ ou (hétéroaryle en C₃-C₂₀) carbonyle, dont chacun est non substitué ou substitué par un ou plusieurs groupes halogène, phényle, (alkyle en C₁-C₂₀)phényle, (alcoxy en C₁-C₈)phényle, halogénoalkyle en C₁-C₄, CN, NO₂, OR⁴, SR⁹ ou NR¹⁰R¹¹ ; ou
M est alkylène en C₁-C₂₀ interrompu par un ou plusieurs groupes O, S, NR¹⁰, CO, SO ou SO₂, qui est non substitué ou substitué par cycloalkyle en C₃-C₈, OH, SH, O(CO)-R^{3a}, COOR⁴, CONR¹⁰R¹¹, aryle en C₆-C₂₀, hétéroaryle en C₃-C₂₀, aroyle en C₆-C₂₀ ou (hétéroaryle en C₃-C₂₀)carbonyle, dans lequel aryle en C₆-C₂₀, hétéroaryle en C₃-C₂₀, aroyle en C₆-C₂₀ ou (hétéroaryle en C₃-C₂₀) carbonyle est non substitué ou substitué par un ou plusieurs groupes halogène, alkyle en C₁-C₈, OR⁴, SR⁹ ou NR¹⁰R¹¹ ; ou
M est alcénylène en C₂-C₁₂ ou cycloalkylène en C₃-C₂₀, dont chacun est ininterrompu ou interrompu par un ou plusieurs groupes O, S, CO, NR¹⁰ ou COOR⁴ ; ou
M est arylène en C₆-C₂₀ ou hétéroarylène en C₃-C₂₀, dont chacun est non substitué ou substitué par un ou plusieurs groupes halogène, alkyle en C₁-C₂₀, halogénoalkyle en C₁-C₄, phényle, (alkyle en C₁-C₂₀) phényle, (alcoxy en C₁-C₈)phényle, CN, NO₂, OR⁴, SR⁹, NR¹⁰R¹¹, COOR⁴, (CO)-R^{3a} ou SO₂-R^{3a} ;
M' est une liaison directe ou un groupe de liaison divalent choisi parmi alkylène en C₁-C₂₀ ou alkylène en C₁-C₂₀ substitué par un ou plusieurs groupes halogène, OR⁴, SR⁹, NR¹⁰R¹¹, CN, COOR⁴, CONR¹⁰R¹¹, cycloalkyle en C₃-C₈ qui est ininterrompu ou interrompu par un ou plusieurs groupes O, S, CO ou NR¹⁰ ; ou par un ou plusieurs groupes aryle en C₆-C₂₀, hétéroaryle en C₃-C₂₀, aroyle en C₆-C₂₀ ou (hétéroaryle en C₃-C₂₀) carbonyle, dont chacun est non substitué ou substitué par un ou plusieurs groupes halogène, phényle, (alkyle en C₁-C₂₀)phényle, (alcoxy en C₁-C₈) phényle, halogénoalkyle en C₁-C₄, CN, NO₂, OR⁴, SR⁹ ou NR¹⁰R¹¹ ; ou
M' est alkylène en C₁-C₂₀ interrompu par un ou plusieurs groupes O, S, NR¹⁰, CO, SO ou SO₂, qui est non substitué ou substitué par cycloalkyle en C₃-C₈, OH, SH, O(CO)-R^{3a}, COOR⁴, CONR¹⁰R¹¹, aryle en C₆-C₂₀, hétéroaryle en C₃-C₂₀, aroyle en C₆-C₂₀ ou (hétéroaryle en C₃-C₂₀) carbonyle, dans lequel aryle en C₆-C₂₀, hétéroaryle en C₃-C₂₀, aroyle en C₆-C₂₀ ou (hétéroaryle en C₃-C₂₀) carbonyle est non substitué ou substitué par un ou plusieurs groupes halogène, alkyle en C₁-C₈, OR⁴, SR⁹ ou NR¹⁰R¹¹ ; ou
M' est alcénylène en C₂-C₁₂ ou cycloalkylène en C₃-C₂₀, dont chacun est ininterrompu ou interrompu par un ou plusieurs groupes O, S, CO, NR¹⁰ ou COOR⁴ ; ou
M' est arylène en C₆-C₂₀ ou hétéroarylène en C₃-C₂₀, dont chacun est non substitué ou substitué par un ou plusieurs groupes halogène, alkyle en C₁-C₂₀, halogénoalkyle en C₁-C₄, phényle, (alkyle en C₁-C₂₀) phényle, (alcoxy en C₁-C₈) phényle, CN, NO₂, OR⁴, SR⁹, NR¹⁰R¹¹, COOR⁴, (CO)-R^{3a} ou SO₂-R^{3a} ;
M" est une liaison directe ou un groupe de liaison divalent choisi parmi alkylène en C₁-C₂₀ ou alkylène en C₁-C₂₀ substitué par un ou plusieurs groupes halogène, OR⁴, SR⁹, NR¹⁰R¹¹, CN, COOR⁴, CONR¹⁰R³¹, cycloalkyle en C₃-C₈ qui est ininterrompu ou interrompu par un ou plusieurs groupes O, S, CO ou NR¹⁰ ; ou par un ou plusieurs groupes aryle en C₆-C₂₀, hétéroaryle en C₃-C₂₀, aroyle en C₆-C₂₀ ou (hétéroaryle en C₃-C₂₀) carbonyle, dont chacun est non substitué ou substitué par un ou plusieurs groupes halogène, phényle, (alkyle en C₁-C₂₀) phényle, (alcoxy en C₁-C₈) phényle, halogénoalkyle en C₁-C₄, CN, NO₂, OR⁴, SR⁹ ou NR¹⁰R¹¹ ; ou
M" est alkylène en C₁-C₂₀ interrompu par un ou plusieurs groupes O, S, NR¹⁰, CO, SO ou SO₂, qui est non substitué ou substitué par cycloalkyle en C₃-C₈, OH, SH, O(CO)-R^{3a}, COOR⁴, CONR¹⁰R¹¹, aryle en C₆-C₂₀, hétéroaryle en C₃-C₂₀, aroyle en C₆-C₂₀ ou (hétéroaryle en C₃-C₂₀) carbonyle, dans lequel aryle en C₆-C₂₀, hétéroaryle en C₃-C₂₀, aroyle en C₆-C₂₀ ou (hétéroaryle en C₃-C₂₀) carbonyle est non substitué ou substitué par un ou plusieurs groupes halogène, alkyle en C₁-C₈, OR⁴, SR⁹ ou NR¹⁰R¹¹ ; ou
M" est alcénylène en C₂-C₁₂ ou cycloalkylène en C₃-C₂₀, dont chacun est ininterrompu ou interrompu par un ou plusieurs groupes O, S, CO, NR¹⁰ ou COOR⁴ ; ou
M" est arylène en C₆-C₂₀ ou hétéroarylène en C₃-C₂₀, dont chacun est non substitué ou substitué par un ou plusieurs groupes halogène, alkyle en C₁-C₂₀, halogénoalkyle en C₁-C₄, phényle, (alkyle en C₁-C₂₀) phényle, (alcoxy en C₁-C₈) phényle, CN, NO₂, OR⁴, SR⁹, NR¹⁰R¹¹, COOR⁴, (CO)-R^{3a} ou SO₂-R^{3a} ;
m est 1 ou 2 ;
Q est CO ou une liaison directe ; et
x, y et z, indépendamment les uns des autres, sont un entier 1, 2, 3 ou 4.

2. Composés des formules I, II, III et IV selon la revendication 1, les composés étant des composés de formule Ia, IIa, IIIa ou IVa : 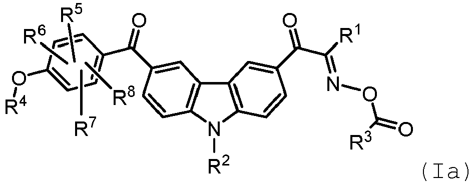 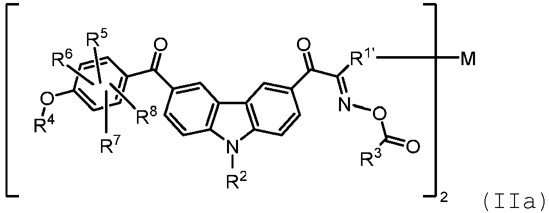 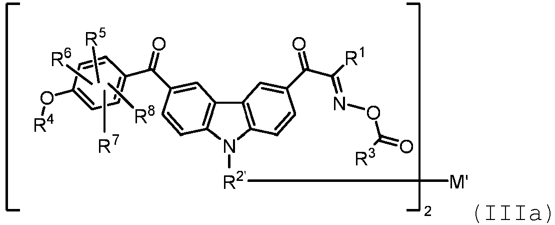 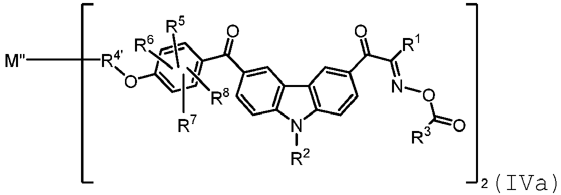 dans lesquels
R¹ à R⁸, R¹', R²', R⁴', M, M' et M" sont tels que définis dans la revendication 1.

3. Composés des formules I, II, III et IV selon la revendication 1 ou 2, les composés étant des composés de formule Ib, IIb, IIIb ou IVb : 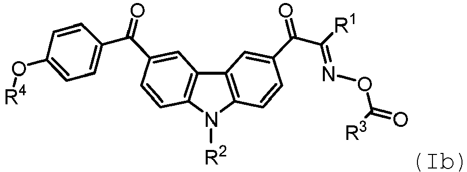 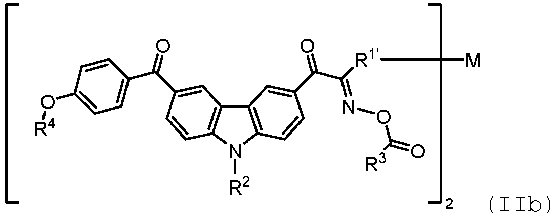 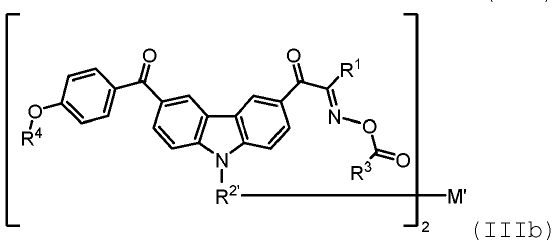 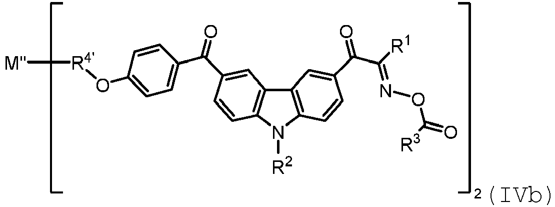 dans lesquels
R¹ est hydrogène, alkyle en C₁-C₂₀, (alkyle en C₁-C₆) - (cycloalkyle en C₃-C₆), cycloalkyle en C₃-C₂₀, alcényle en C₂-C₁₂, dans lequel cycloalkyle en C₃-C₂₀ ou alcényle en C₂-C₁₂ est ininterrompu ou interrompu par un ou plusieurs groupes O, S, CO, NR¹⁰ ou COOR⁴ ; ou
R¹ est alkyle en C₂-C₂₀ qui est interrompu par un ou plusieurs groupes O, CO, S, C(O)O, OC(O), SO, SO₂, phénylène, naphtylène ou NR¹⁰, dans lequel l'alkyle en C₂-C₂₀ interrompu est non substitué ou substitué par un ou plusieurs groupes halogène, cycloalkyle en C₃-C₈, OH, SH, OR⁴, SR⁹, COOR⁴, O(CO)-R^{3a}, CONR¹⁰R¹¹, NR¹⁰R¹¹, aryle en C₆-C₂₀, hétéroaryle en C₃-C₂₀, aroyle en C₆-C₂₀ ou (hétéroaryle en C₃-C₂₀) carbonyle, dans lequel aryle en C₆-C₂₀, hétéroaryle en C₃-C₂₀, aroyle en C₆-C₂₀ ou (hétéroaryle en C₃-C₂₀)carbonyle est non substitué ou substitué par un ou plusieurs groupes halogène, alkyle en C₁-C₈, OR⁴, SR⁹ ou NR¹⁰R¹¹ ; ou
R¹ est aryle en C₆-C₂₀ ou hétéroaryle en C₃-C₂₀ dont chacun est non substitué ou substitué par un ou plusieurs groupes alkyle en C₁-C₂₀ ;
R¹' est alkylène en C₁-C₂₀, alkylène en C₁-C₂₀ qui est interrompu par un ou plusieurs groupes O, S, (CO)O, O(CO), phénylène, naphtylène ou NR⁸, dans lequel l'alkylène en C₁-C₂₀ et l'alkylène en C₁-C₂₀ interrompu sont non substitués ou substitués par halogène ou OR⁴, ou
R² est hydrogène, alkyle en C₁-C₂₀ qui est non substitué ou substitué par un ou plusieurs groupes halogène, OR⁴, SR⁹, COOR⁴ CONR¹⁰R¹¹, NR¹⁰R¹¹, PO(OR^{3a})₂ ; (CO)-R^{3a}, ou
R² est alkyle en C₂-C₂₀ ou (alkyle en C₁-C₆)-(cycloalkyle en C₃-C₆) qui est interrompu par un ou plusieurs groupes O, CO, S, C(O)O, OC(O), SO, SO₂, phénylène, naphtylène ou NR¹⁰ ; ou
R² est aryle en C₆-C₂₀ ou hétéroaryle en C₃-C₂₀ dont chacun est non substitué ou substitué par un ou plusieurs groupes alkyle en C₁-C₁₂, (CO)-R^{3a} ;
R²' est alkylène en C₁-C₂₀, alkylène en C₁-C₂₀ qui est interrompu par un ou plusieurs groupes O, S, (CO)O, O(CO), phénylène, naphtylène ou NR⁸, dans lequel l'alkylène en C₁-C₂₀ et l'alkylène en C₁-C₂₀ interrompu sont non substitués ou substitués par halogène ou OR⁴, R³ est hydrogène ou alkyle en C₁-C₂₀ ; ou
R³ est alkyle en C₁-C₂₀ substitué par un ou plusieurs groupes halogène, OR⁴, SR⁹, NR¹⁰R¹¹, CN, COOR⁴, CONR¹⁰R¹¹, cycloalkyle en C₃-C₈ qui est ininterrompu ou interrompu par un ou plusieurs groupes O, S, CO ou NR¹⁰ ; ou par un ou plusieurs groupes aryle en C₆-C₂₀, hétéroaryle en C₃-C₂₀, aroyle en C₆-C₂₀ ou (hétéroaryle en C3-C20) carbonyle, dont chacun est non substitué ou substitué par un ou plusieurs groupes halogène, phényle, (alkyle en C₁-C₂₀)phényle, (alcoxy en C₁-C₈) phényle, halogénoalkyle en C₁-C₄, CN, NO₂, OR⁴, SR⁹ ou NR¹⁰R¹¹ ; ou
R^{3a} est hydrogène ou alkyle en C₁-C₂₀ ; ou
R^{3a} est alkyle en C₁-C₂₀ interrompu par un ou plusieurs groupes O, S, NR^{10a}, CO, SO ou SO₂, qui est non substitué ou substitué par cycloalkyle en C₃-C₈, OH, SH, O(CO)-(alkyle en C₁-C₈), COOR^{4a}, CONR^{10a}R^{11a}, aryle en C₆-C₂₀, hétéroaryle en C₃-C₂₀, aroyle en C₆-C₂₀ ou (hétéroaryle en C₃-C₂₀)carbonyle, dans lequel aryle en C₆-C₂₀, hétéroaryle en C₃-C₂₀, aroyle en C₆-C₂₀ ou (hétéroaryle en C₃-C₂₀)carbonyle est non substitué ou substitué par un ou plusieurs groupes halogène, alkyle en C₁-C₈, OR^{4a}, SR^{9a} ou NR^{10a}R^{11a} ; ou
R^{3a} est aryle en C₆-C₂₀ ou hétéroaryle en C₃-C₂₀, dont chacun est non substitué ou substitué par un ou plusieurs groupes halogène, alkyle en C₁-C₂₀, halogénoalkyle en C₁-C₄, phényle, (alkyle en C₁-C₂₀)phényle, (alcoxy en C₁-C₈)phényle, CN, NO₂, OR^{4a}, SR^{9a}, NR^{10a}R^{11a}, COOR^{4a}, (CO)-(alkyle en C₁-C₈) ou SO₂- (halogénoalkyle en C₁-C₄) ;
R⁴ est hydrogène, (CO)-R^{3a}, COOR^{4a}, CONR¹⁰R¹¹, S(O)ₘ-R^{3a} ou PO(OR^{3a})₂ ;
R⁴' est -(CO)-, -(CO)O-, -(CO)N-, -S(O)ₘ-, -PO(O)₂-, ou - OP(O)₂ ;
R^{4a} est hydrogène, alkyle en C₁-C₂₀, (CO)O(alkyle en C₁-C₈) ou CON (alkyle en C₁-C₈)₂ ; ou
R^{4a} est alkyle en C₂-C₂₀ interrompu par un ou plusieurs groupes O, S, N(alkyle en C₁-C₈), CO, SO ou SO₂, qui est non substitué ou substitué par cycloalkyle en C₃-C₈, OH, SH, O(CO)(alkyle en C₁-C₈), (CO)O (alkyle en C₁-C₈), (CO)N(alkyle en C₁-C₈)₂, aryle en C₆-C₂₀, hétéroaryle en C₃-C₂₀, aroyle en C₆-C₂₀ ou (hétéroaryle en C₃-C₂₀)carbonyle, dans lequel aryle en C₆-C₂₀, hétéroaryle en C₃-C₂₀, aroyle en C₆-C₂₀ ou (hétéroaryle en C₃-C₂₀)carbonyle est non substitué ou substitué par un ou plusieurs groupes halogène, alkyle en C₁-C₈, alcoxy en C1-C8, (alkyle en C₁-C₈)sulfanyle ou N(alkyle en C₁-C₈)₂ ; ou
R^{4a} est aryle en C₆-C₂₀, hétéroaryle en C₃-C₂₀, aroyle en C₆-C₂₀ ou (hétéroaryle en C₃-C₂₀)carbonyle, dont chacun est non substitué ou substitué par un ou plusieurs groupes halogène, CN, NO₂, OH, alkyle en C₁-C₈, halogénoalkyle en C₁-C₄, alcoxy en C₁-C₈, phényl- (alkyloxy en C₁-C₃), phénoxy, (alkyle en C₁-C₈₎sulfanyle, phénylsulfanyle, N(alkyle en C₁-C₈)₂, diphénylamino, (CO)O(alkyle en C₁-C₈), (CO)-(alkyle en C₁-C₈) ou (CO)N(C₁-C₈)₂, phényle ou benzoyle ;
R⁸ est hydrogène ; alkyle en C₁-C₂₀ ; ou aryle en C₆-C₂₀ ;
R⁹ est hydrogène ou alkyle en C₁-C₂₀ ;
R^{9a} est hydrogène ou alkyle en C₁-C₂₀ ;
R¹⁰ et R¹¹, indépendamment l'un de l'autre, sont hydrogène, alkyle en C₁-C₂₀, S(O)ₘ-R^{3a}, O(CO)-R^{3a}, (CO)-R^{3a} ou CONR^{10a}R^{11a} ;
R^{10a} et R^{11a}, indépendamment l'un de l'autre, sont hydrogène, alkyle en C₁-C₂₀, S(O)ₘ-(alkyle en C₁-C₈), O(CO)(alkyle en C₁-C₈), (CO)(alkyle en C₁-C₈), (CO)O(alkyle en C₁-C₈) ou CON (alkyle en C₁-C₈)₂ ;
M est une liaison directe ou un groupe de liaison divalent alkylène en C₁-C₂₀ ou alkylène en C₁-C₂₀ substitué par un ou plusieurs groupes halogène, OR⁴, SR⁹, NR¹⁰R¹¹, CN, COOR⁴, CONR¹⁰R¹¹, cycloalkyle en C₈-C₈ qui est ininterrompu ou interrompu par un ou plusieurs groupes O, S, CO ou NR¹⁰ ; ou par un ou plusieurs groupes aryle en C₆-C₂₀, hétéroaryle en C₃-C₂₀, aroyle en C₆-C₂₀ ou (hétéroaryle en C₃-C₂₀)carbonyle, dont chacun est non substitué ou substitué par un ou plusieurs groupes halogène, phényle, (alkyle en C₃-C₂₀) phényle, (alcoxy en C₁-C₈) phényle, halogénoalkyle en C₁-C₄, CN, NO₂, OR⁴, SR⁹ ou NR¹⁰R¹¹ ;
M est une liaison directe ou un groupe de liaison divalent choisi parmi alkylène en C₃-C₂₀ ou alkylène en C₃-C₂₀ substitué par un ou plusieurs groupes halogène, OR⁴, SR⁹, NR¹⁰R¹¹, CN, COOR⁴, CONR¹⁰R¹¹, cycloalkyle en C₃-C₈ qui est ininterrompu ou interrompu par un ou plusieurs groupes O, S, CO ou NR¹⁰ ; ou par un ou plusieurs groupes aryle en C₆-C₂₀, hétéroaryle en C₃-C₂₀, aroyle en C₆-C₂₀ ou (hétéroaryle en C₃-C₂₀)carbonyle, dont chacun est non substitué ou substitué par un ou plusieurs groupes halogène, phényle, (alkyle en C₁-C₂₀)phényle, (alcoxy en C₁-C₈)phényle, halogénoalkyle en C₁-C₄, CN, NO₂, OR⁴, SR⁹ ou NR¹⁰R¹¹ ;
M" est une liaison directe ou un groupe de liaison divalent choisi parmi alkylène en C₁-C₂₀ ou alkylène en C₁-C₂₀ substitué par un ou plusieurs groupes halogène, OR⁴, SR⁹, NR¹⁰R¹¹, CN, COOR⁴, CONR¹⁰R¹¹, cycloalkyle en C₃-C₈ qui est ininterrompu ou interrompu par un ou plusieurs groupes O, S, CO ou NR¹⁰ ; ou par un ou plusieurs groupes aryle en C₆-C₂₀, hétéroaryle en C₃-C₂₀, aroyle en C₆-C₂₀ ou (hétéroaryle en C₃-C₂₀) carbonyle, dont chacun est non substitué ou substitué par un ou plusieurs groupes halogène, phényle, (alkyle en C₁-C₂₀)phényle, (alcoxy en C₁-C₈)phényle, halogénoalkyle en C₁-C₄, CN, NO₂, OR⁴, SR⁹ ou NR¹⁰R¹¹ ; et
m est 1 ou 2.

4. Composés des formules I, II, III et IV selon la revendication 3, les composés étant des composés de formule Ib, dans lesquels
R¹ est alkyle en C₁-C₂₀, (alkyle en C₁-C₈)-(cycloalkyle en C₃-C₆), cycloalkyle en C₃-C₂₀ ; ou
R¹ est alkyle en C₂-C₂₀ qui est interrompu par un ou plusieurs groupes O, CO, S, C(O)O, OC(O), SO, ou SO₂ ; ou
R¹ est aryle en C₆-C₂₀ qui est non substitué ou substitué par un ou plusieurs groupes alkyle en C₁-C₂₀ ;
R² est alkyle en C₁-C₂₀ ; ou
R² est alkyle en C₂-C₂₀ ou (alkyle en C₁-C₆)-(cycloalkyle en C₃-C₆) qui est interrompu par un ou plusieurs groupes O, CO, S, C(O)O, OC(O), SO ou SO₂ ; ou
R² est aryle en C₆-C₂₀ qui est non substitué ou substitué par un ou plusieurs groupes alkyle en C₁-C₁₂ ou (CO)-R^{3a} ;
R³ est alkyle en C₁-C₂₀ ;
R^{3a} est alkyle en C₁-C₂₀, aryle en C₆-C₂₀ ou hétéroaryle en C₃-C₂₀ ;
R⁴ est (CO)-R^{3a}, COOR^{4a}, CONR¹⁰R¹¹, S(O)ₘ-R^{3a} ou PO(OR^{3a})₂ ;
R^{4a} est alkyle en C₁-C₂₀, aryle en C₆-C₂₀ ou hétéroaryle en C₃-C₂₀ ;
R¹⁰ et R¹¹ sont alkyle en C₁-C₂₀ ; et
m est 1 ou 2.

5. Composés des formules I, II, III et IV selon l'une quelconque des revendications 1 à 4, dans lesquels
(i) plus de 50 %, de préférence 55 % ou plus, plus préférablement 75 % ou plus, encore plus préférablement 90 % ou plus, tel que 95 % ou plus, des doubles liaisons C=N présentes dans les groupes ester d'oxime dans la molécule sont dans la configuration Z ; ou
(ii) plus de 50 %, de préférence 55 % ou plus, plus préférablement 75 % ou plus, encore plus préférablement 90 % ou plus, tel que 95 % ou plus, des doubles liaisons C=N présentes dans les groupes ester d'oxime dans la molécule sont dans la configuration E.

6. Composition photopolymérisable comprenant
(a) au moins un composé photopolymérisable éthyléniquement insaturé et
(b) en tant que photoamorceur, au moins un composé de formule I, II, III ou IV tel que défini dans l'une quelconque des revendications 1 to 5.

7. Composition photopolymérisable selon la revendication 6, dans laquelle le composant (a) est une résine obtenue par la réaction d'un anhydride d'acide polybasique saturé ou insaturé avec un produit de la réaction d'une résine époxy et d'un acide monocarboxylique insaturé ; et/ou
dans laquelle, en plus du photoamorceur (b), composition photopolymérisable comprend au moins un photoamorceur supplémentaire (c), et/ou d'autres additifs (d).

8. Composition photopolymérisable selon la revendication 6, comprenant, en tant qu'additif supplémentaire (d) un pigment, ou un mélange de pigments, ou un colorant, ou un mélange de colorants, ou un mélange d'un ou plusieurs pigments avec un ou plusieurs colorants ; de préférence, comprenant, en tant qu'additif supplémentaire (d) un dispersant ou un mélange de dispersants.

9. Composition photopolymérisable selon l'une quelconque des revendications 6 à 8, comprenant 0,05 à 25 % en poids du photoamorceur (b), ou des photoamorceurs (b) et (c), par rapport au poids de la composition ; et/ou
comprenant, en tant qu'additif supplémentaire (d) un photosensibilisateur, en particulier un composé choisi dans le groupe constitué par la benzophénone, des dérivés de benzophénone, la thioxanthone, des dérivés de thioxanthone, l'anthraquinone, des dérivés d'anthraquinone, la coumarine et des dérivés de coumarine ; et/ou
comprenant en outre un polymère liant (e), en particulier un copolymère de méthacrylate et d'acide méthacrylique.

10. Procédé de préparation d'un composé de formule I, II, III ou IV selon la revendication 1 par réaction du composé oxime correspondant avec un halogénure d'acyle de formule I' ou un anhydride de formule I" 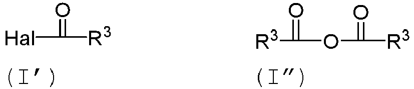 dans lequel Hal est un halogène, en particulier Cl, et R³ est tel que défini dans la revendication 1, en présence d'une base ou d'un mélange de bases.

11. Procédé de photopolymérisation de composés contenant des doubles liaisons éthyléniquement insaturées, qui comprend l'irradiation d'une composition selon l'une quelconque des revendications 6 à 9 avec un rayonnement électromagnétique dans la plage de 150 à 600 nm, ou avec un faisceau d'électrons ou avec des rayons X.

12. Procédé selon la revendication 11 pour la production de peintures et de vernis pigmentés et non pigmentés, revêtements en poudre, encres d'impression, plaques d'impression, adhésifs, adhésifs autocollants, compositions dentaires, enduits gélifiés, résines photosensibles pour l'électronique, réserves de galvanoplastie, réserves de gravure, films liquides et secs, réserves de soudure, résines pour la fabrication de filtres de couleur pour diverses applications d'affichage, résines pour la génération de structures dans les processus de fabrication de panneaux d'affichage à plasma, écrans électroluminescents et LCD, espaceurs pour LCD, pour le stockage de données holographiques (HDS), comme composition pour l'encapsulation de composants électriques et électroniques, pour la production de matériaux d'enregistrement magnétique, pièces micromécaniques, guides d'ondes, commutateurs optiques, masques de placage, masques de gravure, systèmes d'épreuves couleur, revêtements de câbles en fibre de verre, pochoirs de sérigraphie, pour la production d'objets tridimensionnels au moyen d'une stéréolithographie, comme matériau d'enregistrement d'image, pour des enregistrements holographiques, circuits microélectroniques, matériaux de décoloration, matériaux de décoloration pour matériaux d'enregistrement d'image, pour des matériaux d'enregistrement d'image utilisant des microcapsules, comme matériau de résine photosensible pour un système d'imagerie directe laser UV et visible, comme matériau de résine photosensible utilisé pour former des couches diélectriques dans une couche de construction séquentielle d'une carte de circuit imprimé.

13. Substrat revêtu qui est revêtu sur au moins une surface avec une composition selon l'une quelconque des revendications 6 à 9.

14. Procédé de production photographique d'images en relief, dans lequel un substrat revêtu selon la revendication 13 est soumis à une exposition selon une image, puis les parties non exposées sont éliminées avec un révélateur.

15. Filtre coloré préparé par fourniture d'éléments d'image rouges, verts et bleus et d'une matrice noire, tous comprenant une résine photosensible et un pigment sur un substrat transparent et fourniture d'une électrode transparente soit sur la surface du substrat, soit sur la surface de la couche de filtre coloré, dans lequel ladite résine photosensible comprend un monomère acrylate polyfonctionnel, un liant polymère organique et un amorceur de photopolymérisation de formule I, II, III ou IV tel que défini dans la revendication 1.

16. Utilisation d'un composé de formule I, II, III ou IV tel que défini dans la revendication 1 pour la photopolymérisation d'une composition comprenant au moins un composé photopolymérisable éthyléniquement insaturé.

17. Composés des formules IA, IIA, IIIA et IVA 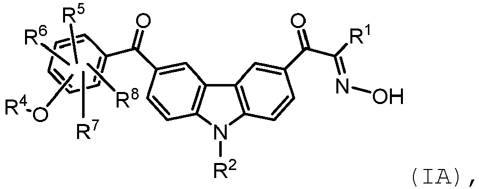 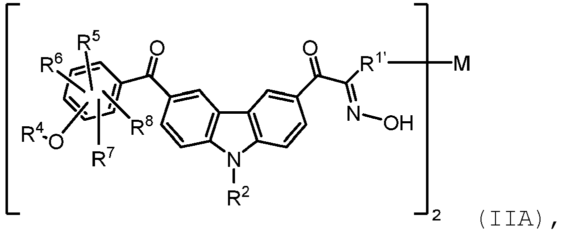 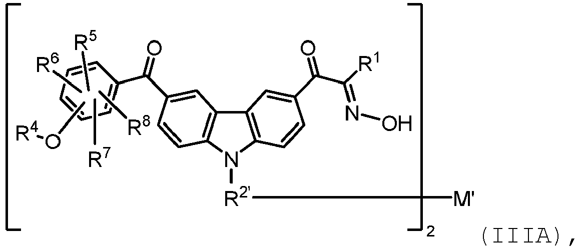 et 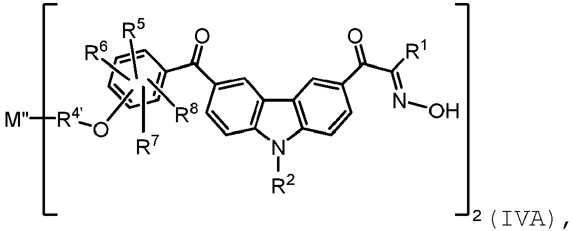 dans lesquels
R¹, R², R⁴ to R⁸, R¹' , R²' , R⁴' , M, M' et M" sont tels que définis dans l'une quelconque des revendications 1 à 4.
